# EUROPEAN PATENT APPLICATION

(11) **EP 4 851 158 A1**
(43) Date of publication of application: **09.12.2026**
(21) Application number: 25747822.2
(22) Date of filing: 24.01.2025
(51) Int. Cl.: C07D 413/14, C07D 401/14, C07D 519/00, C07D 498/04, A61K 31/4523, A61K 31/4525, A61P 35/00

(54) **COMPOUND CONTAINING TRICYCLIC RING**

(30) Priority: 03.02.2024 CN 202410159662; 05.09.2024 CN 202411245407; 16.01.2025 CN 202510073391; 21.01.2025 CN 202510097532
(71) Applicant: Chia Tai Tianqing Pharmaceutical Group Co., Ltd., Lianyungang, Jiangsu 222062 (CN)
(72) Inventor: XU, Sheng, Lianyungang, Jiangsu 222062 (CN); REN, Jing, Lianyungang, Jiangsu 222062 (CN); HUANG, Yongkang, Lianyungang, Jiangsu 222062 (CN); HU, Kongquan, Lianyungang, Jiangsu 222062 (CN); WEI, Xiaochao, Lianyungang, Jiangsu 222062 (CN); WEI, Jian, Lianyungang, Jiangsu 222062 (CN)
(74) Representative: Lorenz Seidler Gossel Part. mbB
(86) International application number: PCT/CN2025/074679
(87) International publication number: WO 2025/162254

(57) **Abstract**

The present invention relates to a compound containing a tricyclic ring, and specifically relates to a compound of formula I, a preparation method therefor, a pharmaceutical composition containing the compound, and the use thereof in the treatment of related diseases (e.g., cancer). Formula I is PTM-L-CLM, wherein CLM is an E3 ubiquitin ligase binding moiety, L is a linking group, and PTM is a binding moiety targeting a target protein (e.g., GSPT1).

PTM-L-CLM Formula I

## Description

### TECHNICAL FIELD

The present application relates to a compound containing a tricyclic ring, a preparation method therefor, a pharmaceutical composition containing the compound, and use thereof for treating a related disease (e.g., cancer).

### BACKGROUND

The transcription factor terminator (G1 to S phase transition 1, GSPT1) is a translation termination factor that mediates the recognition of a termination codon and the release of a peptide chain that promotes transcription from ribosomes. In addition to its role in transcription termination, GSPT1 is also closely associated with a variety of important cellular activities, such as cell cycle regulation, cytoskeleton formation, apoptosis, etc. GSPT1 is considered an oncogenic factor for a variety of tumors, and has become one of the potentially most attractive antitumor targets. In addition, down-regulation of GSPT1 can also affect the expression of proteins such as AR and AR-V7.

The androgen receptor (AR) is a steroid receptor in the nuclear receptor superfamily. When bound to androgens (such as testosterone and dihydrotestosterone), AR is released from the complex formed by heat shock proteins for a phosphorylation reaction to form a dimer. The dimer is transferred into the nucleus and binds to a DNA fragment associated therewith, thereby stimulating transcription of its target gene. The transcriptional activity of androgen receptors activated by ligand binding is coordinated by proteins known as co-activators. The primary function of AR antagonists is to directly prevent testosterone or dihydrotestosterone from binding to androgen receptors, thereby blocking the effect of androgens on cells. This exerts anti-androgenic and cell growth inhibitory effects, ultimately resulting in cell apoptosis and achieving the important role in treating prostate cancer. Inhibitors of the second-generation AR signaling pathway, abiraterone and enzalutamide, have achieved some success in clinical treatment, but drug resistance has emerged clinically. The ligand-binding region F876L mutation is a missense mutation that confers resistance to enzalutamide, thereby converting it from an antagonist to an agonist. In addition, AR splice mutants, particularly AR-v7 mutations lacking a ligand-binding region, are important reasons for mediating the development of second-generation drug resistance. The treatment of drug-resistant cancer is still a major challenge in cancer treatment, and the existing drug treatments generate drug resistance, wherein AR mutations (including point mutations and splice mutations) are important causes of drug resistance, so there is a need to develop new drugs for treating drug-resistant cancer.

The proteolysis targeting chimera (PROTAC) molecule is a bifunctional compound capable of binding to a target protein and an E3 ubiquitin ligase simultaneously. Such compounds can induce the target protein to be recognized by proteasomes of cells, leading to the degradation of the target protein, thereby effectively reducing the content of the target protein in the cells. The introduction of ligands capable of binding to different target proteins into PROTAC molecules has made it possible to apply the PROTAC technology to the treatment of various diseases, and this technology has received much attention in recent years.

The applicant has developed a series of compounds with GSPT1 degradation/inhibitory activity using the PROTAC technology to treat related diseases.

### SUMMARY

In one aspect, the present application relates to a compound of formula I, a stereoisomer thereof, or a pharmaceutically acceptable salt thereof,

PTM-L-CLM

wherein,
CLM is selected from an E3 ubiquitin ligase-binding moiety;
L is selected from a connecting group;
PTM is selected from a binding moiety targeting a target protein (e.g., GSPT1).

In some embodiments, the E3 ubiquitin ligase-binding moiety is selected from the group consisting of a cereblon E3 ubiquitin ligase-binding moiety, a VHL E3 ubiquitin ligase-binding moiety, an IAP E3 ubiquitin ligase-binding moiety, and an MDM2 E3 ubiquitin ligase-binding moiety. In some embodiments, the E3 ubiquitin ligase-binding moiety is selected from a cereblon E3 ubiquitin ligase-binding moiety.

In some embodiments, CLM is selected from a small molecule compound. In some embodiments, PTM is selected from a small molecule compound.

In some embodiments, CLM is linked to L via a covalent bond. In some embodiments, PTM is linked to L via a covalent bond.

In one aspect, the present application relates to a compound of formula II, a stereoisomer thereof, or a pharmaceutically acceptable salt thereof: 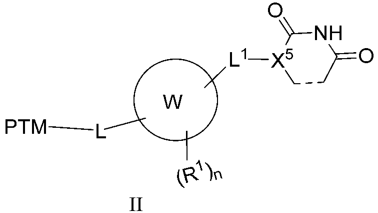 wherein, 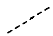
represents a single bond or double bond;
ring W is selected from the group consisting of: 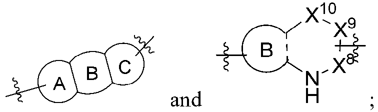
ring A is absent or selected from the group consisting of C₃₋₁₅ cycloalkenyl, 3- to 15-membered heterocycloalkenyl, phenyl, and 5- to 6-membered heteroaryl;
ring B is selected from the group consisting of phenyl and 5- to 6-membered heteroaryl;
ring C is absent or selected from 5- to 6-membered heteroaryl;
X⁸ is selected from the group consisting of -C(O)- and -CH₂-;
X⁹ is selected from the group consisting of -C(R^{d}R^{e})-, -N(R^{e})-, -O-, and -S-;
X¹⁰ is selected from the group consisting of a bond, -CH₂-, -NH-, -O-, and -S-;
R^{d} and R^{e} are each independently selected from the group consisting of hydrogen, deuterium, halogen, -OH, -NH₂, -CN, C₁₋₁₀ alkyl, C₁₋₁₀ alkoxy, (C₁₋₁₀ alkyl)NH-, (C₁₋₁₀ alkyl)₂N-, and C₁₋₁₀ haloalkyl, or R^{d} and R^{e} are linked to each other to form C₃₋₁₀ cycloalkyl or 3- to 10-membered heterocycloalkyl;
each R¹ is independently selected from the group consisting of deuterium, halogen, -OH, -NH₂, -CN, and the following groups optionally substituted with one or more substituents: C₁₋₁₀ alkyl, C₁₋₁₀ alkoxy, (C₁₋₁₀ alkyl)NH-, (C₁₋₁₀ alkyl)₂N-, C₁₋₁₀ haloalkyl, C₂₋₁₀ alkenyl, C₂₋₁₀ alkynyl, C₃₋₁₀ cycloalkyl, and 3- to 10-membered heterocycloalkyl;
n is selected from the group consisting of 0, 1, 2, and 3;
X⁵ is selected from the group consisting of C(R^{f}) and N;
R^{f} is selected from the group consisting of H, halogen, deuterium, and C₁₋₆ alkyl optionally substituted with one or more substituents;
L¹ is selected from the group consisting of a bond, -NH-, -O-, -S-, -CONH-, and -CON(C₁₋₆ alkyl)-;
L is selected from a connecting group;
PTM is selected from a small molecule compound targeting a target protein (e.g., GSPT1).

In one aspect, the present application also relates to a compound of formula III, a stereoisomer thereof, or a pharmaceutically acceptable salt thereof: 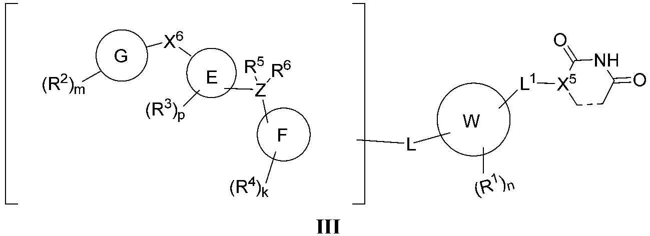 wherein,
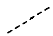 , ring W, L¹, R¹, n, and X⁵ are as defined herein;
L is selected from a connecting group;
X⁶ is selected from the group consisting of a bond, -O-, -S-, -N(R^{t})-, -C₁₋₆ alkylene-, -C₂₋₆ alkenylene-, -C₂₋₆ alkynylene-, -N(R^{t})C₁₋₆ alkylene-, -OC₁₋₆ alkylene-, -SC₁₋₆ alkylene-, and -N(R^{t})C(O)-;
R^{t} is selected from the group consisting of H and C₁₋₆ alkyl;
R², R³, and R⁴ are each independently selected from the group consisting of halogen, -CN, and the following groups optionally substituted with one or more substituents: R^{v}-, R^{v}O-, R^{v}S-, R^{s}R^{v}N-, R^{v}C(O)-, R^{v}S(O)₂-, R^{v}S(O)-, R^{v}=N-, R^{v}OC(O)-, R^{v}C(O)O-, R^{v}S(O)O-, R^{v}OS(O)-, R^{v}S(O)₂O-, R^{v}OS(O)₂-, R^{s}R^{v}NC(O)-, R^{v}C(O)NH-, R^{v}OC(O)NH-, R^{v}NHC(O)O-, R^{v}S(O)NH-, R^{s}R^{v}NS(O)-, R^{v}S(O)₂NH-, R^{s}R^{v}NS(O)₂-, and R^{s}R^{v}S(O)=N-;
R^{s} and R^{v} are each independently selected from the group consisting of H, C₁₋₁₂ alkyl, C₂₋₁₂ alkenyl, C₂₋₁₂ alkynyl, C₃₋₁₂ cycloalkyl, C₃₋₁₂ cycloalkyl C₁₋₃ alkylene-, 3- to 12-membered heterocycloalkyl, 3- to 12-membered heterocycloalkyl C₁₋₃ alkylene-, C₃₋₁₂ cycloalkenyl, C₃₋₁₂ cycloalkenyl C₁₋₃ alkylene-, 3- to 12-membered heterocycloalkenyl, 3- to 12-membered heterocycloalkenyl C₁₋₃ alkylene-, C₆₋₁₂ aryl, C₆₋₁₂ aryl C₁₋₃ alkylene-, 5- to 12-membered heteroaryl, and 5- to 12-membered heteroaryl C₁₋₃ alkylene-;
m, p, and k are each independently selected from the group consisting of 0, 1, 2, 3, 4, 5, and 6;
ring G is selected from the group consisting of C₃₋₁₂ cycloalkyl, 3- to 12-membered heterocycloalkyl, C₃₋₁₂ cycloalkenyl, 3- to 12-membered heterocycloalkenyl, C₆₋₁₂ aryl, and 5- to 12-membered heteroaryl;
ring E and ring F are each independently selected from the group consisting of C₆₋₁₂ aryl and 5- to 12-membered heteroaryl;
Z is selected from the group consisting of -C-, -O-, -S-, -N-, -OC₁₋₆ alkylene-, and -NC(O)-;
R⁵ and R⁶ are each independently absent or each independently selected from the group consisting of H, =O, halogen, -OH, -NH₂, -CN, C₁₋₁₀ alkyl, C₂₋₁₀ alkenyl, and C₂₋₁₀ alkynyl, or R⁵ and R⁶ are linked to each other to form the following group optionally substituted with one or more substituents: C₃₋₁₀ cycloalkyl or 3- to 10-membered heterocycloalkyl.

In some embodiments, CLM is selected from 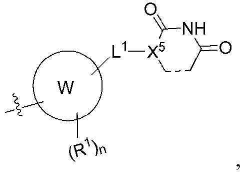 wherein 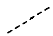 , ring W, R¹, n, L¹, and X⁵ are as defined herein.

In some embodiments, CLM is selected from 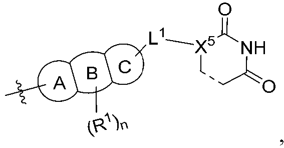 wherein 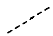 , ring A, ring B, ring C, R¹, n, L¹, and X⁵ are as defined herein.

In some embodiments, PTM is selected from 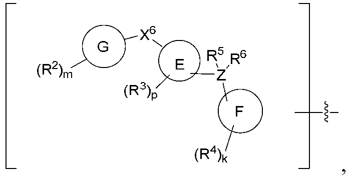 wherein X⁶, Z, R², R³, R⁴, m, p, k, ring G, ring E, ring F, R⁵, and R⁶ are as defined herein.

In some embodiments, ring W is selected from the group consisting of: 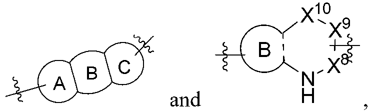 wherein ring A, ring B, ring C, X⁸, X⁹, and X¹⁰ are as defined herein.

In some embodiments, ring A is absent or selected from the group consisting of C₃₋₁₂ cycloalkenyl, 3- to 12-membered heterocycloalkenyl, phenyl, and 5- to 6-membered heteroaryl. In some embodiments, the C₃₋₁₂ cycloalkenyl is selected from C₃₋₆ cycloalkenyl. In some embodiments, the 3- to 12-membered heterocycloalkenyl is selected from 4- to 9-membered heterocycloalkenyl.

In some embodiments, ring A is absent or selected from the group consisting of C₅₋₁₅ cycloalkenyl, 5- to 15-membered heterocycloalkenyl, phenyl, and 5- to 6-membered heteroaryl.

In some embodiments, ring A is absent or selected from the group consisting of C₅₋₁₀ cycloalkenyl, 5- to 10-membered heterocycloalkenyl, phenyl, and 5- to 6-membered heteroaryl.

In some embodiments, ring A is absent or selected from the group consisting of C₅₋₉ cycloalkenyl, 5- to 9-membered heterocycloalkenyl, phenyl, and 5- to 6-membered heteroaryl.

In some embodiments, ring A is absent or selected from the group consisting of C₅₋₇ cycloalkenyl, 5- to 9-membered heterocycloalkenyl, phenyl, and 5- to 6-membered heteroaryl.

In some embodiments, ring A is absent or selected from the group consisting of C₅₋₆ cycloalkenyl, 5- to 10-membered heterocycloalkenyl, phenyl, and 5- to 6-membered heteroaryl.

In some embodiments, ring A is absent or selected from the group consisting of C₅₋₆ cycloalkenyl, 5- to 9-membered heterocycloalkenyl, phenyl, and 5- to 6-membered heteroaryl.

In some embodiments, ring A is absent or selected from the group consisting of C₅₋₆ cycloalkenyl, 5- to 9-membered heterocycloalkenyl, phenyl, and 5-membered heteroaryl.

In some embodiments, ring A is absent or selected from the group consisting of C₅₋₆ cycloalkenyl, 5- to 9-membered heterocycloalkenyl, phenyl, pyrrolyl, pyrazolyl, furanyl, and oxazolyl.

In some embodiments, ring A is absent or selected from the group consisting of C₅ cycloalkenyl, C₆ cycloalkenyl, 5-membered, 6-membered, 7-membered, 8-membered, and 9-membered heterocycloalkenyl, phenyl, pyrrolyl, pyrazolyl, furanyl, and oxazolyl.

In some embodiments, ring A is absent or selected from the group consisting of cyclopentenyl, monocyclohexenyl, bicyclohexenyl, dihydropyrrolyl, tetrahydropyridinyl, tetrahydroazepinyl, azaspirooctenyl, azaspirononenyl, phenyl, pyrrolyl, pyrazolyl, furanyl, oxazolyl, and dihydrooxazinyl. In some embodiments, ring A is selected from the group consisting of cyclopentenyl and monocyclohexenyl. In some embodiments, ring A is selected from the group consisting of dihydropyrrolyl, tetrahydropyridinyl, and tetrahydroazepinyl.

In some specific embodiments, ring A is selected from the group consisting of C₅₋₁₀ cycloalkenyl and 5- to 10-membered heterocycloalkenyl.

In some specific embodiments, ring A is selected from the group consisting of C₅₋₇ cycloalkenyl and 5- to 10-membered heterocycloalkenyl. In some specific embodiments, ring A is selected from the group consisting of C₅₋₇ cycloalkenyl and 5- to 10-membered heterocycloalkenyl containing 1-3 heteroatoms selected from the group consisting of N, O, and S.

In some specific embodiments, ring A is selected from the group consisting of C₅₋₆ cycloalkenyl and 5- to 7-membered heterocycloalkenyl. In some specific embodiments, ring A is selected from the group consisting of C₅₋₆ cycloalkenyl and 5- to 6-membered heterocycloalkenyl. In some specific embodiments, ring A is selected from the group consisting of C₅ cycloalkenyl and 5- to 6-membered heterocycloalkenyl. In some specific embodiments, ring A is selected from the group consisting of C₅ cycloalkenyl and 5- to 6-membered heterocycloalkenyl containing 1-2 N atoms or O atoms. In some embodiments, ring A is selected from the group consisting of dihydropyrrolyl and tetrahydropyridinyl.

In some embodiments, ring B is selected from the group consisting of phenyl and 6-membered heteroaryl. In some embodiments, ring B is selected from the group consisting of phenyl and 6-membered heteroaryl containing 1-3 heteroatoms selected from the group consisting of N, O, and S. In some embodiments, ring B is selected from the group consisting of phenyl and 6-membered heteroaryl containing 1-2 N atoms. In some embodiments, ring B is selected from the group consisting of phenyl and pyridinyl. In some embodiments, ring B is phenyl.

In some embodiments, ring C is absent or selected from 5-membered heteroaryl. In some embodiments, ring C is selected from 5-membered heteroaryl. In some embodiments, ring C is selected from 5-membered heteroaryl containing 1-3 (e.g., 1-2) heteroatoms selected from the group consisting of N, O, and S. In some embodiments, ring C is absent or selected from the group consisting of isoxazolyl, pyrazolyl, and furanyl. In some embodiments, ring C is selected from the group consisting of isoxazolyl and furanyl. In some embodiments, ring C is isoxazolyl. In some embodiments, ring C is absent.

In some embodiments, the substitution position for R¹ is selected from the group consisting of ring A and ring B. In some embodiments, the substitution position for R¹ is selected from ring A. In some embodiments, the substitution position for R¹ is selected from ring B. In some embodiments, the substitution position for R¹ is selected from ring C. In some embodiments, R¹ is absent (i.e., n is 0).

In some embodiments, ring A is linked to L. In some embodiments, ring B is linked to L. In some embodiments, ring C is linked to L. In some embodiments, ring A is linked to L¹. In some embodiments, ring B is linked to L¹. In some embodiments, ring C is linked to L¹. In some embodiments, ring A is linked to fragment 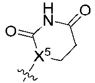 (when L¹ is selected from a bond). In some embodiments, ring B is linked to fragment 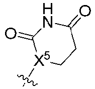 (when L¹ is selected from a bond). In some embodiments, ring C is linked to fragment 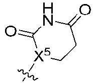 (when L¹ is selected from a bond).

In some embodiments, the structural fragment 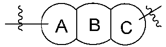 is selected from the group consisting of

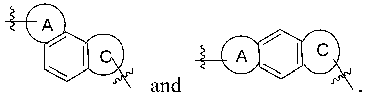

In some embodiments, the structural fragment 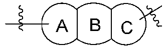 is selected from the group consisting of

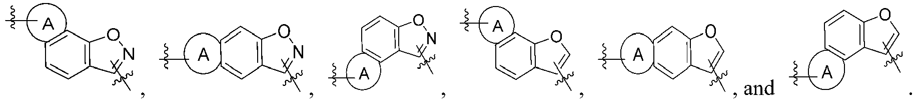

In some embodiments, the structural fragment 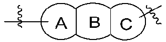 is selected from the group consisting of

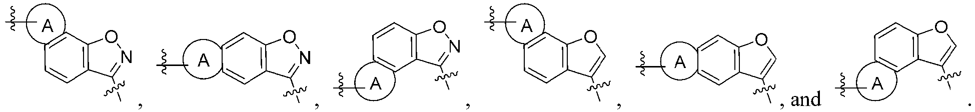

In some embodiments, the structural fragment 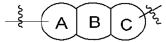 is selected from the group consisting of 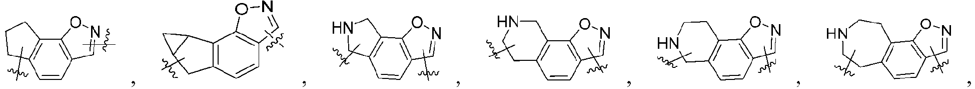 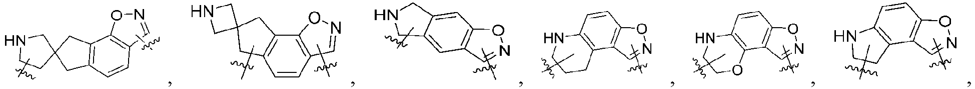 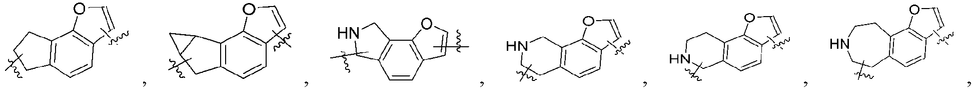 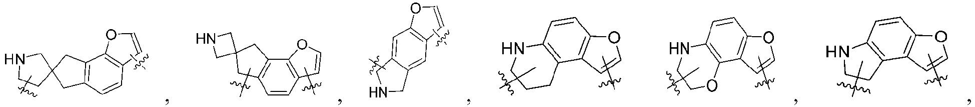 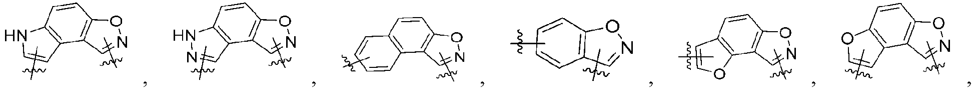 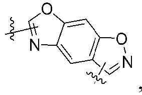 phenyl, pyridinyl,

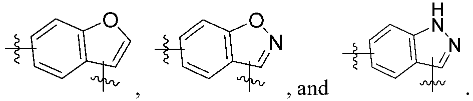

In some embodiments, the structural fragment 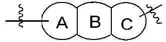 is selected from the group consisting of 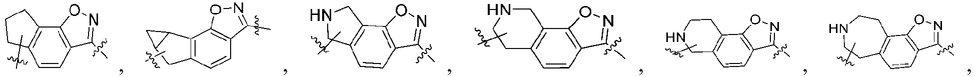 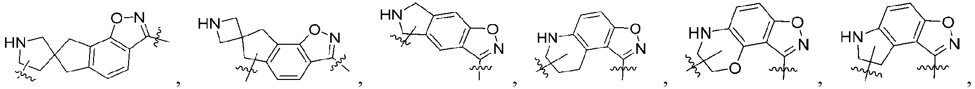 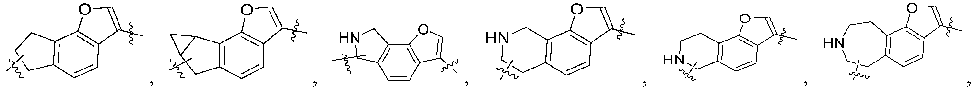 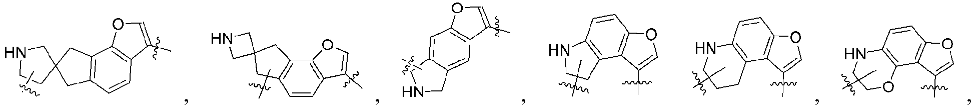 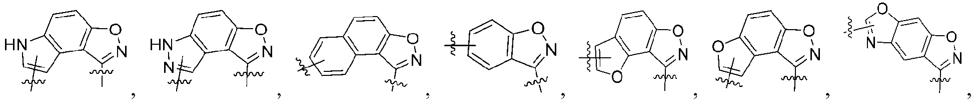 phenyl, pyridinyl,

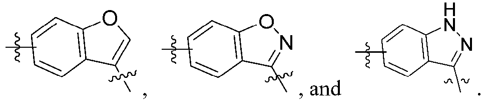

In some embodiments, the structural fragment 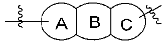 is selected from the group consisting of 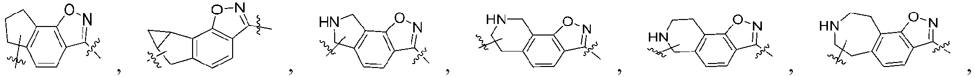 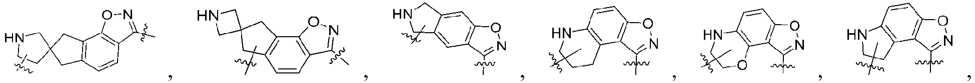 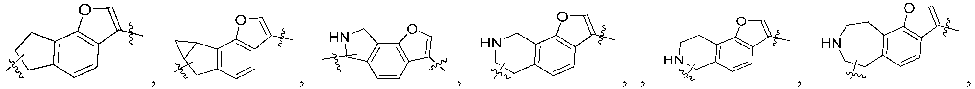 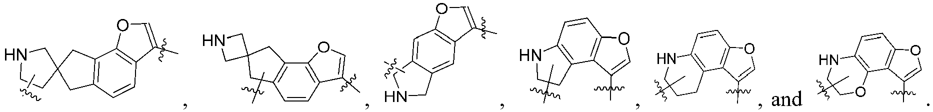 In some embodiments, the structural fragment 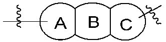 is selected from the group consisting of 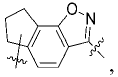 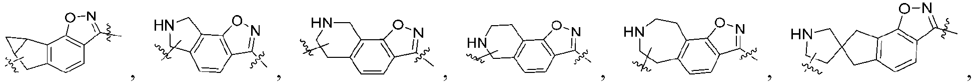 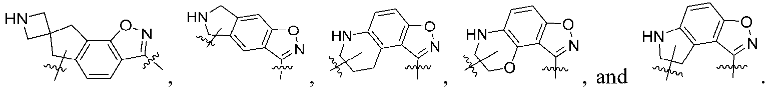 In some embodiments, the structural fragment 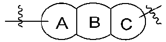 is selected from the group consisting of 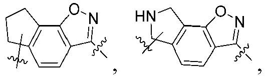 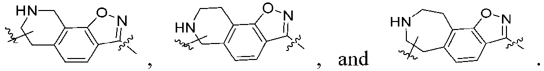 In some embodiments, the structural fragment 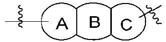 is selected from the group consisting of phenyl and pyridinyl. In some embodiments, the structural fragment 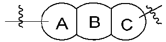 is selected from the group consisting of 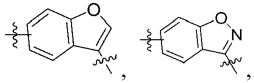 and

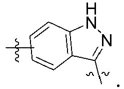

In some embodiments, the structural fragment 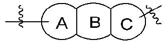 is selected from the group consisting of 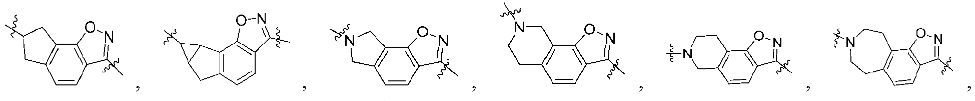 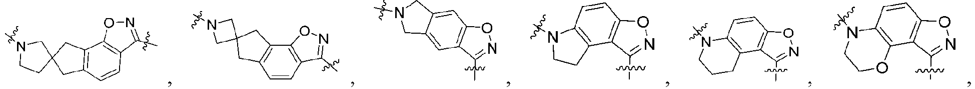 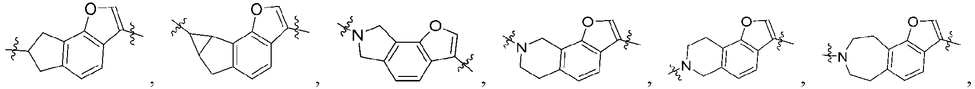 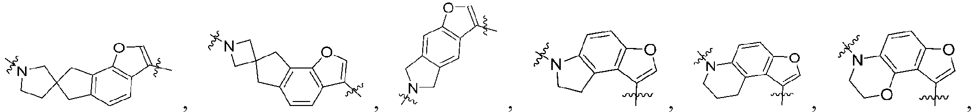 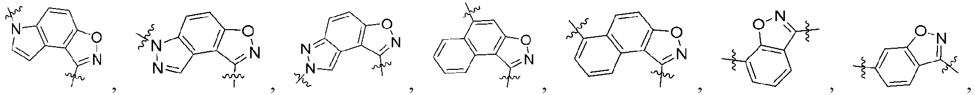 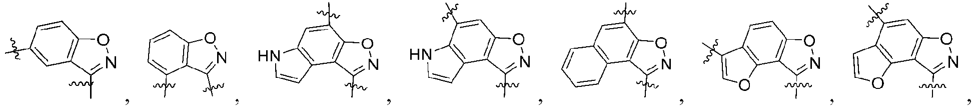 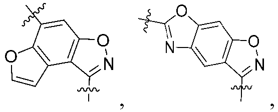 phenyl, pyridinyl,

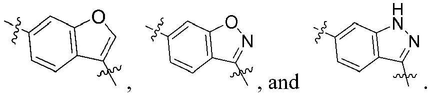

In some embodiments, the structural fragment 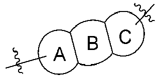 is selected from the group consisting of

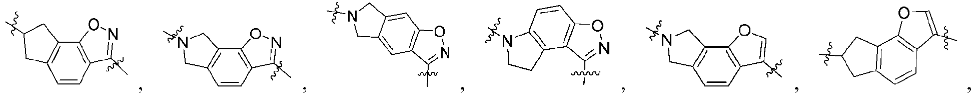

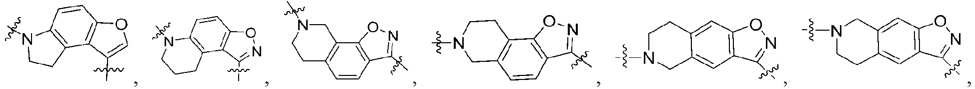

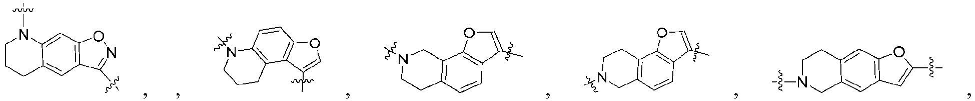

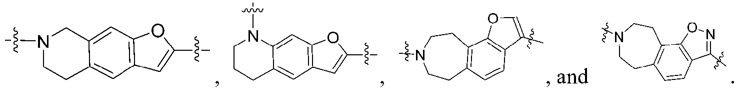

In some embodiments, the structural fragment 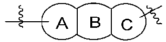 is selected from the group consisting of 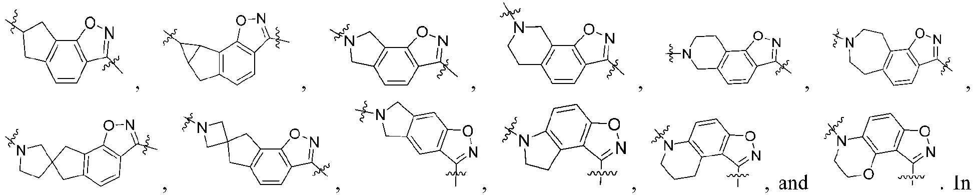 some embodiments, the structural fragment 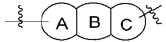 is selected from the group consisting of 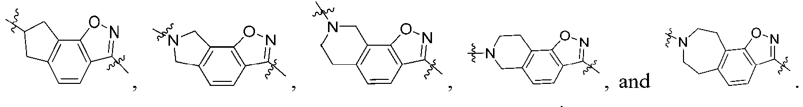 In some embodiments, the structural fragment 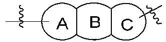 is selected from 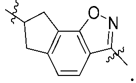 In some embodiments, the structural fragment 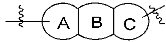 is selected from 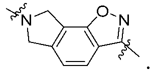 In some embodiments, the structural fragment 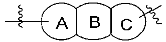 is selected from 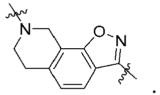 In some embodiments, the structural fragment 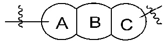 is selected from 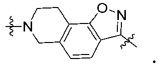 In some embodiments, the structural fragment 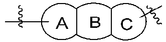 is selected from 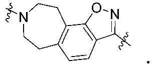 In some embodiments, the structural fragment 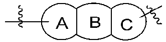 is selected from the group consisting of phenyl and pyridinyl. In some embodiments, the structural fragment 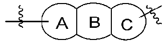 is selected from the group consisting of 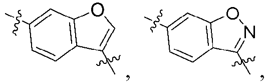 and

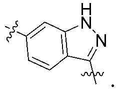

In some embodiments, the structural fragment 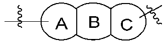 is independently substituted with n R¹. In some embodiments, n is 0 or 1. In some embodiments, n is 0.

In some embodiments, the structural fragment 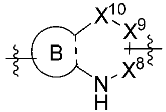 is independently substituted with n R¹.

In some embodiments, the structural fragment 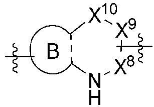 is selected from the group consisting of

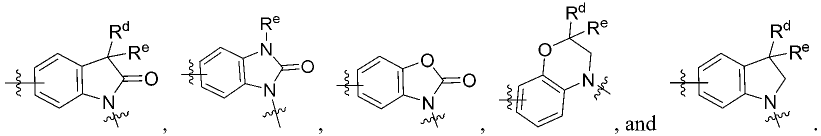

In some embodiments, X⁹ is selected from the group consisting of -C(R^{d}R^{e})-, -N(R^{e})-, and -O-.

In some embodiments, X⁹ is selected from the group consisting of -C(R^{d}R^{e})- and -O-.

In some embodiments, X¹⁰ is selected from the group consisting of a bond and -O-.

In some embodiments, R^{d} and R^{e} are each independently selected from the group consisting of hydrogen, deuterium, halogen, -OH, -NH₂, -CN, C₁₋₆ alkyl, C₁₋₆ alkoxy, (C₁₋₆ alkyl) NH-, (C₁₋₆ alkyl)₂N-, and C₁₋₆ haloalkyl, or R^{d} and R^{e} are linked to each other to form C₃₋₆ cycloalkyl or 3- to 6-membered heterocycloalkyl.

In some embodiments, R^{d} and R^{e} are each independently selected from the group consisting of hydrogen, deuterium, halogen, -OH, -NH₂, -CN, and C₁₋₆ alkyl, or R^{d} and R^{e} are linked to each other to form C₃₋₄ cycloalkyl.

In some embodiments, R^{d} and R^{e} are each independently selected from the group consisting of hydrogen, F, and methyl, or R^{d} and R^{e} are linked to each other to form cyclopropyl.

In some embodiments, the structural fragment 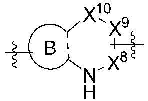 is selected from

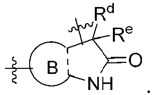

In some embodiments, the structural fragment 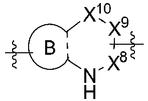 is selected from the group consisting of

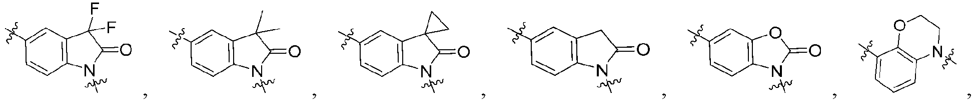

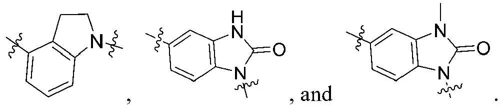

In some embodiments, each R¹ is independently selected from the group consisting of deuterium, halogen, -OH, - NH₂, -CN, and the following groups optionally substituted with one or more substituents: C₁₋₆ alkyl, C₁₋₆ alkoxy, (C₁₋₁₀ alkyl) NH-, (C₁₋₁₀ alkyl)₂N-, C₁₋₆ haloalkyl, C₂₋₆ alkenyl, C₂₋₆ alkynyl, C₃₋₆ cycloalkyl, and 3- to 6-membered heterocycloalkyl.

In some embodiments, each R¹ is independently selected from the group consisting of deuterium, halogen, -OH, - NH₂, -CN, and the following groups optionally substituted with one or more substituents: C₁₋₆ alkyl, C₁₋₆ alkoxy, and C₁₋₆ haloalkyl.

In some embodiments, each R¹ is independently selected from the group consisting of halogen, -OH, -NH₂, -CN, C₁₋₄ alkyl, C₁₋₄ alkoxy, and C₁₋₄ haloalkyl.

In some embodiments, each R¹ is independently selected from the group consisting of halogen, -OH, -NH₂, -CN, C₁₋₃ alkyl, C₁₋₃ alkoxy, and C₁₋₃ haloalkyl. In some embodiments, each R¹ is independently selected from the group consisting of halogen, -OH, -NH₂, -CN, and C₁₋₃ alkyl. In some embodiments, each R¹ is independently selected from the group consisting of halogen and C₁₋₃ alkyl. In some embodiments, each R¹ is independently selected from the group consisting of fluorine, chlorine, bromine, -OH, -NH₂, and -CN. In some embodiments, each R¹ is independently selected from the group consisting of fluorine, chlorine, and bromine. In some embodiments, each R¹ is independently selected from the group consisting of fluoro and methyl.

In some embodiments, n is selected from the group consisting of 0, 1, and 2. In some embodiments, n is selected from the group consisting of 0 and 1.

In some specific embodiments, n is selected from 0.

In some embodiments, the structural fragment 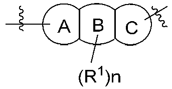 is selected from the group consisting of 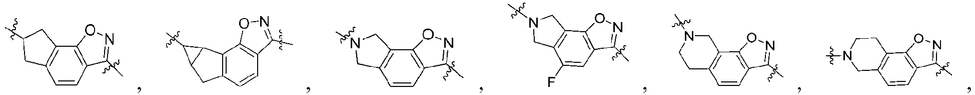 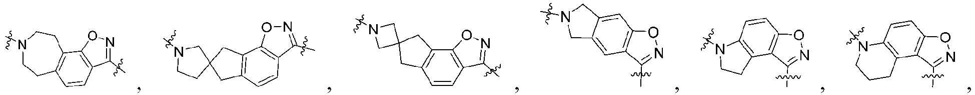 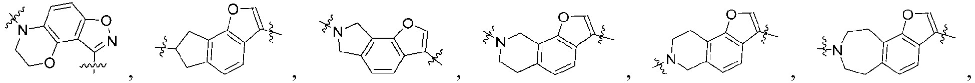 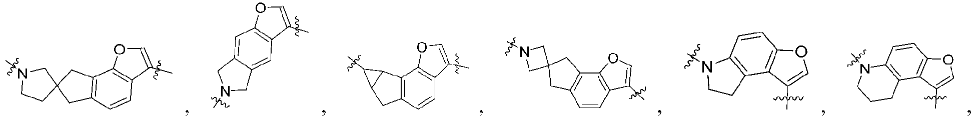 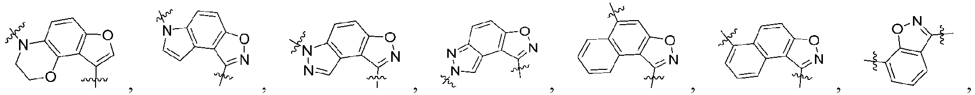 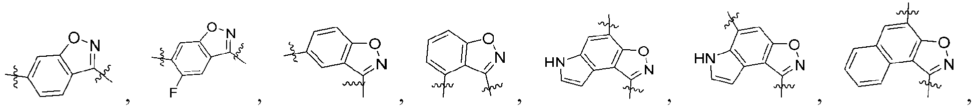 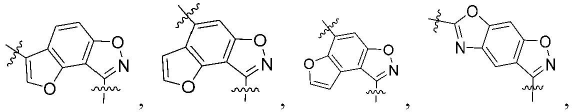 phenyl, 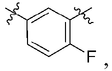 pyridinyl,

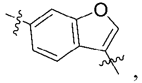

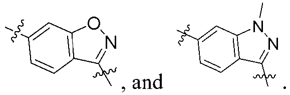

In some embodiments, the structural fragment 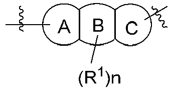 is selected from the group consisting of 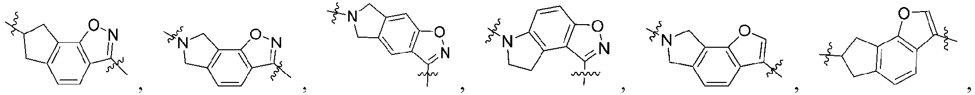 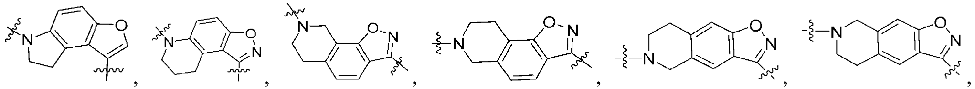 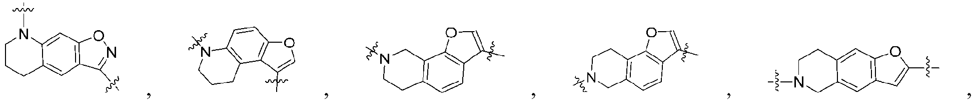 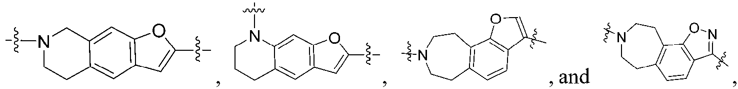 more preferably from the group consisting of

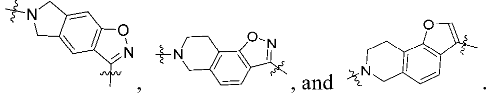

In some embodiments, X⁵ is selected from C(R^{f}).

In some embodiments, R^{f} is selected from the group consisting of H, fluorine, chlorine, bromine, deuterium, and C₁₋₃ alkyl optionally substituted with one or more substituents.

In some embodiments, R^{f} is selected from the group consisting of H, fluorine, chlorine, bromine, deuterium, and C₁₋₃ alkyl, wherein the C₁₋₃ alkyl is optionally substituted with one or more of the following groups: halogen, -OH, -NH₂, and -CN.

In some embodiments, R^{f} is selected from the group consisting of H, fluorine, chlorine, bromine, deuterium, and C₁₋₃ alkyl. In some embodiments, R^{f} is selected from the group consisting of H, fluorine, deuterium, and methyl. In some embodiments, R^{f} is selected from H.

In some embodiments, X⁵ is selected from the group consisting of CH and N.

In some embodiments, X⁵ is selected from CH.

In some embodiments, 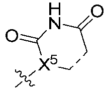 is selected from the group consisting of

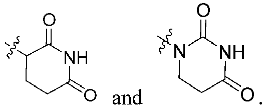

In some embodiments, CLM, 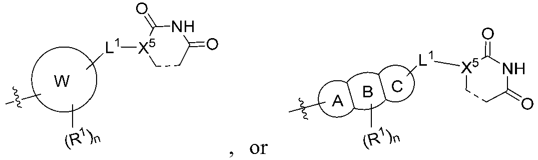 is selected from

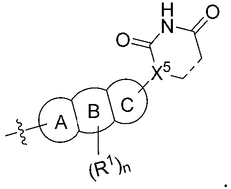

In some embodiments, CLM or 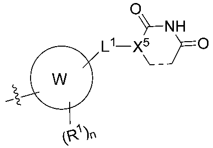 is selected from the group consisting of

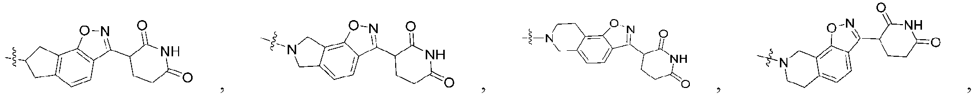

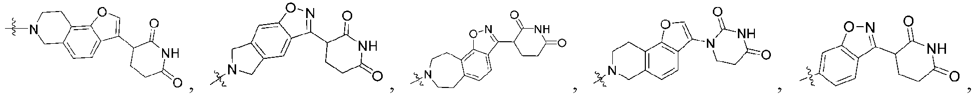

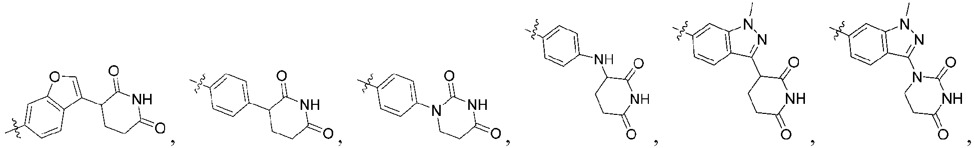

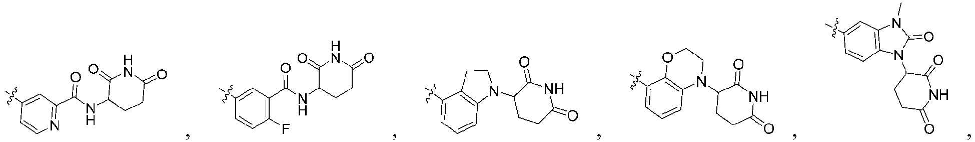

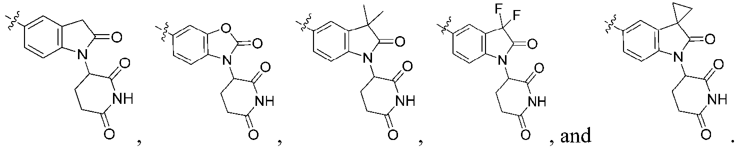

In some embodiments, CLM, 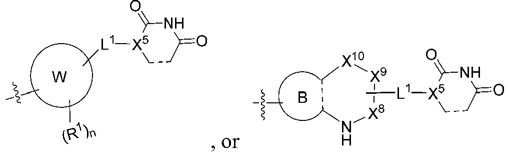 is selected from the group consisting of

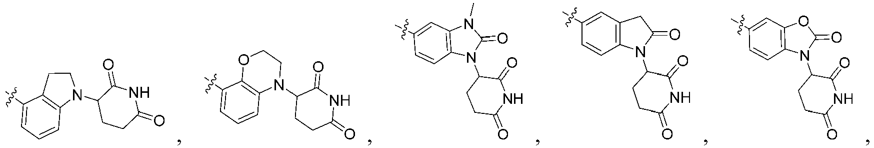

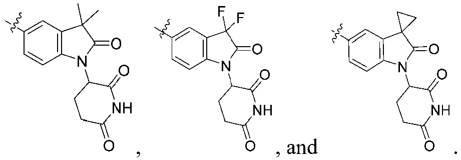

In some embodiments, CLM, 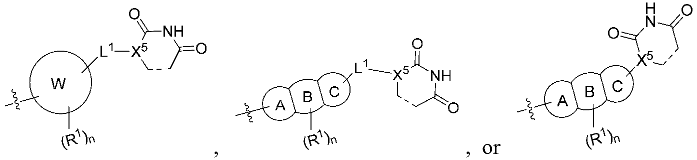 is selected from the group consisting of

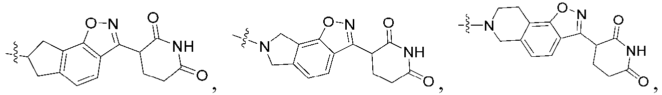

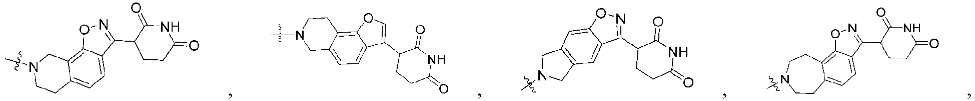

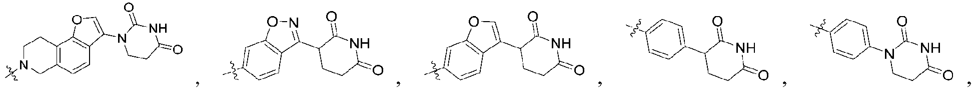

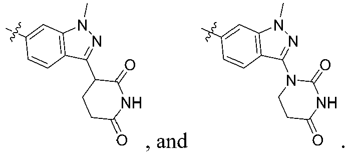

In some embodiments, CLM or 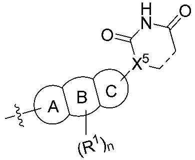 is selected from the group consisting of 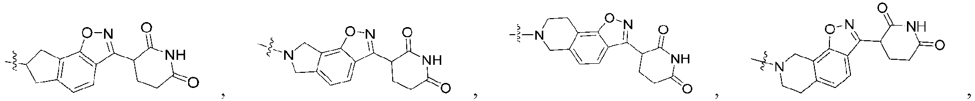 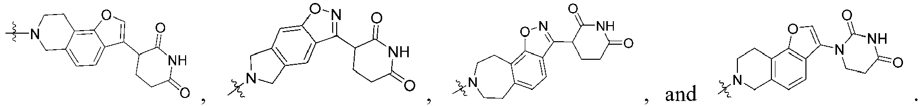 In some embodiments, CLM or 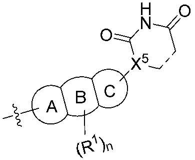 is selected from the group consisting of 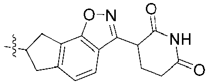 and 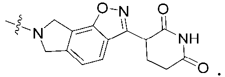 In some embodiments, CLM or 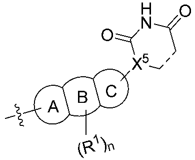 is selected from the group consisting of

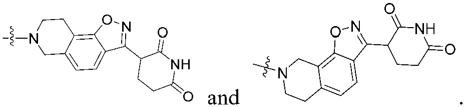

In some embodiments, L¹ is selected from the group consisting of a bond, -NH- and -CONH-. In some embodiments, L¹ is selected from a bond.

In some embodiments, L is selected from the group consisting of the following groups optionally substituted with one or more substituents: C₁₋₅₀ alkylene, C₂₋₅₀ alkenylene, and C₂₋₅₀ alkynylene, wherein optionally, 1 or more -CH₂-of the C₁₋₅₀ alkylene, C₂₋₅₀ alkenylene, or C₂₋₅₀ alkynylene are independently optionally replaced by -O-, C₃₋₁₅ cycloalkyl, 3- to 15-membered heterocycloalkyl, 4- to 15-membered heterocycloalkenyl, C₆₋₁₅ aryl, 5- to 15-membered heteroaryl, -NH-, -N(C₁₋₆ alkyl)-, or -S-.

In some embodiments, L is selected from the group consisting of the following groups optionally substituted with one or more substituents: C₁₋₃₀ alkylene, C₂₋₃₀ alkenylene, and C₂₋₃₀ alkynylene, wherein optionally, 1 or more -CH₂-of the C₁₋₃₀ alkylene, C₂₋₃₀ alkenylene, or C₂₋₃₀ alkynylene are independently optionally replaced by -O-, C₃₋₁₂ cycloalkyl, 3- to 12-membered heterocycloalkyl, 4- to 12-membered heterocycloalkenyl, C₆₋₁₂ aryl, 5- to 12-membered heteroaryl, -NH-, -N(C₁₋₆ alkyl)-, or -S-.

In some embodiments, L is selected from the group consisting of the following groups optionally substituted with one or more substituents: C₁₋₂₀ alkylene, C₂₋₂₀ alkenylene, and C₂₋₂₀ alkynylene, wherein optionally, 1 or more -CH₂-of the C₁₋₂₀ alkylene, C₂₋₂₀ alkenylene, or C₂₋₂₀ alkynylene are independently optionally replaced by -O-, C₃₋₁₀ cycloalkyl, 3- to 11-membered heterocycloalkyl, 4- to 10-membered heterocycloalkenyl, C₆₋₁₀ aryl, 5- to 10-membered heteroaryl, -NH-, -N(C₁₋₆ alkyl)-, or -S-.

In some embodiments, L is selected from the group consisting of the following groups optionally substituted with one or more substituents: C₁₋₁₅ alkylene, C₂₋₁₅ alkenylene, and C₂₋₁₅ alkynylene, wherein optionally, 1 or more -CH₂-of the C₁₋₁₅ alkylene, C₂₋₁₅ alkenylene, or C₂₋₁₅ alkynylene are independently optionally replaced by -O-, C₃₋₉ cycloalkyl, 3- to 11-membered heterocycloalkyl, 4- to 8-membered heterocycloalkenyl, C₆₋₈ aryl, 5- to 8-membered heteroaryl, -NH-, -N(C₁₋₄ alkyl)-, or -S-.

In some embodiments, L is selected from the group consisting of the following groups optionally substituted with one or more substituents: C₁₋₁₀ alkylene, C₂₋₁₀ alkenylene, and C₂₋₁₀ alkynylene, wherein optionally, 1 or more -CH₂-of the C₁₋₁₀ alkylene, C₂₋₁₀ alkenylene, or C₂₋₁₀ alkynylene are independently optionally replaced by -O-, C₃₋₉ cycloalkyl, 3- to 11-membered heterocycloalkyl, 4- to 6-membered heterocycloalkenyl, C₆ aryl, 5- to 6-membered heteroaryl, -NH-, -N(C₁₋₃ alkyl)-, or -S-.

In some embodiments, L is selected from the group consisting of the following groups optionally substituted with one or more substituents: C₁₋₆ alkylene, C₂₋₆ alkenylene, and C₂₋₆ alkynylene, wherein optionally, 1 or more -CH₂-of the C₁₋₆ alkylene, C₂₋₆ alkenylene, or C₂₋₆ alkynylene are independently optionally replaced by -O-, C₃₋₉ cycloalkyl, 3- to 11-membered heterocycloalkyl, 4- to 6-membered heterocycloalkenyl, C₆ aryl, 5- to 6-membered heteroaryl, - NH-, -N(C₁₋₃ alkyl)-, or -S-.

In some embodiments, L is selected from the group consisting of the following groups optionally substituted with one or more substituents: C₁₋₄ alkylene, C₂₋₄ alkenylene, and C₂₋₄ alkynylene, wherein optionally, 1 or more (e.g., 1 or 2, 1 or 3, etc.) -CH₂- of the C₁₋₄ alkylene, C₂₋₄ alkenylene, or C₂₋₄ alkynylene are independently optionally replaced by -O-, C₄₋₆ cycloalkyl, 4- to 6-membered heterocycloalkyl, 4- to 6-membered heterocycloalkenyl, C₆ aryl, 5- to 6-membered heteroaryl, -NH-, -N(C₁₋₃ alkyl)-, or -S-.

In some embodiments, L is selected from the group consisting of C₁₋₁₀ alkylene and C₂₋₁₀ alkynylene optionally substituted with one or more substituents, wherein optionally, 1 or more -CH₂- of the C₁₋₁₀ alkylene or C₂₋₁₀ alkynylene are independently optionally replaced by a group selected from the group consisting of -O-, C₃₋₁₂ cycloalkyl, 4- to 12-membered heterocycloalkyl, 4- to 12-membered heterocycloalkenyl, -NH-, -N(C₁₋₆ alkyl)-, and -S-.

In some embodiments, L is selected from the group consisting of C₁₋₆ alkylene and C₂₋₆ alkynylene optionally substituted with one or more substituents, wherein optionally, 1 or more -CH₂- of the C₁₋₆ alkylene or C₂₋₆ alkynylene are independently optionally replaced by a group selected from the group consisting of -O-, C₃₋₁₀ cycloalkyl, 4- to 11-membered heterocycloalkyl, 5- to 6-membered heterocycloalkenyl, -NH-, -N(C₁₋₃ alkyl)-, and -S-.

In some embodiments, L is selected from the group consisting of C₁₋₆ alkylene (e.g., C₁, C₂, C₃, C₄, C₅, or C₆ alkylene) and C₂₋₆ alkynylene (e.g., C₂, C₃, C₄, C₅, or C₆ alkynylene) optionally substituted with one or more substituents, wherein optionally, 1 or more -CH₂- of the C₁₋₆ alkylene (e.g., C₁, C₂, C₃, C₄, C₅, or C₆ alkylene) or C₂₋₆ alkynylene (e.g., C₂, C₃, C₄, C₅, or C₆ alkynylene) are independently optionally replaced by a group selected from the group consisting of -O-, C₃₋₁₀ cycloalkyl (e.g., C₃, C₄, C₅, C₆, C₇, C₈, C₉, or C₁₀ cycloalkyl), 4- to 11-membered heterocycloalkyl (e.g., 4-, 5-, 6-, 7-, 8-, 9-, 10-, or 11-membered heterocycloalkyl), -NH-, -N(C₁₋₃ alkyl)-, and -S-. In some embodiments, in the definition of L, the substituent is selected from the group consisting of halogen, =O, - OH, -NH₂, -CN, C₁₋₆ alkyl, C₁₋₆ alkoxy, C₁₋₆ haloalkyl, (C₁₋₆ alkyl)NH-, (C₁₋₆ alkyl)₂N-, C₃₋₁₂ cycloalkyl, and 4- to 12-membered heterocycloalkyl.

In some embodiments, in the definition of L, the substituent is selected from the group consisting of halogen, =O, - OH, -NH₂, -CN, C₁₋₄ alkyl, C₁₋₄ alkoxy, C₁₋₄ haloalkyl, (C₁₋₄ alkyl)NH-, (C₁₋₄ alkyl)₂N-, C₃₋₁₀ cycloalkyl, and 4- to 10-membered heterocycloalkyl.

In some embodiments, in the definition of L, the substituent is selected from the group consisting of halogen, =O, - OH, -NH₂, -CN, C₁₋₆ alkyl, and C₁₋₆ alkoxy. In some embodiments, in the definition of L, the substituent is selected from the group consisting of =O, -OH, -NH₂, halogen, and -CN. In some embodiments, in the definition of L, the substituent is =O.

In some embodiments, in the definition of L: the cycloalkyl is selected from the group consisting of cyclobutyl, cyclopentyl, cyclohexyl, and spirononyl.

In some embodiments, in the definition of L: the heterocycloalkyl is selected from the group consisting of azetidinyl, pyrrolidinyl, piperidinyl, piperazinyl, monoazaspiroheptyl, monoazaspirooctyl, monoazaspirononyl, diazaspirononyl, monoazaspirodecyl, diazaspirodecyl, monoazaspiroundecyl, diazaspiroundecyl, monoazabicyclohexyl, octahydrocyclopentapyrrolyl, diazabicyclooctyl, and monoazabicyclononyl.

In some embodiments, in the definition of L: the heterocycloalkenyl is selected from tetrahydropyridinyl.

In some embodiments, in the definition of L: the heteroaryl is selected from the group consisting of pyridinyl, pyrimidinyl, pyridazinyl, pyrazinyl, tetrazolyl, triazolyl, pyrazolyl, imidazolyl, oxazolyl, isoxazolyl, furanyl, thienyl, pyrrolyl, and the like.

In some embodiments, in the definition of L: the aryl is selected from phenyl.

In some embodiments, L is selected from -LNK¹-Cy¹-LNK-Cy²-LNK²-Cy³-, wherein
Cy¹, Cy², or Cy³ is each independently selected from the group consisting of a bond and the following groups optionally substituted with one or more R^{b}: C₃₋₁₂ cycloalkyl, 4- to 12-membered heterocycloalkyl, and 4- to 12-membered heterocycloalkenyl;
LNK, LNK¹, and LNK² are each independently selected from the group consisting of a bond, -NH-, -O-, -S-, and the following groups optionally substituted with one or more R^{c}: C₁₋₁₂ alkylene, C₂₋₁₂ alkenylene, C₂₋₁₂ alkynylene, and C₁₋₁₂ heteroalkylene;
R^{b} and R^{c} are each independently selected from the group consisting of halogen, =O, -OH, -NH₂, -CN, C₁₋₆ alkyl, C₁₋₆ alkoxy, C₁₋₆ haloalkyl, (C₁₋₆ alkyl) NH-, (C₁₋₆ alkyl)₂N-, C₃₋₁₂ cycloalkyl, and 4- to 12-membered heterocycloalkyl.
In some embodiments, Cy¹, Cy², and Cy³ are not bonds simultaneously. In some embodiments, Cy² and Cy³ are bonds. In some embodiments, LNK¹ and LNK² are selected from a bond.

In some embodiments, L is selected from the group consisting of -Cy¹-, -Cy²-, -LNK¹-, -Cy¹-LNK-, -Cy¹-Cy²-, - LNK¹-Cy¹-LNK-, -LNK-Cy²-LNK²-, -Cy¹-Cy²-LNK²-, -LNK¹-Cy¹-Cy²-, -Cy¹-LNK-Cy²-, -LNK¹-Cy¹-Cy²-LNK²-, -LNK¹-Cy¹-LNK-Cy²-, -Cy¹-LNK-Cy²-LNK²-, -Cy¹-Cy²-Cy³-, and -Cy¹-Cy²-LNK²-Cy³-.

In some embodiments, LNK, LNK¹, and LNK² are each independently selected from the group consisting of a bond, -NH-, -O-, -S-, and the following groups optionally substituted with one or more R^{c}: C₁₋₁₀ alkylene, C₂₋₁₀ alkenylene, C₂₋₁₀ alkynylene, and C₁₋₁₀ heteroalkylene.

In some embodiments, LNK, LNK¹, and LNK² are each independently selected from the group consisting of a bond, -NH-, -O-, -S-, and the following groups optionally substituted with one or more R^{c}: C₁₋₆ alkylene, C₂₋₆ alkenylene, C₂₋₆ alkynylene, and C₁₋₆ heteroalkylene.

In some embodiments, LNK, LNK¹, and LNK² are each independently selected from the group consisting of a bond, -NH-, -O-, -S-, and the following groups optionally substituted with one or more R^{c}: C₁₋₄ alkylene, C₂₋₄ alkenylene, C₂₋₄ alkynylene, and C₁₋₄ heteroalkylene.

In some embodiments, LNK, LNK¹, and LNK² are each independently selected from the group consisting of a bond, -NH-, -O-, -S-, and the following groups optionally substituted with one or more R^{c}: C₁₋₆ alkylene, C₂₋₆ alkynylene, and C₁₋₆ heteroalkylene.

In some embodiments, LNK, LNK¹, and LNK² are each independently selected from the group consisting of a bond, -NH-, -O-, -S-, and the following groups optionally substituted with one or more R^{c}: C₁₋₄ alkylene, C₂₋₄ alkynylene, and C₁₋₄ heteroalkylene.

In some embodiments, LNK, LNK¹, and LNK² are each independently selected from the group consisting of a bond, -NH-, -O-, and the following groups optionally substituted with one or more R^{c}: C₁₋₃ alkylene, C₂₋₃ alkynylene, and C₁₋₃ heteroalkylene.

In some embodiments, LNK, LNK¹, and LNK² are each independently selected from the group consisting of a bond, -NH-, -O-, and the following groups optionally substituted with one or more R^{c}: C₁₋₂ alkylene, C₂ alkynylene, and C₁₋₂ heteroalkylene.

In some embodiments, LNK, LNK¹, and LNK² are each independently selected from the group consisting of a bond, -NH-, -O-, -NHCH₂-, -CH₂NHCH₂-, -CH₂-, -CH₂CH₂-, ethynylene, -C(O)-, and -C(O)CH₂-.

In some embodiments, Cy¹, Cy², or Cy³ is each independently selected from the group consisting of a bond and the following groups optionally substituted with one or more R^{b}: C₃₋₁₁ cycloalkyl, 4- to 12-membered heterocycloalkyl, and 4- to 11-membered heterocycloalkenyl.

In some embodiments, Cy¹, Cy², or Cy³ is each independently selected from the group consisting of a bond and the following groups optionally substituted with one or more R^{b}: C₄₋₁₀ cycloalkyl, 4- to 11-membered heterocycloalkyl, and 5- to 6-membered heterocycloalkenyl.

In some embodiments, Cy¹, Cy², or Cy³ is each independently selected from the group consisting of a bond and the following groups optionally substituted with one or more R^{b}: C₄₋₉ cycloalkyl, 4- to 11-membered heterocycloalkyl, and 5- to 6-membered heterocycloalkenyl.

In some embodiments, Cy¹, Cy², or Cy³ is each independently selected from the group consisting of a bond and the following groups optionally substituted with one or more R^{b}: C₄₋₆ cycloalkyl, C₉ cycloalkyl, 4- to 11-membered heterocycloalkyl, and 6-membered heterocycloalkenyl.

In some specific embodiments, Cy¹, Cy², or Cy³ is each independently selected from the group consisting of a bond and the following group optionally substituted with one or more R^{b}: C₄₋₆ cycloalkyl or 4- to 6-membered heterocycloalkyl.

In some embodiments, Cy¹, Cy², or Cy³ is each independently selected from the group consisting of a bond and the following groups optionally substituted with one or more R^{b}: cyclobutyl, cyclopentyl, cyclohexyl, spirononyl, azetidinyl, pyrrolidinyl, piperidinyl, tetrahydropyridinyl, piperazinyl, monoazaspiroheptyl, monoazaspirooctyl, monoazaspirononyl, diazaspirononyl, monoazaspirodecyl, diazaspirodecyl, monoazaspiroundecyl, diazaspiroundecyl, monoazabicyclohexyl, octahydrocyclopentapyrrolyl, diazabicyclooctyl, and monoazabicyclononyl.

In some embodiments, Cy¹, Cy², or Cy³ is each independently selected from the group consisting of a bond,

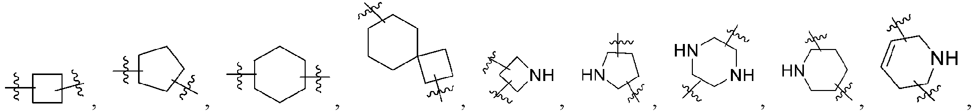

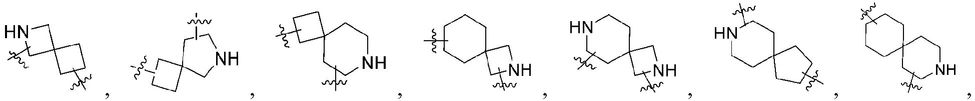

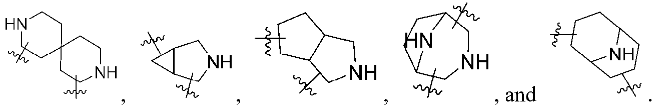

In some embodiments, Cy¹, Cy², or Cy³ is each independently selected from the group consisting of a bond,

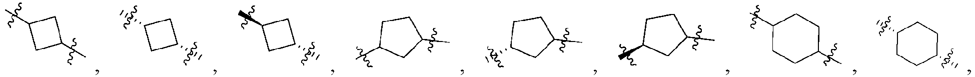

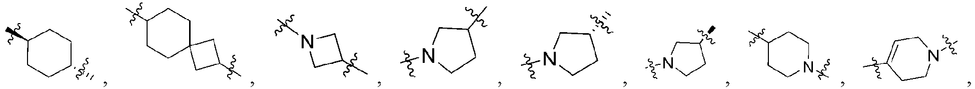

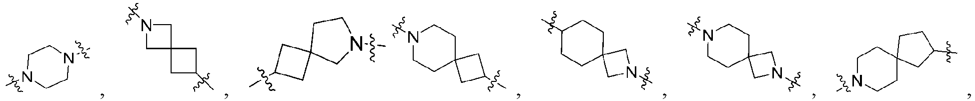

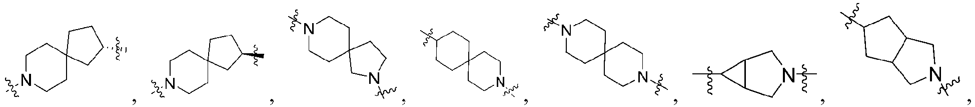

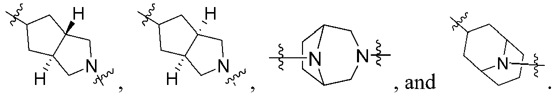

In some embodiments, R^{b} and R^{c} are each independently selected from the group consisting of halogen, =O, -OH, - NH₂, -CN, C₁₋₆ alkyl, C₁₋₆ alkoxy, C₁₋₆ haloalkyl, (C₁₋₆ alkyl)NH-, (C₁₋₆ alkyl)₂N-, C₃₋₁₀ cycloalkyl, and 4- to 10-membered heterocycloalkyl.

In some embodiments, R^{b} and R^{c} are each independently selected from the group consisting of halogen, =O, -OH, - NH₂, -CN, C₁₋₆ alkyl, C₁₋₆ alkoxy, C₁₋₆ haloalkyl, (C₁₋₆ alkyl)NH-, and (C₁₋₆ alkyl)₂N-.

In some embodiments, R^{b} and R^{c} are each independently selected from the group consisting of halogen, =O, -OH, - NH₂, -CN, C₁₋₄ alkyl, C₁₋₄ alkoxy, C₁₋₄ haloalkyl, (C₁₋₄ alkyl)NH-, and (C₁₋₄ alkyl)₂N-.

In some embodiments, R^{b} and R^{c} are each independently selected from the group consisting of halogen, =O, -OH, - NH₂, -CN, and C₁₋₃ alkyl.

In some embodiments, R^{b} and R^{c} are each independently selected from the group consisting of halogen, =O, -OH, - NH₂, and -CN.

In some embodiments, R^{b} and R^{c} are each independently selected from =O.

In some embodiments, L or -LNK¹- is selected from the group consisting of a bond, -O-, -NHCH₂-, -CH₂NHCH₂-, -CH₂-, -CH₂CH₂-, ethynylene, -C(O)-, and -C(O)CH₂-.

In some embodiments, L is selected from the group consisting of a bond, -NHCH₂-, -CH₂NHCH₂-, -CH₂-, -C(O)CH₂-,

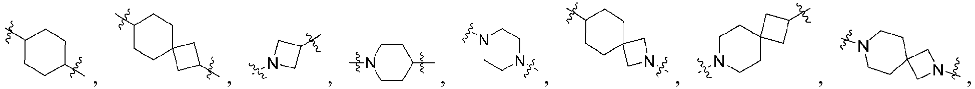

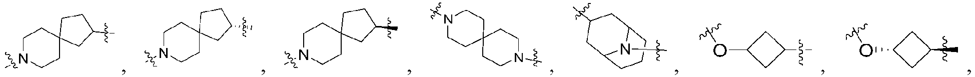

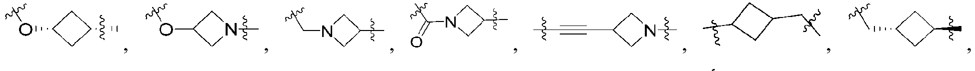

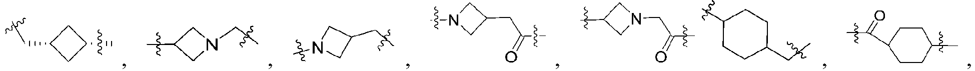

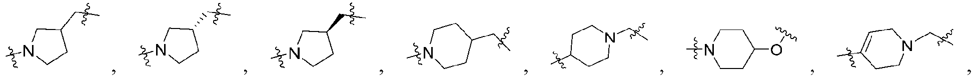

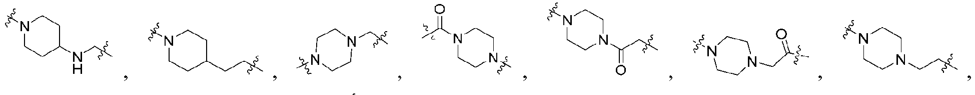

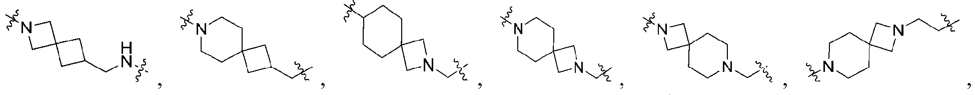

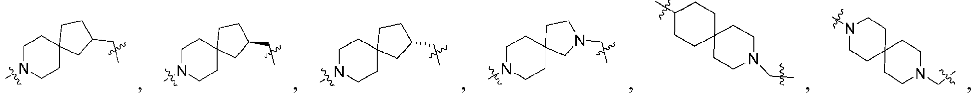

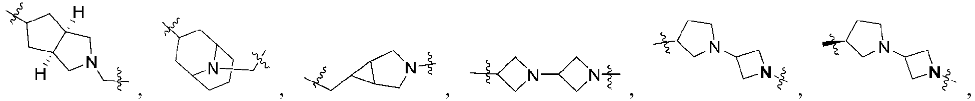

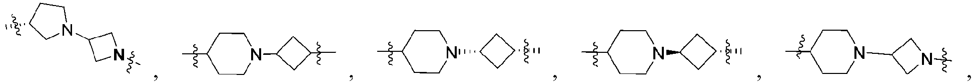

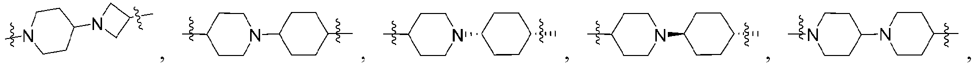

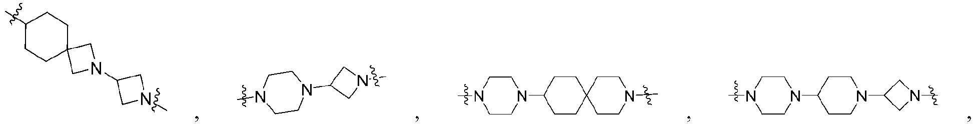

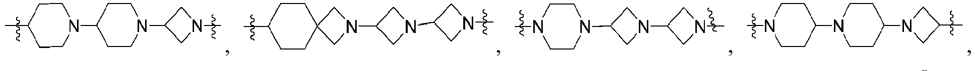

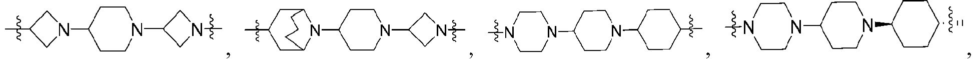

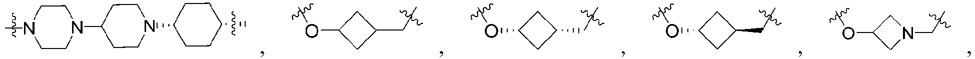

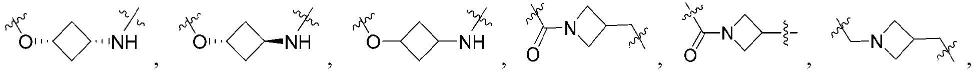

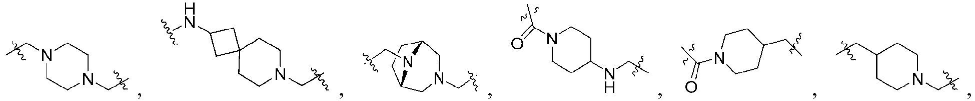

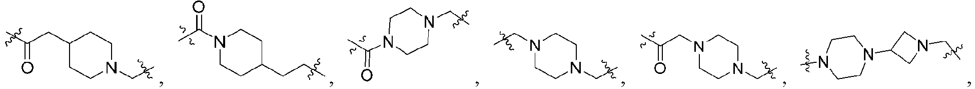

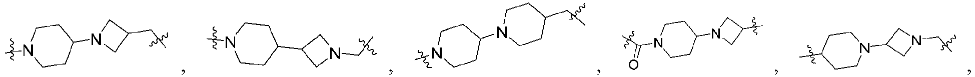

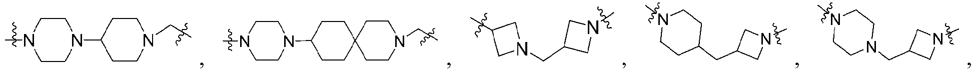

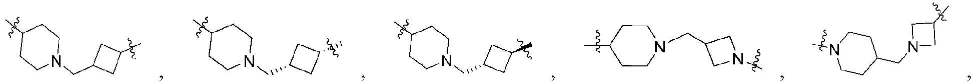

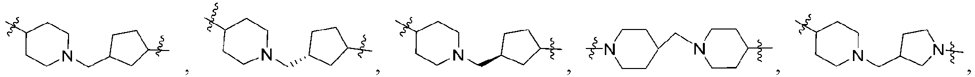

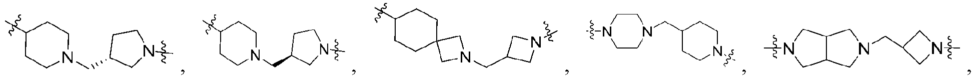

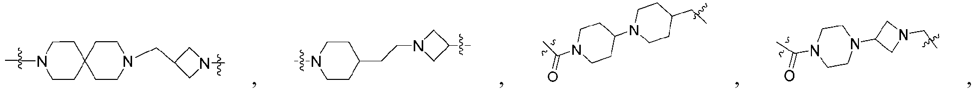

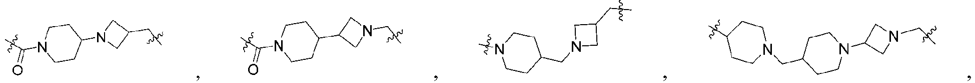

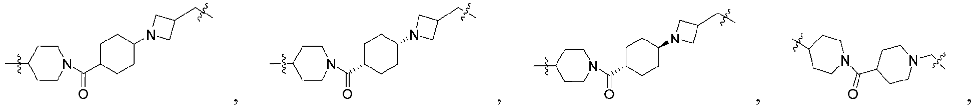

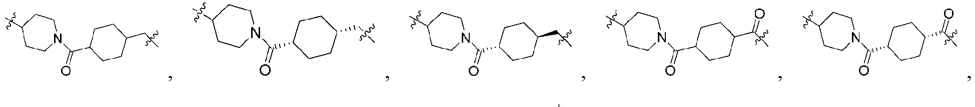

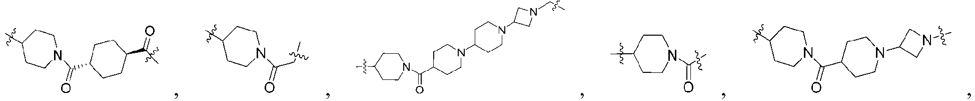

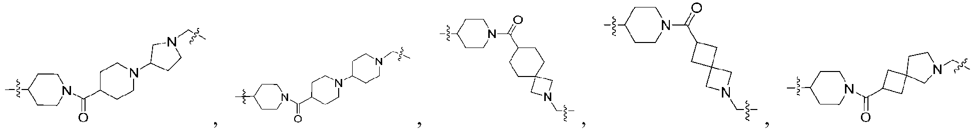

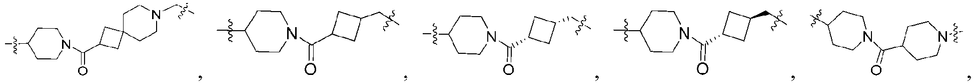

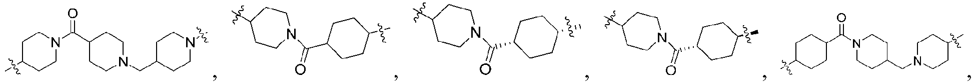

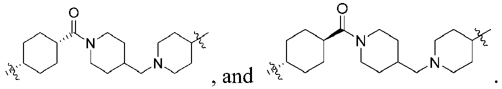

In some embodiments, L is selected from the group consisting of

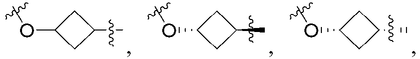

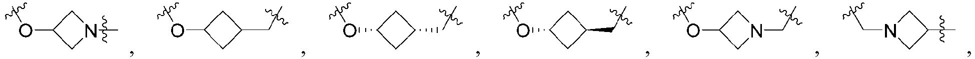

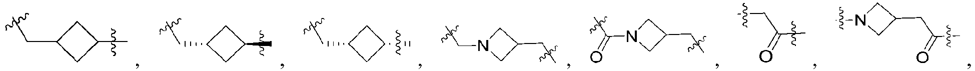

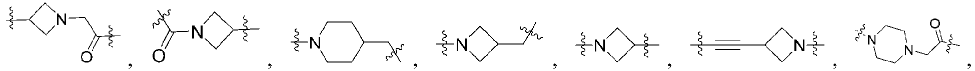

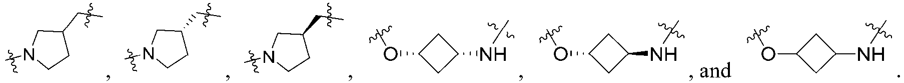

In some embodiments, L is selected from the group consisting of 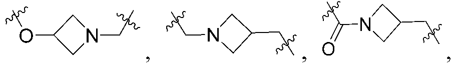 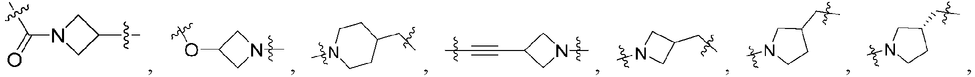 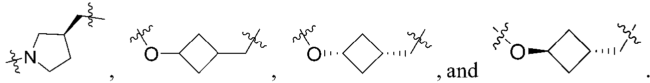 In some embodiments, L is selected from the group consisting of 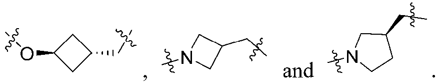 In some embodiments, L is selected from the group consisting of 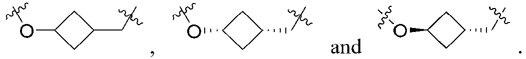 In some embodiments, L is selected from 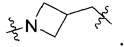 In some embodiments, L is selected from the group consisting of

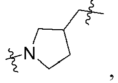

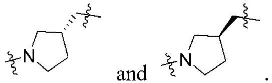

In some embodiments, ring F is linked to L.

In some embodiments, PTM is selected from 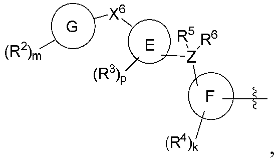 wherein X⁶, Z, R², R³, R⁴, m, p, k, ring G, ring E, ring F, R⁵, and R⁶ are as defined herein; optionally, the structural moieties 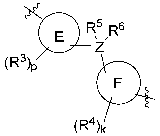 and 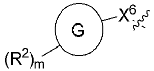 are as defined herein.

In some embodiments, X⁶ is selected from the group consisting of a bond, -O-, -S-, -N(R^{t})-, -C₁₋₄ alkylene-, -C₂₋₄ alkenylene-, -C₂₋₄ alkynylene-, -N(R^{t})C₁₋₄ alkylene-, -OC₁₋₄ alkylene-, -SC₁₋₄ alkylene-, and -N(R^{t})C(O)-.

In some embodiments, X⁶ is selected from the group consisting of a bond, -O-, -S-, -N(R^{t})-, -C₁₋₃ alkylene-, -C₂₋₃ alkenylene-, -C₂₋₃ alkynylene-, -N(R^{t})C₁₋₃ alkylene-, -OC₁₋₃ alkylene-, -SC₁₋₃ alkylene-, and -N(R^{t})C(O)-.

In some embodiments, X⁶ is selected from the group consisting of a bond, -O-, -S-, -N(R^{t})-, -C₁₋₃ alkylene-, -C₂₋₃ alkenylene-, -C₂₋₃ alkynylene-, -N(R^{t})C₁₋₃ alkylene-, -OC₁₋₃ alkylene-, and -N(R^{t})C(O)-.

In some embodiments, X⁶ is selected from the group consisting of a bond, -O-, -S-, -N(R^{t})-, -CH₂-, -C₂ alkynylene-, -N(R^{t})C₁₋₂ alkylene-, -OC₁₋₂ alkylene-, and -N(R^{t})C(O)-.

In some embodiments, X⁶ is selected from the group consisting of a bond, -O-, -S-, -NH-, -CH₂-, -NHCH₂-, - N(CH₃)CH₂-, -OCH₂-, -OCH(CH₃)-, and -NHC(O)-.

In some embodiments, X⁶ is selected from the group consisting of -O-, -S-, -NH-, -C₂₋₄ alkynylene-, -NHC₁₋₄ alkylene-, and -OC₁₋₄ alkylene-.

In some embodiments, X⁶ is selected from the group consisting of a bond, -O-, -C₂₋₃ alkynylene-, and -OC₁₋₃ alkylene-. In some embodiments, X⁶ is selected from the group consisting of a bond, -O-, -C₂₋₃ alkynylene-, and - C₂₋₃ alkenylene-.

In some embodiments, X⁶ is selected from the group consisting of a bond, -O-, -ethynylene-, and -OCH₂-.

In some specific embodiments, X⁶ is selected from the group consisting of a bond, -O-, -NHC₁₋₃ alkylene-, and - OC₁₋₃ alkylene-.

In some specific embodiments, X⁶ is selected from the group consisting of a bond, -O-, -NHCH₂-, and -OCH₂-.

In some specific embodiments, X⁶ is selected from the group consisting of -O-, -C₂₋₃ alkynylene-, and -OC₁₋₃ alkylene-. In some specific embodiments, X⁶ is selected from the group consisting of -O-, -ethynylene-, and -OCH₂-. In some specific embodiments, X⁶ is selected from -O-. In some embodiments, X⁶ is selected from a bond. In some specific embodiments, X⁶ is selected from -OCH₂-. In some specific embodiments, X⁶ is selected from -ethynylene-. In some embodiments, R^{t} is selected from the group consisting of H and C₁₋₆ alkyl. In some embodiments, R^{t} is selected from the group consisting of H and C₁₋₃ alkyl. In some embodiments, R^{t} is selected from the group consisting of H and methyl.

In some embodiments, each R² is independently selected from the group consisting of halogen, -CN, and the following groups optionally substituted with one or more R': R^{v}-, R^{v}O-, R^{v}S-, R^{s}R^{v}N-, R^{v}C(O)-, R^{v}S(O)₂-, R^{v}S(O)-, R^{v}=N-, R^{v}OC(O)-, R^{v}C(O)O-, R^{v}S(O)O-, R^{v}OS(O)-, R^{v}S(O)₂O-, R^{v}OS(O)₂-, R^{s}R^{v}NC(O)-, R^{v}C(O)NH-, R^{v}OC(O)NH-, R^{v}NHC(O)O-, R^{v}S(O)NH-, R^{s}R^{v}NS(O)-, R^{v}S(O)₂NH-, R^{s}R^{v}NS(O)₂-, and R^{s}R^{v}S(O)=N-.

In some other embodiments, each R² is independently selected from the group consisting of halogen, -CN, and the following groups optionally substituted with one or more R': R^{v}-, R^{v}O-, R^{v}C(O)-, R^{v}=N-, R^{v}S(O)₂NH-, and R^{s}R^{v}S(O)=N-.

In some embodiments, the R' substitution is on R^{s} or R^{v}.

In some embodiments, R^{s} and R^{v} are each independently selected from the group consisting of H, C₁₋₁₀ alkyl, C₂₋₁₀ alkenyl, C₂₋₁₀ alkynyl, C₃₋₁₂ cycloalkyl, C₃₋₁₂ cycloalkyl C₁₋₃ alkylene-, 3- to 12-membered heterocycloalkyl, 3- to 12-membered heterocycloalkyl C₁₋₃ alkylene-, C₃₋₁₂ cycloalkenyl, C₃₋₁₂ cycloalkenyl C₁₋₃ alkylene-, 3- to 12-membered heterocycloalkenyl, 3- to 12-membered heterocycloalkenyl C₁₋₃ alkylene-, C₆₋₁₀ aryl, C₆₋₁₀ aryl C₁₋₃ alkylene, 5- to 10-membered heteroaryl, and 5- to 10-membered heteroaryl C₁₋₃ alkylene-.

In some embodiments, R^{s} and R^{v} are each independently selected from the group consisting of H, C₁₋₆ alkyl, C₂₋₆ alkenyl, C₂₋₆ alkynyl, C₃₋₁₀ cycloalkyl, C₃₋₁₀ cycloalkyl C₁₋₃ alkylene-, 3- to 10-membered heterocycloalkyl, 3- to 10-membered heterocycloalkyl C₁₋₃ alkylene-, C₃₋₁₀ cycloalkenyl, C₃₋₁₀ cycloalkenyl C₁₋₃ alkylene-, 3- to 10-membered heterocycloalkenyl, 3- to 10-membered heterocycloalkenyl C₁₋₃ alkylene-, C₆₋₁₀ aryl, C₆₋₁₀ aryl C₁₋₃ alkylene-, 5- to 10-membered heteroaryl, and 5- to 10-membered heteroaryl C₁₋₃ alkylene-.

In some embodiments, R^{s} and R^{v} are each independently selected from the group consisting of H, C₁₋₆ alkyl, C₂₋₆ alkenyl, C₃₋₆ cycloalkyl, 3- to 10-membered heterocycloalkyl, C₆₋₁₀ aryl, and 5- to 6-membered heteroaryl.

In some specific embodiments, each R^{s} is independently selected from the group consisting of H and C₁₋₆ alkyl. In some embodiments, each R^{s} is independently selected from H.

In some embodiments, R^{s} and R^{v} are each independently selected from the group consisting of H, methyl, ethyl, n-propyl, isopropyl, isobutyl, ethenyl, cyclopropyl, cyclobutyl, cyclopentyl, oxetanyl, azetidinyl, pyrrolidinyl, tetrahydrothienyl, 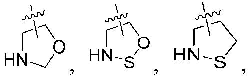 piperidinyl, 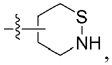 morpholinyl, piperazinyl, 1,4-oxathianyl, 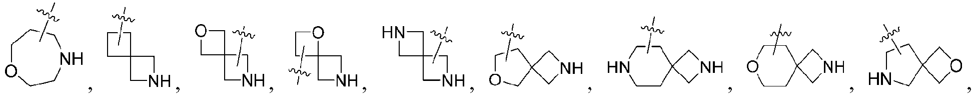 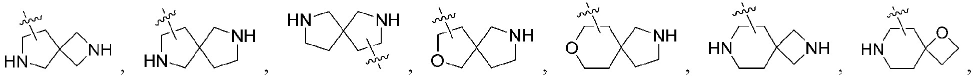 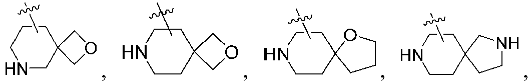 pyrazolyl, imidazolyl, oxazolyl, isoxazolyl, triazolyl, oxadiazolyl, thiadiazolyl, and phenyl.

In some embodiments, R^{s} and R^{v} are each independently selected from the group consisting of H, methyl, ethyl, n-propyl, isopropyl, isobutyl, ethenyl, cyclopropyl, cyclobutyl, cyclopentyl, oxetanyl (e.g., 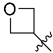 ), azetidinyl (e.g., 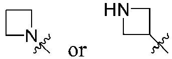 ), pyrrolidinyl (e.g., 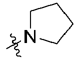 ), 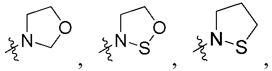 piperidinyl (e.g., 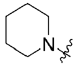 or 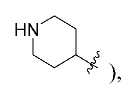 ), 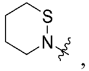 morpholinyl (e.g., 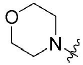 ), piperazinyl (e.g., 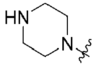 ), 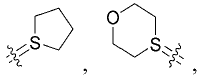 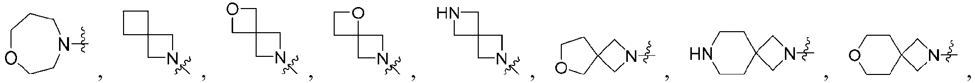 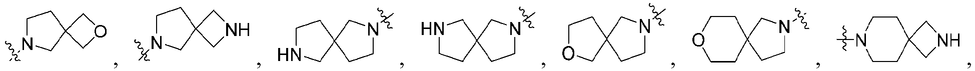 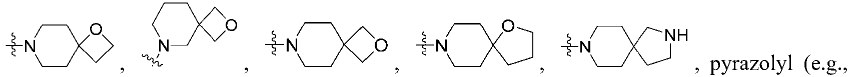 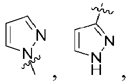 or 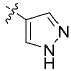 ), imidazolyl (e.g., 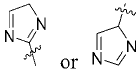 ), oxazolyl (e.g., 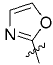 ), isoxazolyl (e.g., 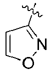 ), triazolyl (e.g., 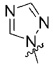 or 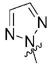 ), oxadiazolyl (e.g., 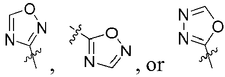 ), thiadiazolyl (e.g., 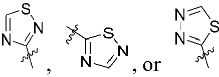 ), and phenyl.

In some other embodiments, R^{s} and R^{v} are each independently selected from the group consisting of H, C₁₋₄ alkyl, C₃₋₄ cycloalkyl, 4- to 7-membered heterocycloalkyl, and 5- to 6-membered heteroaryl.

In some other embodiments, R^{s} and R^{v} are each independently selected from the group consisting of H, methyl, ethyl, isopropyl, tetrahydrothienyl, 1,4-oxathianyl, cyclopropyl, oxetanyl, azetidinyl, pyrrolidinyl, morpholinyl, 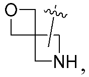 and oxadiazolyl.

In some embodiments, each R² is independently selected from the group consisting of halogen, -OH, -NH₂, -CN, and the following groups optionally substituted with one or more substituents R': C₁₋₆ alkyl, C₁₋₆ alkyl O-, 4- to 10-membered heterocycloalkyl O-, C₁₋₆ alkyl S-, C₁₋₆ alkyl NH-, (C₁₋₆ alkyl)₂N-, C₃₋₁₀ cycloalkyl NH-, 4- to 10-membered heterocycloalkyl NH-, C₁₋₆ alkyl C(O)-, C₃₋₁₀ cycloalkyl C(O)-, 5- to 10-membered heterocycloalkyl C(O)-, C₁₋₆ alkyl S(O)₂-, H₂NC(O)-, C₁₋₆ alkyl NHC(O)-, (C₁₋₆ alkyl)₂NC(O)-, C₂₋₆ alkenyl C(O)NH-, C₁₋₆ alkyl S(O)₂NH-, C₃₋₁₀ cycloalkyl-S(O)₂NH-, H₂NS(O)₂-, (C₁₋₆ alkyl)₂S(O)=N-, 4- to 12-membered heterocycloalkyl=N-, C₃₋₁₀ cycloalkyl, 4- to 12-membered heterocycloalkyl, and 5- to 10-membered heteroaryl.

In some embodiments, each R² is independently selected from the group consisting of halogen, -OH, -NH₂, -CN, and the following groups optionally substituted with one or more substituents R': C₁₋₆ alkyl, C₁₋₆ alkyl O-, 4- to 6-membered heterocycloalkyl O-, C₁₋₆ alkyl S-, C₁₋₆ alkyl NH-, (C₁₋₆ alkyl)₂N-, C₃₋₆ cycloalkyl NH-, 4- to 6-membered heterocycloalkyl NH-, C₁₋₆ alkyl C(O)-, C₃₋₆ cycloalkyl C(O)-, 5- to 6-membered heterocycloalkyl C(O)-, C₁₋₆ alkyl S(O)₂-, H₂NC(O)-, C₁₋₆ alkyl NHC(O)-, (C₁₋₆ alkyl)₂NC(O)-, C₂₋₆ alkenyl C(O)NH-, C₁₋₆ alkyl S(O)₂NH-, C₃₋₆ cycloalkyl-S(O)₂NH-, H₂NS(O)₂-, (C₁₋₆ alkyl)₂S(O)=N-, 4- to 10-membered heterocycloalkyl=N-, C₃₋₆ cycloalkyl, 4- to 10-membered heterocycloalkyl, and 5- to 6-membered heteroaryl.

In some embodiments, each R² is independently selected from the group consisting of halogen, -OH, -NH₂, -CN, and the following groups optionally substituted with one or more substituents R': methyl, ethyl, n-propyl, isopropyl, isobutyl, 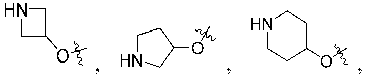 CH₃S-, CH₃NH-, CH₃CH₂NH-, (CH₃)₂CHNH-, (CH₃)₂N-, 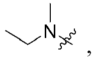 cyclopropyl-NH-, 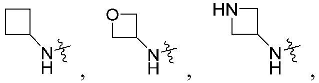 CH₃C(O)-, CH₃CH₂C(O)-, (CH₃)₂CHC(O)-, cyclopropyl-C(O)-, cyclobutyl-C(O)-, cyclopentyl-C(O)-, 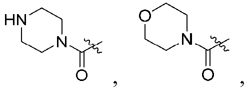 CH₃S(O)₂-, H₂NC(O)-, CH₃NHC(O)-, CH₃CH₂NHC(O)-, (CH₃)₂NC(O)-, 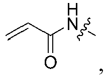 CH₃S(O)₂NH-, cyclopropyl-S(O)₂NH-, H₂NS(O)₂-, (CH₃)₂S(O)=N-, 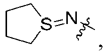 cyclopropyl, 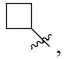 azetidinyl (e.g., 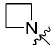 ), pyrrolidinyl (e.g., 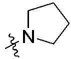 ), 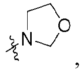 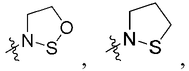 piperidinyl (e.g., 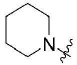 ), 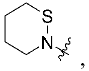 morpholinyl (e.g., 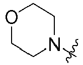 ), piperazinyl (e.g., 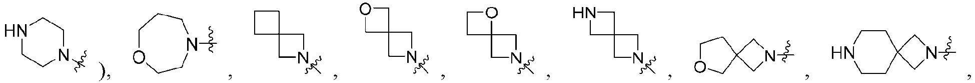 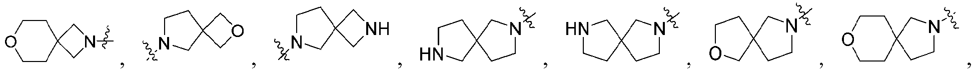 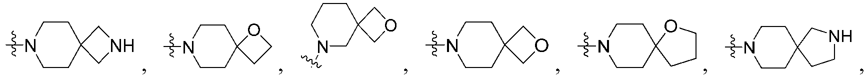 pyrazolyl (e.g., 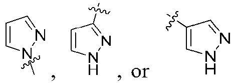 ), imidazolyl (e.g., 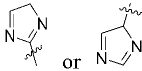 ), oxazolyl (e.g., 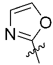 ), isoxazolyl (e.g., 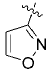 ), triazolyl (e.g., 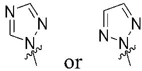 ), oxadiazolyl (e.g., 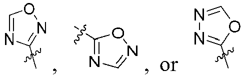 ), and thiadiazolyl (e.g., 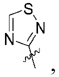 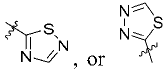 ); in some embodiments, each R² is independently selected from the group consisting of the following groups optionally substituted with one or more substituent R': CH₃O-, CH₃CH₂O-,

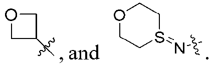

In some embodiments, each R' is independently selected from the group consisting of halogen, -OH, -NH₂, -CN, =O, and the following groups optionally substituted with one or more substituents: C₁₋₆ alkyl, C₁₋₆ alkyl O-C₁₋₆ alkyl-, C₁₋₆ alkyl O-, C₁₋₆ alkyl S-, (C₁₋₆ alkyl)NH-, (C₁₋₆ alkyl)₂N-, C₁₋₆ alkyl C(O)-, C₁₋₆ alkyl S(O)₂-, C₁₋₆ alkyl C(O)NH-, C₁₋₆ alkyl S(O)₂NH-, C₁₋₆ alkyl S(O)NH-, (C₁₋₆ alkyl)₂S(O)=N-, C₁₋₆ alkyl NHC(O)-, C₁₋₆ alkyl NHS(O)₂-, C₁₋₆ alkyl NHS(O)-, C₁₋₆ alkyl C(O)O-, C₁₋₆ alkyl S(O)₂O-, C₁₋₆ alkyl S(O)O-, C₁₋₆ alkyl OC(O)-, C₁₋₆ alkyl OS(O)₂-, C₁₋₆ alkyl OS(O)-, C₁₋₆ alkyl NHC(O)O-, C₁₋₆ alkyl OC(O)NH-, C₃₋₁₀ cycloalkyl, 3- to 10-membered heterocycloalkyl, 3- to 10-membered heterocycloalkenyl, C₆₋₁₀ aryl, and 5- to 10-membered heteroaryl.

In some embodiments, each R' is independently selected from the group consisting of halogen, -OH, -NH₂, -CN, =O, C₁₋₆ alkyl optionally substituted with one or more hydroxyl or halogen, C₁₋₆ alkyl OC₁₋₆ alkylene-, C₁₋₆ alkyl O-, (C₁₋₆ alkyl)NH-, (C₁₋₆ alkyl)₂N-, C₁₋₆ alkyl S(O)₂-, C₁₋₆ alkyl S(O)₂NH-, C₃₋₆ cycloalkyl, and 3- to 6-membered heterocycloalkyl optionally substituted with one or more halogens (e.g., F, Cl, Br, or I).

In some embodiments, each R' is independently selected from the group consisting of -F, -OH, -NH₂, -CN, =O, methyl, ethyl, HOCH₂-, CH₃OCH₂-, CH₃CH₂O-, (CH₃)₂N-, CH₃S(O)₂-, cyclopropyl, cyclobutyl, cyclopentyl, azetidinyl (e.g., 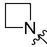 ), and 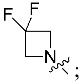 in some embodiments, each R' is independently selected from the group consisting of -Cl and CH₃S(O)₂NH-.

In some specific embodiments, each R' is optionally substituted with one or more substituents. In some specific embodiments, each R' is optionally substituted with one or more of the following substituents: halogen, -OH, -NH₂, -CN, =O, C₁₋₆ alkyl, C₁₋₆ alkyl O-, C₁₋₆ alkyl S-, (C₁₋₆ alkyl) NH-, (C₁₋₆ alkyl)₂N-, C₃₋₁₀ cycloalkyl, 3- to 10-membered heterocycloalkyl, C₆₋₁₀ aryl, and 5- to 10-membered heteroaryl.

In some specific embodiments, each R' is optionally substituted with one or more of the following substituents: halogen, -OH, -NH₂, -CN, =O, C₁₋₆ alkyl, C₁₋₆ alkyl O-, C₁₋₆ alkyl S-, (C₁₋₆ alkyl) NH-, and (C₁₋₆ alkyl)₂N-.

In some specific embodiments, each R' is optionally substituted with one or more of the following substituents: halogen, -OH, -NH₂, and -CN.

In some embodiments, each R² is independently selected from the group consisting of halogen, C₁₋₆ haloalkyl, C₁₋₆ alkyl, C₁₋₆ alkyl O-, C₁₋₆ haloalkyl O-, hydroxyl-substituted C₁₋₆ alkyl-, C₃₋₆ cycloalkyl optionally substituted with hydroxyl, C₁₋₆ alkyl S(O)₂NH-, C₃₋₆ cycloalkyl-S(O)₂NH-, 4- to 9-membered heterocycloalkyl containing 1-3 heteroatoms selected from the group consisting of N, O, and S (including monocyclic or spiro rings), C₁₋₆ alkylsubstituted 4- to 8-membered heterocycloalkyl containing 1-3 heteroatoms selected from the group consisting of N, O, and S (including monocyclic or spiro rings), C₁₋₆ alkyl S(O)₂NH-heterocycloalkyl- (wherein heterocycloalkyl is 4- to 8-membered heterocycloalkyl containing 1-3 heteroatoms selected from the group consisting of N, O, and S), C₁₋₆ alkyl S(O)₂-heterocycloalkyl- (wherein heterocycloalkyl is 4- to 8-membered heterocycloalkyl containing 1-3 heteroatoms selected from the group consisting of N, O, and S), hydroxyl-substituted azetidinyl, hydroxyl-substituted oxetanyl, 3-hydroxyl-thietanyl,

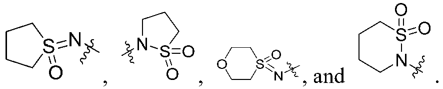

In some embodiments, each R² is independently selected from the group consisting of -F, -CN, -NH₂, methyl, CH₃CH₂-, CH₃CH(NH₂)-, CH₃CH(OH)-, (CH₃)₂C(OH)-, CH₃CH₂CH(OH)-, (CH₃)₂CHCH(OH)-, 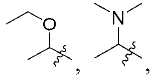 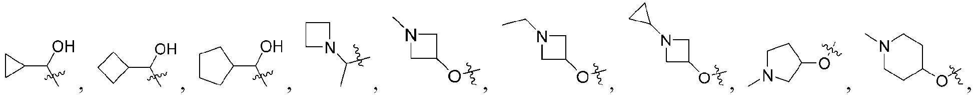 CH₃S-, CH₃NH-, CH₃CH₂NH-, (CH₃)₂CHNH-, 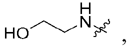 (CH₃)₂N-, 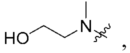 cyclopropyl-NH-, 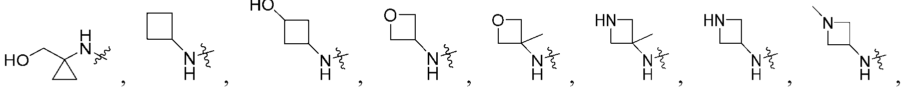 CH₃C(O)-, CH₃CH₂C(O)-, (CH₃)₂CHC(O)-, cyclopropyl-C(O)-, cyclobutyl-C(O)-, cyclopentyl-C(O)-, 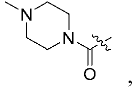 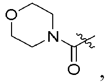 CH₃S(O)₂-, H₂NC(O)-, CH₃NHC(O)-, CH₃CH₂NHC(O)-, (CH₃)₂NC(O)-, 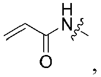 CH₃S(O)₂NH-, cyclopropyl-S(O)₂NH-, H₂NS(O)₂-, (CH₃)₂S(O)=N-, 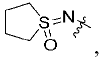 cyclopropyl, 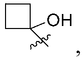 azetidinyl (e.g., 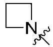 or 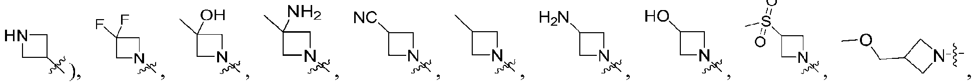 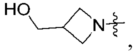 pyrrolidinyl (e.g., 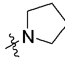 ), 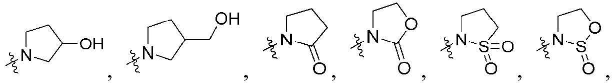 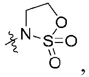 piperidinyl (e.g., 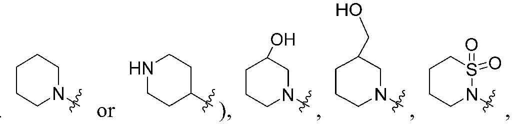 morpholinyl (e.g., 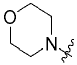 ), 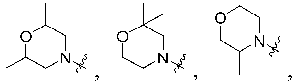 piperazinyl (e.g., 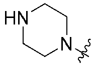 ), 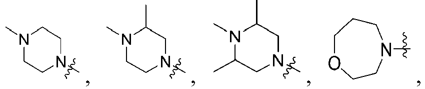 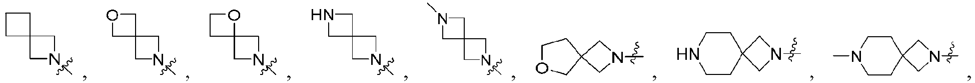 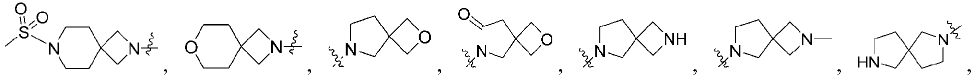 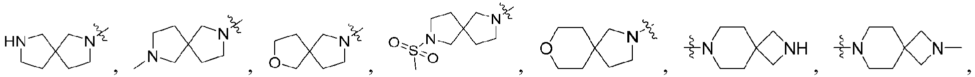 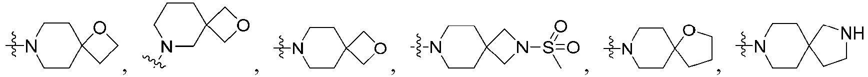 pyrazolyl (e.g., 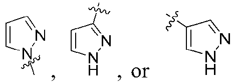 ), imidazolyl (e.g., 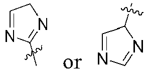 ), oxazolyl (e.g., 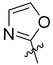 ), isoxazolyl (e.g., 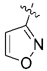 ), triazolyl (e.g., 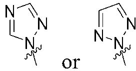 ), oxadiazolyl (e.g., 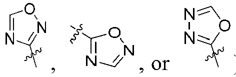 ), thiadiazolyl (e.g., 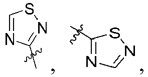 or 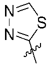 ), 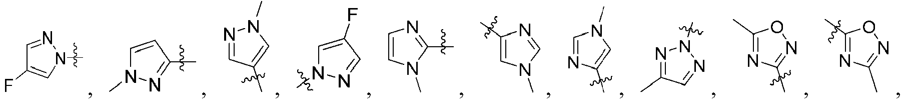 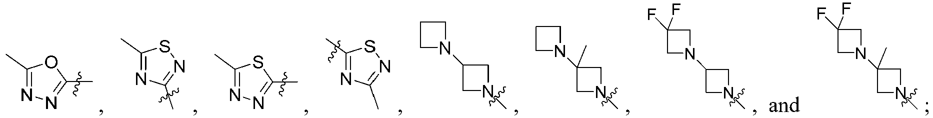 in some specific embodiments, each R² is independently selected from the group consisting of -Cl, -CHF₂, -CF₃, CH₃O-, CHF₂O-, CF₃O-, CF₃CH₂O-,

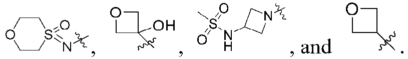

In some specific embodiments, each R² is independently selected from the group consisting of halogen and the following groups optionally substituted with one or more R': C₁₋₆ alkyl, C₁₋₆ alkyl O-, C₁₋₆ alkyl C(O)-, C₁₋₆ alkyl S(O)₂NH-, (C₁₋₆ alkyl)₂S(O)=N-, C₃₋₆ cycloalkyl, 4- to 7-membered heterocycloalkyl, and 5- to 6-membered heteroaryl. In some other embodiments, each R² is independently selected from the group consisting of halogen and the following groups optionally substituted with one or more R': C₁₋₄ alkyl, C₁₋₄ alkyl O-, C₁₋₃ alkyl C(O)-, C₁₋₃ alkyl S(O)₂NH-, (C₁₋₃ alkyl)₂S(O)=N-, C₃₋₄ cycloalkyl, 4- to 7-membered heterocycloalkyl (e.g., 6- to 7-membered heterocycloalkyl), and 5-membered heteroaryl.

In some embodiments, R² is selected from the group consisting of halogen, -CHF₂, -CF₃, methyl, CH₃O-, CHF₂O-, CF₃O-, CF₃CH₂O-, CH₃CH(OH)-, (CH₃)₂C(OH)-, CH₃CH₂CH(OH)-, cyclopropyl, 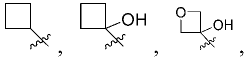 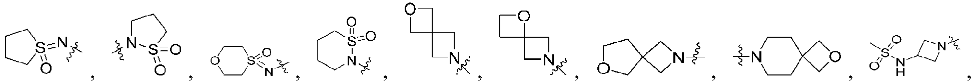 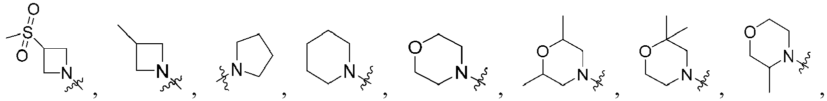 CH₃S(O)₂NH-, and cyclopropyl-S(O)₂NH-. In some specific embodiments, each R² is independently selected from the group consisting of -F, -Cl, CH₃O-, CH₃CH(OH)-, (CH₃)₂C(OH)-, cyclopropyl, 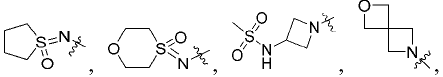 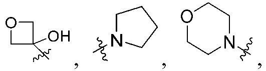 CHF₂O-, CF₃O-, CF₃CH₂O-, -CF₃, -CHF₂, and CH₃S(O)₂NH-.

In some other embodiments, R' is selected from the group consisting of halogen, -OH, -NH₂, -CN, =O, C₁₋₆ alkyl, C₁₋₆ alkyl O-, (C₁₋₆ alkyl) NH-, (C₁₋₆ alkyl)₂N-, C₁₋₆ alkyl S(O)₂NH-, C₃₋₆ cycloalkyl, and 3- to 6-membered heterocycloalkyl.

In some other embodiments, each R' is independently selected from the group consisting of halogen (e.g., F, Cl, Br, or I), -OH, -NH₂, -CN, =O, C₁₋₆ alkyl, C₁₋₆ alkyl O-, (C₁₋₆ alkyl) NH-, C₁₋₆ alkyl S(O)₂NH-, C₃₋₆ cycloalkyl, and 3-to 6-membered heterocycloalkyl.

In some other embodiments, R' is selected from the group consisting of halogen, -OH, -NH₂, -CN, =O, C₁₋₃ alkyl, C₁₋₃ alkyl O-, (C₁₋₃ alkyl) NH-, C₁₋₃ alkyl S(O)₂NH-, and (C₁₋₃ alkyl)₂N-.

In some other embodiments, each R' is independently selected from the group consisting of halogen (e.g., F, Cl, Br, or I), -OH, -NH₂, -CN, =O, C₁₋₄ alkyl, and C₁₋₄ alkyl S(O)₂NH-.

In some other embodiments, each R' is independently selected from the group consisting of -F, -Cl, -OH, =O, methyl, and CH₃S(O)₂NH-.

In some specific embodiments, R' is selected from the group consisting of halogen (F, Cl, Br, or I), -OH, -NH₂, -CN, and C₁₋₃ alkyl. In some specific embodiments, R' is selected from the group consisting of=O and C₁₋₃ alkyl S(O)₂NH-. In some other embodiments, each R² is independently selected from the group consisting of halogen and the following groups optionally substituted with one or more R': C₁₋₆ alkyl, C₁₋₆ alkyl O-, C₁₋₆ alkyl C(O)-, C₁₋₆ alkyl S(O)₂NH-, (C₁₋₆ alkyl)₂S(O)=N-, 5- to 7-membered heterocycloalkyl=N-, C₃₋₆ cycloalkyl, 4- to 7-membered heterocycloalkyl, and 5- to 6-membered heteroaryl.

In some other embodiments, each R² is independently selected from the group consisting of the following groups: halogen, C₁₋₄ alkyl, C₁₋₄ alkyl O-, C₁₋₃ alkyl C(O)-, C₁₋₃ alkyl S(O)₂NH-, (C₁₋₃ alkyl)₂S(O)=N-, 5- to 6-membered heterocycloalkyl=N-, C₃₋₄ cycloalkyl, 4- to 7-membered heterocycloalkyl, and 5-membered heteroaryl, wherein the C₁₋₄ alkyl, C₁₋₄ alkyl O-, C₁₋₃ alkyl C(O)-, C₁₋₃ alkyl S(O)NH-, (C₁₋₃ alkyl)₂S(O)=N-, 5- to 6-membered heterocycloalkyl=N-, C₃₋₄ cycloalkyl, 4- to 7-membered heterocycloalkyl, or 5-membered heteroaryl is optionally substituted with one or more of the following groups: halogen (e.g., F, Cl, Br, or I), -OH, -NH₂, -CN, C₁₋₃ alkyl, =O, and C₁₋₃ alkyl S(O)₂NH-.

In some other embodiments, each R² is independently selected from the group consisting of -F, CH₃CH(OH)-, (CH₃)₂C(OH)-, -CHF₂, -CF₃, CH₃O-, CHF₂O-, CF₃O-, CF₃CH₂O-, CH₃C(O)-, CH₃S(O)₂NH-, (CH₃)₂S(O)=N-, 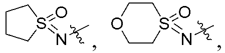 cyclopropyl,

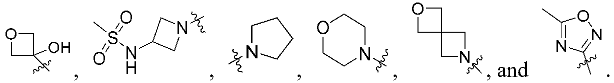

In some specific embodiments, each R² is independently selected from the group consisting of the following groups: C₁₋₄ alkyl, C₁₋₃ alkyl C(O)-, C₁₋₃ alkyl S(O)₂NH-, (C₁₋₃ alkyl)₂S(O)=N-, 6- to 7-membered heterocycloalkyl, and 5-membered heteroaryl, wherein the C₁₋₄ alkyl, 6- to 7-membered heterocycloalkyl, or 5-membered heteroaryl is optionally substituted with one or more of the following groups: halogen, -OH, -NH₂, -CN, and C₁₋₃ alkyl.

In some specific embodiments, each R² is independently selected from the group consisting of the following groups: C₁₋₃ alkyl, C₁₋₂ alkyl C(O)-, C₁₋₂ alkyl S(O)₂NH-, (C₁₋₂ alkyl)₂S(O)=N-, 7-membered heterocycloalkyl, and 5-membered heteroaryl, wherein the C₁₋₃ alkyl or 5-membered heteroaryl is optionally substituted with one or more of the following groups: -OH and C₁₋₃ alkyl.

In some specific embodiments, each R² is independently selected from the group consisting of the following groups: CH₃CH₂-, CH₃C(O)-, CH₃S(O)₂NH-, (CH₃)₂S(O)=N-, 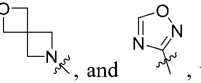 wherein the CH₃CH₂- or 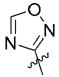 is optionally substituted with one or more of the following groups: -OH and methyl.

In some specific embodiments, each R² is independently selected from the group consisting of CH₃CH(OH)-, CH₃C(O)-, CH₃S(O)₂NH-, (CH₃)₂S(O)=N-, 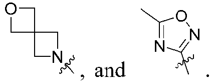 In some specific embodiments, each R² is independently selected from the group consisting of CH₃CH(OH)- and CH₃C(O)-. In some specific embodiments, each R² is independently selected from the group consisting of CH₃S(O)₂NH- and (CH₃)₂S(O)=N-. In some specific embodiments, each R² is independently selected from the group consisting of

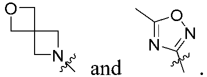

In some embodiments, each R³ is independently selected from the group consisting of halogen, -OH, -NH₂, -CN, C₁₋₆ alkyl, C₁₋₆ alkoxy, C₁₋₆ haloalkyl, C₃₋₆ cycloalkyl, 3- to 6-membered heterocycloalkyl, C₆₋₁₀ aryl, and 5- to 6-membered heteroaryl.

In some embodiments, each R³ is independently selected from the group consisting of halogen, -OH, -NH₂, -CN, C₁₋₆ alkyl, C₁₋₆ alkoxy, and C₁₋₆ haloalkyl.

In some embodiments, each R³ is independently selected from the group consisting of halogen, -OH, -NH₂, -CN, and methyl.

In some embodiments, each R³ is independently selected from halogen. In some embodiments, each R³ is independently selected from F.

In some embodiments, each R⁴ is independently selected from the group consisting of halogen, -OH, -NH₂, -CN, C₁₋₆ alkyl, C₁₋₆ alkoxy, C₁₋₆ haloalkyl, C₃₋₆ cycloalkyl, 3- to 6-membered heterocycloalkyl, C₆₋₁₀ aryl, and 5- to 6-membered heteroaryl.

In some embodiments, each R⁴ is independently selected from the group consisting of halogen, -OH, -NH₂, -CN, C₁₋₆ alkyl, C₁₋₆ alkoxy, and C₁₋₆ haloalkyl.

In some embodiments, each R⁴ is independently selected from the group consisting of halogen, -OH, -NH₂, -CN, and methyl.

In some embodiments, each R⁴ is independently selected from the group consisting of halogen, -OH, -NH₂, and - CN.

In some embodiments, each R⁴ is independently selected from the group consisting of halogen and -CN.

In some embodiments, each R⁴ is independently selected from the group consisting of -F, -Cl, and -CN.

In some embodiments, m is selected from the group consisting of 0, 1, 2, and 3. In some embodiments, m is selected from the group consisting of 1 and 2. In some embodiments, m is 1.

In some embodiments, p is selected from the group consisting of 0, 1, and 2. In some embodiments, p is selected from the group consisting of 0 and 1. In some embodiments, p is 0.

In some embodiments, k is selected from the group consisting of 0, 1, 2, and 3. In some embodiments, k is selected from the group consisting of 0, 1, and 2. In some embodiments, k is 0 or 1.

In some embodiments, m is selected from 1, p is selected from 0, and k is selected from the group consisting of 0, 1, and 2.

In some embodiments, ring G is selected from the group consisting of C₄₋₆ cycloalkyl, 4- to 9-membered heterocycloalkyl, phenyl, 5- to 9-membered heteroaryl, and 9- to 10-membered heterocycloalkenyl. In some embodiments, ring G is selected from the group consisting of C₄₋₆ cycloalkyl, 4- to 9-membered heterocycloalkyl, phenyl, 5- to 8-membered heteroaryl, and 9- to 10-membered heterocycloalkenyl. In some embodiments, ring G is selected from the group consisting of C₄₋₆ cycloalkyl, 4- to 9-membered heterocycloalkyl, phenyl, 5- to 8-membered heteroaryl, tetrahydroisoquinolinyl, tetrahydropyridopyrimidinyl, tetrahydropyridopyrazinyl, pyrrolopyrimidinyl, and pyrimidopyrrolidinyl.

In some embodiments, ring G is selected from the group consisting of phenyl, 5- to 6-membered heteroaryl, and 9-to 10-membered heterocycloalkenyl. In some embodiments, ring G is selected from the group consisting of pyrimidine and tetrahydropyridopyrimidinyl.

In some embodiments, ring G is selected from the group consisting of cyclobutyl, cyclopentyl, cyclohexyl, oxetanyl, azetidinyl, pyrrolidinyl, tetrahydrofuranyl, tetrahydropyranyl, piperidinyl, morpholinyl, dioxanyl, 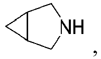 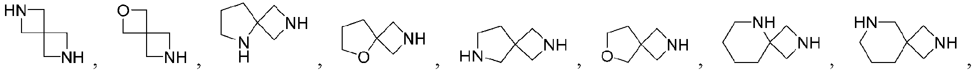 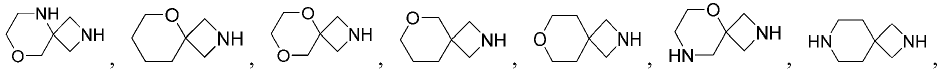 phenyl, thiazolyl, isothiazolyl, oxazolyl, isoxazolyl, imidazolyl, pyrazolyl, triazolyl, oxadiazolyl, thiadiazolyl, pyridinyl, pyrimidinyl, pyrazinyl, pyridazinyl,

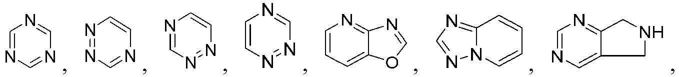

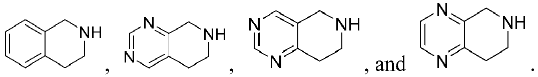

In some embodiments, ring G is selected from the group consisting of cyclobutyl, cyclopentyl, cyclohexyl, oxetanyl, azetidinyl, pyrrolidinyl, tetrahydrofuranyl, tetrahydropyranyl, piperidinyl, morpholinyl, dioxanyl,

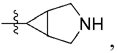

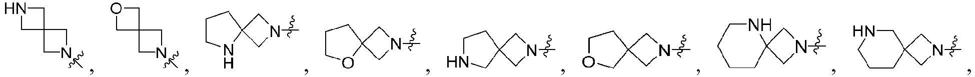

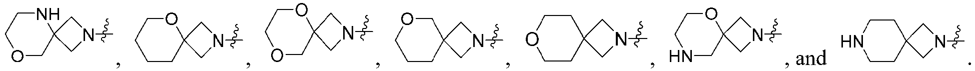

In some embodiments, ring G is selected from the group consisting of phenyl, thiazolyl, isothiazolyl, oxazolyl, isoxazolyl, imidazolyl, pyrazolyl, triazolyl, oxadiazolyl, thiadiazolyl, pyridinyl, pyrimidinyl, pyrazinyl, pyridazinyl, 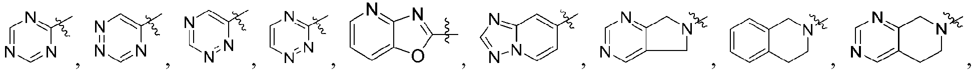 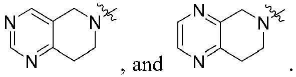 In some embodiments, ring G is selected from the group consisting of pyridinyl, pyrimidinyl, pyrazinyl, pyridazinyl, 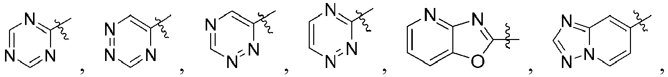 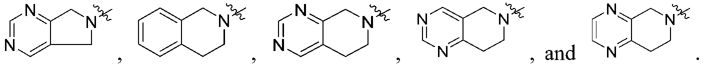 In some embodiments, ring G is selected from the group consisting of pyridinyl, pyrimidinyl, pyridazinyl, 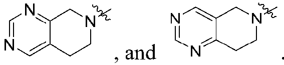 In some embodiments, ring G is selected from the group consisting of 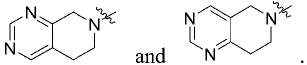 In some embodiments, ring G is selected from the group consisting of pyrimidinyl and pyridazinyl. In some embodiments, ring G is selected from the group consisting of 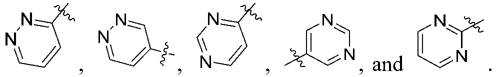 In some embodiments, ring G is selected from the group consisting of

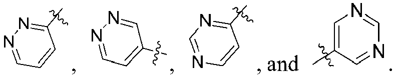

In some embodiments, ring G is selected from the group consisting of phenyl and 5- to 10-membered heteroaryl. In some embodiments, ring G is selected from the group consisting of phenyl and 5- to 10-membered heteroaryl containing 1-3 heteroatoms selected from the group consisting of N, O, and S.

In some embodiments, ring G is selected from the group consisting of phenyl and 6-membered heteroaryl. In some embodiments, ring G is selected from the group consisting of phenyl and 6-membered N-containing heteroaryl. In some embodiments, ring G is selected from the group consisting of phenyl and 5- to 6-membered heteroaryl containing 1-3 N atoms.

In some embodiments, ring G is selected from the group consisting of phenyl, pyrimidinyl, pyridazinyl, pyridinyl, and pyrazinyl.

In some embodiments, ring G is selected from the group consisting of

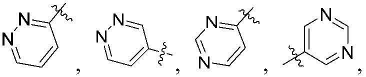

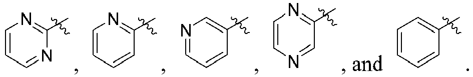

In some embodiments, ring G is selected from the group consisting of 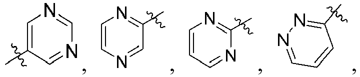 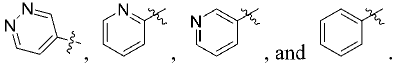 In some embodiments, ring E is selected from the group consisting of C₆₋₁₂ aryl and 5- to 10-membered heteroaryl. In some embodiments, ring E is selected from the group consisting of phenyl and 5- to 6-membered heteroaryl. In some embodiments, ring E is selected from the group consisting of phenyl and 6-membered heteroaryl. In some embodiments, ring E is selected from the group consisting of phenyl and 6-membered N-containing heteroaryl. In some embodiments, ring E is selected from the group consisting of phenyl and 6-membered heteroaryl containing 1-2 N atoms. In some embodiments, ring E is selected from the group consisting of phenyl and pyridinyl. In some embodiments, ring E is selected from the group consisting of 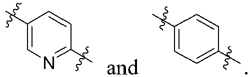 In some embodiments, ring E is selected from

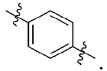

In some embodiments, ring F is selected from the group consisting of C₆₋₁₂ aryl and 5- to 10-membered heteroaryl. In some embodiments, ring F is selected from the group consisting of phenyl and 5- to 6-membered heteroaryl. In some embodiments, ring F is selected from the group consisting of phenyl and 6-membered heteroaryl. In some embodiments, ring F is selected from the group consisting of phenyl and 6-membered N-containing heteroaryl. In some embodiments, ring F is selected from the group consisting of phenyl and 6-membered heteroaryl containing 1-2 N atoms. In some embodiments, ring F is selected from the group consisting of phenyl and pyridinyl. In some embodiments, ring F is selected from the group consisting of 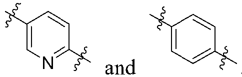 . In some embodiments, ring F is selected from

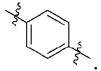

In some embodiments, 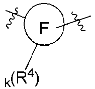 is selected from the group consisting of

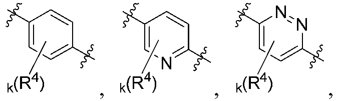

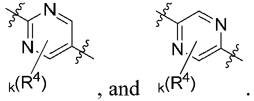

In some embodiments, 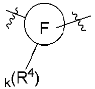 is selected from the group consisting of

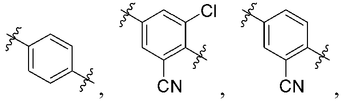

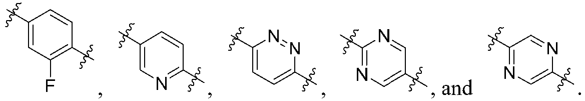

In some embodiments, Z is selected from -C-.

In some embodiments, R⁵ and R⁶ are each independently selected from the group consisting of H, =O, halogen, -OH, -NH₂, -CN, C₁₋₆ alkyl, and C₂₋₆ alkenyl, or R⁵ and R⁶ are linked to each other to form the following group optionally substituted with one or more substituents such as R": C₃₋₆ cycloalkyl or 3- to 6-membered heterocycloalkyl.

In some embodiments, R⁵ and R⁶ are each independently selected from the group consisting of H, C₁₋₃ alkyl, and C₂₋₃ alkenyl, or R⁵ and R⁶ are linked to each other to form the following group optionally substituted with one or more R": C₃₋₆ cycloalkyl or 4- to 6-membered heterocycloalkyl.

In some embodiments, R⁵ and R⁶ are each independently selected from the group consisting of H, methyl, ethyl, and ethenyl, or R⁵ and R⁶ are linked to each other to form the following group optionally substituted with one or more R": cyclopropyl, cyclobutyl, cyclopentyl, cyclohexyl, tetrahydropyranyl, or oxetanyl.

In some embodiments, R" is selected from the group consisting of halogen, -OH, -NH₂, -CN, C₁₋₃ alkyl, and C₁₋₃ haloalkyl.

In some embodiments, R⁵ and R⁶ are each independently selected from the group consisting of H, methyl, ethyl, and ethenyl, or R⁵ and R⁶ are linked to each other to form the following group: cyclopropyl, cyclobutyl, cyclopentyl, cyclohexyl, tetrahydropyranyl, or oxetanyl.

In some embodiments, R⁵ and R⁶ are each independently selected from the group consisting of H and C₁₋₆ alkyl, or R⁵ and R⁶ are linked to each other to form the following group: C₃₋₆ cycloalkyl or 4- to 6-membered heterocycloalkyl. In some embodiments, R⁵ and R⁶ are each independently selected from the group consisting of H and C₁₋₃ alkyl, or R⁵ and R⁶ are linked to each other to form the following group: C₃₋₄ cycloalkyl or 4-membered heterocycloalkyl. In some embodiments, R⁵ and R⁶ are each independently selected from the group consisting of H and C₁₋₃ alkyl, or R⁵ and R⁶ are linked to each other to form 4-membered heterocycloalkyl. In some embodiments, R⁵ and R⁶ are each independently selected from the group consisting of H and C₁₋₃ alkyl.

In some embodiments, R⁵ and R⁶ are each independently selected from methyl, or R⁵ and R⁶ are linked to each other to form oxetanyl.

In some embodiments, the structural moiety 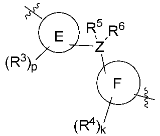 is selected from the group consisting of 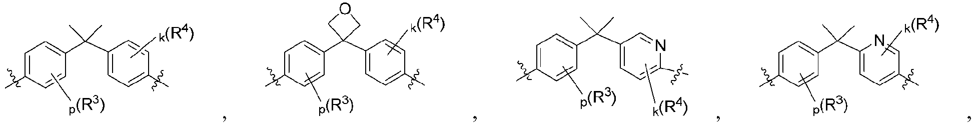 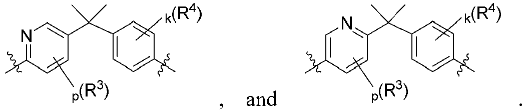 In some embodiments, the structural moiety 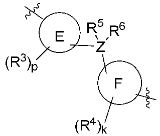 is selected from the group consisting of 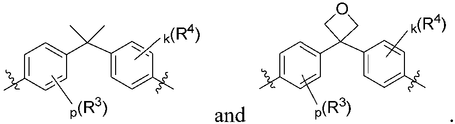 In some embodiments, the structural moiety 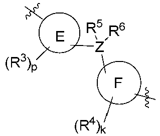 is selected from the group consisting of 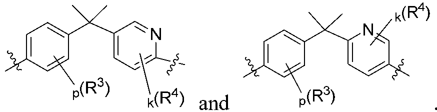 In some embodiments, the structural moiety 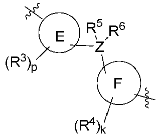 is selected from the group consisting of

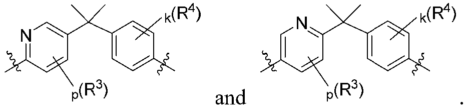

In some embodiments, the structural moiety 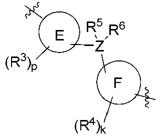 is selected from the group consisting of 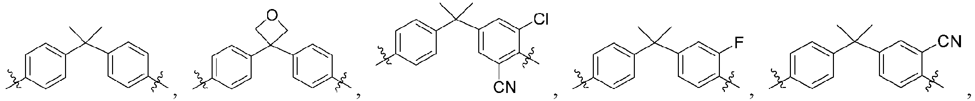 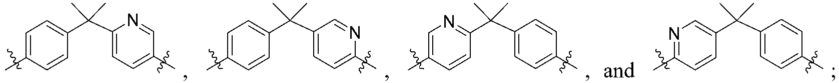 optionally, in the fragment, the left ring is ring E, and the right ring is ring F.

In some specific embodiments, the structural moiety 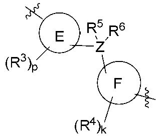 is selected from the group consisting of

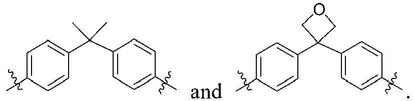

In some embodiments, the structural moiety 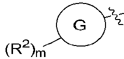 is selected from the group consisting of

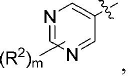

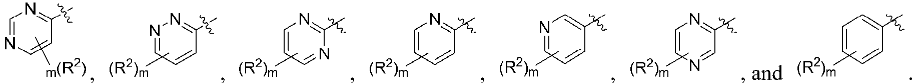

In some embodiments, the structural moiety 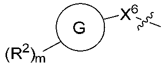 is selected from the group consisting of

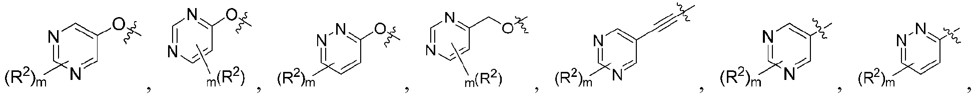

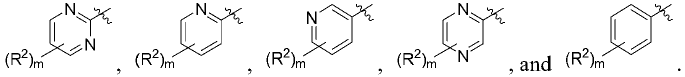

In some embodiments, the structural moiety 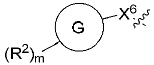 is selected from the group consisting of

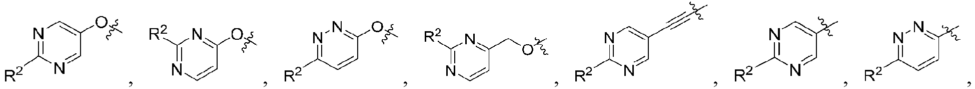

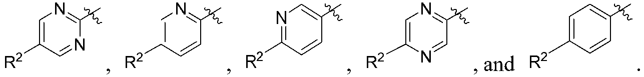

In some embodiments, the structural moiety 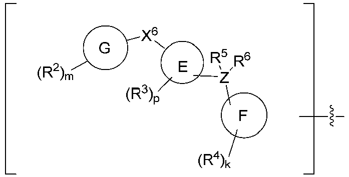 (or PTM) is selected from the group consisting of 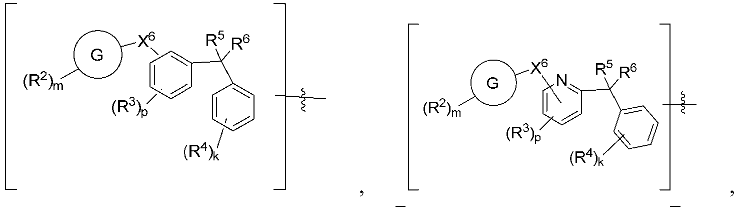 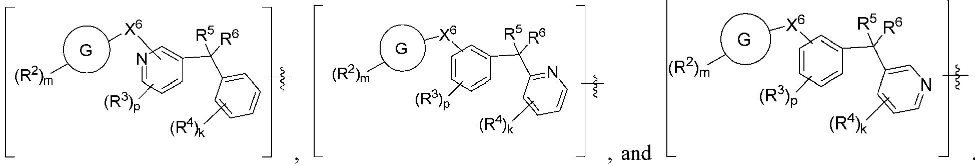 In some embodiments, the structural moiety 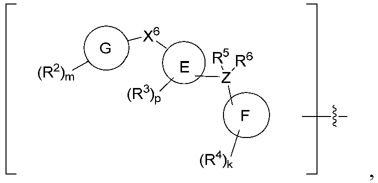 or PTM is

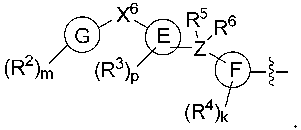

In some embodiments, the structural moiety 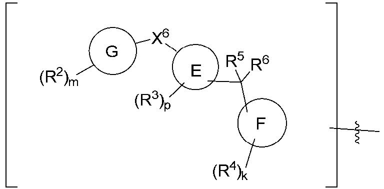 (or PTM) is selected from the group consisting of

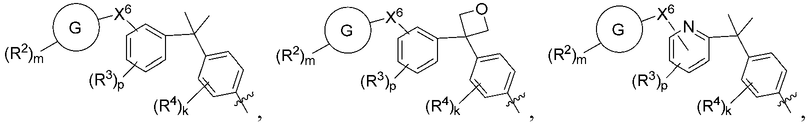

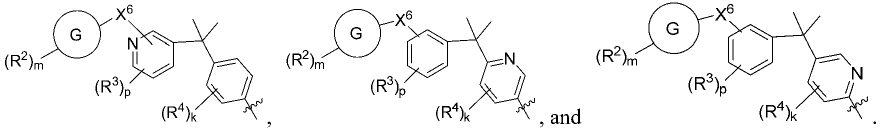

In some embodiments, the structural moiety 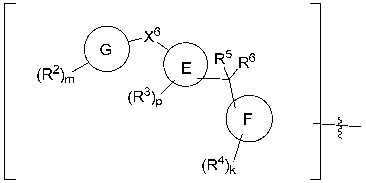 (or PTM) is selected from the group consisting of

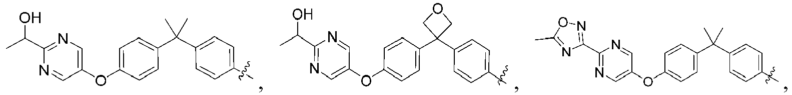

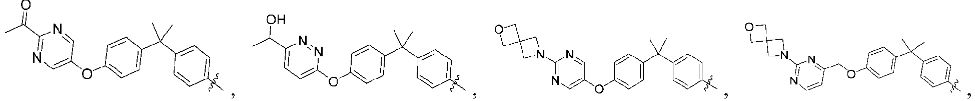

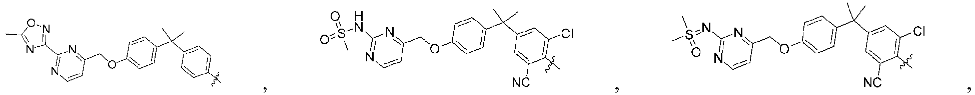

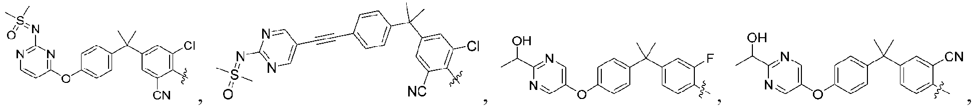

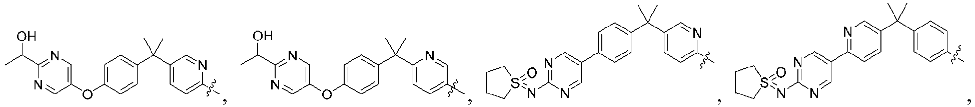

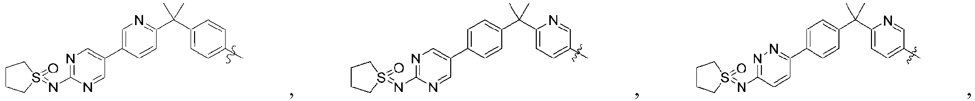

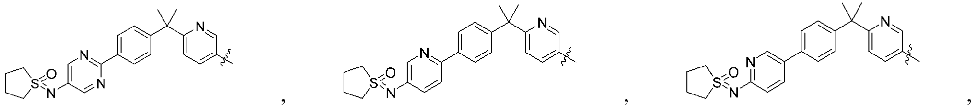

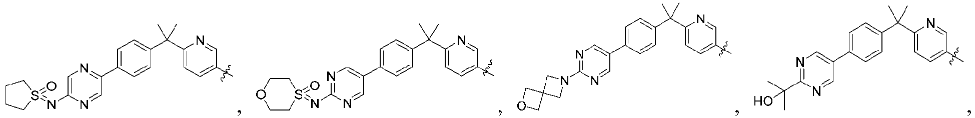

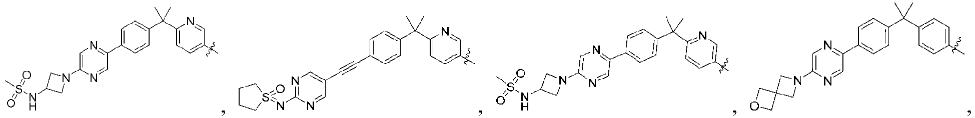

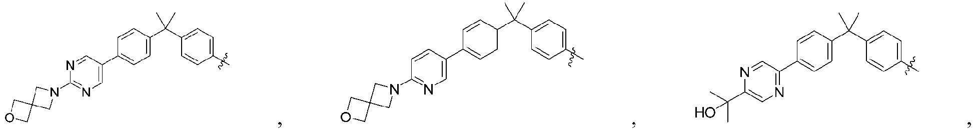

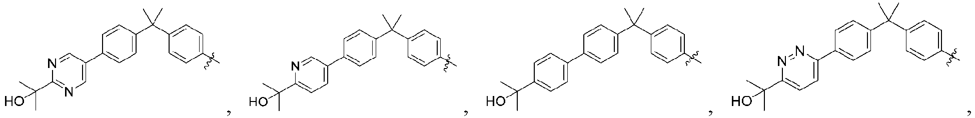

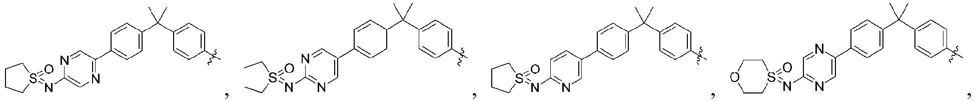

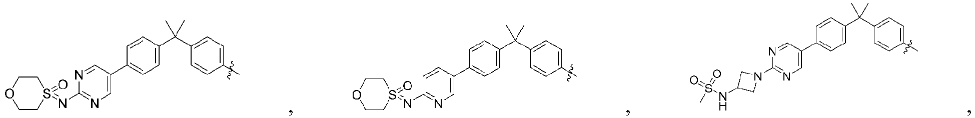

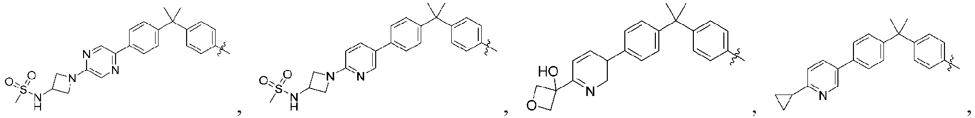

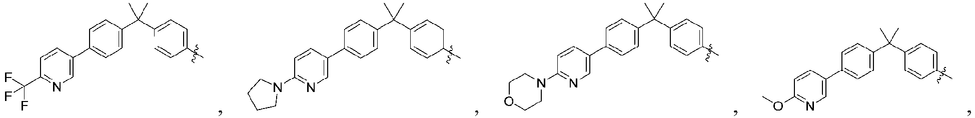

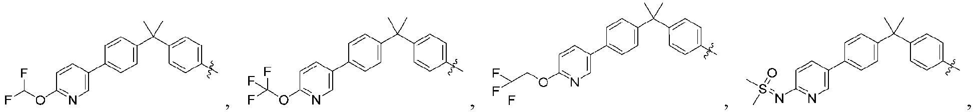

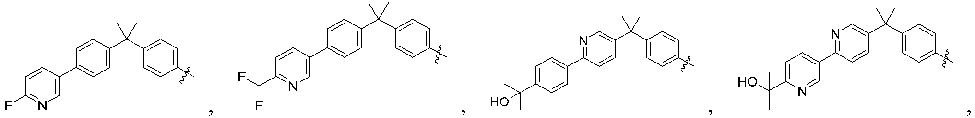

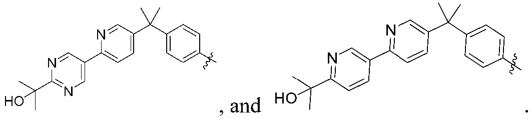

In some specific embodiments, the structural moiety 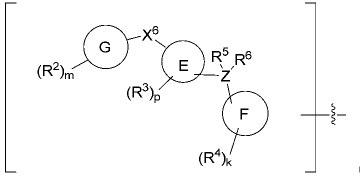 (or PTM) is selected from the group consisting of 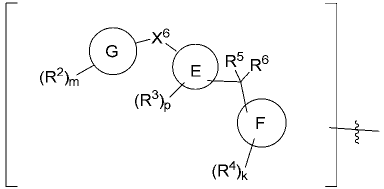 and

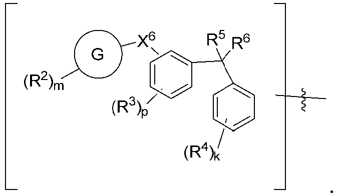

In some specific embodiments, the structural moiety 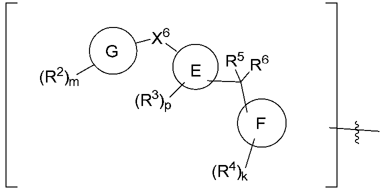 (or PTM) is selected from

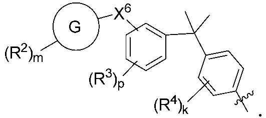

In some specific embodiments, the structural moiety 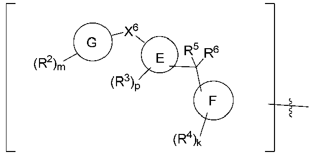 (or PTM) or 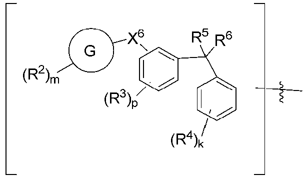 is selected from the group consisting of 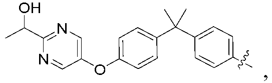 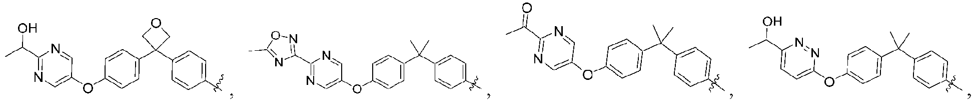 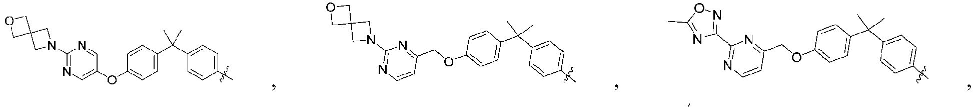 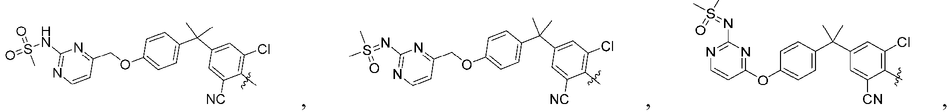 and

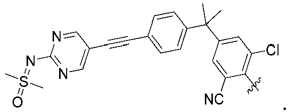

In some embodiments, the structural moiety 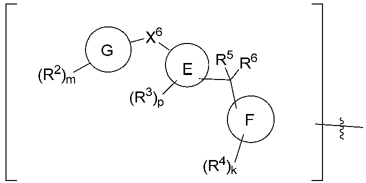 (or PTM) is selected from 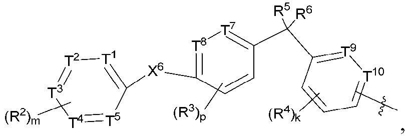 wherein T¹, T², T³, T⁴, T⁵, T⁷, T⁸, T⁹, and T¹⁰ are each independently selected from the group consisting of CH and N, one or more of which are selected from N, and X⁶ is selected from the group consisting of O and a bond; when X⁶ is selected from O, one or more of T⁷, T⁸, and T¹⁰ are selected from N, wherein R², R³, R⁴, m, p, k, R⁵, and R⁶ are as defined herein. In some embodiments, the structural moiety 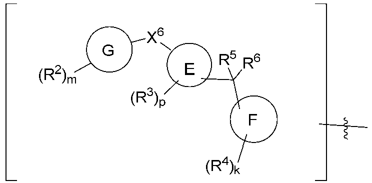 (or PTM) is selected from 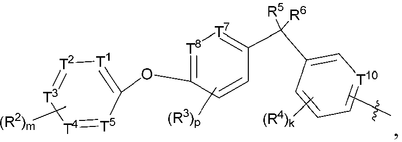 wherein T¹, T², T³, T⁴, T⁵, T⁷, T⁸, and T¹⁰ are each independently selected from the group consisting of CH and N, and one or more of T⁷, T⁸, and T¹⁰ are selected from N, wherein R², R³, R⁴, m, p, k, R⁵, and R⁶ are as defined herein.

In some embodiments, the structural moiety 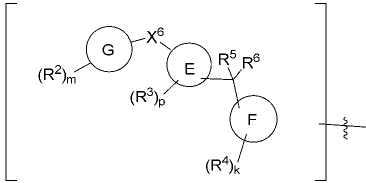 (or PTM) is selected from 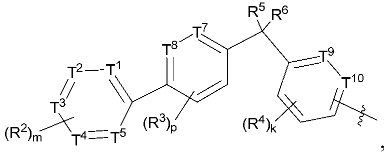 wherein T¹, T², T³, T⁴, T⁵, T⁷, T⁸, T⁹ and T¹⁰ are each independently selected from the group consisting of CH and N, one or more of which are selected from N, wherein R², R³, R⁴, m, p, k, R⁵, and R⁶ are as defined herein.

In some embodiments, T¹, T², T³, T⁴, and T⁵ are each independently selected from the group consisting of CH and N, one or more of which are selected from N.

In some embodiments, one or two of T¹, T², T³, T⁴, and T⁵ are selected from N, and the others are selected from CH. In some embodiments, T² and T⁴ are each independently selected from N, and T¹, T³, and T⁵ are each independently selected from the group consisting of CH and N.

In some embodiments, T⁷, T⁸, T⁹, and T¹⁰ are each independently selected from the group consisting of CH and N, one or two of which are selected from N, and the others are selected from CH.

In some embodiments, T⁷ and T⁸ are each independently selected from the group consisting of CH and N, one of which is selected from CH, and the other is selected from the group consisting of CH and N.

In some embodiments, T⁷ and T⁸ are selected from the group consisting of CH and N, one of which is selected from CH, and the other is selected from N.

In some embodiments, T⁷ and T⁸ are selected from CH.

In some embodiments, T⁹ and T¹⁰ are each independently selected from the group consisting of CH and N, one of which is selected from CH, and the other is selected from the group consisting of CH and N.

In some embodiments, T⁹ and T¹⁰ are selected from the group consisting of CH and N, one of which is selected from CH, and the other is selected from N.

In some embodiments, T⁹ and T¹⁰ are selected from CH.

In some embodiments, T¹⁰ is selected from N.

In some embodiments, T¹⁰ is selected from CH.

In some embodiments, 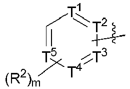 is selected from the group consisting of

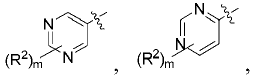

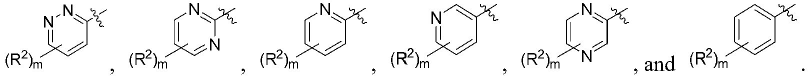

In some embodiments, the structural moiety 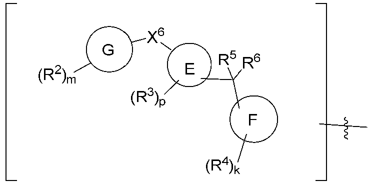 (or PTM) is selected from the group consisting of

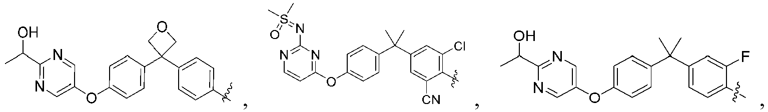

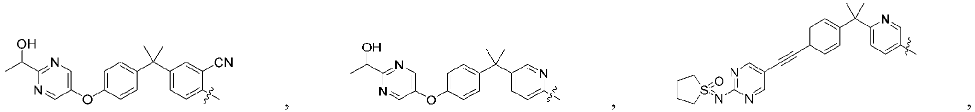

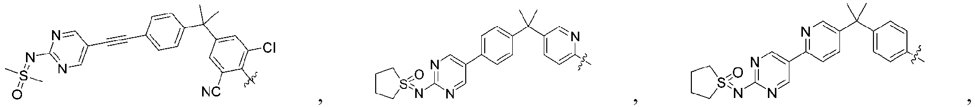

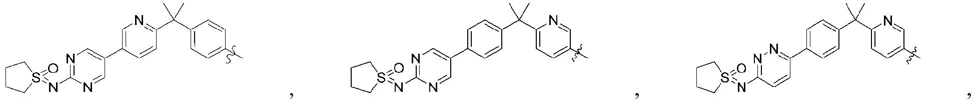

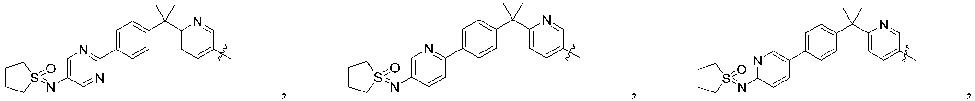

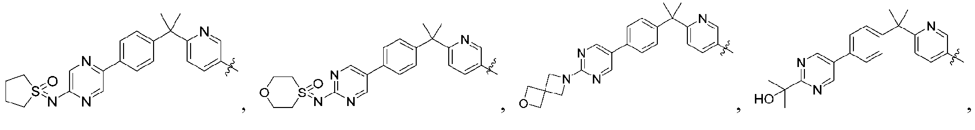

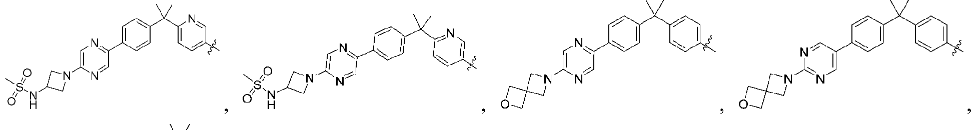

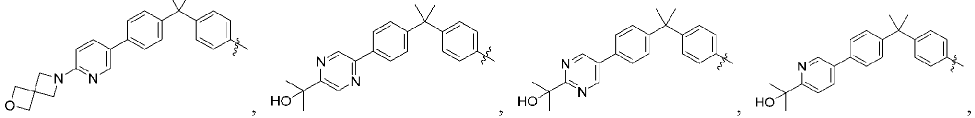

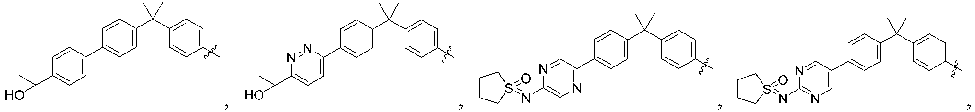

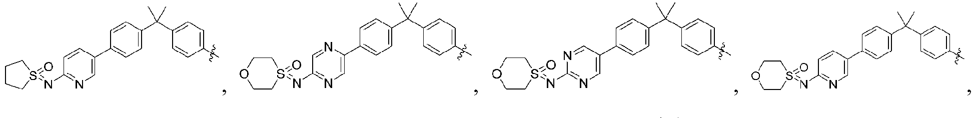

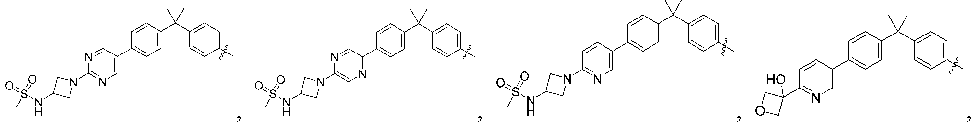

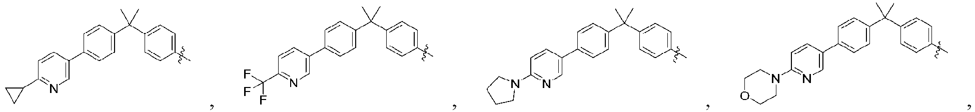

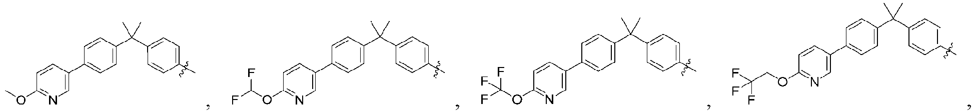

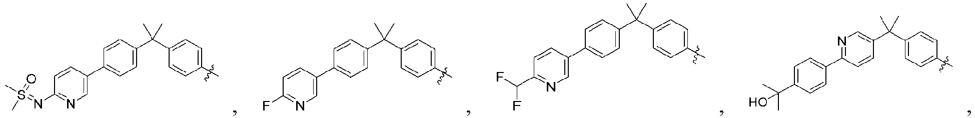

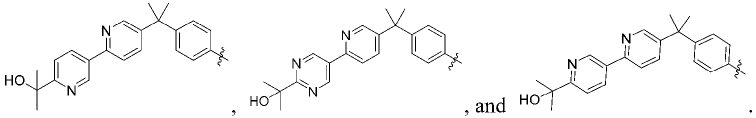

In some embodiments, the structural moiety 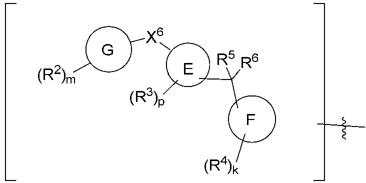 (or PTM) is selected from the group consisting of

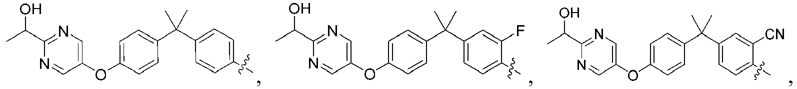

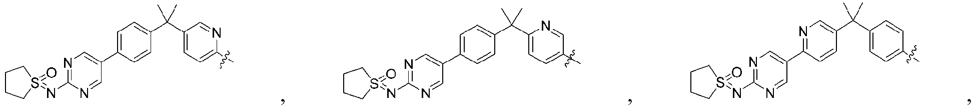

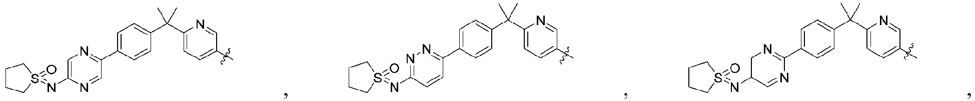

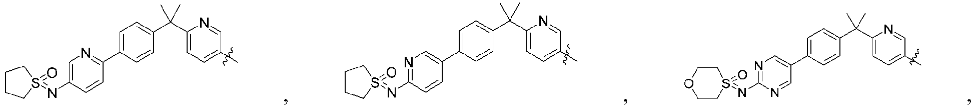

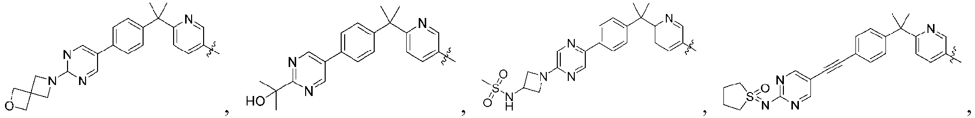

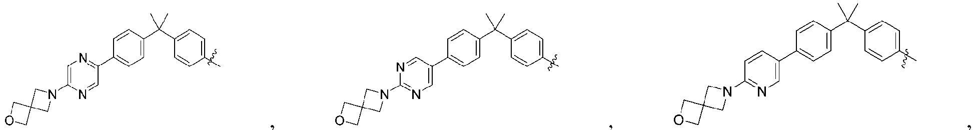

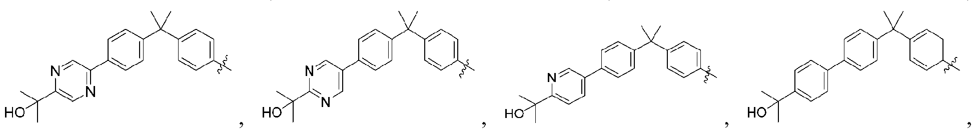

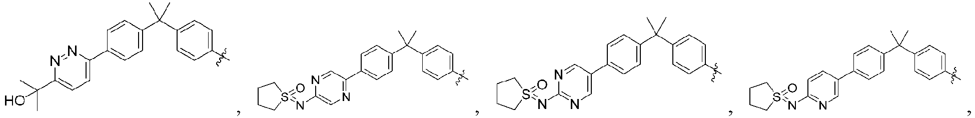

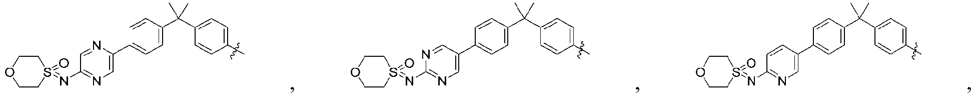

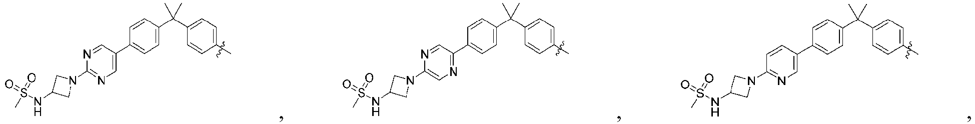

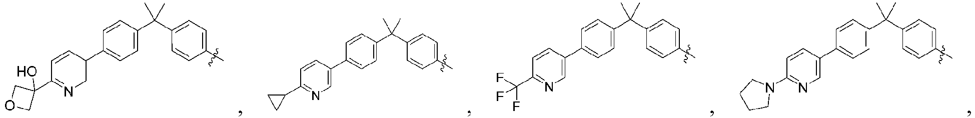

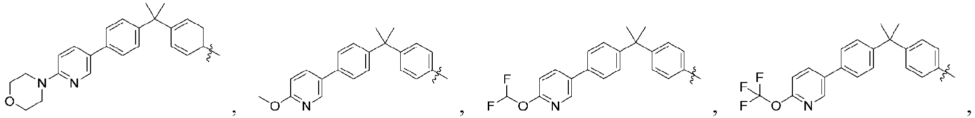

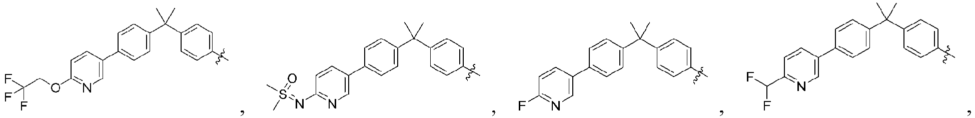

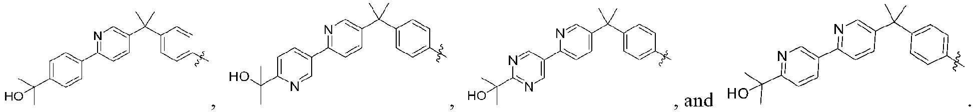

In some embodiments, the compound of formula II, the stereoisomer thereof, or the pharmaceutically acceptable salt thereof is selected from a compound of formula IIA, a stereoisomer thereof, or a pharmaceutically acceptable salt thereof: 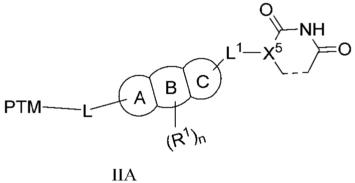 wherein ring A, ring B, ring C, R¹, n, X⁵, L¹, L, and PTM are as defined herein.

In some embodiments, in the compound of formula IIA,
X³ is C(R^{f}), and R^{f} is selected from the group consisting of H, halogen, deuterium, and C₁₋₃ alkyl; preferably, X⁵ is C(R^{f}), and R^{f} is H or deuterium; more preferably, X⁵ is CH;
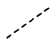 represents a single bond;
L¹ is a bond;
ring A is selected from 5- to 6-membered heterocycloalkenyl containing 1-2 N atoms or O atoms (e.g., dihydropyrrolyl or tetrahydropyridinyl), ring B is selected from the group consisting of phenyl and pyridinyl (e.g., phenyl), and ring C is 5-membered heteroaryl containing 1-2 heteroatoms selected from the group consisting of N, O, and S (e.g., isoxazolyl, pyrazolyl, or furanyl); preferably, 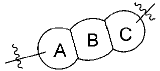 is selected from the group consisting of 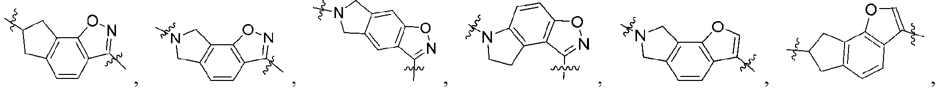 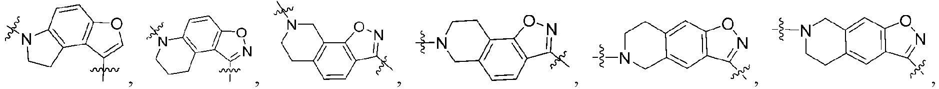 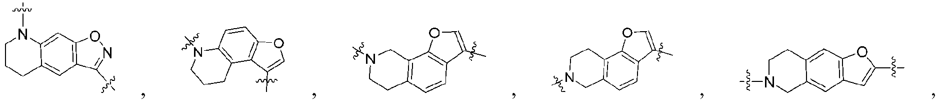 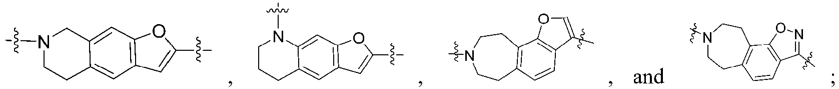 more preferably, 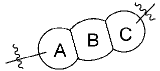 is selected from the group consisting of 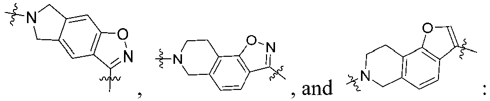
R¹ is selected from the group consisting of deuterium, halogen, -OH, and -NH₂ (preferably, R¹ is selected from deuterium); n is 0 or 1 (preferably, n is 0); preferably, 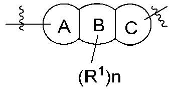 is selected from the group consisting of 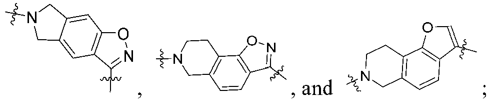
L is selected from the group consisting of 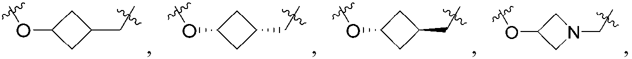 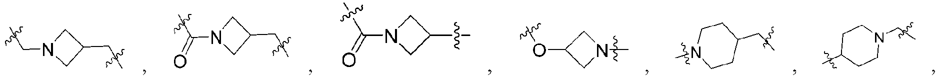 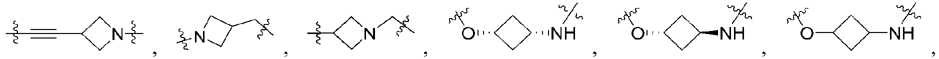 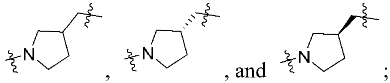 preferably, L is selected from the group consisting of 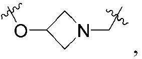 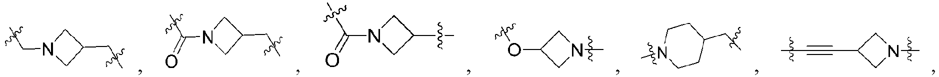 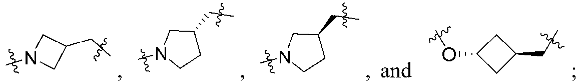 more preferably, L is selected from the group consisting of 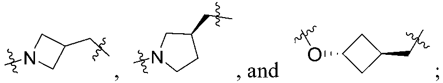
PTM is 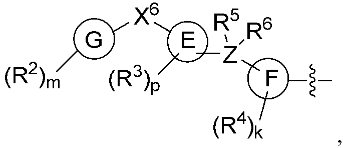 preferably 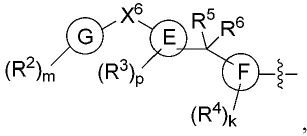 and more preferably 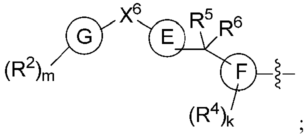 preferably,
R² is selected from the group consisting of halogen, -CHF₂, -CF₃, methyl, CH₃O-, CHF₂O-, CF₃O-, CF₃CH₂O-, CH₃CH(OH)-, (CH₃)₂C(OH)-, CH₃CH₂CH(OH)-, cyclopropyl, 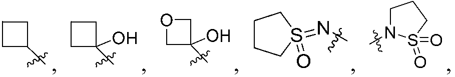 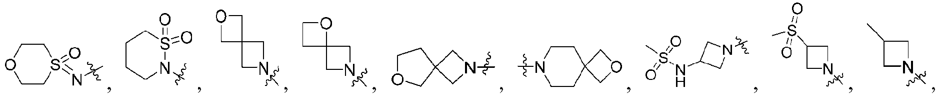 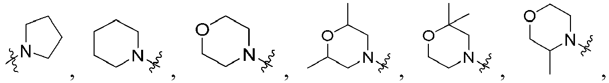 CH₃S(O)₂NH-, and cyclopropyl-S(O)₂NH-; preferably, R² is selected from the group consisting of -CHF₂, -CF₃, CH₃O-, CHF₂O-, CF₃O-, CF₃CH₂O-, CH₃CH(OH)-, (CH₃)₂C(OH)-, 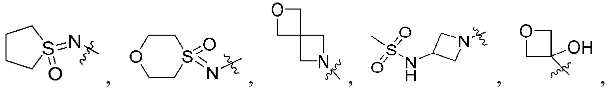 cyclopropyl, 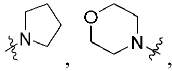 (CH₃)₂S(O)=N-, -F, and -Cl; m is 1;
ring G is selected from the group consisting of phenyl and 5- to 6-membered heteroaryl containing 1-3 N atoms; preferably, ring G is selected from the group consisting of 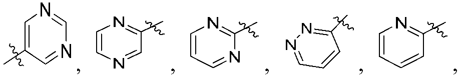 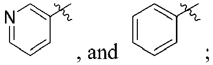
X⁶ is selected from the group consisting of a bond, -O-, -S-, -C₂₋₃ alkenylene-, and -C₂₋₃ alkynylene- (for example,
X⁶ is selected from the group consisting of a bond, -O-, -S-, and -ethynylene-); preferably, X⁶ is selected from the group consisting of -O-, a bond (e.g., a single bond), and -ethynylene-;
ring E is selected from the group consisting of phenyl and 6-membered heteroaryl containing 1-2 N atoms; preferably,
ring E is selected from the group consisting of 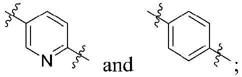
p is 0;
Z is selected from -C-;
R³ and R⁶ are each independently selected from the group consisting of C₁₋₃ alkyl and C₂₋₃ alkenyl (e.g., methyl, ethyl, or propyl); preferably, R⁵ and R⁶ are each independently C₁₋₃ alkyl; more preferably, R⁵ and R⁶ are each independently methyl;
ring F is selected from the group consisting of phenyl and 6-membered heteroaryl containing 1-2 N atoms; preferably,
ring F is selected from the group consisting of 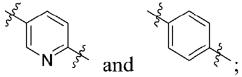
R⁴ is selected from the group consisting of halogen and -CN; preferably, R⁴ is selected from the group consisting of -F and -CN;
k is 0 or 1 (preferably, 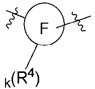 is selected from the group consisting of

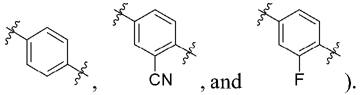

In some embodiments, PTM is selected from the group consisting of

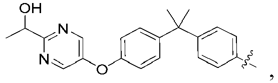

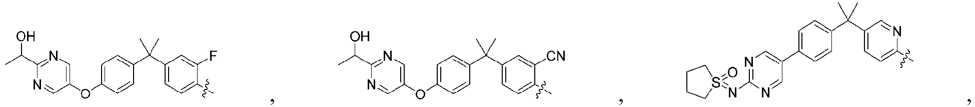

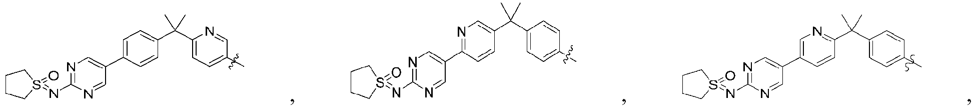

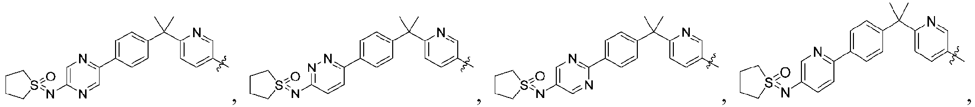

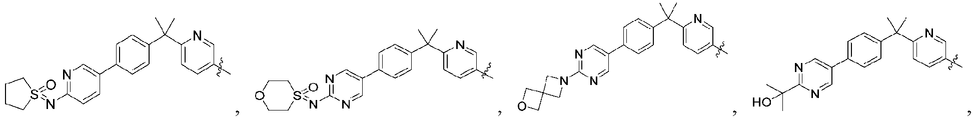

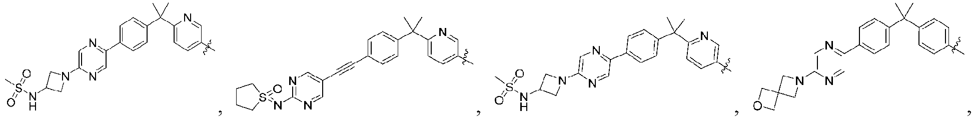

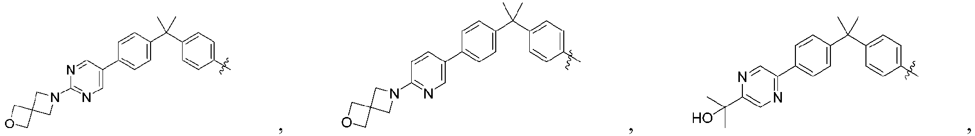

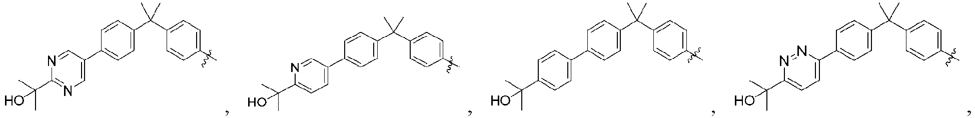

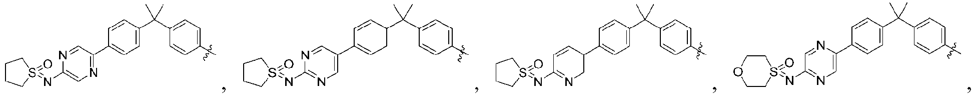

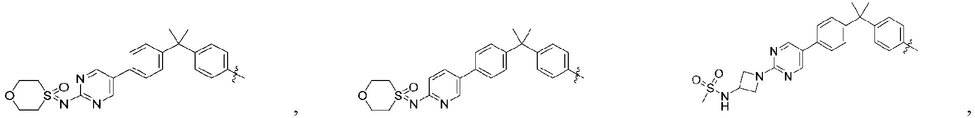

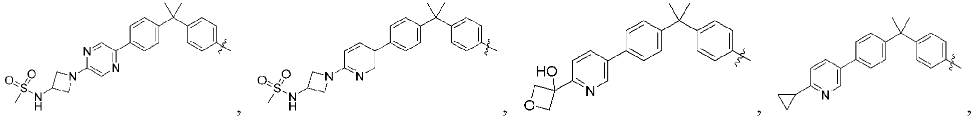

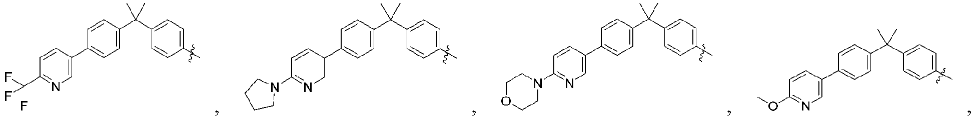

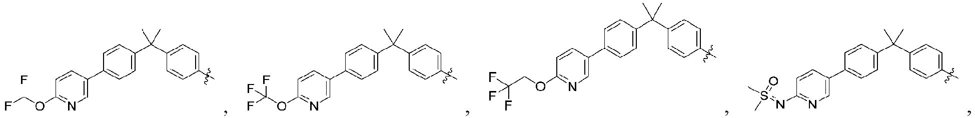

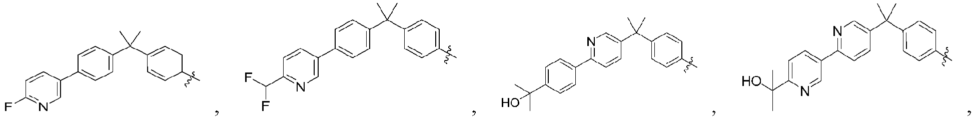

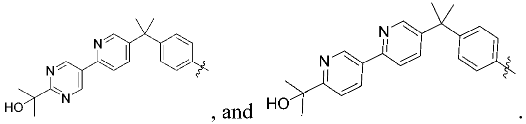

In some embodiments, the compound of formula III, the stereoisomer thereof, or the pharmaceutically acceptable salt thereof is selected from a compound of formula IIIA, a stereoisomer thereof, or a pharmaceutically acceptable salt thereof: 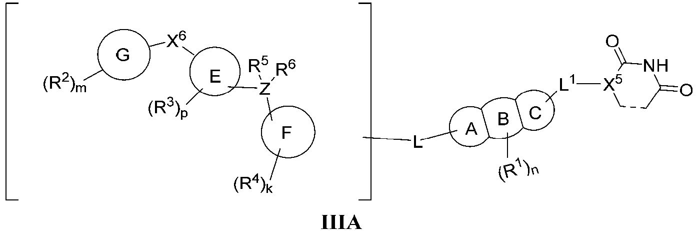 wherein ring A, ring B, ring C, R¹, n, X⁵, L¹, L, X⁶, Z, R², R³, R⁴, m, p, k, ring G, ring E, ring F, R⁵, and R⁶ are as defined herein.

In some embodiments, the compound of formula I, formula II, or formula III, the stereoisomer thereof, or the pharmaceutically acceptable salt thereof is selected from a compound of formula IIIB, a stereoisomer thereof, or a pharmaceutically acceptable salt thereof: 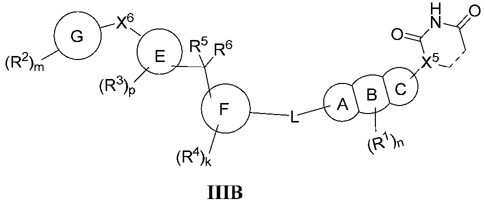 wherein ring A, ring B, ring C, R¹, n, X⁵, L, X⁶, R², R³, R⁴, m, p, k, ring G, ring E, ring F, R⁵, and R⁶ are as defined herein.

In some embodiments, in the compound of formula IIIB, 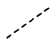 represents a single bond; X⁵ is C(R^{f}), R^{f} is H or deuterium, and preferably, X⁵ is CH; 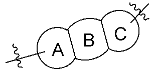 is selected from the group consisting of 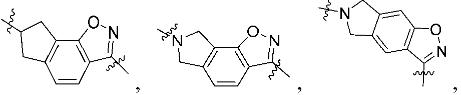 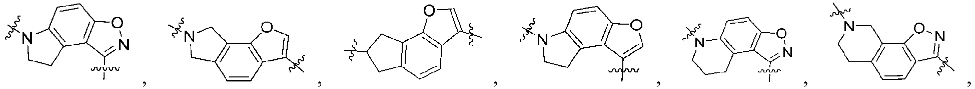 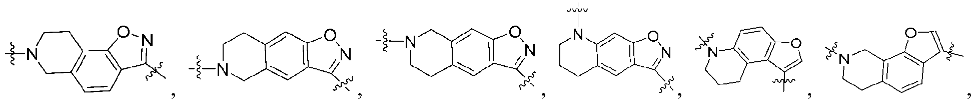 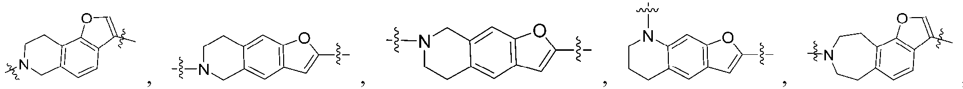 and 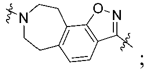 more preferably, 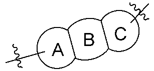 is selected from the group consisting of 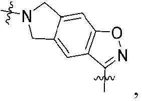 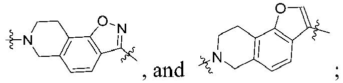
n is 0;
L is selected from the group consisting of 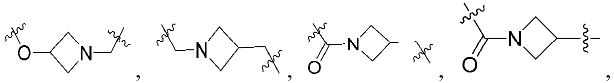 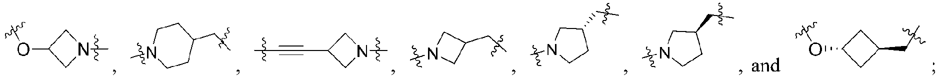 preferably, L is selected from the group consisting of 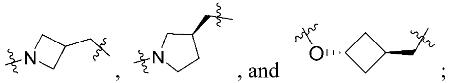
R² is selected from the group consisting of -CHF₂, -CF₃, CH₃O-, CHF₂O-, CF₃O-, CF₃CH₂O-, CH₃CH(OH)-, (CH₃)₂C(OH)-, 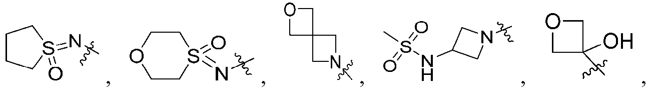 cyclopropyl, 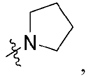 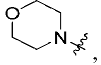 (CH₃)₂S(O)=N-, -F, and -Cl;
m is 1;
ring G is selected from the group consisting of phenyl and 5- to 6-membered heteroaryl containing 1-3 N atoms; preferably, ring G is selected from the group consisting of 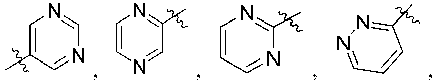 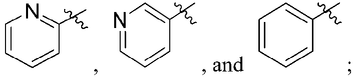
X⁶ is selected from the group consisting of a bond, -O-, -S-, -C₂₋₃ alkenylene-, and -C₂₋₃ alkynylene- (for example,
X⁶ is selected from the group consisting of a bond, -O-, -S-, and -ethynylene-); preferably, X⁶ is selected from the group consisting of -O-, a bond (e.g., a single bond), and -ethynylene-;
ring E is selected from the group consisting of phenyl and 6-membered heteroaryl containing 1-2 N atoms; preferably,
ring E is selected from the group consisting of 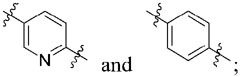
p is 0;
R⁵ and R⁶ are each independently C₁₋₃ alkyl; preferably, R⁵ and R⁶ are each independently methyl; preferably, ring F is selected from the group consisting of 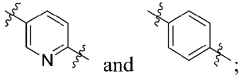
R⁴ is selected from the group consisting of halogen and -CN; preferably, R⁴ is selected from the group consisting of -F and -CN;
k is 0 or 1.

In some embodiments, the compound of formula I, formula II, formula III, or formula IIIA, the stereoisomer thereof, or the pharmaceutically acceptable salt thereof is selected from the group consisting of compounds of formula IV, formula V, formula VI, formula VII, formula VIII-1, formula VIII-2, formula VIII-3, and formula VIII-4, stereoisomers thereof, or pharmaceutically acceptable salts thereof: 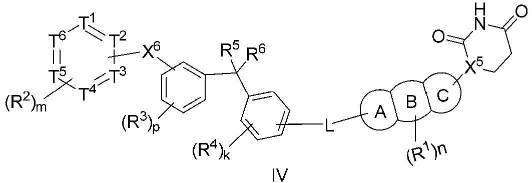 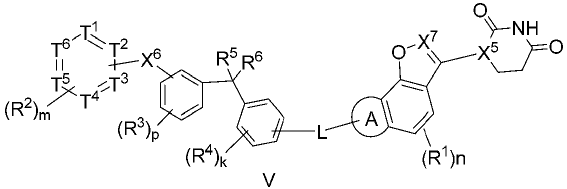 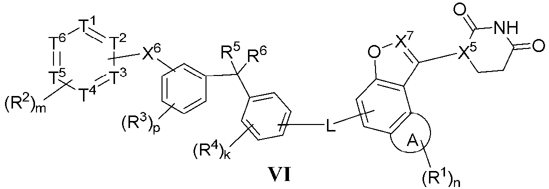 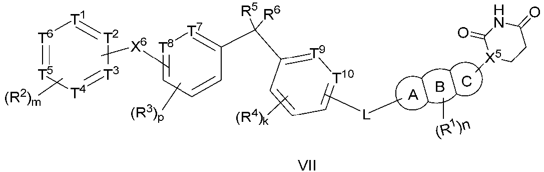 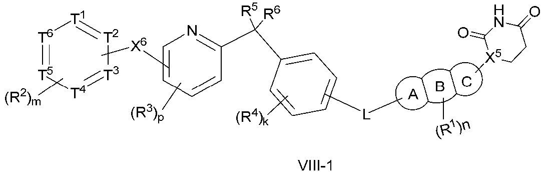 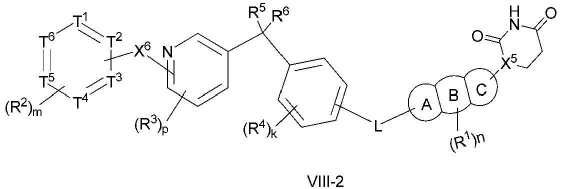 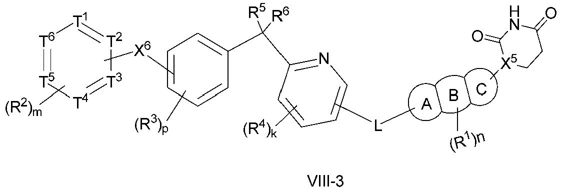 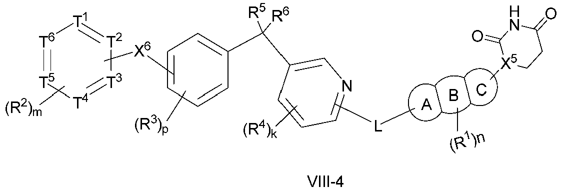
wherein ring A, ring B, ring C, R¹, n, X⁵, L, X⁶, R², R³, R⁴, m, p, k, ring G, ring E, ring F, R⁵, and R⁶ are as defined herein;
wherein T¹, T², T³, T⁴, T⁵, and T⁶ are each independently selected from the group consisting of CH and N;
X⁷ is selected from the group consisting of CH and N;
T⁷, T⁸, T⁹, and T¹⁰ are each independently selected from the group consisting of CH and N.

In some embodiments, two of T¹, T², T³, T⁴, T⁵, and T⁶ are selected from N, and the others are selected from CH.

In some embodiments, T⁷ and T⁸ are each independently selected from the group consisting of CH and N, one of which is selected from CH, and the other is selected from the group consisting of CH and N.

In some embodiments, T⁷ and T⁸ are selected from the group consisting of CH and N, one of which is selected from CH, and the other is selected from N.

In some embodiments, T⁷ and T⁸ are selected from CH.

In some embodiments, T⁹ and T¹⁰ are each independently selected from the group consisting of CH and N, one of which is selected from CH, and the other is selected from the group consisting of CH and N.

In some embodiments, T⁹ and T¹⁰ are selected from the group consisting of CH and N, one of which is selected from CH, and the other is selected from N.

In some embodiments, T⁹ and T¹⁰ are selected from CH.

In some embodiments, 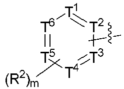 is selected from 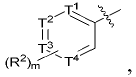 wherein T¹, T², T³, and T⁴ are each independently selected from the group consisting of CH and N. In some embodiments, 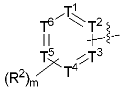 is selected from the group consisting of 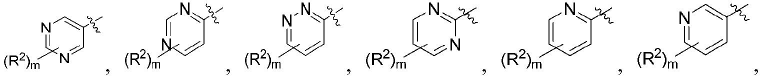 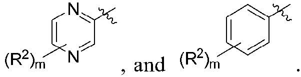 In some embodiments, 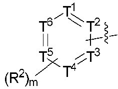 is selected from the group consisting of

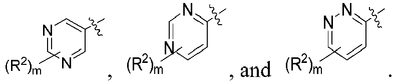

Others such as the structural moiety 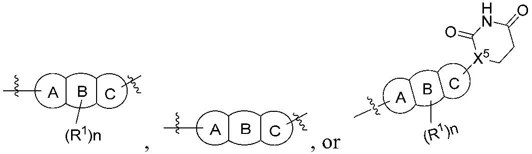 are as described herein.

In some embodiments, the heterocycloalkenyl, heteroaryl, heterocycloalkyl, or heteroalkylene described above comprises one or more heteroatoms or heteroatom groups independently selected from the group consisting of -O-, -NH-, -N-, -S-, -C(=O)-, -C(=O)NH-, -C(=O)O-, -S(=O)-, and -S(=O)₂-; in some embodiments, the heterocycloalkenyl, heteroaryl, heterocycloalkyl, or heteroalkylene described above comprises one or more heteroatoms or heteroatom groups independently selected from the group consisting of -O-, -NH-, -N-, and -S-; in some embodiments, the heterocycloalkenyl, heteroaryl, heterocycloalkyl, or heteroalkylene described above comprises one or more heteroatoms or heteroatom groups independently selected from the group consisting of -O-, -NH-, and -N-. In some embodiments, the number of the heteroatoms or heteroatom groups is independently selected from the group consisting of 1, 2, 3, 4, 5, and 6, or is selected from the group consisting of 1, 2, 3, and 4, or is selected from the group consisting of 1, 2, and 3, or is selected from the group consisting of 1 and 2.

In some embodiments, the heteroatom in the heterocycloalkenyl described above is selected from the group consisting of N, NH, O, and S. In some embodiments, the heteroatom in the heterocycloalkenyl described above is selected from the group consisting of N, O, and S. In some specific embodiments, the heteroatom in the heterocycloalkenyl described above is selected from the group consisting of N and O. In some embodiments, the number of the heteroatoms in the heterocycloalkenyl described above is selected from the group consisting of 1, 2, 3, 4, 5, and 6. In some embodiments, the number of the heteroatoms in the heterocycloalkenyl described above is selected from the group consisting of 1, 2, 3, and 4. In some embodiments, the number of the heteroatoms in the heterocycloalkenyl described above is selected from the group consisting of 1, 2, and 3. In some specific embodiments, the number of the heteroatoms in the heterocycloalkenyl described above is selected from the group consisting of 1 and 2.

In some embodiments, the halo is selected from the group consisting of fluoro, chloro, and bromo. In some embodiments, the halo is selected from the group consisting of fluoro and chloro. In some embodiments, the halo is selected from fluoro.

In some embodiments, the C₁₋₁₀ is selected from the group consisting of C₁₋₉, C₁₋₈, C₁₋₇, C₁₋₆, C₁₋₄, C₁₋₃, and C₁₋₂. In some embodiments, the C₁₋₆ is selected from the group consisting of C₁₋₄, C₁₋₃, and C₁₋₂. In some embodiments, the C₁₋₄ is selected from the group consisting of C₄, C₃, C₂, and C₁. In some embodiments, the C₁₋₃ is selected from the group consisting of C₃, C₂, and C₁.

In some embodiments, the C₂₋₁₀ is selected from the group consisting of C₂₋₈, C₂₋₆, C₂₋₅, C₂₋₄, and C₂₋₃. In some embodiments, the C₂₋₆ is selected from the group consisting of C₂₋₄ and C₂₋₃. In some embodiments, the C₂₋₄ is selected from the group consisting of C₄, C₃, and C₂.

In some embodiments, the C₃₋₆ is selected from the group consisting of C₃₋₅, C₃₋₄, C₄₋₆, C₄₋₅, and C₅₋₆. In some embodiments, the C₆₋₁₀ is selected from the group consisting of C₆₋₉, C₆₋₈, C₆₋₇, C₇₋₁₀, C₇₋₉, C₇₋₈, C₈₋₁₀, C₈₋₉, and C₉₋₁₀. In some embodiments, the C₃₋₁₀ is selected from the group consisting of C₃₋₉, C₃₋₈, C₃₋₇, C₃₋₆, C₃₋₅, C₃₋₄, C₄₋₁₀, C₄₋₉, C₄₋₈, C₄₋₇, C₄₋₆, C₄₋₅, C₅₋₁₀, C₅₋₉, C₅₋₈, C₅₋₇*,* C₅₋₆*,* C₆₋₁₀, C₆₋₉, C₆₋₈, C₆₋₇, C₇₋₁₂, C₇₋₁₀, C₇₋₉, C₇₋₈, C₈₋₁₂, C₈₋₁₀, C₈₋₉, C₉₋₁₂, and C₉₋₁₀. In some embodiments, the C₃₋₁₅ is selected from the group consisting of C₃₋₁₂ and C₃₋₁₀. In some embodiments, the C₃₋₁₂ is selected from C₃₋₁₀. In some embodiments, the C₆₋₁₂ is selected from C₆₋₁₀.

In some embodiments, the 3- to 6-membered is selected from the group consisting of 3- to 5-membered, 3- to 4-membered, 4- to 6-membered, 4- to 5-membered, and 5- to 6-membered. In some embodiments, the 5- to 10-membered is selected from the group consisting of 5- to 8-membered, 5- to 7-membered, 5- to 6-membered, 6- to 10-membered, 6- to 9-membered, 6- to 8-membered, 6- to 7-membered, 7- to 10-membered, 7- to 9-membered, 7-to 8-membered, 8- to 10-membered, 8- to 9-membered, and 9- to 10-membered. In some embodiments, the 3- to 10-membered is selected from the group consisting of 3- to 9-membered, 3- to 8-membered, 3- to 7-membered, 3- to 6-membered, 3- to 5-membered, 3- to 4-membered, 4- to 10-membered, 4- to 9-membered, 4- to 8-membered, 4- to 7-membered, 4- to 6-membered, 4- to 5-membered, 5- to 10-membered, 5- to 9-membered, 5- to 8-membered, 5- to 7-membered, 5- to 6-membered, 6- to 10-membered, 6- to 9-membered, 6- to 8-membered, 6- to 7-membered, 7- to 10-membered, 7- to 9-membered, 7- to 8-membered, 8- to 10-membered, 8- to 9-membered, and 9- to 10-membered. In some embodiments, the 3- to 15-membered is selected from the group consisting of 3- to 12-membered and 3- to 10-membered. In some embodiments, the 3- to 12-membered is selected from 3- to 10-membered. In some embodiments, the 5- to 12-membered is selected from 5- to 10-membered.

It should be understood that any embodiment of the compounds of the present application as described above, and any specific substituents set forth herein with respect to particular ring A, ring B, ring C, R¹, n, X⁵, L, X⁶, R², R³, R⁴, ring G, ring E, ring F, R⁵, and R⁶ substituents in the compounds of the present application as described above, may be independently combined with other embodiments of the present application and/or substituents of the compounds to form embodiments of the present disclosure not specifically set forth above. In addition, where substituent ranges are disclosed in the specific embodiments and/or claims with respect to any particular ring A, ring B, ring C, R¹, n, X⁵, L, X⁶, R², R³, R⁴, ring G, ring E, ring F, R⁵, and R⁶ substituents, it should be understood that one or more substituents may be deleted from the ranges, and the remaining substituent ranges should also be considered as the embodiments of the present application.

In some embodiments, the VHL E3 ubiquitin ligase-binding moiety is selected from the group consisting of:

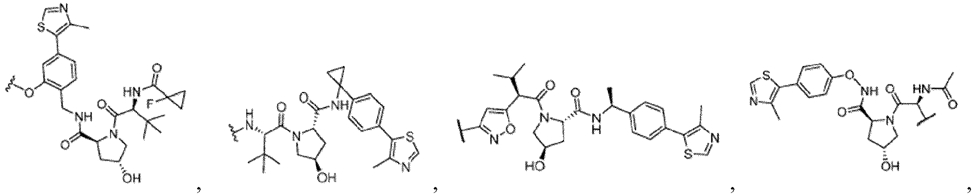

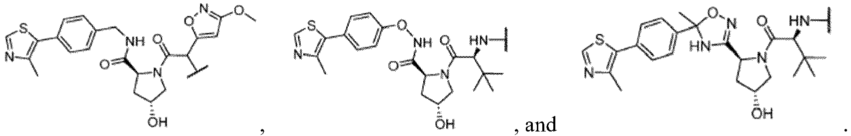

In some embodiments, the IAP E3 ubiquitin ligase-binding moiety is selected from the group consisting of:

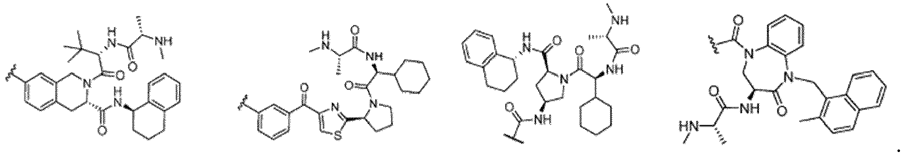

In some embodiments, the MDM2 E3 ubiquitin ligase-binding moiety is selected from the group consisting of:

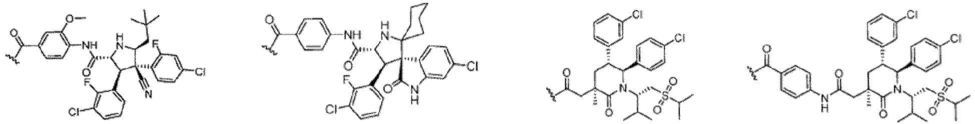

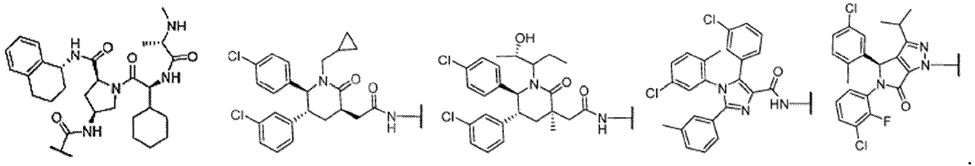

In some embodiments, CLM is selected from the group consisting of the following structural fragments: 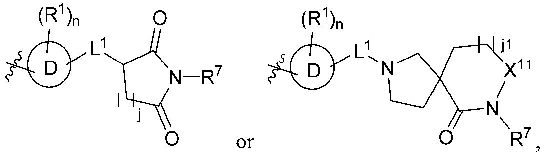
wherein j and j1 are each independently selected from the group consisting of 0, 1, and 2;
ring D is selected from a 5- to 20-membered ring (e.g., aryl, heteroaryl, cycloalkyl, partially unsaturated heterocyclyl, or heterocycloalkyl);
each R⁷ is independently selected from the group consisting of H and C₁₋₆ alkyl;
X¹¹ is selected from the group consisting of a bond, O, S, CH₂, and NH;
R¹, n, and L¹ are as defined herein.

In some embodiments, ring D is selected from a 5- to 15-membered ring; in some embodiments, ring D is selected from a 5- to 12-membered ring; in some embodiments, ring D is selected from a 5- to 10-membered ring; in some embodiments, ring D is selected from a 5- to 7-membered ring; in some embodiments, ring D is selected from the group consisting of a 5- to 15-membered ring, C₆₋₁₅ aryl, 5- to 15-membered heteroaryl, C₅₋₁₅ cycloalkyl, and 5- to 15-membered heterocycloalkyl; in some embodiments, ring D is selected from the group consisting of a 5- to 12-membered ring, C₆₋₁₂ aryl, 5- to 12-membered heteroaryl, C₅₋₁₂ cycloalkyl, and 5- to 12-membered heterocycloalkyl; in some embodiments, ring D is selected from the group consisting of a 5- to 10-membered ring, C₆₋₁₀ aryl, 5- to 10-membered heteroaryl, C₅₋₁₀ cycloalkyl, and 5- to 10-membered heterocycloalkyl; in some embodiments, ring D is selected from the group consisting of a 5- to 7-membered ring, C₆₋₁₀ aryl, 5- to 7-membered heteroaryl, C₅₋₇ cycloalkyl, and 5- to 7-membered heterocycloalkyl.

In some embodiments, each R⁷ is independently selected from the group consisting of H and C₁₋₃ alkyl; in some embodiments, each R⁷ is independently selected from H. The heteroatoms in the heteroaryl, heterocyclyl, or heterocycloalkyl are selected from the group consisting of nitrogen, oxygen, and sulfur, and the number of the heteroatoms is optionally 1, 2, 3, 4, 5, or 6.

In some embodiments, CLM is selected from the group consisting of the following structural fragments: 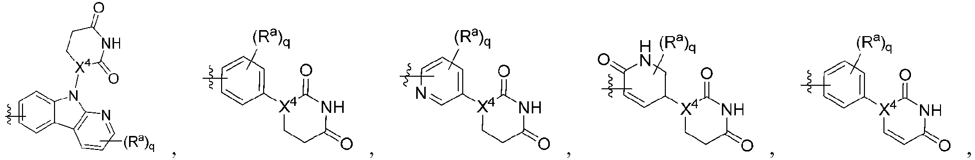 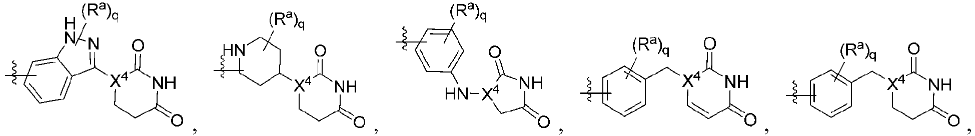 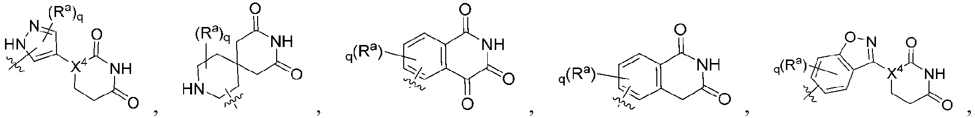 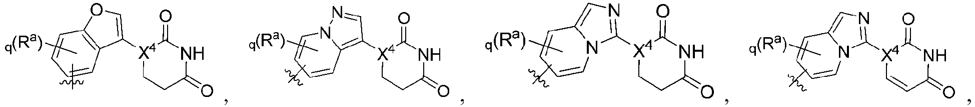 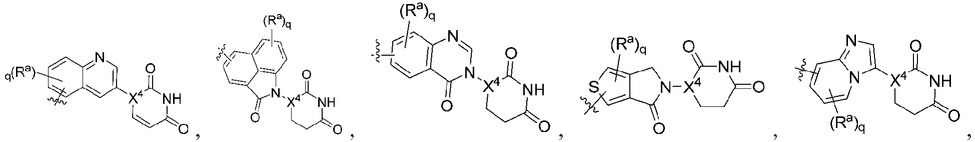 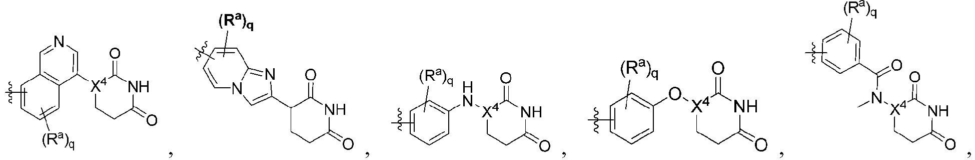 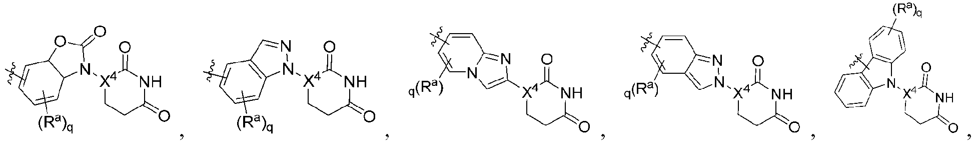 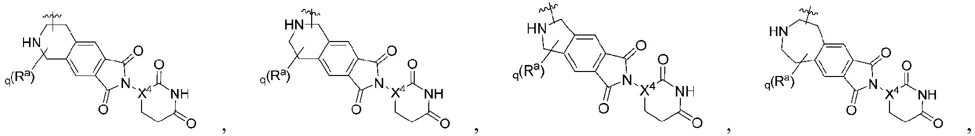 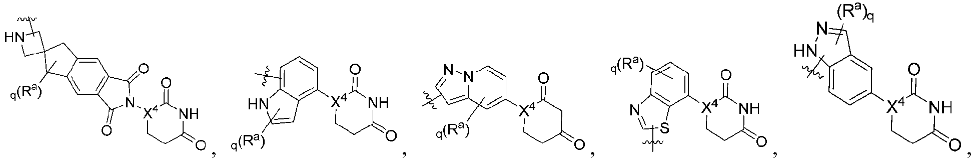 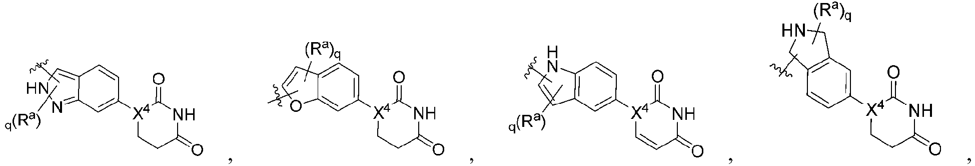 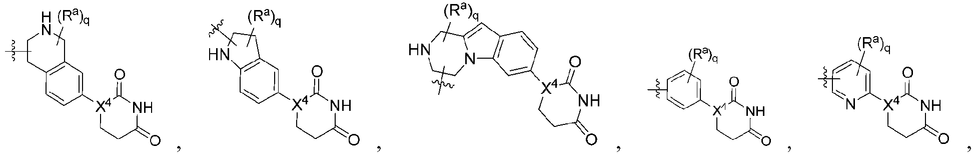 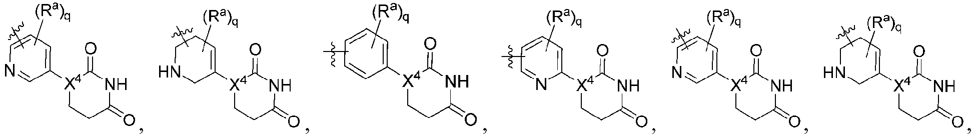 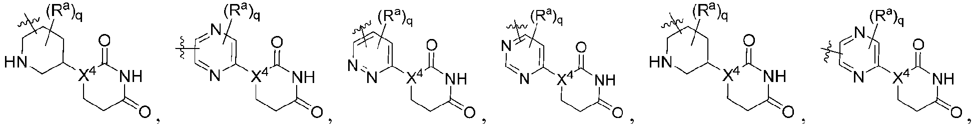 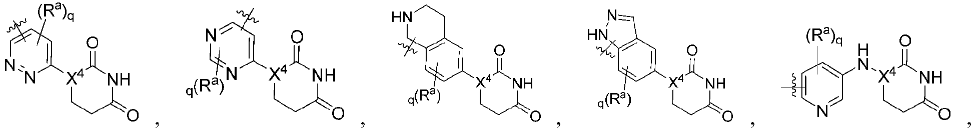 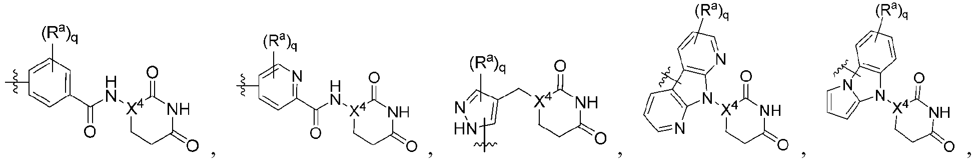 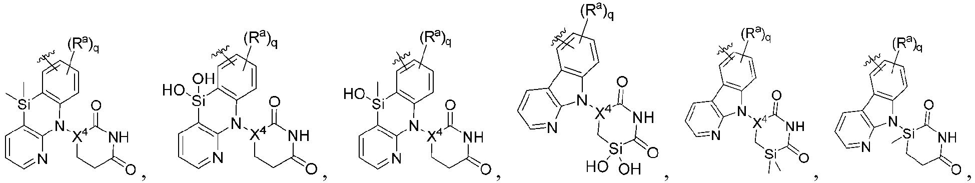 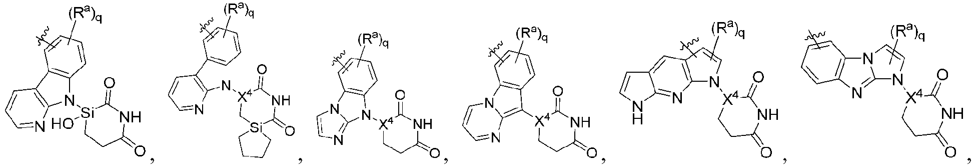 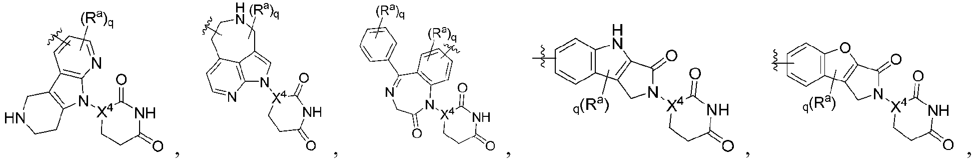 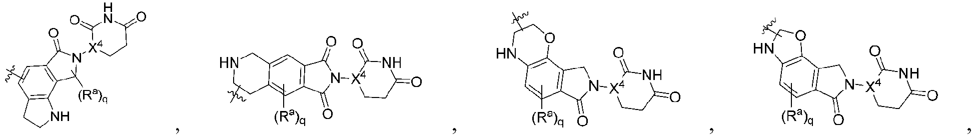 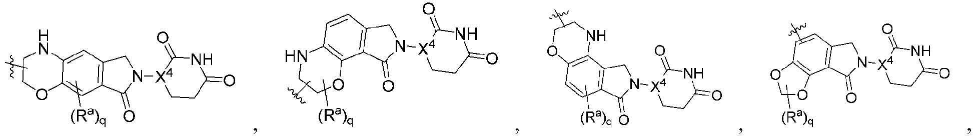 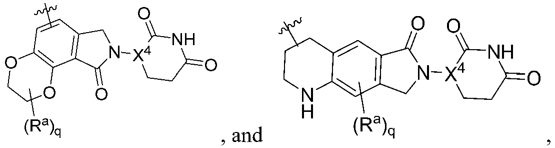
wherein each R^{a} is independently selected from the group consisting of hydroxyl, halogen, amino, cyano, and C₁₋₈ alkyl;
q is selected from the group consisting of 0, 1, 2, and 3;
X⁴ is selected from the group consisting of N and optionally substituted CH.

In some embodiments, each R^{a} is independently selected from the group consisting of hydroxyl, halogen, amino, cyano, and C₁₋₆ alkyl.

In some embodiments, each R^{a} is independently selected from the group consisting of hydroxyl, halogen, amino, cyano, and C₁₋₄ alkyl.

In some embodiments, each R^{a} is independently selected from the group consisting of hydroxyl, halogen, amino, cyano, and C₁₋₃ alkyl.

In some embodiments, each R^{a} is independently selected from the group consisting of hydroxyl, halogen, amino, and cyano.

In some embodiments, each R^{a} is independently selected from the group consisting of halogen and amino.

In some embodiments, q is selected from the group consisting of 0, 1, and 2. In some embodiments, q is selected from the group consisting of 0 and 1.

In some embodiments, X⁴ is selected from the group consisting of N and CH, wherein CH is optionally substituted with the following substituent: hydroxyl, halogen, amino, cyano, or C₁₋₄ alkyl.

In some embodiments, X⁴ is selected from N. In some embodiments, X⁴ is selected from CH.

In some embodiments, CLM is selected from the group consisting of the following structural fragments: 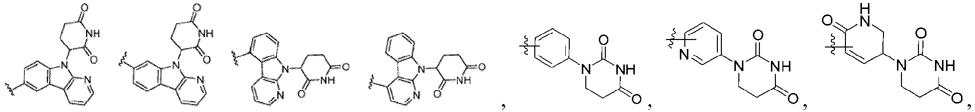 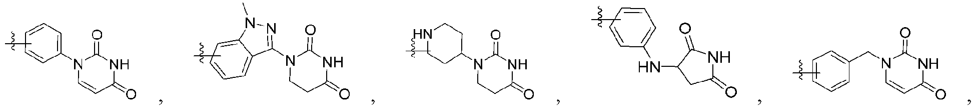 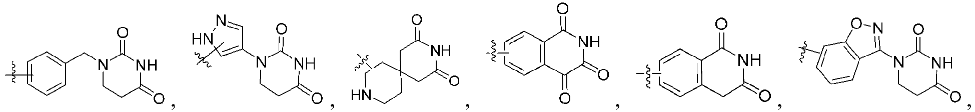 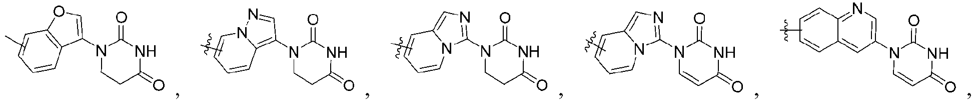 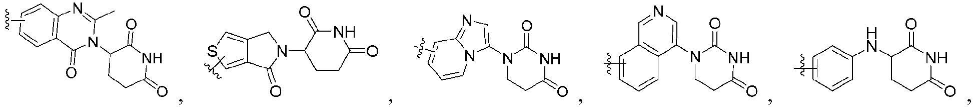 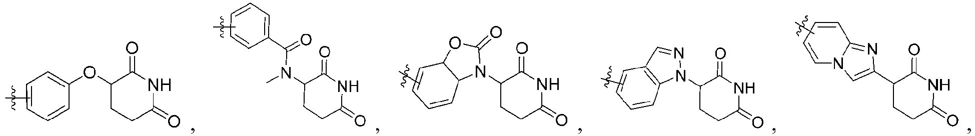 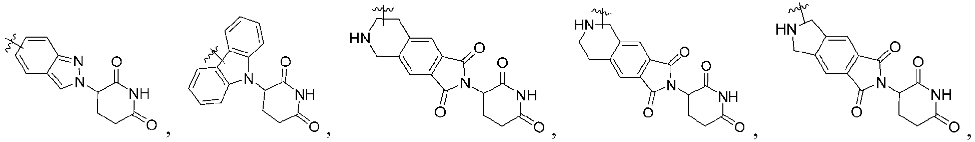 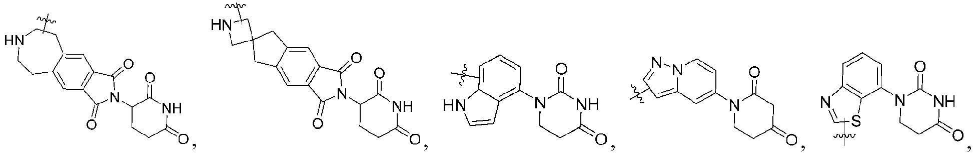 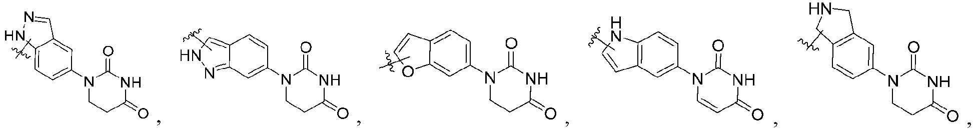 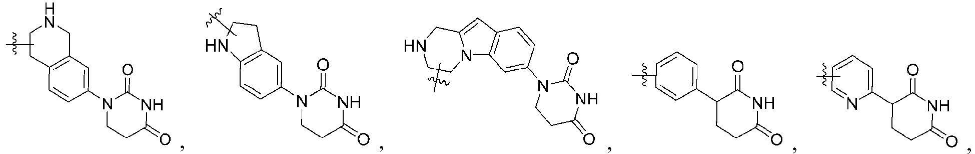 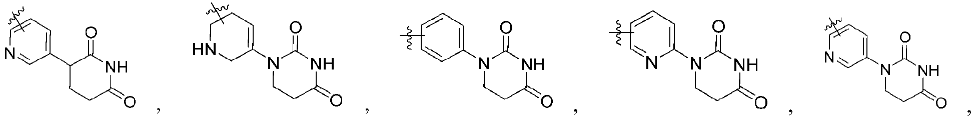 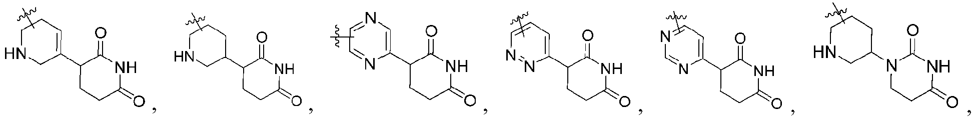 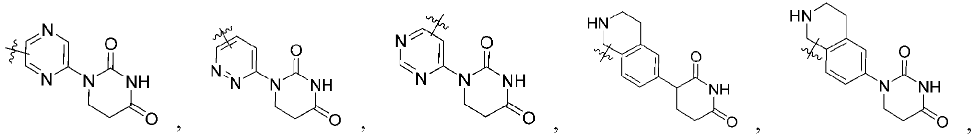 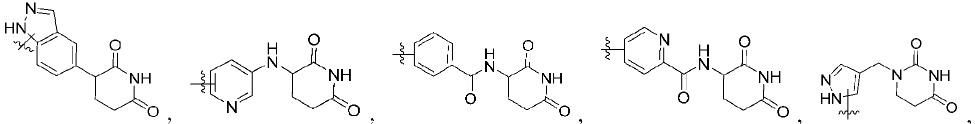 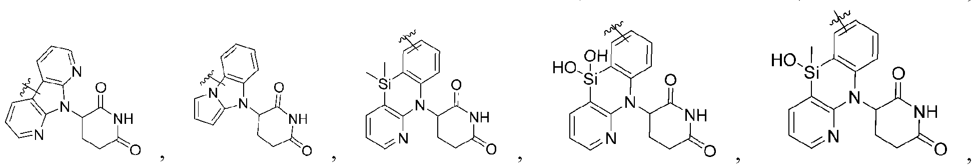 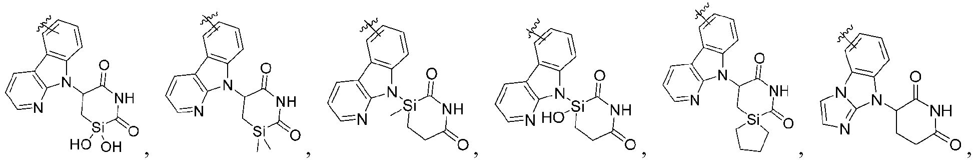 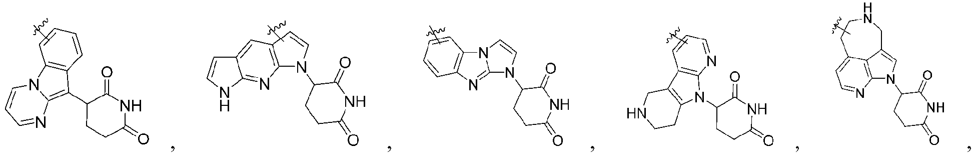 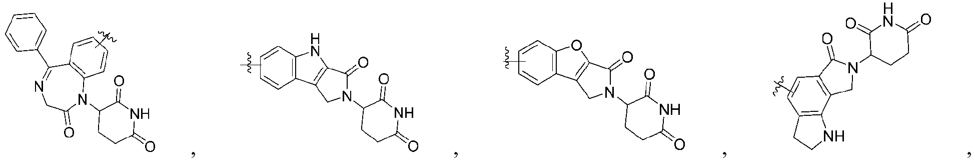 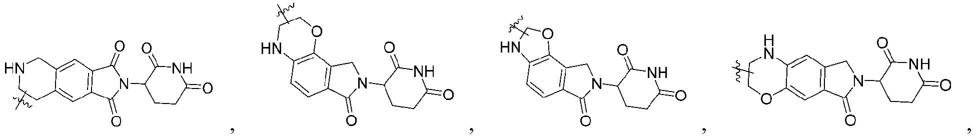 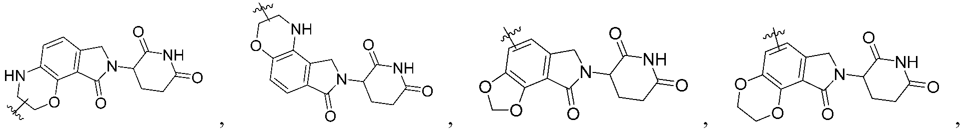 and

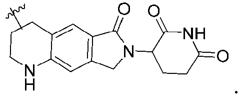

The present application also relates to a compound of a formula selected from the group consisting of the following formulas, a stereoisomer thereof, or a pharmaceutically acceptable salt thereof: 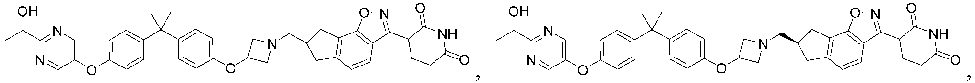 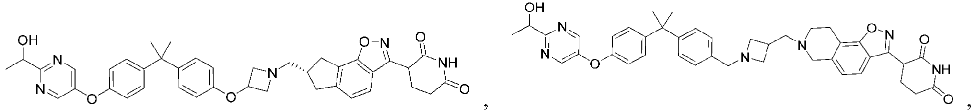 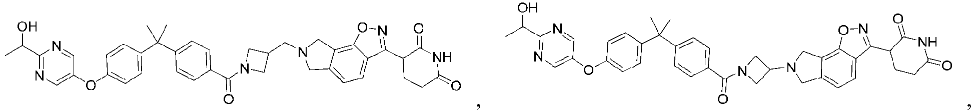 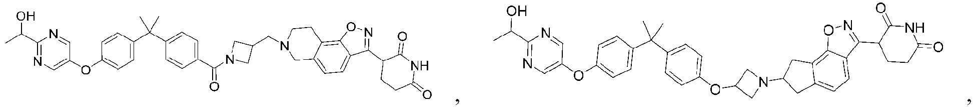 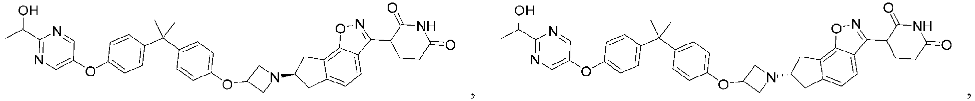 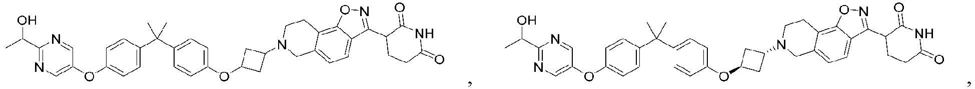 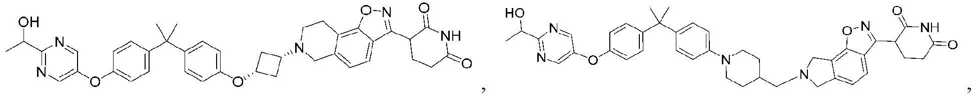 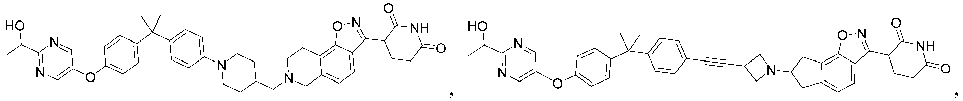 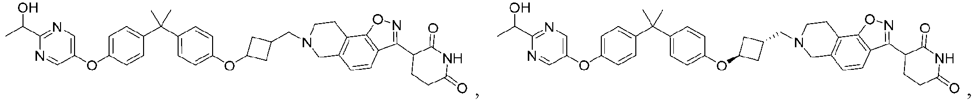 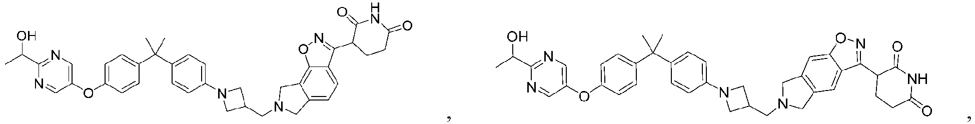 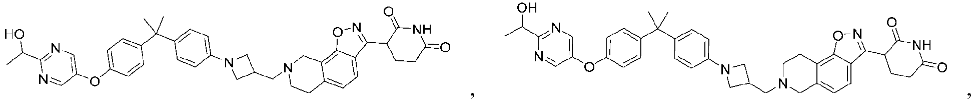 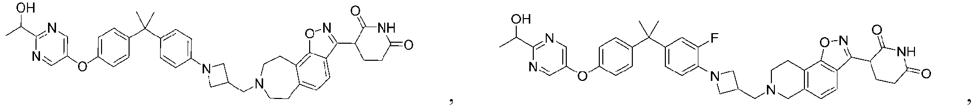 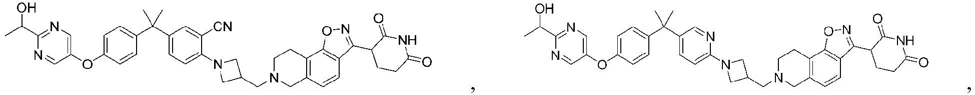 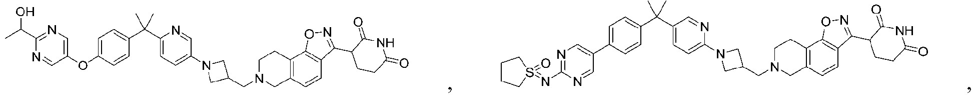 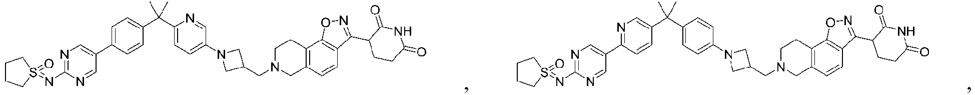 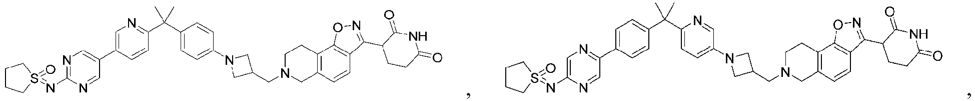 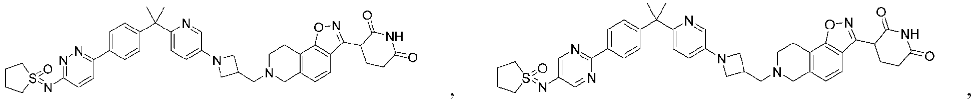 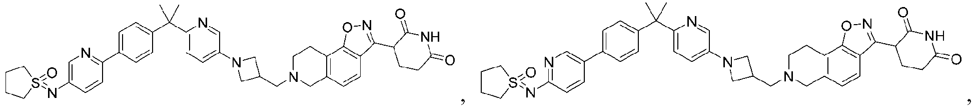 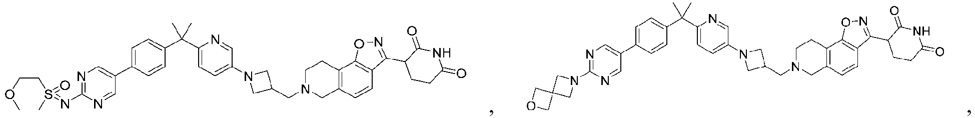 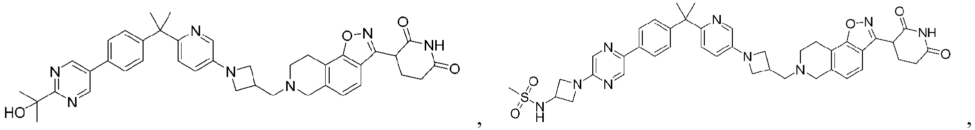 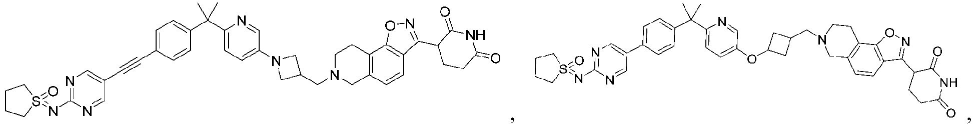 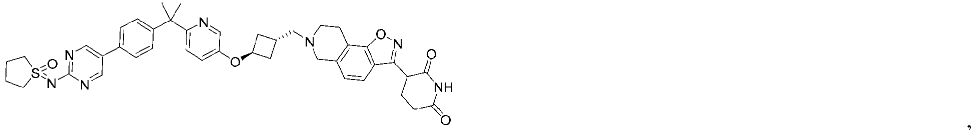 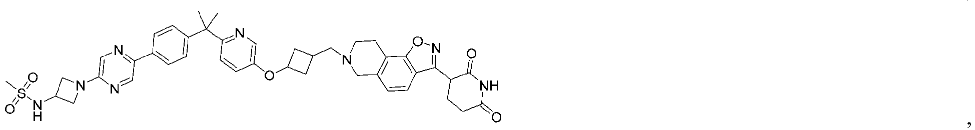 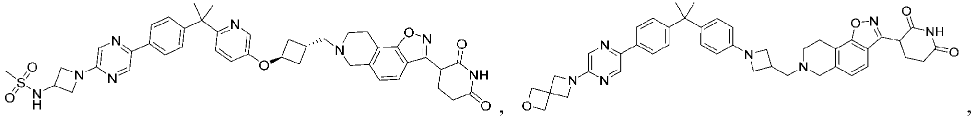 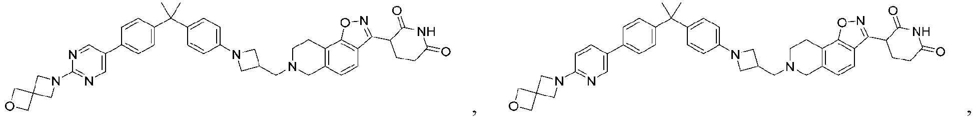 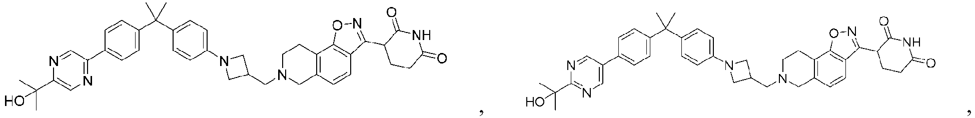 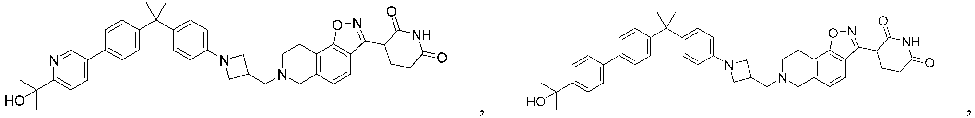 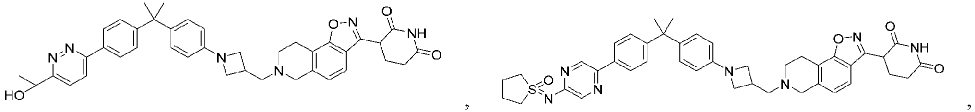 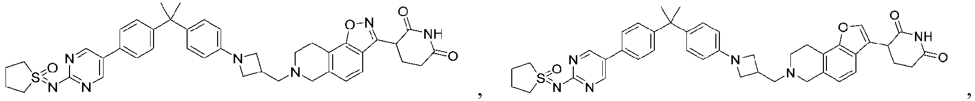 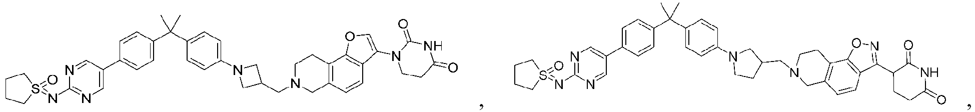 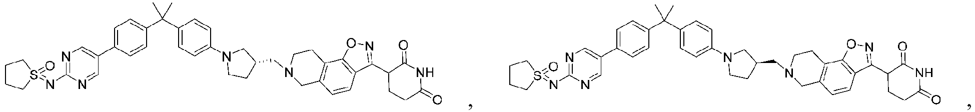 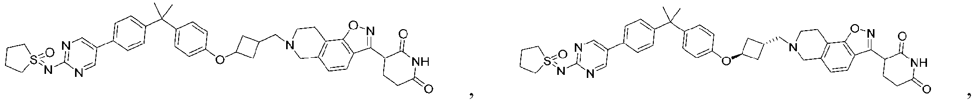 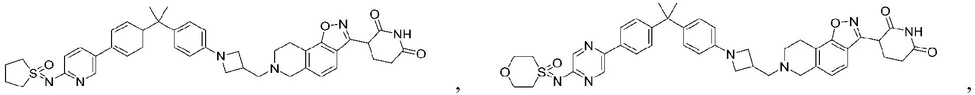 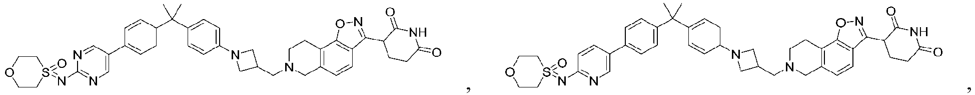 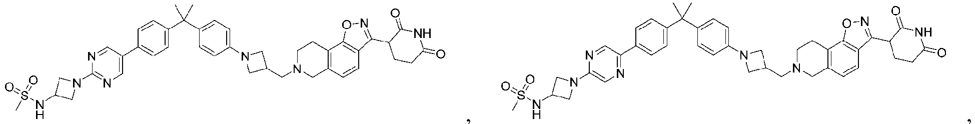 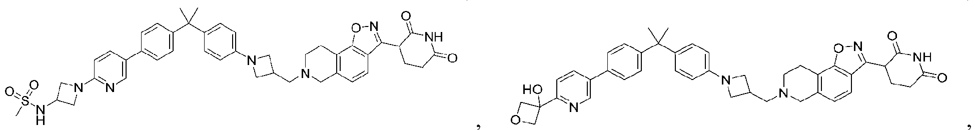 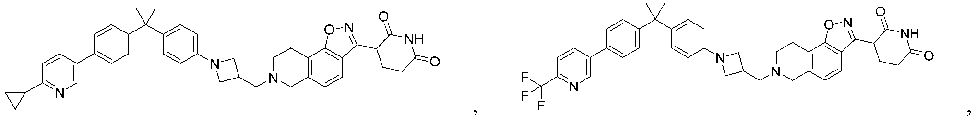 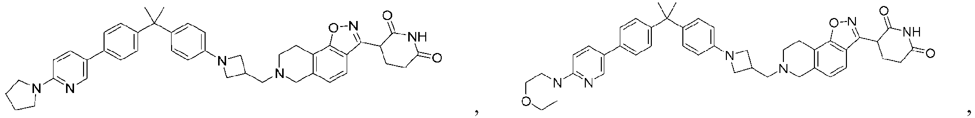 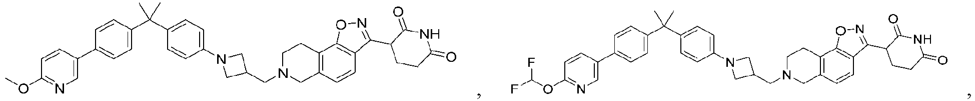 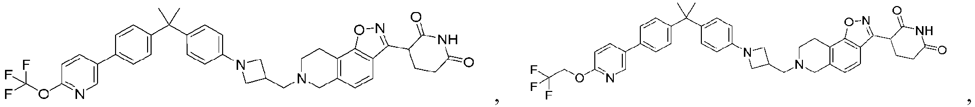 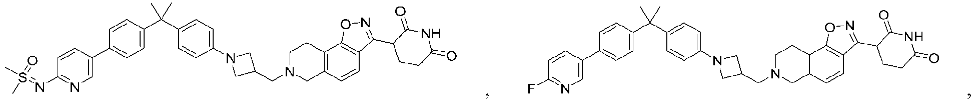 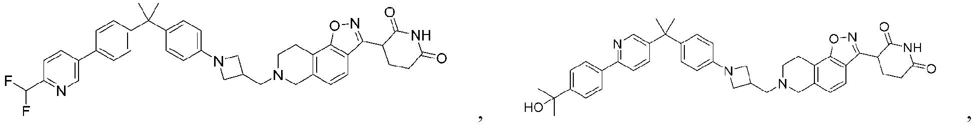 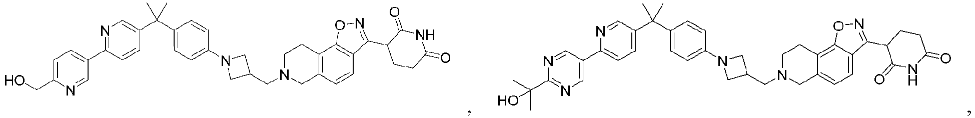 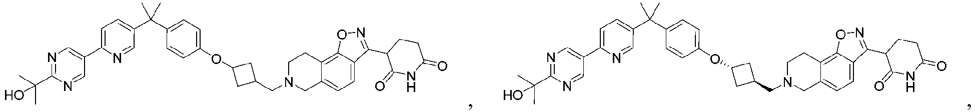 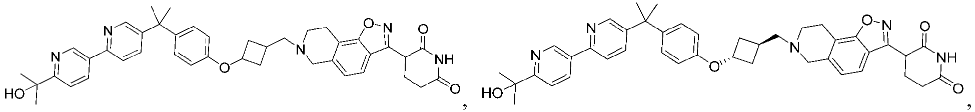 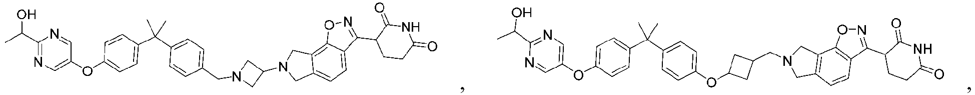 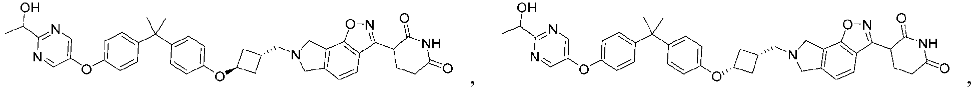 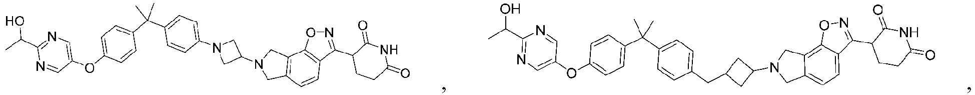 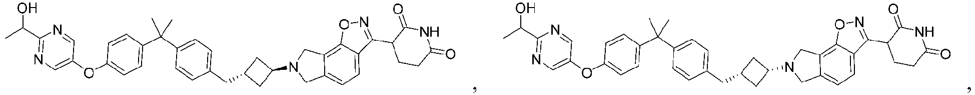 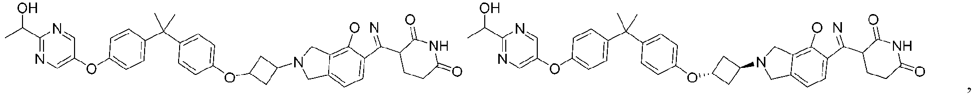 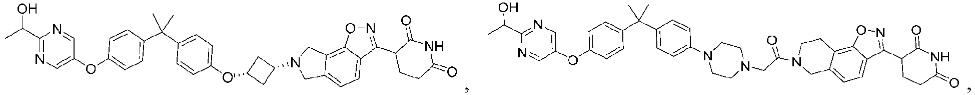 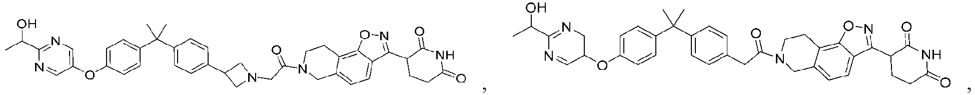 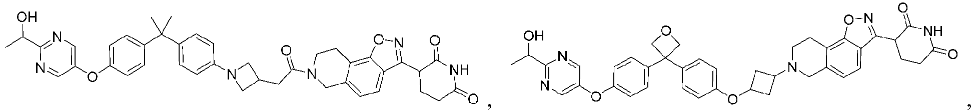 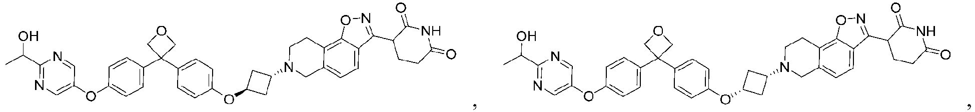 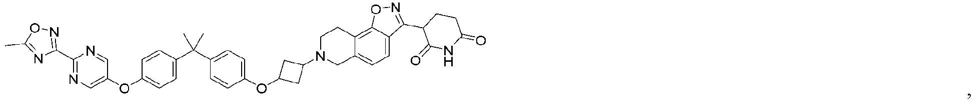 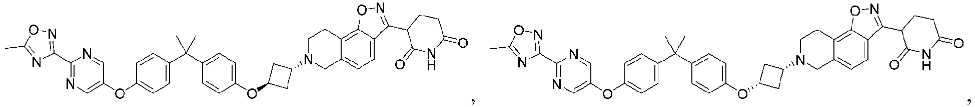 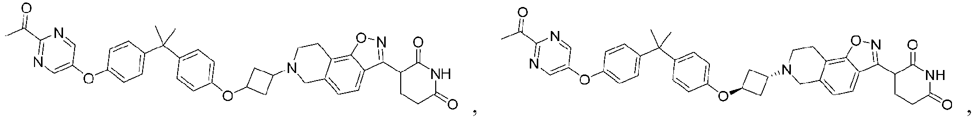 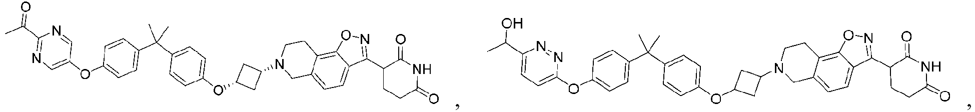 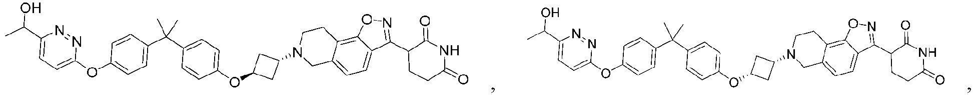 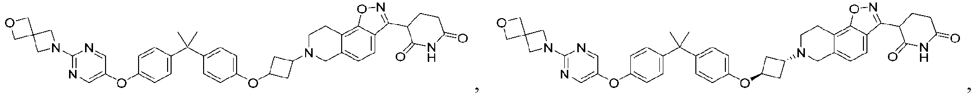 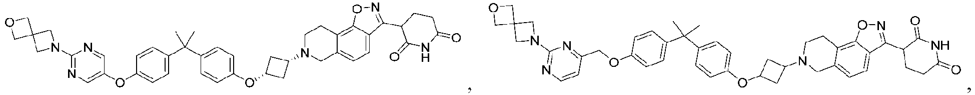 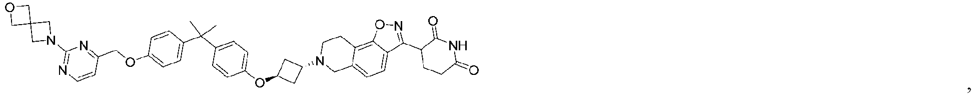 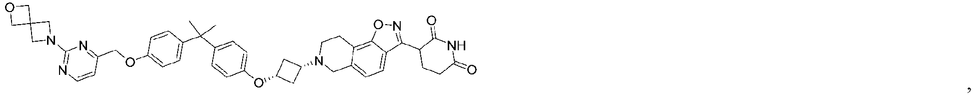 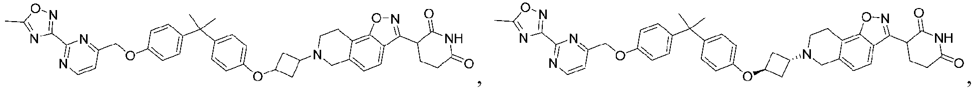 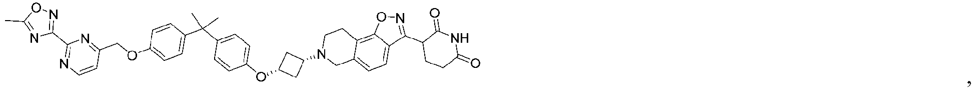 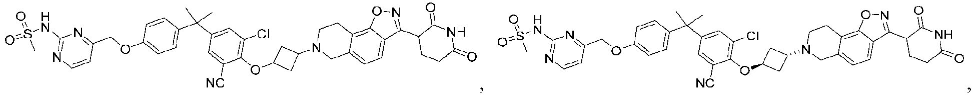 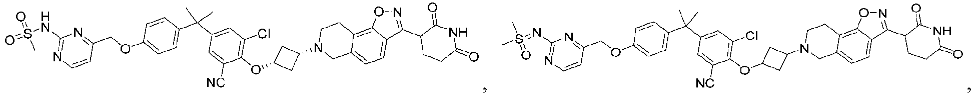 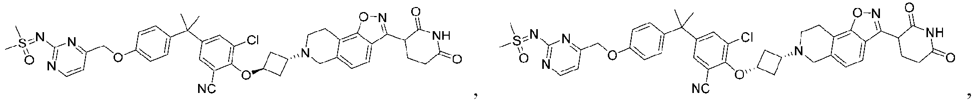 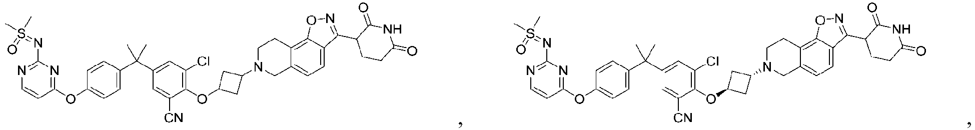 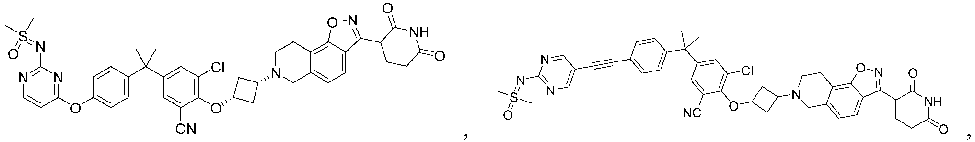 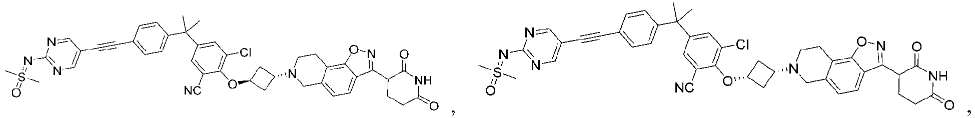 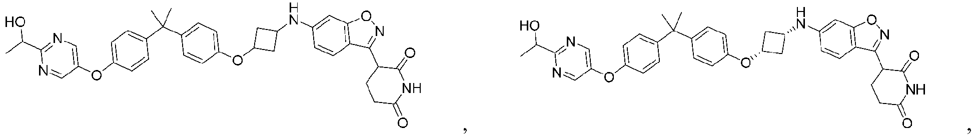 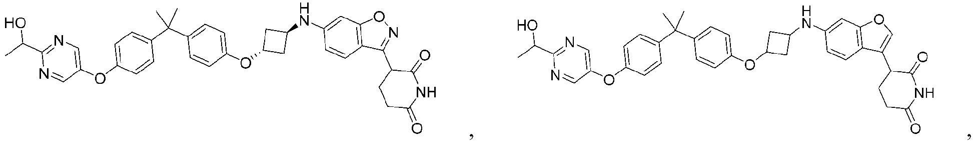 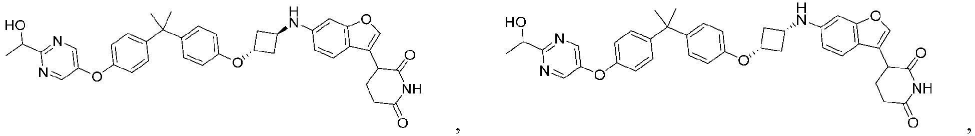 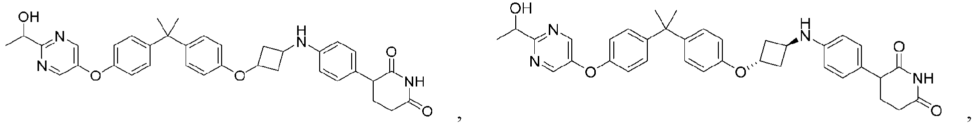 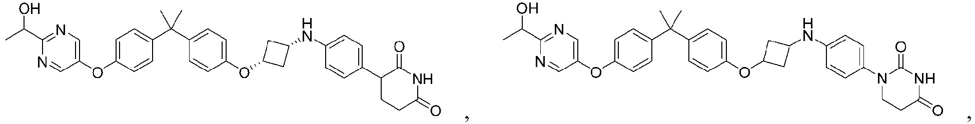 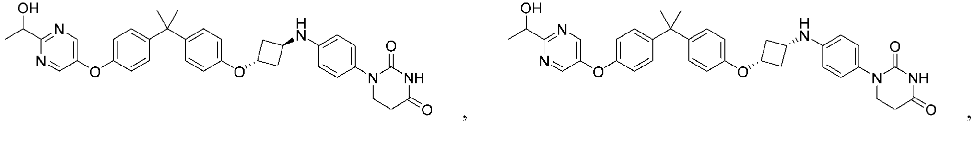 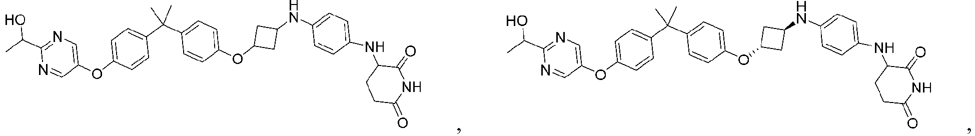 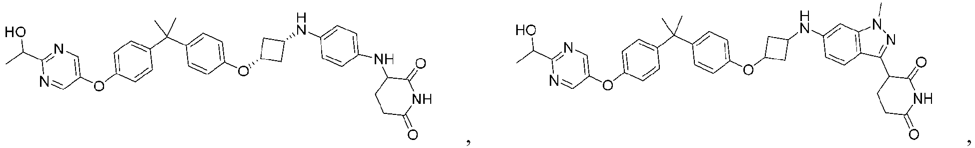 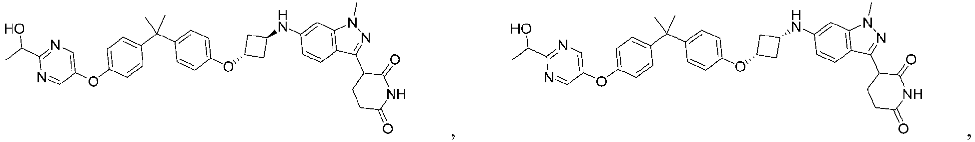 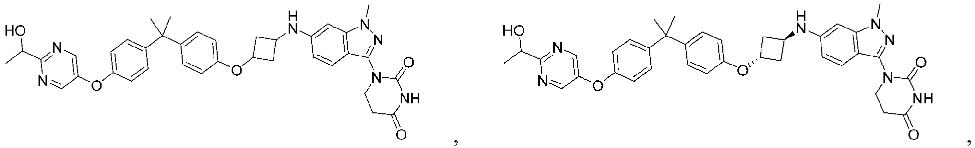 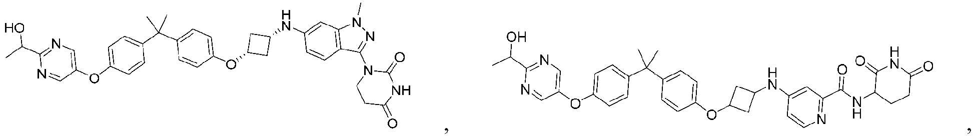 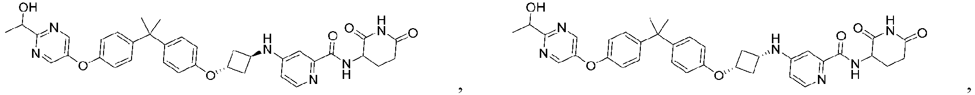 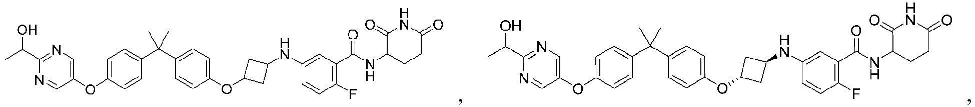 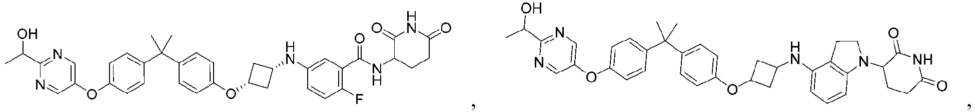 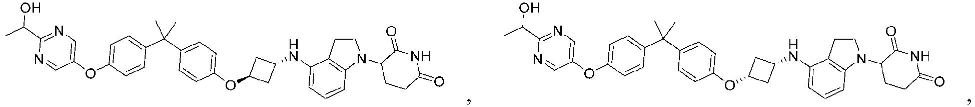 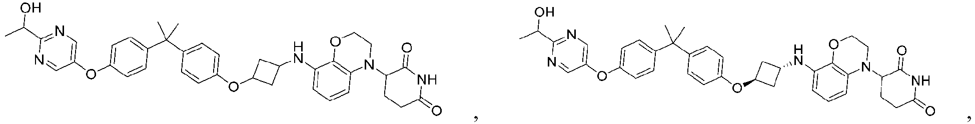 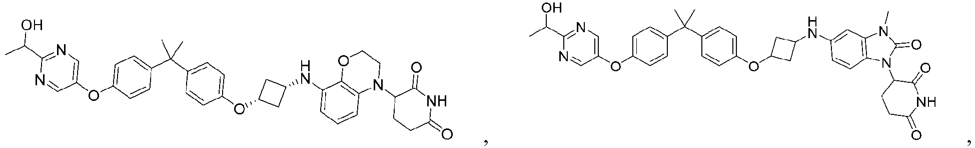 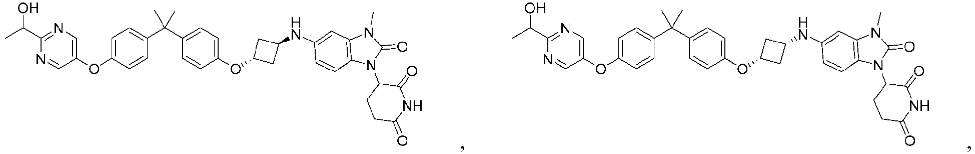 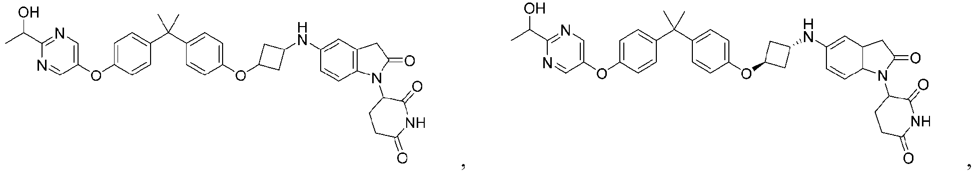 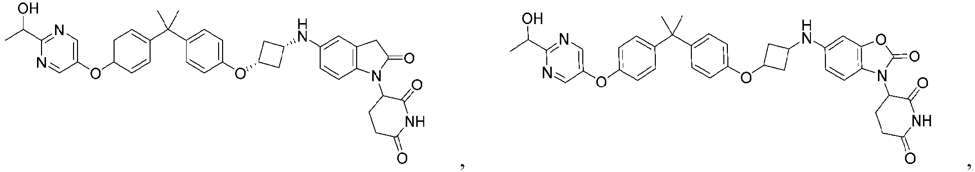 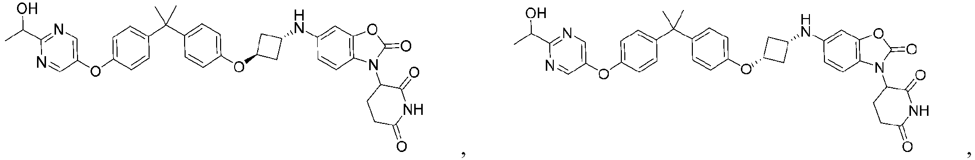 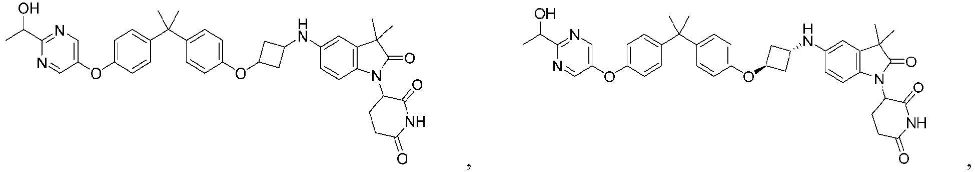 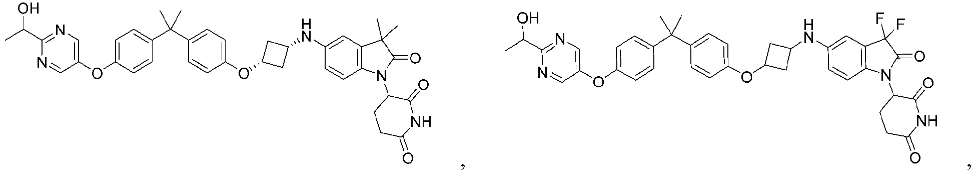 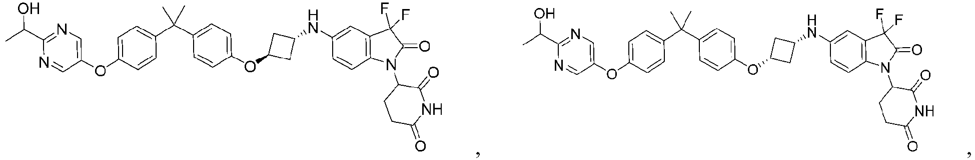 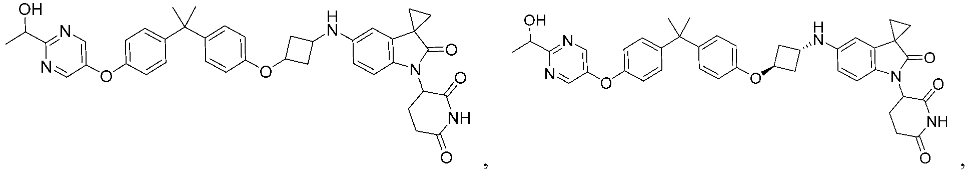 and

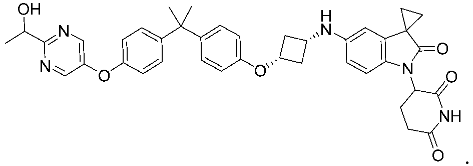

The present application relates to a compound of formula I-a, a structural moiety thereof, a derivative (e.g., Protac) thereof, a stereoisomer thereof, or a pharmaceutically acceptable salt thereof: 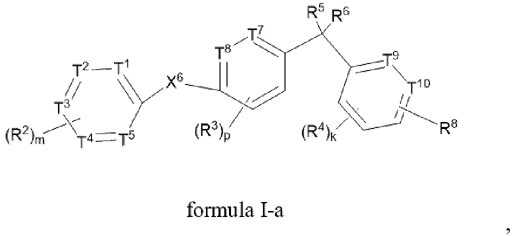 wherein,
T¹, T², T³, T⁴, T⁵, T⁷, T⁸, T⁹, and T¹⁰ are each independently selected from the group consisting of CH and N, and X⁶ is selected from the group consisting of O and a bond, provided that when X⁶ is selected from O, one or more of T⁷, T⁸, and T¹⁰ are selected from N;
R⁸ is selected from the group consisting of hydrogen, C₃₋₁₂ cycloalkyl, 4- to 12-membered heterocycloalkyl, -OC₃₋₁₂ cycloalkyl, -NHC₃₋₁₂ cycloalkyl, and -SC₃₋₁₂ cycloalkyl, wherein the C₃₋₁₂ cycloalkyl, 4- to 12-membered heterocycloalkyl, -OC₃₋₁₂ cycloalkyl, -NHC₃₋₁₂ cycloalkyl, or -SC₃₋₁₂ cycloalkyl is optionally substituted with HC(O)- or C₁₋₆ alkyl OC(O)-;
wherein R², R³, R⁴, m, p, k, R⁵, and R⁶ are as defined herein.

In some embodiments, X⁶ is selected from a bond.

In some embodiments, at least one of T¹, T², T³, T⁴, T⁵, T⁷, T⁸, T⁹, and T¹⁰ is selected from N.

In some embodiments, one or two of T¹, T², T³, T⁴, and T⁵ are selected from N, and the others are selected from CH. In some embodiments, T⁷, T⁸, T⁹, and T¹⁰ are each independently selected from the group consisting of CH and N, one or two of which are selected from N, and the others are selected from CH.

In some embodiments, T¹ is selected from N. In some embodiments, T² is selected from N. In some embodiments, T³ is selected from N. In some embodiments, T⁴ is selected from N. In some embodiments, T⁵ is selected from N. In some embodiments, T⁶ is selected from N. In some embodiments, T⁷ is selected from N. In some embodiments, T⁸ is selected from N. In some embodiments, T⁹ is selected from N. In some embodiments, T¹⁰ is selected from N.

In some embodiments, R⁸ is selected from the group consisting of hydrogen, C₃₋₁₀ cycloalkyl, 4- to 10-membered heterocycloalkyl, -OC₃₋₉ cycloalkyl, -NHC₃₋₁₀ cycloalkyl, and -SC₃₋₁₀ cycloalkyl, wherein the C₃₋₁₀ cycloalkyl, 4- to 10-membered heterocycloalkyl, -OC₃₋₉ cycloalkyl, -NHC₃₋₁₀ cycloalkyl, or -SC₃₋₁₀ cycloalkyl is optionally substituted with HC(O)- or C₁₋₄ alkyl OC(O)-.

In some embodiments, R⁸ is selected from the group consisting of hydrogen, C₄₋₆ cycloalkyl, 4- to 6-membered heterocycloalkyl, -OC₃₋₆ cycloalkyl, -NHC₃₋₆ cycloalkyl, and -SC₃₋₆ cycloalkyl, wherein the C₄₋₆ cycloalkyl, 4- to 6-membered heterocycloalkyl, -OC₃₋₆ cycloalkyl, -NHC₃₋₆ cycloalkyl, or -SC₃₋₆ cycloalkyl is optionally substituted with HC(O)- or C₁₋₄ alkyl OC(O)-.

In some embodiments, R⁸ is selected from the group consisting of hydrogen, 4- to 6-membered heterocycloalkyl, and -OC₃₋₆ cycloalkyl, wherein the 4- to 6-membered heterocycloalkyl or -OC₃₋₆ cycloalkyl is optionally substituted with HC(O)- or C₁₋₂ alkyl OC(O)-.

In some embodiments, R⁸ is selected from hydrogen.

The present application also relates to a compound of a formula selected from the group consisting of the following formulas, a structural moiety thereof, a derivative (e.g., Protac) thereof, a stereoisomer thereof, or a pharmaceutically acceptable salt thereof:

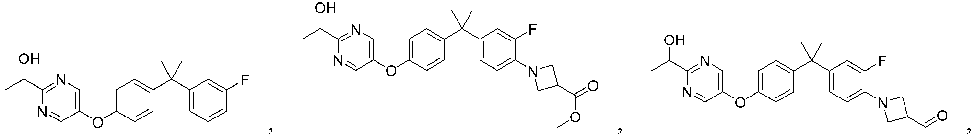

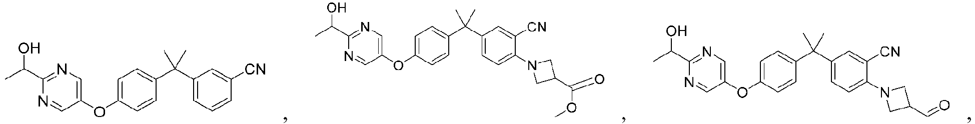

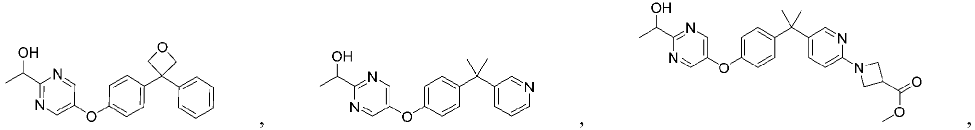

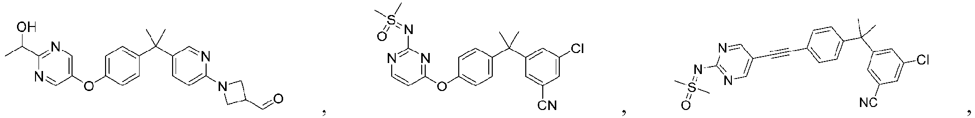

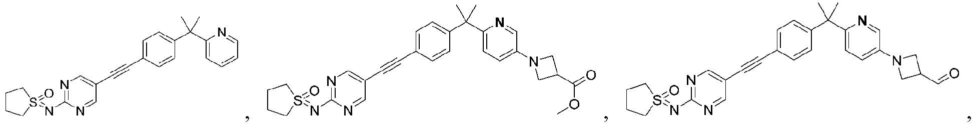

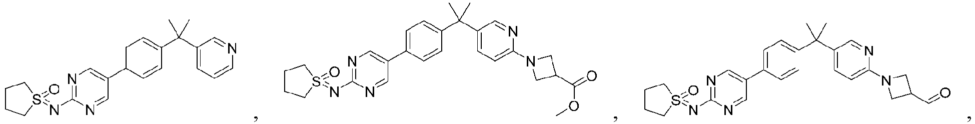

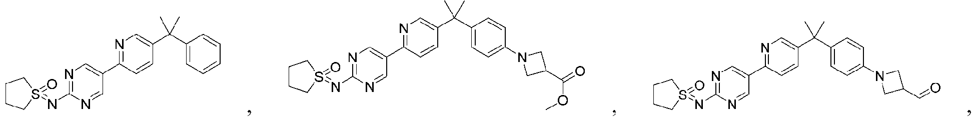

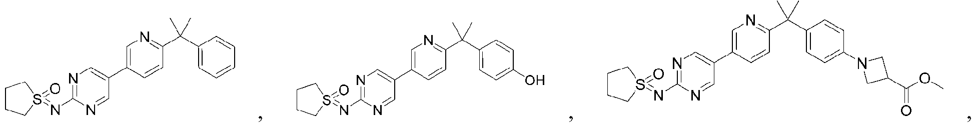

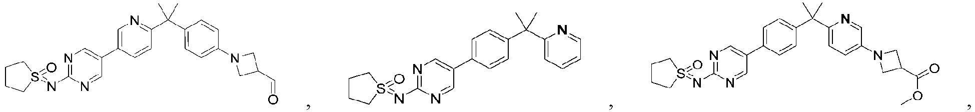

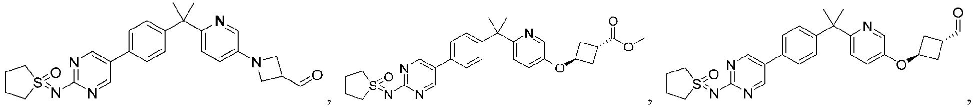

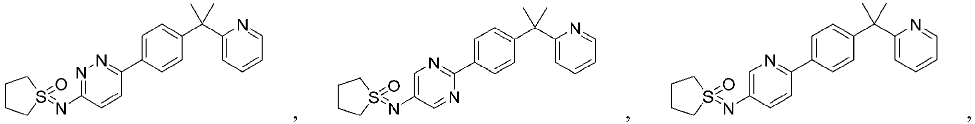

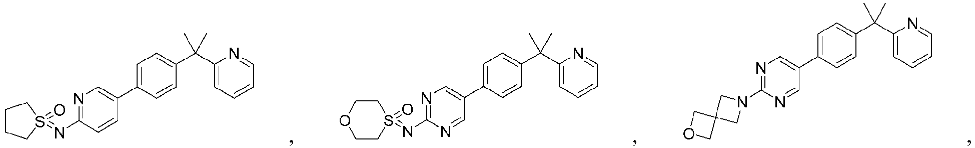

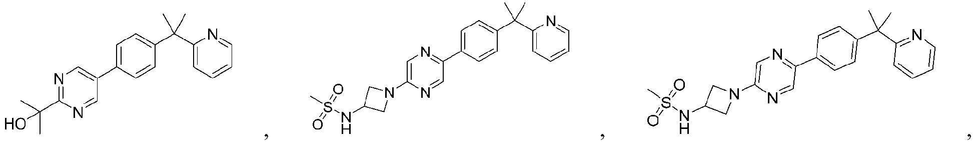

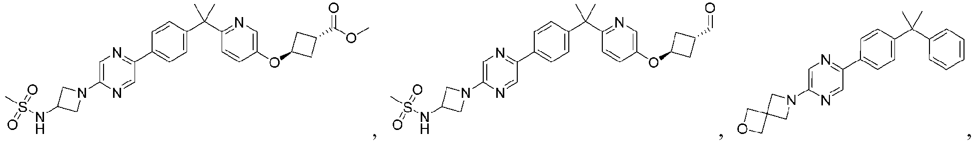

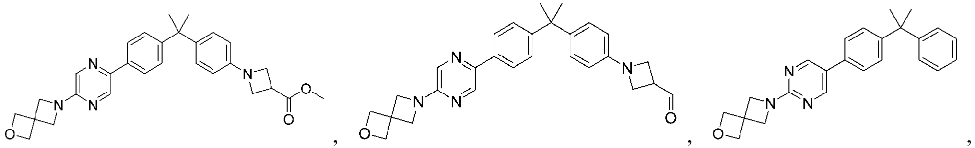

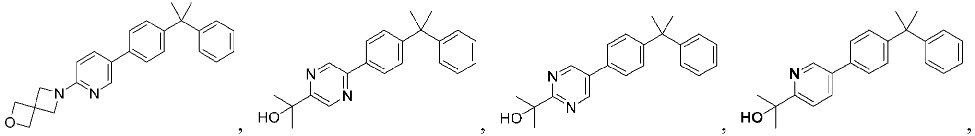

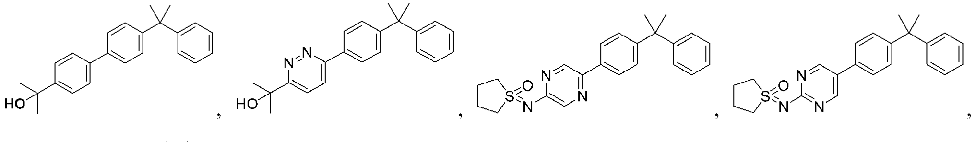

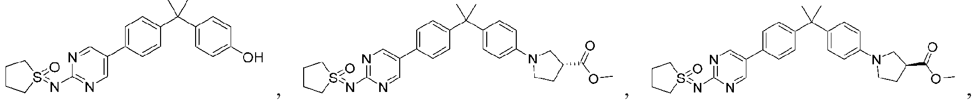

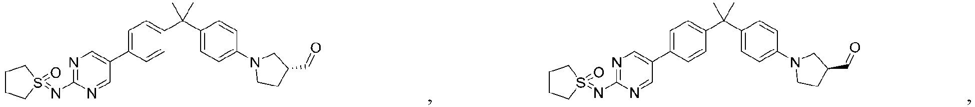

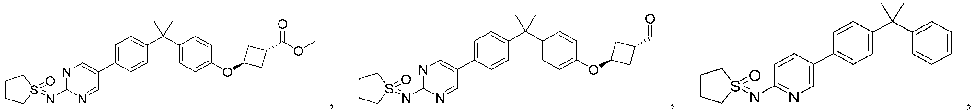

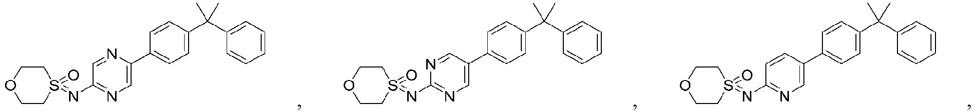

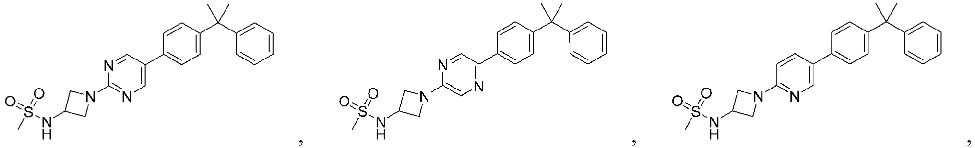

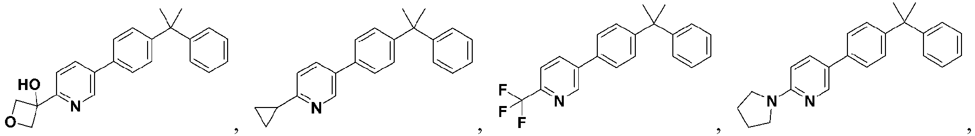

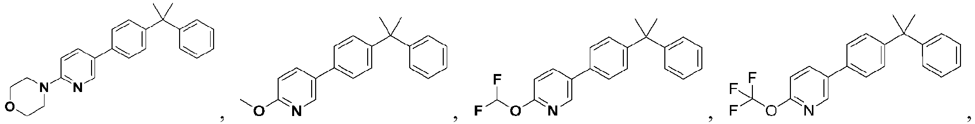

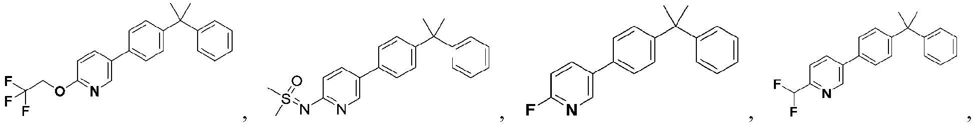

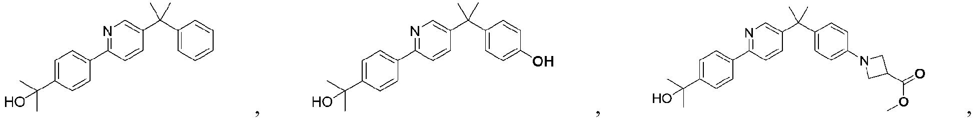

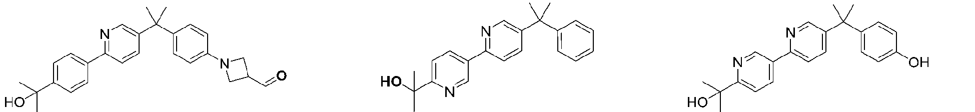

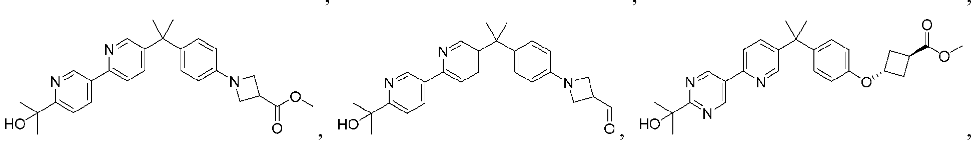

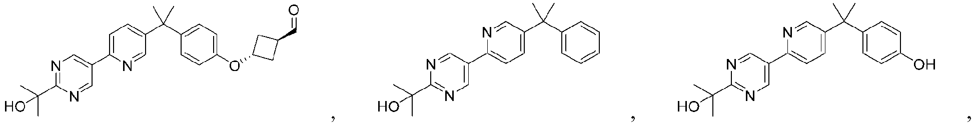

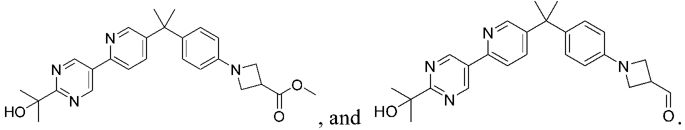

In another aspect, the present application relates to a Protac molecule, comprising a compound of formula I-a, a structural moiety thereof, a derivative thereof, a related specific compound thereof, a stereoisomer thereof, or a pharmaceutically acceptable salt thereof.

Provided is use of the compound of formula I-a, the structural moiety thereof, the derivative thereof, the related specific compound thereof, the stereoisomer thereof, or the pharmaceutically acceptable salt thereof for preparing a Protac (or a protein degrader). Provided are use of the compound of formula I-a, the structural moiety thereof, the derivative thereof, the related specific compound thereof, the stereoisomer thereof, or the pharmaceutically acceptable salt thereof in a Protac molecule, for example, use thereof as a structural moiety of a Protac molecule, existing in the form of a Protac molecule; and use thereof for degrading a protein, i.e., degrading a related protein in the form of a Protac molecule.

The present application also covers solutions obtained by any combination, deletion, or exchange of the embodiments described above.

In another aspect, the present application relates to a pharmaceutical composition, wherein the pharmaceutical composition comprises the compound, the structural moiety thereof, the derivative thereof, the stereoisomer thereof, or the pharmaceutically acceptable salt thereof of the present application described above; optionally, the pharmaceutical composition of the present application also comprises a pharmaceutically acceptable excipient.

In another aspect, the present application relates to use of the compound, the structural moiety thereof, the derivative thereof, the stereoisomer thereof, or the pharmaceutically acceptable salt thereof, or the pharmaceutical composition thereof described above for preparing a medicament for preventing or treating a disease (e.g., cancer).

In another aspect, the present application relates to use of the compound, the structural moiety thereof, the derivative thereof, the stereoisomer thereof, or the pharmaceutically acceptable salt thereof, or the pharmaceutical composition thereof described above for preparing a medicament for preventing or treating a disorder treated by degrading and/or inhibiting a target protein (e.g., GSPT1) that binds to a targeting ligand. In another aspect, the present application relates to use of the compound, the structural moiety thereof, the derivative thereof, the stereoisomer thereof, or the pharmaceutically acceptable salt thereof, or the pharmaceutical composition thereof described above for preparing a medicament for degrading and/or inhibiting a target protein (e.g., GSPT1) that binds to a targeting ligand.

In another aspect, the present application relates to use of the compound, the structural moiety thereof, the derivative thereof, the stereoisomer thereof, or the pharmaceutically acceptable salt thereof, or the pharmaceutical composition thereof described above for preparing a medicament for preventing or treating a disorder treated by binding to cereblon protein *in vivo.*

In another aspect, the present application relates to use of the compound, the structural moiety thereof, the derivative thereof, the stereoisomer thereof, or the pharmaceutically acceptable salt thereof, or the pharmaceutical composition thereof described above for preparing a medicament for preventing or treating a GSPT1-related disease.

In another aspect, the present application relates to use of the compound, the structural moiety thereof, the derivative thereof, the stereoisomer thereof, or the pharmaceutically acceptable salt thereof, or the pharmaceutical composition thereof described above for preparing a medicament for preventing or treating an AR- or ARv7-related disease.

The present application relates to a method for treating or preventing a disease (e.g., cancer), comprising administering to a mammal (preferably, a human) in need of such treatment a therapeutically effective amount of the compound, the structural moiety thereof, the derivative thereof, the stereoisomer thereof, or the pharmaceutically acceptable salt thereof, or the pharmaceutical composition thereof of the present application described above.

The present application relates to a method for treating or preventing a disorder in a mammal treated by degrading and/or inhibiting a target protein (e.g., GSPT1) that binds to a targeting ligand, comprising administering to a mammal (preferably, a human) in need of such treatment a therapeutically effective amount of the compound, the structural moiety thereof, the derivative thereof, the stereoisomer thereof, or the pharmaceutically acceptable salt thereof, or the pharmaceutical composition thereof of the present application described above. The present application relates to a method for degrading and/or inhibiting a target protein (e.g., GSPT1) that binds to a targeting ligand in a mammal, comprising administering to a mammal (preferably, a human) in need of such treatment a therapeutically effective amount of the compound, the structural moiety thereof, the derivative thereof, the stereoisomer thereof, or the pharmaceutically acceptable salt thereof, or the pharmaceutical composition thereof of the present application described above.

The present application relates to a method for treating or preventing a disorder treated by binding to cereblon protein *in vivo,* comprising administering to a mammal (preferably, a human) in need of such treatment a therapeutically effective amount of the compound, the structural moiety thereof, the derivative thereof, the stereoisomer thereof, or the pharmaceutically acceptable salt thereof, or the pharmaceutical composition thereof of the present application described above.

In another aspect, the present application relates to a method for treating a GSPT1-related disease, comprising administering to a mammal (preferably, a human) in need of such treatment a therapeutically effective amount of the compound, the structural moiety thereof, the derivative thereof, the stereoisomer thereof, or the pharmaceutically acceptable salt thereof, or the pharmaceutical composition thereof of the present application described above.

In another aspect, the present application relates to a method for treating an AR- or ARv7-related disease in a mammal, comprising administering to a mammal (preferably, a human) in need of such treatment a therapeutically effective amount of the compound, the structural moiety thereof, the derivative thereof, the stereoisomer thereof, or the pharmaceutically acceptable salt thereof, or the pharmaceutical composition thereof of the present application described above.

In another aspect, the present application relates to the compound, the structural moiety thereof, the derivative thereof, the stereoisomer thereof, or the pharmaceutically acceptable salt thereof, or the pharmaceutical composition thereof described above for use in preventing or treating a disease (e.g., cancer).

In another aspect, the present application relates to the compound, the structural moiety thereof, the derivative thereof, the stereoisomer thereof, or the pharmaceutically acceptable salt thereof, or the pharmaceutical composition thereof described above for use in preventing or treating a disorder treated by degrading and/or inhibiting a target protein (e.g., GSPT1) that binds to a targeting ligand.

In another aspect, the present application relates to the compound, the structural moiety thereof, the derivative thereof, the stereoisomer thereof, or the pharmaceutically acceptable salt thereof, or the pharmaceutical composition thereof described above for use in degrading and/or inhibiting a target protein (e.g., GSPT1) that binds to a targeting ligand. In another aspect, the present application relates to the compound, the structural moiety thereof, the derivative thereof, the stereoisomer thereof, or the pharmaceutically acceptable salt thereof, or the pharmaceutical composition thereof described above for use in preventing or treating a disorder treated by binding to cereblon protein *in vivo.*

In another aspect, the present application relates to the compound, the structural moiety thereof, the derivative thereof, the stereoisomer thereof, or the pharmaceutically acceptable salt thereof, or the pharmaceutical composition thereof described above for use in preventing or treating a GSPT1-related disease.

In another aspect, the present application relates to the compound, the structural moiety thereof, the derivative thereof, the stereoisomer thereof, or the pharmaceutically acceptable salt thereof, or the pharmaceutical composition thereof described above for use in preventing or treating an AR- or ARv7-related disease.

In another aspect, the present application relates to use of the compound, the structural moiety thereof, the derivative thereof, the stereoisomer thereof, or the pharmaceutically acceptable salt thereof, or the pharmaceutical composition thereof described above for preventing or treating a disease (e.g., cancer).

In another aspect, the present application relates to use of the compound, the structural moiety thereof, the derivative thereof, the stereoisomer thereof, or the pharmaceutically acceptable salt thereof, or the pharmaceutical composition thereof described above for preventing or treating a disorder treated by degrading and/or inhibiting a target protein (e.g., GSPT1) that binds to a targeting ligand.

In another aspect, the present application relates to use of the compound, the structural moiety thereof, the derivative thereof, the stereoisomer thereof, or the pharmaceutically acceptable salt thereof, or the pharmaceutical composition thereof described above for degrading and/or inhibiting a target protein (e.g., GSPT1) that binds to a targeting ligand. In another aspect, the present application relates to use of the compound, the structural moiety thereof, the derivative thereof, the stereoisomer thereof, or the pharmaceutically acceptable salt thereof, or the pharmaceutical composition thereof described above for preventing or treating a disorder treated by binding to cereblon protein *in vivo.*

In another aspect, the present application relates to use of the compound, the structural moiety thereof, the derivative thereof, the stereoisomer thereof, or the pharmaceutically acceptable salt thereof, or the pharmaceutical composition thereof described above for preventing or treating a GSPT1-related disease.

In another aspect, the present application relates to use of the compound, the structural moiety thereof, the derivative thereof, the stereoisomer thereof, or the pharmaceutically acceptable salt thereof, or the pharmaceutical composition thereof described above for preventing or treating an AR- or ARv7-related disease.

In some specific embodiments, the disease or disorder described above is a disease treated by degradation and/or inhibition of a target protein (e.g., GSPT1), or by binding to cereblon protein to ubiquitinate the target protein. In some specific embodiments, the disease described above is selected from the group consisting of disorders treated by degrading and/or inhibiting a protein (e.g., GSPT1) that binds to a target protein ligand; in some specific embodiments, the disease described above is selected from the group consisting of disorders treated by binding to cereblon protein *in vivo;* in some embodiments, the disease or disorder described above is selected from the group consisting of cancers, such as prostate cancer.

In some specific embodiments, the disorder treated by binding to cereblon protein *in vivo* and/or by degrading and/or inhibiting a target protein that binds to a targeting ligand described above is selected from the group consisting of a GSPT1-related disease, and an AR- or ARv7-related disease; in some specific embodiments, the GSPT1-related disease, or the AR- or ARv7-related disease described above is selected from the group consisting of cancers, e.g., prostate cancer. In some specific embodiments, the disorder treated by binding to cereblon protein *in vivo* and/or by degrading a target protein that binds to a targeting ligand described above is selected from the group consisting of cancers, e.g., prostate cancer. In some embodiments, the target protein that binds to the targeting ligand is selected from GSPT1. In some specific embodiments, the disease or disorder is selected from an AR- or ARv7-related disease. In some specific embodiments, the disease or disorder is selected from a GSPT1-related disease.

In some specific embodiments, the disease or disorder described above is a disease treated by degradation and/or inhibition of GSPT1, or by binding to cereblon protein to ubiquitinate the target protein.

In some embodiments, the present application encompasses the variables defined above and embodiments thereof, as well as any combination thereof.

### Technical Effects

The compounds of the present application can have degradative activity against GSPT1, and can have antiproliferative activity against VCaP and/or 22RV1 cells. In addition, the compounds of the present application can also have good *in vitro* liver microsome stability and good *in vivo* pharmacokinetic properties (e.g., AUC and other parameters) in mammals (e.g., a mouse, rat, dog, or human), can inhibit the growth of tumors *in vivo,* and show good druggability.

### Definitions

Unless otherwise stated, the following terms used in the present application shall have the following meanings. A certain term, unless otherwise specifically defined, should not be considered uncertain or unclear, but interpreted according to its common meaning in the art. When referring to a trade name, it is intended to refer to its corresponding commercial product or its active ingredient.

"One or more" used herein refers to an integer ranging from one to ten. For example, "one or more" refers to one, two, three, four, five, six, seven, eight, nine, or ten; in some embodiments, the "one or more" is selected from the group consisting of one, two, three, four, five, and six. In some embodiments, the "one or more" is selected from the group consisting of one, two, and three. In some embodiments, the "one or more" is selected from the group consisting of one and two.

Unless otherwise specified, [ ]- is used to indicate that a hydrogen atom at any position of a group within [ ] may be substituted with a group connected via "-", for example, by L.

The term "substituted" means that any one or more hydrogen atoms or lone pair electrons on a specific atom are substituted with a substituent, as long as the valence of the specific atom is normal and the substitution results in a stable compound. When the substituent is oxo (i.e., =O), it means that two hydrogen atoms are substituted; oxo substitutions do not occur on aromatic groups. For example, 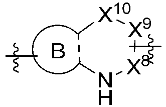 may be substituted with one or more R¹ substituents, wherein R¹ may be substituted on ring B, or on another ring, such as X⁸, X⁹, or X¹⁰, and substituents thereof on the ring, and -NH-.

The term "optional" or "optionally" means that the subsequently described event or circumstance may or may not occur, and the description includes instances where the event or circumstance occurs and instances where it does not. "Optionally substituted" includes unsubstituted and substituted; for example, ethyl being "optionally" substituted with halogen means that the ethyl may be unsubstituted (CH₂CH₃), monosubstituted (e.g., CH₂CH₂F), polysubstituted (e.g., CHFCH₂F, CH₂CHF₂, etc.), or fully substituted (CF₂CF₃). It will be appreciated by those skilled in the art that for any group comprising one or more substituents, no substitution or substitution pattern that is sterically impossible and/or cannot be synthesized is introduced.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms.

When any variable (e.g., R) occurs more than once in the constitution or structure of a compound, the definition of the variable in each case is independent. For example, if a group contains 2 R, the definition of each R is independent. When the number of connecting groups is 0, for example, -(CH₂)₀-, it means that the connecting group is a covalent bond.

When a variable is selected from a covalent bond, it means that the two groups are directly connected. For example, in A-L-Z, when L represents a covalent bond, it means that the structure is actually A-Z.

When a bond is crosslinked to two atoms of a ring (including monocyclic, fused, or spiro ring), the bond may be bonded to any atom on the ring (including monocyclic, fused, or spiro ring). For example, the structural unit 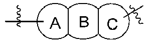 indicates that the bonds on two sides may be linked to any two different atoms on ring A, ring B, or ring C; for another example, 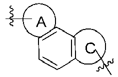 indicates that the bonds on two sides may be linked to any two different atoms on ring A, the middle benzene ring, or ring C; for further example, 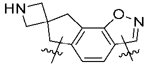 indicates that the bonds on two sides may be linked to any two different atoms of the four rings in the system.

The term "halo" or "halogen" refers to fluorine, chlorine, bromine, and iodine.

The term "hydroxyl" refers to an -OH group.

The term "amino" refers to an -NH₂ group.

The term "cyano" refers to a -CN group.

The term "alkyl" refers to hydrocarbyl with a general formula of CₙH₂ₙ₊₁. The alkyl may be linear or branched. For example, the term "C₁-₆ alkyl" refers to alkyl containing 1 to 6 carbon atoms (e.g., methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, etc.). Similarly, the alkyl moieties (i.e., alkyl) of alkoxy, alkylamino, dialkylamino, alkylsulfonyl, and alkylthio have the same definition as described above.

The term "alkylene" refers to a divalent group formed by the removal of one hydrogen at any position of alkyl, for example, the term "C₁-₆ alkyl" refers to alkylene containing 1 to 6 carbon atoms; the term "C₁-₄ alkyl" refers to alkylene containing 1 to 4 carbon atoms, including but not limited to -CH₂-, -CH₂CH₂-, -CH₂CH₂CH₂-, or - CH₂CH₂CH₂CH₂-.

The term "alkenylene" refers to a divalent group formed by the removal of one hydrogen at any position of alkenyl, for example, the term "C₂-₆ alkenyl" refers to alkenylene containing 2 to 6 carbon atoms; the term "C₂-₄ alkenyl" refers to alkenylene containing 2 to 4 carbon atoms, including but not limited to -CH₂CH=CH-, -CH₂CH₂CH=CH-, or -CH₂CH=CHCH₂-.

The term "alkynylene" refers to a divalent group formed by the removal of one hydrogen at any position of alkynyl, for example, the term "C₂-₆ alkynyl" refers to alkynylene containing 2 to 6 carbon atoms; the term "C₂-₄ alkynyl" refers to alkynylene containing 2 to 4 carbon atoms, including but not limited to -C≡C-, -H₂C-C≡C--H₂C-H₂C-C≡C- , or -H₂C-C≡C-CH₂-.

The term "heteroalkyl" is linear or branched alkyl consisting of a certain number of carbon atoms and at least one heteroatom; it has preferably 1 to 14 carbons, more preferably 1 to 10 carbons, even more preferably 1 to 6 carbons, and most preferably 1 to 3 carbons in the chain, wherein the heteroatom is selected from the group consisting of heteroatoms of Si, P, B, S, O, and N; and it has preferably 1, 2, or 3 heteroatoms selected from the group consisting of S, O, and N. For example, Cₘ heteroalkyl represents alkyl consisting of m carbon atoms and at least one heteroatom (e.g., 1-3 heteroatoms selected from the group consisting of S, O, and N) located between any two carbon atoms and having a heteroatom inserted in the chain. The nitrogen and sulfur atoms are optionally oxidized, and the nitrogen atom is optionally quaternized. The heteroatom or heteroatom group may be located at any internal position of heterohydrocarbyl, including the position where the hydrocarbyl is attached to the rest of the molecule; exemplary heteroalkyl includes alkyl ether, secondary alkylamine and tertiary alkylamine, amide, alkyl sulfide, and the like, including alkoxy, alkylthio, and alkylamino; unless otherwise specified, C₁₋₆ heteroalkyl includes C₁, C₂, C₃, C₄, C₅, and C₆ heteroalkyl, e.g., C₁₋₆ alkoxy, C₁₋₆ alkylthio, or C₁₋₆ alkylamino.

The term "heteroalkylene" refers to a divalent group formed by the removal of one hydrogen at any position of heteroalkyl, e.g., -NHCH₂- or -CH₂NHCH₂-.

The term "alkoxy" refers to -O-alkyl.

The term "alkenyl" refers to linear or branched unsaturated aliphatic hydrocarbyl consisting of carbon atoms and hydrogen atoms and having at least one double bond. Non-limiting examples of the alkenyl include, but are not limited to, ethenyl, 1-propenyl, 2-propenyl, 1-butenyl, isobutenyl, 1,3-butadienyl, and the like.

The term "cycloalkenyl" refers to a non-aromatic carbocyclic ring that is not fully saturated and may exist as a monocyclic ring, a bicyclic bridged ring, or a spiro ring. Unless otherwise specified, the carbocyclic ring is usually a 4- to 16-membered ring, a 4- to 12-membered ring, a 4- to 10-membered ring, or a 4- to 8-membered ring. Non-limiting examples of the cycloalkenyl include, but are not limited to, cyclopentenyl, cyclopentadienyl, cyclohexenyl, cyclohexadienyl, cycloheptenyl, cycloheptadienyl, and the like.

The term "cycloalkyl" refers to a fully saturated carbocyclic ring which may exist in the form of a monocyclic, bridged cyclic or spirocyclic structure. Unless otherwise specified, the carbocyclic ring is usually a 3- to 16-membered ring (e.g., a 3- to 10-membered ring or a 5- to 8-membered ring). Non-limiting examples of cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like.

The term "heterocycloalkyl" refers to a cyclic group that is fully saturated and may exist as a monocyclic ring, a bridged ring, or a spiro ring. Unless otherwise specified, the heterocyclic ring usually contains heteroatoms selected from the group consisting of Si, P, B, S, O, and N; preferably, it is a 3- to 16-membered, 3- to 11-membered, 3- to 10-membered, 3- to 7-membered, 3- to 6-membered, or 3- to 5-membered ring containing 1 to 3 heteroatoms (preferably 1 or 2 heteroatoms) independently selected from the group consisting of sulfur, oxygen, and/or nitrogen. Examples of 3-membered heterocycloalkyl include, but are not limited to, oxiranyl, thiiranyl, and aziranyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, and 1,4-dithianyl; examples of 7-membered heterocycloalkyl include, but are not limited to, azepanyl, oxepanyl, and thiepanyl. Monocyclic heterocycloalkyl having 5 or 6 ring atoms is preferred.

The term "spiro ring" refers to a fully saturated or partially unsaturated polycyclic ring system in which monocyclic rings share one carbon atom (referred to as a spiro atom), including carbocyclic rings and heterocyclic rings. Unless otherwise specified, the spiro ring is 5- to 20-membered, preferably 6- to 14-membered, and more preferably 8- to 12-membered. When the spiro ring is a heterocyclic ring, one or more ring atoms in the polycyclic ring are heteroatoms (preferably 1 or 2 heteroatoms) selected from the group consisting of N, O, S(O)ₙ, and P(O)ₙ (wherein n is 0, 1, or 2), and the remaining ring atoms are carbon atoms.

The term "spirocycloalkyl" refers to a fully saturated all-carbon polycyclic ring in which monocyclic rings share one carbon atom (referred to as a spiro atom). Unless otherwise specified, the spirocycloalkyl is 5- to 20-membered, preferably 6- to 14-membered, and more preferably 8- to 12-membered. According to the number of spiro atoms shared among the rings, the spirocycloalkyl is monospirocycloalkyl, bispirocycloalkyl, or polyspirocycloalkyl, preferably monospirocycloalkyl or bispirocycloalkyl, and more preferably 4-membered/4-membered, 4-membered/5-membered, 4-membered/6-membered, 5-membered/5-membered, or 5-membered/6-membered monospirocycloalkyl. Non-limiting examples of the spirocycloalkyl include 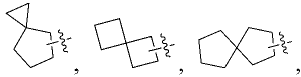 and

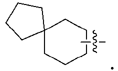

The term "spiro-heterocycloalkyl" refers to a fully saturated polycyclic ring in which monocyclic rings share one carbon atom (referred to as a spiro atom), wherein one or more ring atoms in the polycyclic ring are heteroatoms (preferably 1 or 2 heteroatoms) selected from the group consisting of N, O, S(O)ₙ, and P(O)ₙ (wherein n is 0, 1, or 2), and the remaining ring atoms are carbon atoms. Unless otherwise specified, the spiro-heterocycloalkyl is 5- to 20-membered, preferably 6- to 14-membered, and more preferably 6- to 10-membered. According to the number of spiro atoms shared among the rings, the spiro heterocyclic ring is a monospiro heterocyclic ring, a bispiro heterocyclic ring, or a polyspiro heterocyclic ring, preferably a monospiro heterocyclic ring or a bispiro heterocyclic ring, and more preferably a 4-membered/4-membered, 4-membered/5-membered, 4-membered/6-membered, 5-membered/5-membered, or 5-membered/6-membered monospiro heterocyclic ring. Non-limiting examples of the spiro-heterocycloalkyl include 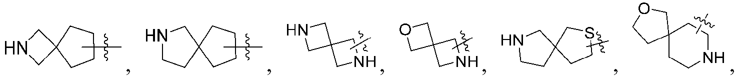 or the like.

The term "heterocycloalkenyl" includes cycloalkenyl in which one or more (e.g., 1-5, 1-4, 1-3, or 1-2) carbon atoms are substituted with a heteroatom, wherein the heteroatom is selected from the group consisting of Si, P, B, S, O, and N; specifically, it includes, for example, cycloalkenyl in which at most 3 carbon atoms, in one embodiment at most 2 carbon atoms, and in another embodiment 1 carbon atom, are each independently replaced by O, S, S(O), or N, provided that at least one cycloalkenyl carbon-carbon double bond is preserved. The heterocycloalkenyl may be a cyclic group that exists as a monocyclic ring, a bridged ring, or a spiro ring and may be a 3- to 20-membered or 3-to 16-membered ring (e.g., a 3- to 12-membered or 5- to 8-membered ring, specifically, e.g., a 5-membered, 6-membered, 7-membered, 8-membered, 9-membered, 10-membered, or 11-membered ring). Examples of the heterocycloalkenyl include, but are not limited to, dihydropyrrolyl, tetrahydropyridinyl, tetrahydroazepinyl, azaspirooctenyl, 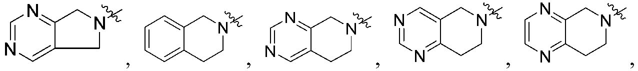 or the like.

The term "aryl" refers to an all-carbon aromatic monocyclic or fused polycyclic group having a conjugated π-electron system. For example, the aryl may have 6-20 carbon atoms, 6-14 carbon atoms, or 6-12 carbon atoms. Non-limiting examples of aryl include, but are not limited to, phenyl, naphthyl, anthryl, 1,2,3,4-tetrahydronaphthalene, and the like.

The term "heterocyclyl" refers to a monocyclic or fused polycyclic system that contains heteroatoms selected from the group consisting of Si, P, B, S, O, and N; preferably, it refers to an unsaturated non-aromatic ring system that contains at least one (e.g., 1-5, 1-4, 1-3, or 1-2) ring atom selected from the group consisting of N, O, and S, with the remaining ring atoms being C, and that may contain no double bond or have at least one or more double bonds. Preferably, the heterocyclyl has a single 4- to 8-membered ring, particularly a 5- to 8-membered (e.g., 5-membered, 6-membered, 7-membered, or 8-membered) ring, or has a plurality of fused rings comprising 6 to 14, particularly 6 to 10 (e.g., 6, 7, 8, 9, or 10), ring atoms.

The term "heteroaryl" refers to a monocyclic or fused polycyclic system that comprises at least one (e.g., 1-5, 1-4, 1-3, or 1-2) ring atom selected from the group consisting of N, O, and S, with the remaining ring atoms being C, and that has at least one aromatic ring. Preferably, the heteroaryl has a single 4- to 8-membered ring, particularly a 5- to 8-membered (e.g., 5-membered, 6-membered, 7-membered, or 8-membered) ring, or has a plurality of fused rings comprising 6 to 14, particularly 6 to 10 (e.g., 6, 7, 8, 9, or 10), ring atoms. Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

The substitution or substitution with a substituent in the term "substituent", "optionally substituted with one or more substituents", or "optionally substituted" includes all substituents mentioned in the text, and may be, for example, the terms "halogen", "deuterium", "O 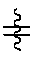 ", "-NH₂", "-NH(C₁₋₄ alkyl)", "-N(C₁₋₄ alkyl)₂", "-OH", "-OC₁₋₄ alkyl","-CN", "C₁₋₄ alkyl", and "3- to 6-membered heterocycloalkyl", and the corresponding non-limiting or exemplary groups mentioned below, wherein some non-limiting examples of the "substituent" include sulfydryl, nitro, nitroso, cyano, azido, a sulfoxide group, a sulfone group, a sulfonamide group, carboxyl, an aldehyde group, an imine group, alkyl, halogenated alkyl, cycloalkyl, halogenated cycloalkyl, alkenyl, halogenated alkenyl, cycloalkenyl, halogenated cycloalkenyl, alkynyl, halogenated alkynyl, cycloalkynyl, halogenated cycloalkynyl, heteroalkyl, halogenated heteroalkyl, alkoxy, alkylthio, aryl, aryloxy, arylthio, arylalkylene, arylalkoxy, arylalkylthio, heteroaryl, heteroaryloxy, heteroarylthio, heteroarylalkylene, heteroarylalkoxy, heteroarylalkylthio, heterocyclyl, heterocyclyloxy, heterocyclylthio, heterocyclylalkylene, heterocyclylalkoxy, heterocyclylalkylthio, acyl, acyloxy, a carbamate group, amido, ureido, an epoxy group, an ester group, oxo, and the like, wherein these substituents are optionally substituted with one or more substituents selected from the group consisting of the following substituents: oxo, hydroxyl, amino, nitro, halogen, cyano, alkyl, alkenyl, alkynyl, alkoxy, haloalkoxy, alkylamino, dialkylamino, haloalkylamino, halodialkylamino, carboxyl, -C(O)O-alkyl, -OC(O)-alkyl, -C(O)NH₂, -C(O)NH-alkyl, - C(O)N(alkyl)₂, -NHC(O)-alkyl, -C(O)-alkyl, -S(O)-alkyl, -S(O)₂-alkyl, -S(O)₂NH₂, -S(O)₂NH-alkyl, - S(O)₂N(alkyl)₂, cycloalkyl, cycloalkylalkylene, cycloalkyloxy, heterocyclyl, heterocyclylalkylene, heterocyclyloxy, heterocycloalkyl, heterocycloalkylalkylene, heterocycloalkyloxy, heteroaryl, heteroarylalkylene, heteroaryloxy, aryl, arylalkylene, and aryloxy.

In some embodiments herein, the substituent is selected from the group consisting of deuterium, tritium, hydroxyl, sulfydryl, halogen, amino, nitro, nitroso, cyano, azido, a sulfoxide group, a sulfone group, a sulfonamide group, carboxyl, an aldehyde group, an imine group, C₁₋₁₂ alkyl, C₁₋₁₂ haloalkyl, 3- to 12-membered cycloalkyl, 3- to 12-membered halocycloalkyl, C₂₋₁₂ alkenyl, C₂₋₁₂ haloalkenyl, 3- to 12-membered cycloalkenyl, 3- to 12-membered halocycloalkenyl, C₂₋₁₂ alkynyl, C₂₋₁₂ haloalkynyl, 8- to 12-membered cycloalkynyl, 8- to 12-membered halocycloalkynyl, C₁₋₁₂ heteroalkyl, C₁₋₁₂ haloheteroalkyl, C₁₋₁₂ alkoxy, C₁₋₁₂ alkylthio, 6- to 10-membered aryl, 6-to 10-membered aryloxy, 6- to 10-membered arylthio, 6- to 10-membered aryl C₁₋₁₂ alkylene, 6- to 10-membered aryl C₁₋₁₂ alkoxy, 6- to 10-membered aryl C₁₋₁₂ alkylthio, 5- to 10-membered heteroaryl, 5- to 10-membered heteroaryloxy, 5- to 10-membered heteroarylthio, 5- to 10-membered heteroarylalkylene, 5- to 10-membered heteroarylalkoxy, 5- to 10-membered heteroarylalkylthio, 3- to 12-membered heterocyclyl, 3- to 12-membered heterocyclyloxy, 3- to 12-membered heterocyclylthio, 3- to 12-membered heterocyclyl C₁₋₁₂ alkylene, 3-to 12-membered heterocyclyl C₁₋₁₂ alkoxy, 3- to 12-membered heterocyclyl C₁₋₁₂ alkylthio, C₁₋₁₂ acyl, C₁₋₁₂ acyloxy, a carbamate group, C₁₋₁₂ amido, ureido, an epoxy group, a C₂₋₁₂ ester group, and oxo, wherein these substituents are optionally substituted with one or more substituents selected from the group consisting of the following substituents: oxo, hydroxyl, amino, nitro, halogen, cyano, C₁₋₁₂ alkyl, C₂₋₁₂ alkenyl, C₂₋₁₂ alkynyl, C₁₋₁₂ alkoxy, C₁₋₁₂ haloalkoxy, C₁₋₁₂ alkylamino, di-C₁₋₁₂ alkylamino, C₁₋₁₂ haloalkylamino, di-C₁₋₁₂ haloalkylamino, carboxyl, -C(O)O-C₁₋₁₂ alkyl, -OC(O)-C₁₋₁₂ alkyl, -C(O)NH₂, -C(O)NH-C₁₋₁₂ alkyl, -C(O)N(C₁₋₁₂ alkyl)₂, -NHC(O)-C₁₋₁₂ alkyl, -C(O)-C₁₋₁₂ alkyl, -S(O)-C₁₋₁₂ alkyl, -S(O)₂-C₁₋₁₂ alkyl, -S(O)₂NH₂, -S(O)₂NH-C₁₋₁₂ alkyl, -S(O)₂N(C₁₋₁₂ alkyl)₂, 3- to 12-membered cycloalkyl, 3- to 12-membered cycloalkyl C₁₋₁₂ alkylene, 3- to 12-membered cycloalkyloxy, 3- to 12-membered heterocyclyl, 3- to 12-membered heterocyclyl C₁₋₁₂ alkylene, 3- to 12-membered heterocyclyloxy, 3- to 12-membered heterocycloalkyl, 3- to 12-membered heterocycloalkyl C₁₋₁₂ alkylene, 3- to 12-membered heterocycloalkyloxy, 5- to 10-membered heteroaryl, 5- to 10-membered heteroaryl C₁₋₁₂ alkylene, 5- to 10-membered heteroaryloxy, 6- to 10-membered aryl, 6- to 10-membered aryl C₁₋₁₂ alkylene, and 6- to 10-membered aryloxy.

Unless otherwise specified, the term "hetero" refers to a heteroatom or a heteroatom group (i.e., a heteroatom-containing group), including atoms other than carbon (C) and hydrogen (H) and groups containing such heteroatoms; for example, heteroatoms include, but are not limited to, oxygen (O), nitrogen (N), sulfur (S), phosphorus (P), silicon (Si), germanium (Ge), aluminum (Al), and boron (B); specific heteroatoms or heteroatom groups are, for example, -O-, -S-, -N=, =O, =S, -P(=O)-, -P(=O)₂-, -P(=O)O-, -P(=O)₂O-, -C(=O)O-, -C(=O)-, -C(=S)-, -S(=O), -S(=O)₂-, and optionally substituted -C(=O)N(H)-, -N(H)-, -C(=NH)-, -S(=O)₂N(H)-, or -S(=O)N(H)-. Preferably, the term "hetero" means that a heteroatom or a heteroatom of a heteroatom group (i.e., a heteroatom-containing group) is selected from the group consisting of oxygen, nitrogen, and sulfur.

The term "derivative" refers to a new compound or a group of new compounds that are produced through one or more chemical reactions or structural evolutions. The derivative retains the basic structure of the parent compound, with changes or modifications occurring in the side chains, functional groups, or substituents only.

In the present application, one of the absolute configurations (e.g., one of 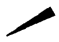 or 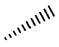 ; specifically, 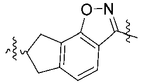 represents 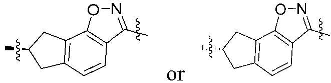 ) or one of the relative configurations (e.g., 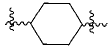 represents 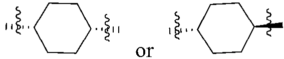 ) of a stereogenic center is represented by a wavy line ( 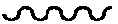 ). Unless otherwise specified, the compounds described herein include both E and Z geometric isomers when they contain olefinic double bonds or other centers of geometric asymmetry. Likewise, all tautomeric forms are included within the scope of the present application.

The group or structural fragment in the present application, such as -LNK¹-Cy¹-LNK-Cy²-LNK²-, -Cy¹-Cy²-LNK²-, LNK, Cy¹, Cy², -Cy¹-LNK-Cy²-, -Cy¹-LNK-, or -LNK-Cy²-, and specific options thereof, optionally can be linked to the left-side group and the right-side group of the group or fragment respectively in the general formula in a left-to-right reading order. For example, in the case that L is selected from -Cy¹-LNK-, when Cy¹ is selected from 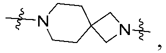 according to a left-to-right reading order, the left side of Cy¹ is linked to the fragment 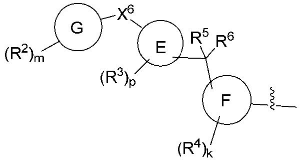 on the corresponding left side in the general formula, and the right side is linked to the fragment 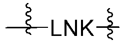 on the right side, resulting in a fragment 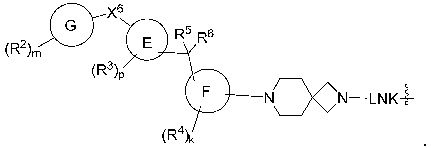 Optionally, the group or structural fragment in the present application, such as -LNK¹-Cy¹-LNK-Cy²-LNK²-, -Cy¹-Cy²-LNK²-, LNK, Cy¹, Cy², -Cy¹-LNK-Cy²-, -Cy¹-LNK-, or -LNK-Cy²-, and specific options thereof, can be linked to the left-side group and the right-side group of the group or fragment respectively in the general formula in a right-to-left reading order. For example, in the case that L is selected from -Cy¹-LNK-, when Cy¹ is selected from 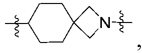 according to a right-to-left reading order, the right side of Cy¹ is linked to the fragment 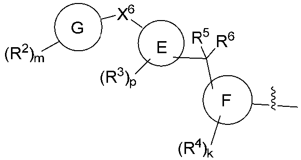 on the corresponding left side in the general formula, and the left side is linked to the fragment 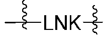 on the corresponding right side in the general formula, resulting in a fragment 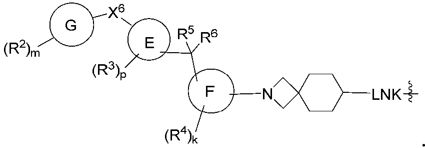 The other groups are as described above.

The term "treat", "treating", or "treatment" means administering the compound or formulation of the present application to ameliorate or eliminate a disease or one or more symptoms associated with the disease, and includes: (i) inhibiting the disease or disease state, i.e., arresting its progression; (ii) relieving the disease or disease state, i.e., causing regression of the disease or disease state.

The term "prevent", "preventing", or "prevention" means administering the compound or formulation of the present application to prevent a disease or one or more symptoms associated with the disease, and includes: preventing the occurrence of the disease or disease state in a mammal, particularly when such a mammal is predisposed to the disease state but has not yet been diagnosed with it.

The term "therapeutically effective amount" refers to an amount of the compound of the present application for (i) treating or preventing a specific disease, condition, or disorder; (ii) alleviating, ameliorating, or eliminating one or more symptoms of a specific disease, condition, or disorder, or (iii) preventing or delaying onset of one or more symptoms of a specific disease, condition, or disorder described herein. The amount of the compound of the present application composing the "therapeutically effective amount" varies depending on the compound, the disease state and its severity, the mode of administration, and the age of the mammal to be treated, but may be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organism.

The term "pharmaceutically acceptable excipient" refers to those that do not have a significant irritating effect on an organism and do not impair the biological activity and properties of the active compound. Suitable excipients are well known to those skilled in the art, such as carbohydrates, wax, water-soluble and/or water-swellable polymers, hydrophilic or hydrophobic materials, gelatin, oil, solvents, and water.

The word "comprise" and variations thereof such as "comprises" or "comprising" should be understood in an open, non-exclusive sense, i.e., "including but not limited to".

Unless otherwise specified clearly in the context, singular terms herein encompass plural referents, and vice versa. Similarly, unless otherwise specified clearly in the context, the word "or" herein is intended to include "and".

Unless otherwise stated, all numbers expressing the amounts of ingredients, measurements, or reaction conditions used herein should be understood as being modified in all instances by the term "about". The term "about", when connected to a percentage, may represent, for example, ±1%, preferably +0.5%, and more preferably ±0.1%.

The compounds and intermediates of the present application may also exist in different tautomeric forms, and all such forms are included within the scope of the present application. The term "tautomer" or "tautomeric form" refers to structural isomers of different energies that can interconvert via a low energy barrier. For example, a proton tautomer (also referred to as a prototropic tautomer) includes interconversion via proton transfer, such as keto-enol isomerization and imine-enamine isomerization. A specific example of a proton tautomer is an imidazole moiety in which a proton can transfer between two ring nitrogens. A valence tautomer includes the interconversion via recombination of some bonding electrons. Specifically, any of the compounds of the present disclosure in which, for example, pyrazolyl is independent or as part of heterocyclyl, may exist in the form of any tautomer or a mixture of two tautomers in any amount, i.e., 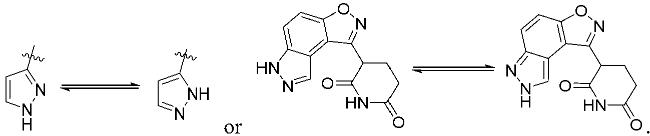 The present disclosure includes all possible tautomers of the compounds of the present disclosure, either as a single tautomer or any mixture of the tautomers in any ratio.

Unless otherwise stated, the "bond" in the definition of a substituent (e.g., L¹ or X⁶) herein refers to a single bond. The present application also includes isotopically labeled compounds of the present application, which are identical to those recited herein but have one or more atoms replaced by an atom having an atomic mass or mass number different from the atomic mass or mass number generally found in nature. Examples of isotopes that can be incorporated into the compounds of the present application include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Certain isotopically labeled compounds of the present application (e.g., those labeled with ³H and ¹⁴C) can be used to analyze compounds and/or substrate tissue distribution. Tritiated (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present application can generally be prepared by following procedures analogous to those disclosed in the schemes and/or examples below while substituting a non-isotopically labeled reagent with an isotopically labeled reagent. The compound of the present application may be asymmetrical, for example, having one or more stereoisomers (e.g., *cis-trans* isomers, or the like). Unless otherwise stated, all stereoisomers include, for example, enantiomers and diastereoisomers. The compound of the present application containing asymmetrical carbon atoms may be separated in an optically pure form or in a racemic form. The optically pure form can be resolved from a racemic mixture or can be synthesized using a chiral raw material or a chiral reagent.

The pharmaceutical composition of the present application may be prepared by combining the compound of the present application with a suitable pharmaceutically acceptable excipient.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous, and intravenous administration.

The pharmaceutical composition of the present application may be manufactured using methods well known in the art, such as conventional mixing, dissolving, granulating, dragee-making, levigating, emulsifying, and lyophilizing. In some embodiments, the pharmaceutical composition is in an oral form.

A solid oral composition can be prepared by conventional mixing, filling, or tableting. For example, the solid oral composition can be obtained by the following method: mixing the active compound with solid excipients, optionally grinding the resulting mixture, adding additional suitable excipients if desired, and then processing the mixture into granules to obtain the core parts of tablets or dragees.

The pharmaceutical composition may also be suitable for parenteral administration.

In all of the administration methods of the compound of general formula I described herein, the daily dose administered is from 0.001 mg/kg body weight to 2000 mg/kg body weight, given in individual or separated doses. The compounds of the present application can be prepared using a variety of synthetic methods well known to those skilled in the art, including the specific embodiments listed below, embodiments formed by combining them with other chemical synthetic methods, and equivalent substitutions well known to those skilled in the art. The preferred embodiments include, but are not limited to, the examples of the present application.

The chemical reactions in the specific embodiments of the present application are conducted in a proper solvent that must be suitable for the chemical changes in the present application and the reagents and materials required. In order to obtain the compounds of the present application, it is sometimes necessary for those skilled in the art to modify or select a synthetic procedure or a reaction process based on the existing embodiments.

An important consideration in synthetic route planning in the art is the selection of suitable protective groups for reactive functional groups (e.g., amino in the present application); for example, reference may be made to Greene's Protective Groups in Organic Synthesis (4th Ed.) Hoboken, New Jersey: John Wiley & Sons, Inc.

In some embodiments, the compounds of the present application can be prepared by those skilled in the art of organic synthesis through the following routes using the following intermediates or salts thereof: 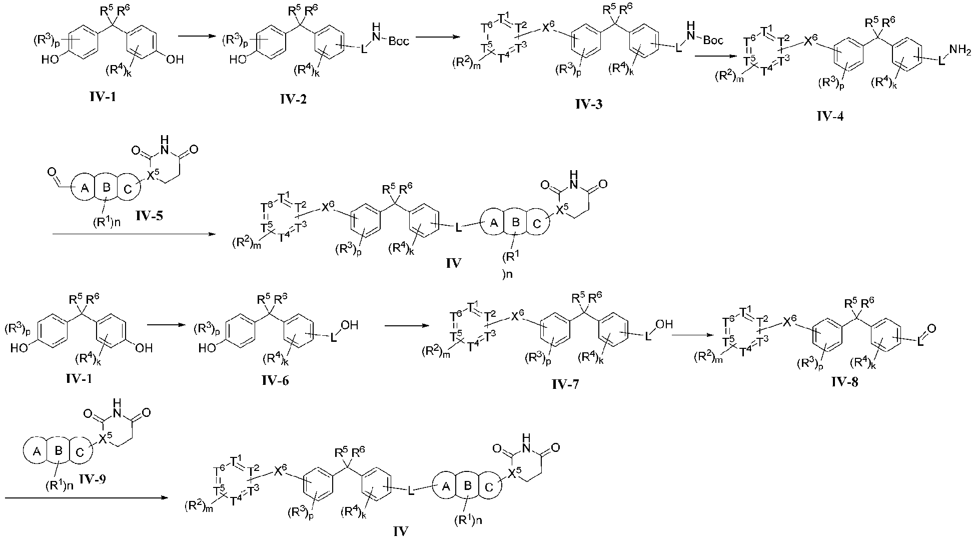 wherein ring A, ring B, ring C, R¹, n, X⁵, L, X⁶, R², R³, R⁴, m, p, k, R⁵, R⁶, T¹, T², T³, T⁴, T⁵, and T⁶ are as defined herein.

A compound of general formula **IV-1** is subjected to a Mitsunobu reaction to obtain a compound of general formula **IV-2,** the compound of general formula **IV-2** is subjected to a coupling reaction to obtain a compound of general formula **IV-3,** then a deprotection Boc reaction is performed to obtain a compound of general formula **IV-4,** and the compound of general formula **IV-4** is subjected to a reductive amination reaction to obtain a compound of general formula **IV.**

The compound of general formula **IV-1** is subjected to a Mitsunobu reaction to obtain a compound of general formula **IV-6,** the compound of general formula **IV-2** is subjected to a coupling reaction to obtain a compound of general formula **IV-7,** then an oxidation reaction is performed to obtain a compound of general formula **IV-8,** and the compound of general formula **IV-8** is subjected to a reductive amination reaction to obtain the compound of general formula **IV.**

### The following abbreviations are used in the present application:

DMSO represents dimethyl sulfoxide; DMF represents N,N-dimethylformamide; DIPEA represents N,N'-diisopropylethylamine; HATU represents O-(7-azabenzotriazol-1-yl)-N,N,N',N'-tetramethyluronium hexafluorophosphate; DCE represents dichloroethane; Boc represents tert-butoxycarbonyl; DCM represents dichloromethane; DEAD represents diethyl azodicarboxylate; EA represents ethyl acetate; DMAP represents 4-dimethylaminopyridine; TBS represents tert-butyldimethylsilyl; Tf represents trifluoromethanesulfonyl; THF represents tetrahydrofuran; Dess-martin oxidant represents 1,1,1-tris(acetyloxy)-1,1-dihydro-1,2-benziodoxol-3-(1H)-one; DC₅₀ represents the drug concentration at which the degradation rate reaches 50%; Dmax represents the maximum degradation rate.

PdCl₂(PPh₃)₂ represents bis(triphenylphosphine)palladium(II) dichloride; XPhos-Pd-G3 represents methanesulfonato(2-dicyclohexylphosphino-2',4',6'-triisopropyl-1,1'-biphenyl)(2'-amino-1,1'-biphenyl-2-yl)palladium(II); Xphos represents 2-dicyclohexylphosphonium-2',4',6'-triisopropylbiphenyl; Pd₂(dba)₃ represents tris(dibenzylideneacetone)dipalladium(0); TBAF represents tetrabutylammonium fluoride; DMF represents N,N-dimethylformamide; DIBAL-H represents diisobutylaluminum hydride; DCE represents dichloroethane; iPrOH represents isopropanol; TIPS-Cl represents triisopropylchlorosilane; TIPS represents triisopropylsilyl; PdCl₂(dppf)-CH₂Cl₂ represents [1,1'-bis(diphenylphosphino)ferrocene]palladium(II) dichloride dichloromethane complex; DIAD represents diisopropyl azodicarboxylate; RuPhos represents 2-dicyclohexylphosphino-2',6'-diisopropoxy-1,1'-biphenyl.

For clarity, the present disclosure is further described with the following examples, which are, however, not intended to limit the scope of the present application. All reagents used in the present application are commercially available and can be used without further purification.

### DETAILED DESCRIPTION

### Preparation Example 1

### Synthesis of intermediate z8

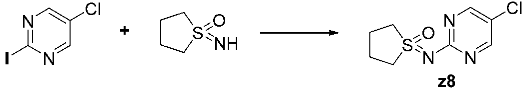

Intermediates 5-chloro-2-iodopyrimidine (8.04 g), cesium carbonate (21.68 g), XPhos-Pd-G3 (2.64 g), 1-iminotetrahydrothiophene (7.93 g), and 1,4-dioxane (100 mL) were added to a reaction flask in sequence. The mixture was reacted at 110 °C for 2 h under nitrogen atmosphere. After the reaction was completed, the reaction solution was purified by silica gel column chromatography to give compound z8 (6.8 g). MS (ESI, [M+H]⁺) m/z: 232.10.

For the synthesis of intermediates **z9-z17,** reference was made to the synthetic method for intermediate **z8** to synthesize the following compounds:

| **Intermediate No.** | **Starting material** | **Compound structure** | **MS (ESI) m/z [M+H]⁺:** |
|---|---|---|---|
| **z9** | 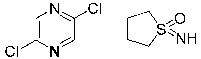 | 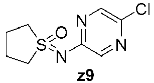 | 232.35 |
| **z10** | 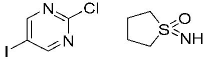 | 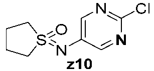 | 232.41 |
| **z11** | 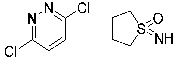 | 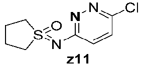 | 232.33 |
| **z12** | 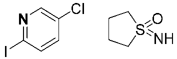 | 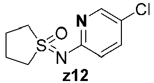 | 231.23 |
| **z13** | 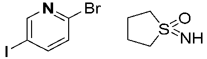 | 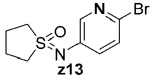 | 275.18 |
| **z14** | 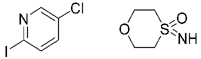 | 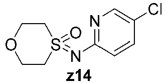 | 247.31 |
| **z15** | 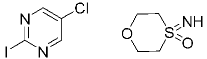 | 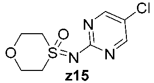 | 248.22 |
| **z16** | 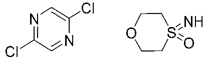 | 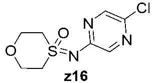 | 248.31 |
| **z17** | 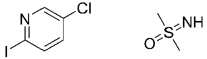 | 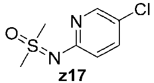 | 205.31 |

### Preparation Example 2: Synthesis of Intermediate z18

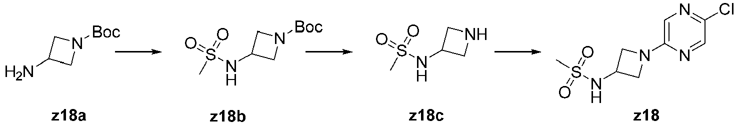

### Step 1: Preparation of compound z18b

**z18a** (1.0 g), triethylamine (1.5 g), and dichloromethane (20 mL) were added to a reaction flask in sequence. The mixture was cooled to 0 °C, and methanesulfonic anhydride (1.3 g) was slowly added. The mixture was reacted at 0 °C for 2 h. After the reaction was completed, water was added to the reaction solution to quench the reaction. The organic phase was separated, washed with saturated brine, and concentrated to give **z18b** (1.2 g).

### Step 2: Preparation of compound z18c

**z18b** (1.2 g), dichloromethane (20 mL), and trifluoroacetic acid (5 mL) were added to a reaction flask in sequence, and the mixture was reacted at room temperature for 2 h. After the reaction was completed, the reaction solution was directly concentrated to give **z18c** (0.8 g).

### Step 3: Preparation of compound z18

**z18c** (0.2 g), 2,5-dichloropyrazine (0.17 g), potassium carbonate (0.3 g), and acetonitrile (5 mL) were added to a reaction flask in sequence, and the mixture was reacted at 50 °C for 2 h. After the reaction was completed, the reaction solution was purified by silica gel column chromatography to give compound **z18** (0.12 g). MS (ESI, [M+H]⁺) m/z: 263.21.

For the synthesis of intermediates **z19-z23,** reference was made to the synthetic method for intermediate **z18** to synthesize the following compounds:

| **Intermediate No.** | **Starting material** | **Compound structure** | **MS (ESI) m/z [M+H]⁺:** |
|---|---|---|---|
| **z19** | 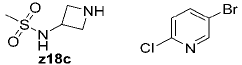 | 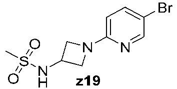 | 306.22 |
| **z20** | 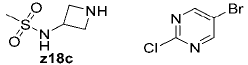 | 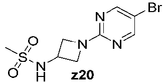 | 307.23 |
| **z21** | 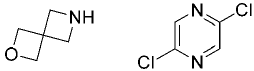 | 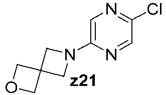 | 212.31 |
| **z22** | 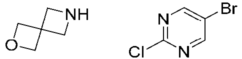 | 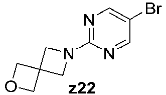 | 256.12 |
| **z23** | 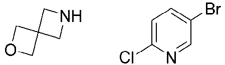 | 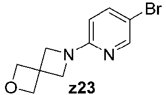 | 255.25 |

### Example 1: Synthesis of Compound 1

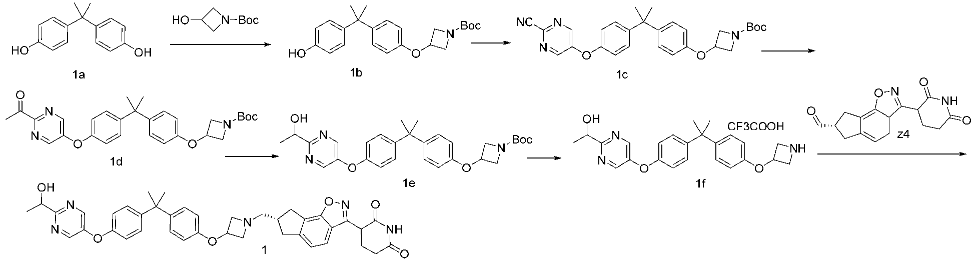

### Step 1: Preparation of intermediate 1b

Intermediate **1a** (2 g), N-Boc-3-hydroxyazetidine (1.5 g), triphenylphosphine (2.8 g), and a solvent THF (30 mL) were mixed, and a reagent diethyl azodicarboxylate (1.8 g) was added slowly and dropwise to the reaction system under N₂ atmosphere. The mixture was reacted at 60 °C for 16 h. After the reaction was completed, the reaction solution was concentrated and purified by silica gel column chromatography to give intermediate **1b** (1.37 g). MS (ESI) *m*/*z* [M+H]⁺: 384.1.

### Step 2: Preparation of intermediate 1c

Intermediate **1b** (500 mg), 5-bromo-2-cyanopyrimidine (360 mg), copper(I) iodide (49 mg), 2-pyridinecarboxylic acid (32 mg), potassium phosphate (454 mg), and a solvent DMSO (10 mL) were mixed, and the mixture was reacted at 100 °C for 16 h under N₂ atmosphere. After the reaction was completed, the reaction solution was extracted with ethyl acetate and a saturated aqueous ammonium chloride solution. The organic phase was concentrated and then purified by silica gel column chromatography to give intermediate **1c** (530 mg). MS (ESI) *m*/*z* [M+H]⁺: 487.3.

### Step 3: Preparation of intermediate 1d

Intermediate **1c** (530 mg) and a solvent THF (10 mL) were mixed, and a reagent methylmagnesium bromide (3.27 mL, 1 M in THF) was added slowly and dropwise to the reaction system under N₂ atmosphere. The mixture was reacted at 25 °C for 3 h. After the reaction was completed, the reaction solution was extracted with ethyl acetate and a saturated aqueous ammonium chloride solution. The organic phase was concentrated and then purified by silica gel column chromatography to give intermediate **1d** (480 mg). MS (ESI) *m*/*z* [M+H]⁺: 504.4.

### Step 4: Preparation of intermediate 1e

Intermediate **1d** (480 mg) and methanol (10 mL) were mixed, and sodium borohydride (108 mg) was added to the reaction system in an ice-water bath. The mixture was reacted for 1 h. After the reaction was completed, ammonium chloride was added to the reaction solution to quench the reaction, and the resulting mixture was extracted with ethyl acetate. The organic phase was concentrated and purified by silica gel column chromatography to give intermediate **1e** (420 mg). MS (ESI) *m*/*z* [M+H]⁺: 506.4.

### Step 5: Preparation of intermediate 1f

Intermediate **1e** (420 mg) and dichloromethane (10 mL) were mixed, and trifluoroacetic acid (2 mL) was added to the reaction system. The mixture was reacted for 1 h. After the reaction was completed, the reaction solution was concentrated to give intermediate **1f** (430 mg). MS (ESI) *m*/*z* [M+H]⁺: 406.5.

### Step 6: Preparation of compound 1

Intermediate **1f** (120 mg), **z4** (70 mg, i.e., intermediate **40e** in Example 40 of WO2023088406, see the preparation process thereof), and a solvent dichloromethane/isopropanol (v:v 5/1, 10 mL) were mixed, and sodium triacetoxyborohydride (148 mg) was added to the reaction system. The mixture was reacted for 1 h. After the reaction was completed, the reaction solution was concentrated and purified by silica gel column chromatography to give compound **1** (53 mg). MS (ESI) *m*/*z* [M+H]⁺: 688.5. ¹H NMR (500 MHz, DMSO) δ 11.07 (s, 1H), 8.57 (s, 2H), 7.60 (d*, J =* 8.0 Hz, 1H), 7.32-7.18 (m, 3H), 7.18-7.07 (m, 2H), 7.07-6.95 (m, 2H), 6.75 (d*, J =* 8.8 Hz, 2H), 5.21 (d*, J =* 5.5 Hz, 1H), 4.78 (dp, *J* = 17.2, 6.0 Hz, 2H), 4.56 (dd, *J* = 11.8, 5.0 Hz, 1H), 3.85-3.72 (m, 2H), 3.17 (ddd, *J* = 35.9, 16.3, 7.8 Hz, 2H), 3.04-2.96 (m, 2H), 2.92-2.70 (m, 3H), 2.68-2.52 (m, 4H), 2.46 (dd, *J =* 12.0, 4.4 Hz, 1H), 2.18 (dd, *J =* 12.6, 5.6 Hz, 1H), 1.61 (s, 6H), 1.41 (d, *J =* 6.5 Hz, 3H).

### Example 2: Synthesis of Compound 2

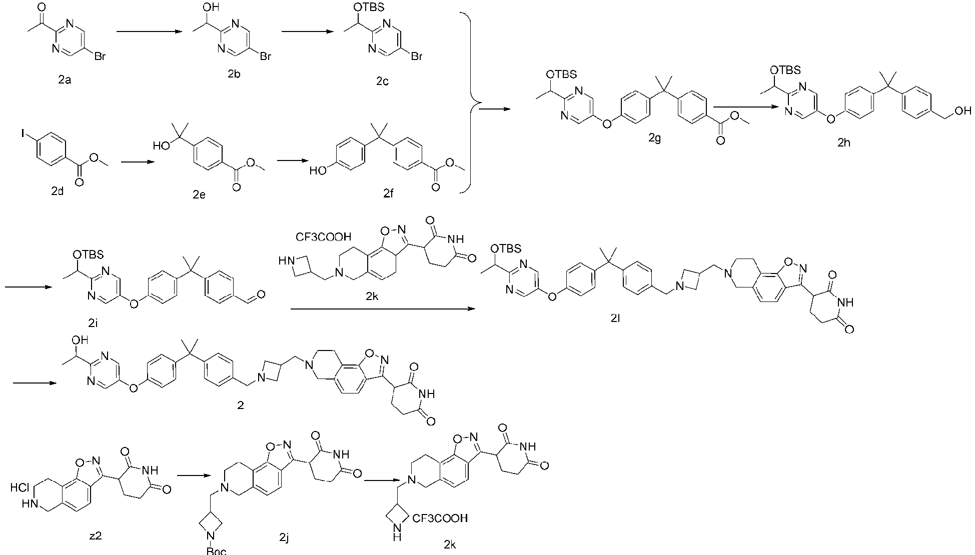

### Step 1: Preparation of intermediate 2b

Intermediate **2a** (30 g) and methanol (300 mL) were mixed, and sodium borohydride (1.56 g) was added to the reaction system in an ice bath. The mixture was reacted at 0 °C for 1 h until the reaction was completed. A saturated ammonium chloride solution was added to quench the reaction, and the resulting mixture was extracted with DCM. The organic phases were combined, dried, and concentrated to give intermediate **2b** (g). MS (ESI) *m*/*z* [M+H]⁺: 203.1.

### Step 2: Preparation of intermediate 2c

Intermediate **2b** (15 g), imidazole (15 g), and DCM (200 mL) were mixed, and TBSCl (16.7 g) was added to the reaction system in an ice bath. The mixture was reacted at 25 °C for 16 h until the reaction was completed. The reaction solution was concentrated and then purified by silica gel column chromatography to give intermediate **2c** (17.3 g). MS (ESI) *m*/*z* [M+H]⁺: 317.2.

### Step 3: Preparation of intermediate 2e

**2d** (15 g) and THF (150 mL) were mixed and cooled to -20 °C. A 1 M solution of isopropylmagnesium chloride in THF (60 mL) was added dropwise to the reaction system under N₂ atmosphere, and the mixture was reacted at - 20°C for 1 h. Acetone (4.95 g) was added, and the mixture was warmed to room temperature and reacted completely. The reaction solution was poured into a saturated aqueous ammonium chloride solution, and the mixture was extracted with ethyl acetate, concentrated, and purified by silica gel column chromatography to give intermediate **2e** (5.54 g). MS (ESI) *m*/*z* [M+H]⁺: 195.1.

### Step 4: Preparation of intermediate 2f

Intermediate **2e** (5.54 g), phenol (2.81 g), and carbon tetrachloride (100 mL) were mixed, and the mixture was cooled to -20 °C. Boron trifluoride diethyl etherate (23.82 g) was added dropwise to the reaction system under N₂ atmosphere, and the mixture was warmed to room temperature and reacted completely. The reaction solution was poured into water, and the mixture was extracted with ethyl acetate, concentrated, and purified by silica gel column chromatography to give intermediate **2f** (5.69 g). MS (ESI) *m*/*z* [M+H]⁺: 271.2.

### Step 5: Preparation of intermediate 2g

Intermediate **2c** (2 g), intermediate **2f** (1.74 g), CuI (600 mg), 2-pyridinecarboxylic acid (388 mg), potassium phosphate (4 g), and a solvent DMSO (20 mL) were mixed, and the mixture was reacted at 100 °C for 16 h under N₂ atmosphere until the reaction was completed. The mixture was extracted with saturated brine and EA, and the organic phase was dried and concentrated to give a crude product, which was separated by silica gel column chromatography to give intermediate **2g** (730 mg). MS (ESI) *m*/*z* [M+H]⁺: 507.3.

### Step 6: Preparation of intermediate 2h

Intermediate **2g** (300 mg) and a solvent THF (10 mL) were mixed, and LiAlH₄ (22 mg) was added to the reaction system in an ice bath. The mixture was reacted at 0 °C for 1 h until the reaction was completed. A 5% aqueous sodium hydroxide solution was added to quench the reaction, and the resulting mixture was dried over anhydrous sodium sulfate and filtered under vacuum. The filtrate was then concentrated to give intermediate **2h** (210 mg). MS (ESI) *m*/*z* [M+H]⁺: 479.2.

### Step 7: Preparation of intermediate 2i

Intermediate **2h** (210 mg) and DCM (10 mL) were mixed, and a Dess-martin oxidant (279 mg) was added to the reaction system in an ice bath. The mixture was reacted at 25 °C for 1 h until the reaction was completed. A saturated sodium thiosulfate solution was added to quench the reaction, and the resulting mixture was extracted with DCM. The organic phase was dried, concentrated, and then separated by silica gel column chromatography to give intermediate **2i** (180 mg). MS (ESI) *m*/*z* [M+H]⁺: 477.5.

### Step 8: Preparation of intermediate 2j

Intermediate **z2** (260 mg), tert-butyl 3-formylazetidine-1-carboxylate (150 mg), sodium acetate (66 mg), and a solvent isopropanol/DCM (1:5, 10 mL) were mixed, and sodium triacetoxyborohydride (515 mg) was added to the reaction system. The mixture was reacted at 25 °C for 1 h until the reaction was completed. The reaction solution was concentrated and separated by silica gel column chromatography to give intermediate **2j** (370 mg). MS (ESI) *m*/*z* [M+H]⁺: 455.3.

### Step 9: Preparation of intermediate 2k

Intermediate **2j** (370 mg) and a solvent DCM (10 mL) were mixed, and trifluoroacetic acid (2 mL) was added. The mixture was reacted at 25 °C for 1 h until the reaction was completed. The reaction solution was concentrated to give intermediate **2k** (420 mg). MS (ESI) *m*/*z* [M+H]⁺: 355.2.

### Step 10: Preparation of intermediate 2l

Intermediate **2k** (177 mg), intermediate **2i** (180 mg), and a solvent isopropanol/DCE (v:v = 1:5, 10 mL) were mixed, and sodium triacetoxyborohydride (240 mg) was added to the reaction system. The mixture was reacted at 70 °C for 16 h until the reaction was completed. The reaction solution was concentrated and separated by silica gel column chromatography to give intermediate **2l** (85 mg). MS (ESI) *m*/*z* [M+H]⁺: 815.5.

### Step 11: Preparation of compound 2

Intermediate **2l** (85 mg), formic acid (2 mL), and water (2 mL) were mixed, and the mixture was reacted at 25 °C for 1 h until the reaction was completed. A saturated sodium bicarbonate solution was added to the reaction solution to quench the reaction, and the resulting mixture was extracted with DCM. The organic phase was filtered, concentrated, and separated by silica gel column chromatography to give compound **2** (62 mg).

MS (ESI) *m*/*z* [M+H]⁺: 701.5. ¹H NMR (500 MHz, DMSO) δ 11.07 (s, 1H), 8.57 (s, 2H), 7.56 (d, *J* = 8.2 Hz, 1H), 7.29-7.22 (m, 2H), 7.21-7.12 (m, 4H), 7.07 (d, *J* = 8.3 Hz, 1H), 7.04-6.97 (m, 2H), 5.20 (d, *J* = 5.5 Hz, 1H), 4.79 (p, *J* = 6.4 Hz, 1H), 4.54 (dd, *J* = 11.9, 5.0 Hz, 1H), 3.65 (s, 2H), 3.50 (s, 2H), 3.37 (d, *J* = 8.0 Hz, 2H), 2.95 (t, *J =* 5.9 Hz, 2H), 2.81 (d, *J* = 19.2 Hz, 2H), 2.77-2.66 (m, 6H), 2.60 (dt, *J* = 17.3, 4.2 Hz, 1H), 2.46 (dt, *J =* 12.3, 6.3 Hz, 1H), 2.17 (dq, *J =* 13.6, 4.9 Hz, 1H), 1.62 (s, 6H), 1.41 (d, *J* = 6.6 Hz, 3H).

### Example 3: Synthesis of Compound 3

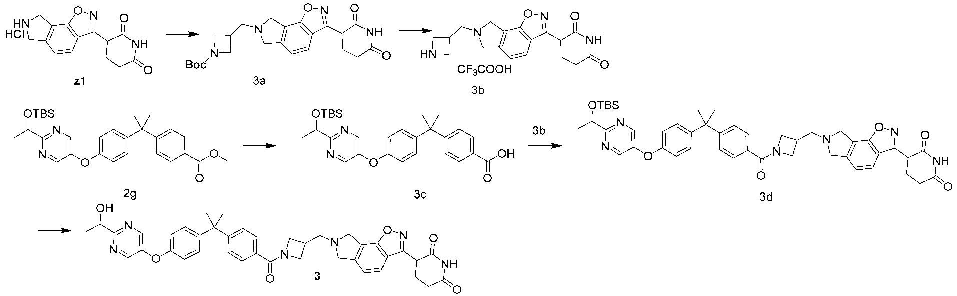

### Step 1: Preparation of intermediate 3a

Intermediate **z1** (200 mg), tert-butyl 3-formylazetidine-1-carboxylate (180 mg), sodium acetate (270 mg), and DCE/isopropanol (v:v = 5:1, 20 mL) were mixed, and sodium triacetoxyborohydride (110 mg) was then added. The mixture was reacted at room temperature until the reaction was completed. The reaction solution was concentrated and purified by silica gel column chromatography to give intermediate **3a** (210 mg). MS (ESI) *m*/*z* [M+H]⁺: 441.1.

### Step 2: Preparation of intermediate 3b

Intermediate **3a** (210 mg), DCM (5 mL), and trifluoroacetic acid (2 mL) were mixed, and the mixture was reacted at room temperature until the reaction was completed. The reaction solution was concentrated to give intermediate **3b** (170 mg). MS (ESI) *m*/*z* [M+H]⁺: 341.1.

### Step 3: Preparation of intermediate 3c

Intermediate **2g** (400 mg), methanol (10 mL), water (2 mL), and sodium hydroxide (158 mg) were mixed, and the mixture was reacted at 70 °C for 5 h. After the reaction was completed, a 1 M aqueous hydrochloric acid solution was added to the reaction solution to adjust the pH to acidity, and the mixture was extracted with ethyl acetate. The organic phase was dried and concentrated to give intermediate **3c** (340 mg). MS (ESI) *m*/*z* [M+H]⁺: 493.1.

### Step 4: Preparation of intermediate 3d

Intermediate **3b** (120 mg), intermediate **3c** (110 mg), DIPEA (150 mg), and DMF (5 mL) were mixed, and HATU (110 mg) was then added. The mixture was reacted at room temperature until the reaction was completed. The reaction solution was poured into water, and the mixture was extracted with ethyl acetate and purified by silica gel column chromatography to give intermediate **3d** (150 mg). MS (ESI) *m*/*z* [M+H]⁺: 815.4.

### Step 5: Preparation of compound 3

Intermediate **3d** (150 mg), water (2 mL), and formic acid (2 mL) were mixed, and the mixture was reacted at room temperature until the reaction was completed. The reaction solution was purified by silica gel column chromatography to give compound **3** (80 mg).

MS (ESI) *m*/*z* [M+H]⁺: 701.5. ¹H NMR (500 MHz, DMSO) δ 11.08 (s, 1H), 8.58 (s, 2H), 7.71 (d, *J =* 8.0 Hz, 1H), 7.61-7.56 (m, 2H), 7.33-7.27 (m, 4H), 7.26 (d, *J =* 2.1 Hz, 1H), 7.05-7.01 (m, 2H), 5.21 (d, *J =* 5.5 Hz, 1H), 4.82-4.77 (m, 1H), 4.60 (dd, *J* = 11.9, 5.0 Hz, 1H), 4.44 (t, *J* = 8.2 Hz, 1H), 4.16 (d, *J* = 8.4 Hz, 3H), 4.04 (t, *J* = 2.5 Hz, 3H), 3.77 (dd, *J* = 9.9*,* 5.5 Hz, 1H) , 3.00 (d, *J =* 8.4 Hz, 2H), 2.92 (p, *J =* 6.8 Hz, 1H), 2.77 (ddd, *J =* 17.2, 12.0, 5.3 Hz, 1H), 2.61 (dt, *J* = 17.3, 4.3 Hz, 1H), 2.49-2.45 (m, 1H), 2.20 (dt, *J* = 13.1, 4.7 Hz, 1H), 1.66 (s, 6H), 1.41 (d, *J* = 6.5 Hz, 3H).

### Example 4: Synthesis of Compound 4

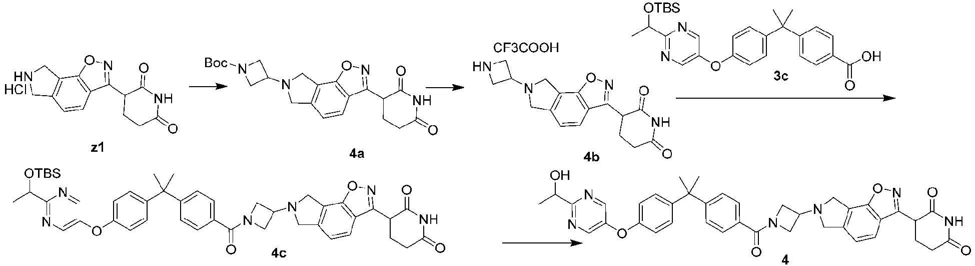

### Step 1: Preparation of intermediate 4a

Intermediate **z1** (200 mg), 1-Boc-3-azetidinone (160 mg), sodium acetate (270 mg), and DCE/isopropanol (v:v = 5:1, 20 mL) were mixed, and sodium triacetoxyborohydride (110 mg) was then added. The mixture was reacted at room temperature until the reaction was completed. The reaction solution was concentrated and purified by silica gel column chromatography to give intermediate **4a** (180 mg). MS (ESI) *m*/*z* [M+H]⁺: 427.3.

### Step 2: Preparation of intermediate 4b

Intermediate **4a** (180 mg), DCM (5 mL), and trifluoroacetic acid (2 mL) were mixed, and the mixture was reacted at room temperature until the reaction was completed. The reaction solution was concentrated to give intermediate **4b** (160 mg). MS (ESI) *m*/*z* [M+H]⁺: 327.1.

### Step 3: Preparation of intermediate 4c

Intermediate **3c** (130 mg), intermediate **4b** (110 mg), DIPEA (140 mg), and DMF (5 mL) were mixed, and HATU (110 mg) was then added. The mixture was reacted at room temperature until the reaction was completed. The reaction solution was poured into water, and the mixture was extracted with ethyl acetate and purified by silica gel column chromatography to give intermediate **4c** (120 mg). MS (ESI) *m*/*z* [M+H]⁺: 801.4.

### Step 4: Preparation of compound 4

Intermediate **4c** (120 mg), water (2 mL), and formic acid (2 mL) were mixed, and the mixture was reacted at room temperature until the reaction was completed. The reaction solution was separated by silica gel column chromatography to give compound 4 (60 mg).

MS (ESI) *m*/*z* [M+H]⁺: 687.5. ¹H NMR (500 MHz, DMSO) δ 11.09 (s, 1H), 8.59 (s, 2H), 7.73 (d, *J =* 8.1 Hz, 1H), 7.62-7.58 (m, 2H), 7.34-7.26 (m, 5H), 7.06-7.02 (m, 2H), 5.21 (d, *J =* 5.5 Hz, 1H), 4.83-4.77 (m, 1H), 4.60 (dd, *J* = 11.9, 5.0 Hz, 1H), 4.42 (t, *J =* 8.1 Hz, 1H), 4.34 (s, 1H), 4.21 (d, *J =* 18.9 Hz, 3H), 4.13-4.03 (m, 3H), 3.82 (dq, *J* = 9.8, 3.7 Hz, 1H), 2.77 (ddd, *J =* 17.2, 12.0, 5.4 Hz, 1H), 2.61 (dt*, J =* 17.3, 4.1 Hz, 1H), 2.53 (dd*, J =* 12.3, 4.3 Hz, 1H), 2.20 (dq, *J* = 13.5, 4.6 Hz, 1H), 1.67 (s, 6H), 1.41 (d, *J =* 6.5 Hz, 3H).

### Example 5: Synthesis of Compound 5

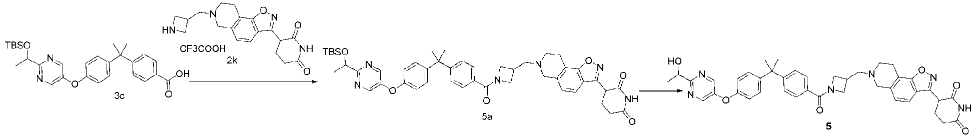

### Step 1: Preparation of intermediate 5a

Intermediate **3c** (130 mg), intermediate **2k** (110 mg), DIPEA (140 mg), and DMF (5 mL) were mixed, and HATU (110 mg) was then added. The mixture was reacted at room temperature until the reaction was completed. The reaction solution was poured into water, and the mixture was extracted with ethyl acetate and purified by silica gel column chromatography to give intermediate **5a** (100 mg). MS (ESI) *m*/*z* [M+H]⁺: 829.3.

### Step 2: Preparation of compound 5

Intermediate **5a** (100 mg) and formic acid/water (v:v = 1:1, 3 mL) were mixed and reacted at room temperature. After the reaction was completed, dichloromethane and water were added to the system for dilution, and a saturated aqueous sodium bicarbonate solution was added to adjust the pH to weak alkalinity. The organic phase was separated, and the aqueous phase was extracted with ethyl acetate. The organic phases were combined, washed with saturated brine, dried over anhydrous sodium sulfate, filtered, and concentrated by evaporation under reduced pressure to remove the solvent, and the residue was purified by silica gel column chromatography to give compound **5** (40 mg). MS (ESI, [M+H]⁺) *m*/*z:* 715.3. ¹H NMR (500 MHz, DMSO-*d*₆) *δ* 11.07 (s, 1H), 8.58 (d, J = 1.5 Hz, 2H), 7.57 (d, J = 8.1 Hz, 3H), 7.33-7.24 (m, 4H), 7.08 (d, J = 8.2 Hz, 1H), 7.03 (dd, J = 8.5, 1.6 Hz, 2H), 5.21 (d, J = 5.5 Hz, 1H), 4.79 (p, J = 6.3 Hz, 1H), 4.55 (dd, J = 11.9, 4.9 Hz, 1H), 4.42 (t, J = 8.5 Hz, 1H), 4.14 (t, J = 9.2 Hz, 1H), 4.00 (t, J = 7.2 Hz, 1H), 3.82-3.66 (m, 3H), 2.97 (t, J = 5.6 Hz, 3H), 2.85-2.71 (m, 5H), 2.60 (dt, J = 17.3, 4.4 Hz, 1H), 2.46 (dd, J = 12.2, 4.4 Hz, 1H), 2.18 (dq, J = 13.8, 4.8 Hz, 1H), 1.65 (s, 6H), 1.41 (d, J = 6.7 Hz, 3H).

### Example 6: Synthesis of Compound 6

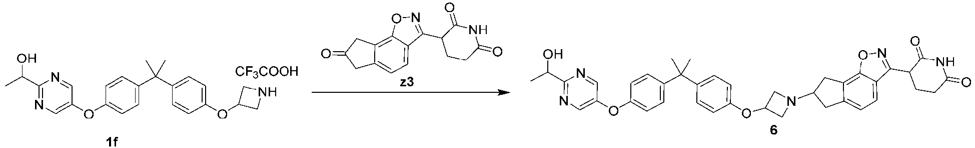

### Step 1: Preparation of compound 6

Intermediate **1f** (200 mg), intermediate **z3** (140 mg), sodium acetate (40 mg), and DCE/isopropanol (v:v = 5:1, 5 mL) were mixed, and then sodium triacetoxyborohydride (314 mg) was added. The mixture was reacted at room temperature. After the reaction was completed, the mixture was concentrated by evaporation under reduced pressure to remove the solvent, and the residue was purified by silica gel column chromatography to give compound **6** (70 mg). MS (ESI, [M+H]⁺) *m*/*z:* 674.3. ¹H NMR (500 MHz, DMSO-*d*₆) *δ* 11.07 (s, 1H), 8.57 (s, 2H), 7.61 (dd, J = 8.1, 2.5 Hz, 1H), 7.25 (dd, J = 8.6, 2.0 Hz, 3H), 7.16-7.09 (m, 2H), 7.05-6.98 (m, 2H), 6.78-6.70 (m, 2H), 5.21 (d, J = 5.5 Hz, 1H), 4.79 (p, J = 6.4 Hz, 1H), 4.70 (p, J = 5.7 Hz, 1H), 4.56 (dd, J = 11.8, 4.9 Hz, 1H), 3.81-3.70 (m, 2H), 3.45-3.37 (m, 1H), 3.34 (s, 1H), 3.12 (dd, J = 14.4, 6.4 Hz, 1H), 3.05 (dt, J = 12.9, 6.4 Hz, 2H), 2.90-2.72 (m, 3H), 2.65-2.57 (m, 1H), 2.49-2.43 (m, 1H), 2.25-2.14 (m, 1H), 1.61 (s, 6H), 1.41 (d, J = 6.5 Hz, 3H).

### Example 7: Synthesis of Compound 7

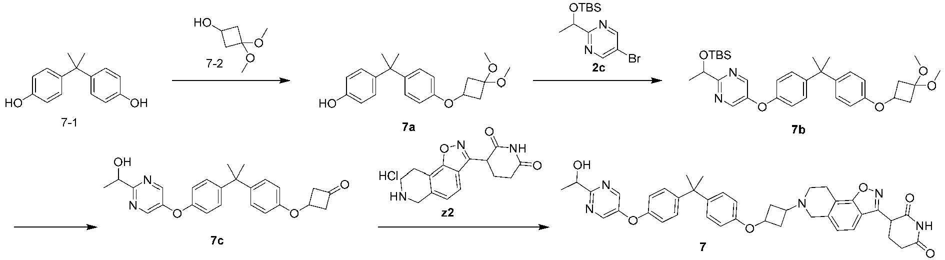

### Step 1: Preparation of intermediate 7a

Intermediate 7-1 (2 g), intermediate 7-2 (1.16 g), triphenylphosphine (3.45 g), and tetrahydrofuran (20 mL) were mixed, and the mixture was purged with nitrogen. DEAD (2.56 mL) was slowly added in an ice bath. After the addition was completed, the mixture was heated to 65 °C and reacted. After the reaction was completed, the reaction solution was cooled to room temperature, and concentrated by evaporation under reduced pressure to remove the solvent, and the residue was purified by silica gel column chromatography to give intermediate **7a** (700 mg). MS (ESI, [M+H]⁺) *m*/*z:* 343.2.

### Step 2: Preparation of intermediate 7b

Intermediate **7a** (700 mg), intermediate **2c** (650 mg), copper(I) iodide (540 mg), pyridine-2-carboxylic acid (350 mg), and DMSO (10 mL) were mixed, and the mixture was purged with nitrogen, heated to 100 °C, and reacted. After the reaction was completed, ethyl acetate and water were added to the system for extraction. The organic phase was separated, and the aqueous phase was extracted with ethyl acetate. The organic phases were combined, washed with saturated brine, dried over anhydrous sodium sulfate, filtered, and concentrated by evaporation under reduced pressure to remove the solvent, and the residue was purified by silica gel column chromatography to give intermediate **7b** (160 mg). MS (ESI, [M+H]⁺) *m*/*z:* 579.2.

### Step 3: Preparation of intermediate 7c

Intermediate **7b** (160 mg) and formic acid/water (v:v = 1:1, 3 mL) were mixed, and the mixture was heated to 90 °C and reacted. After the reaction was completed, dichloromethane and water were added to the system for dilution, and a saturated aqueous sodium bicarbonate solution was added to adjust the pH to weak alkalinity. The organic phase was separated, and the aqueous phase was extracted with ethyl acetate. The organic phases were combined, washed with saturated brine, dried over anhydrous sodium sulfate, filtered, and concentrated by evaporation under reduced pressure to remove the solvent, and the residue was purified by silica gel column chromatography to give intermediate **7c** (90 mg). MS (ESI, [M+H]⁺) *m*/*z:* 419.1.

### Step 4: Preparation of compound 5

Intermediate **7c** (90 mg), intermediate **z2** (74 mg), sodium acetate (14 mg), methanol (2 mL), and sodium cyanoborohydride (41 mg) were mixed, and the mixture was heated to 60 °C and reacted. After the reaction was completed, the reaction solution was concentrated by evaporation under reduced pressure to remove the solvent, and the residue was purified by silica gel column chromatography to give compound **7** (25 mg). MS (ESI, [M+H]⁺) *m*/*z:* 688.3. ¹H NMR (500 MHz, DMSO-*d*₆) *δ* 11.07 (s, 1H), 8.57 (s, 2H), 7.57 (d, J = 8.2 Hz, 1H), 7.25 (d, J = 8.3 Hz, 2H), 7.11 (t, J = 7.2 Hz, 3H), 7.01 (d, J = 8.3 Hz, 2H), 6.78 (d, J = 8.3 Hz, 2H), 5.21 (d, J = 5.5 Hz, 1H), 4.79 (p, J = 6.3 Hz, 1H), 4.51 (ddd, J = 40.8, 13.0, 6.1 Hz, 2H), 3.64 (s, 2H), 2.99 (t, J = 5.9 Hz, 2H), 2.74 (ddq, J = 28.5, 17.2, 5.6 Hz, 6H), 2.60 (dt, J = 17.4, 4.3 Hz, 1H), 2.46 (dd, J = 12.2, 4.4 Hz, 1H), 2.18 (dq, J = 13.7, 4.8 Hz, 1H), 1.94 (q, J = 10.4 Hz, 2H), 1.61 (s, 6H), 1.41 (d, J = 6.5 Hz, 3H).

### Example 8: Synthesis of Compound 8

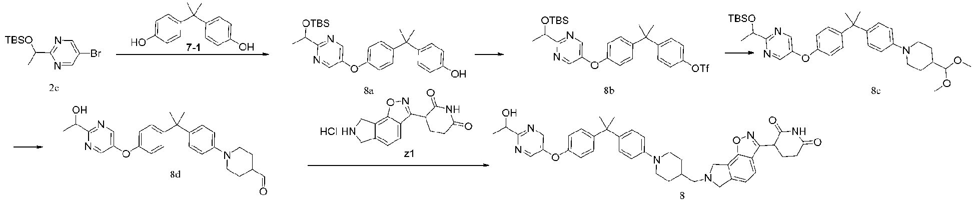

### Step 1: Preparation of compound 8a

Intermediate **2c** (3.50 g), intermediate 7-1 (4.03 g), copper(I) iodide (1.05 g), 2-pyridinecarboxylic acid (0.68 g), potassium phosphate (7.02 g), and DMSO (70 mL) were mixed, and the mixture was reacted at 90 °C for 4 h under N₂ atmosphere. After the reaction was completed, the reaction solution was diluted with water and EA. The organic phase was separated, washed with saturated brine, concentrated, and then purified by silica gel column chromatography to give compound **8a** (2.8 g). MS (ESI, [M+H]⁺) m/z: 465.3.

### Step 2: Preparation of compound 8b

Intermediate **8a** (2.7 g), pyridine (0.76 g), DMAP (0.04 g), and DCM (50 mL) were added to a reaction flask in sequence, and the mixture was cooled to 0 °C. Trifluoromethanesulfonic anhydride (0.81 g) was slowly added, and the mixture was reacted at 0 °C for 2 h. After the reaction was completed, water was added to the reaction solution to quench the reaction. The organic phase was separated, washed with saturated brine, concentrated, and then purified by silica gel column chromatography to give compound **8b** (2.2 g). MS (ESI, [M+H]⁺) m/z: 597.3.

### Step 3: Preparation of compound 8c

Intermediate **8b** (600 mg), 4-(dimethoxymethyl)-piperidine (400 mg), tris(dibenzylideneacetone)dipalladium(0) (90 mg), 2-dicyclohexylphosphonium-2',6'-diisopropoxy-1,1'-biphenyl (500 mg), cesium carbonate (660 mg), and 1,4-dioxane (15 mL) were mixed, and the mixture was reacted at 110 °C for 2 h under N₂ atmosphere. After the reaction was completed, the reaction solution was purified by silica gel column chromatography to give compound **8c** (420 mg). MS (ESI, [M+H]⁺) m/z: 606.4.

### Step 4: Preparation of compound 8d

Intermediate **8c** (400 mg), formic acid (4 mL), and water (4 mL) were mixed, and the mixture was reacted at 80 °C for 2 h. After the reaction was completed, the reaction solution was diluted with water and EA. The organic phase was separated, washed with saturated brine, concentrated, and then purified by silica gel column chromatography to give compound **8d** (320 mg). MS (ESI, [M+H]⁺) m/z: 446.1.

### Step 5: Preparation of compound 8

Intermediate **8d** (100 mg), intermediate **z1** (80 mg), sodium acetate (40 mg), 1,2-dichloroethane (10 mL), and isopropanol (2 mL) were mixed, and the mixture was stirred and reacted at room temperature for 10 min. Sodium triacetoxyborohydride (100 mg) was then added, and the mixture was reacted at room temperature for 1 h. After the reaction was completed, a saturated sodium bicarbonate solution was added to quench the reaction, and the resulting mixture was extracted with dichloromethane, washed with saturated brine, concentrated, and then purified by silica gel column chromatography to give compound **8** (32 mg). MS (ESI, [M+H]⁺) m/z: 701.3. ¹H NMR (500 MHz, DMSO) δ 11.09 (s, 1H), 8.57 (s, 2H), 7.71 (d, *J* = 8.0 Hz, 1H), 7.28 (dd, *J* = 24.5, 8.2 Hz, 3H), 7.03 (dd, *J =* 23.5, 8.3 Hz, 4H), 6.86 (d, *J* = 8.4 Hz, 2H), 5.21 (d, *J =* 5.5 Hz, 1H), 4.79 (p, *J =* 6.3 Hz, 1H), 4.60 (dd, *J =* 11.9, 4.9 Hz, 1H), 4.15 (s, 2H), 4.04 (s, 2H), 3.65 *(d, J =* 11.8 Hz, 2H), 2.77 (ddd, *J =* 17.3, 11.9, 5.3 Hz, 1H), 2.70-2.58 (m, 5H), 2.53 (d, *J* = 10.1 Hz, 1H), 2.26-2.15 (m, 1H), 1.87 (d, *J* = 12.7 Hz, 2H), 1.70 (s, 1H), 1.60 (s, 6H), 1.41 (d, *J* = 6.6 Hz, 3H), 1.33-1.22 (m, 2H).

### Example 9: Synthesis of Compound 9

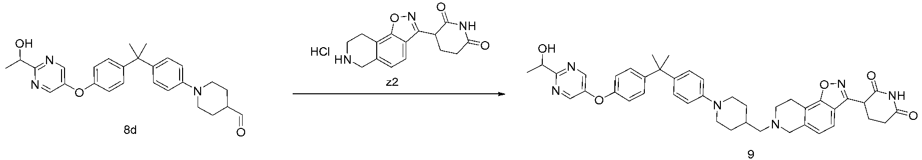

### Step 1: Preparation of compound 9

Referring to the method of step 5 in Example **8,** intermediate **z1** was replaced with intermediate **z2** for synthesis to give compound **9** (70 mg). MS (ESI, [M+H]⁺) m/z: 715.1. ¹H NMR (500 MHz, DMSO) δ 11.07 (s, 1H), 8.57 (s, 2H), 7.57 (d, *J* = 8.1 Hz, 1H), 7.29-7.22 (m, 2H), 7.10 (d, *J* = 8.2 Hz, 1H), 7.08-6.98 (m, 4H), 6.84 (dd, *J* = 9.2, 2.6 Hz, 2H), 5.20 (d, *J* = 5.5 Hz, 1H), 4.79 (p, *J* = 6.4 Hz, 1H), 4.55 (dd, *J* = 11.8, 5.0 Hz, 1H), 3.70 (s, 2H), 3.63 (d, *J* = 12.3 Hz, 2H), 2.99 (t, *J =* 5.9 Hz, 2H), 2.77 (dh, *J =* 12.5, 5.4 Hz, 3H), 2.67-2.57 (m, 3H), 2.46 (dd, *J* = 12.2, 4.4 Hz, 1H), 2.40 (d, *J* = 6.8 Hz, 2H), 2.18 (dq, *J* = 13.7, 4.7 Hz, 1H), 1.81 (t, *J* = 11.8 Hz, 3H), 1.59 (s, 6H), 1.41 (d, *J* = 6.6 Hz, 3H), 1.29-1.22 (m, 2H).

### Example 10: Synthesis of Compound 10

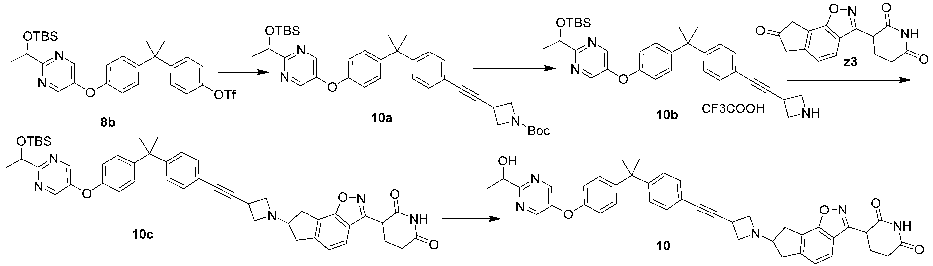

### Step 1: Preparation of intermediate 10a

Intermediate **8b** (350 mg), tert-butyl 3-ethynyl-1-azetidinecarboxylate (90 mg), bis(triphenylphosphine)palladium(II) dichloride (40 mg), copper(I) iodide (20 mg), tetrabutylammonium iodide (240 mg), triethylamine (5 mL), and DMF (5 mL) were mixed, and the mixture was heated to 100 °C and reacted for 3 h under N₂ atmosphere. After the reaction was completed, the reaction solution was poured into water, and the resulting mixture was extracted with ethyl acetate and purified by silica gel column chromatography to give intermediate **10a** (330 mg). MS (ESI) *m*/*z* [M+H]⁺: 628.3.

### Step 2: Preparation of intermediate 10b

Intermediate **10a** (330 mg), DCM (5 mL), and trifluoroacetic acid (2 mL) were mixed, and the mixture was reacted at room temperature until the reaction was completed. The reaction solution was adjusted to about pH 7-8 with a saturated aqueous sodium bicarbonate solution, extracted with ethyl acetate, and purified by silica gel column chromatography to give intermediate **10b** (170 mg). MS (ESI) *m*/*z* [M+H]⁺: 528.2.

### Step 3: Preparation of intermediate 10c

Intermediate **10b** (170 mg), intermediate **z3** (90 mg), and DCE/isopropanol (v:v = 5:1, 10 mL) were mixed, and sodium triacetoxyborohydride (150 mg) was then added. The mixture was reacted at room temperature until the reaction was completed. The reaction solution was concentrated and purified by silica gel column chromatography to give intermediate **10c** (130 mg). MS (ESI) *m*/*z* [M+H]⁺: 796.4.

### Step 4: Preparation of compound 10

Intermediate **10c** (130 mg), water (2 mL), and formic acid (2 mL) were mixed, and the mixture was reacted at room temperature until the reaction was completed. The reaction solution was purified by silica gel column chromatography to give compound **10** (70 mg). MS (ESI) *m*/*z* [M+H]⁺: 682.1. ¹H NMR (500 MHz, DMSO) δ 11.07 (s, 1H), 8.58 (s, 2H), 7.61 (dd, *J* 8.1, 3.1 Hz, 1H), 7.32 (d, *J =* 8.1 Hz, 2H), 7.27-7.23 (m, 3H), 7.20 (d, *J =* 8.3 Hz, 2H), 7.04-7.00 (m, 2H), 5.21 (d, *J =* 5.6 Hz, 1H), 4.79 (p, *J =* 6.4 Hz, 1H), 4.56 (dd, *J* = 11.8, 5.0 Hz, 1H), 3.64-3.56 (m, 2H), 3.35 (q, *J* = 7.7 Hz, 2H), 3.16-3.03 (m, 4H), 2.84 (dd, *J* = 16.2, 3.1 Hz, 1H), 2.81-2.72 (m, 2H), 2.64-2.57 (m, 1H), 2.49-2.43 (m, 1H), 2.18 (dp, J = 13.3, 4.4 Hz, 1H), 1.62 (s, 6H), 1.41 (d, *J =* 6.5 Hz, 3H).

### Example 11: Synthesis of Compound 11

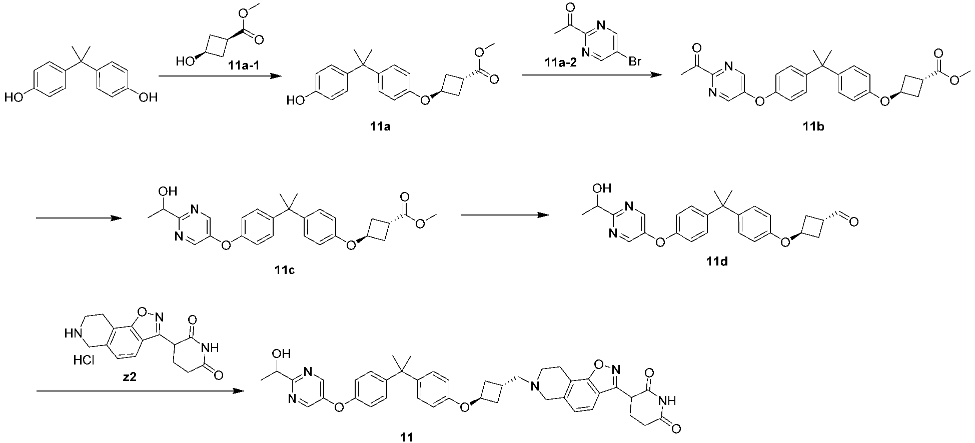

### Step 1: Preparation of intermediate 11a

Bisphenol A (10 g), intermediate **11a-1** (5.7 g), triphenylphosphine (17.23 g), and dioxane (150 mL) were added to a three-necked flask in sequence. DEAD (12.78 mL) was slowly added under nitrogen atmosphere in an ice bath. After the addition was completed, the mixture was heated to 80 °C and reacted. After the reaction was completed, the reaction solution was cooled to room temperature, concentrated under reduced pressure, and purified by column chromatography to give intermediate **11a** (5.5 g). MS (ESI, [M+H]⁺) *m*/*z:* 341.25.

### Step 2: Preparation of intermediate 11b

Intermediate **11a** (1.5 g), intermediate **11a-2** (0.846 g), potassium carbonate (1.745 g), and DMF (15 mL) were added to a single-necked flask in sequence, and the mixture was heated to 80 °C and reacted under N₂ atmosphere. After the reaction was completed, the system was extracted with ethyl acetate and water. The organic phase was separated, washed with saturated brine, dried over anhydrous sodium sulfate, filtered, and concentrated under reduced pressure, and the residue was purified by column chromatography to give intermediate **11b** (0.8 g). MS (ESI, [M+H]⁺) *m*/*z:* 461.28.

### Step 3: Preparation of intermediate 11c

Intermediate **11b** (0.8 g), methanol (5 mL), and sodium borohydride (0.032 g) were added to a single-necked flask in sequence, and the mixture was reacted at room temperature. After the reaction was completed, the reaction solution was slowly poured into a saturated aqueous ammonium chloride solution. The aqueous phase was extracted with dichloromethane. The organic phase was separated, washed with saturated brine, dried over anhydrous sodium sulfate, filtered, and concentrated under reduced pressure to give intermediate **11c** (0.79 g). MS (ESI, [M+H]⁺) *m*/*z:* 463.35.

### Steps 4-5: Preparation of compound 11

Referring to the reactions of steps 9-10 in Example 17, **17h** was replaced with **11c** to finally give compound **11** (60 mg). Compound **11d:** MS (ESI, [M+H]⁺) *m*/*z:* 433.12.

Compound **11:** MS (ESI, [M+H]⁺) *m*/*z:* 702.33. ¹H NMR (500 MHz, DMSO-*d*₆) *δ* 11.07 (s, 1H), 8.57 (s, 2H), 7.57 (d, *J* = 8.2 Hz, 1H), 7.31-7.21 (m, 2H), 7.10 (dd, *J* = 8.7, 6.9 Hz, 3H), 7.06-6.96 (m, 2H), 6.79-6.69 (m, 2H), 5.21 (d, *J* = 5.6 Hz, 1H), 4.79 (p, *J =* 6.3 Hz, 2H), 4.55 (dd, *J =* 11.9, 5.0 Hz, 1H), 3.70 (s, 2H), 2.99 (t, *J =* 5.7 Hz, 2H), 2.87-2.71 (m, 3H), 2.71-2.56 (m, 4H), 2.46 (dd, *J* = 12.1, 4.4 Hz, 1H), 2.34-2.12 (m, 5H), 1.61 (s, 6H), 1.41 (d, *J* = 6.6 Hz, 3H).

### Example 12: Synthesis of Compound 12

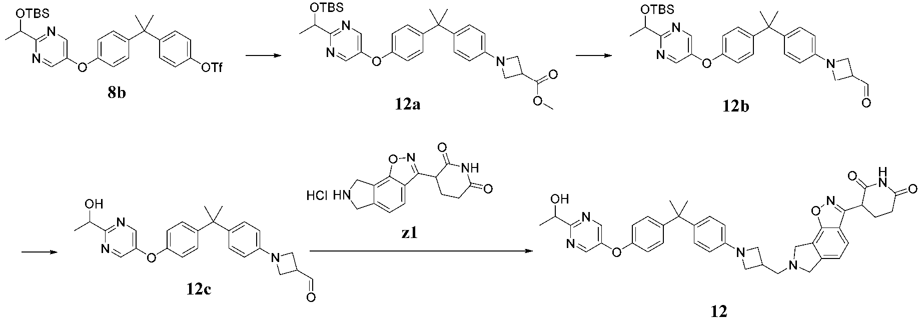

### Step 1: Preparation of compound 12a

Intermediate **8b** (2.60 g), methyl 3-carboxylate azetidine hydrochloride (3.01 g), Pd₂(dba)₃ (0.40 g), RuPhos (0.31 g), cesium carbonate (2.84 g), and 1,4-dioxane (26 mL) were added to a reaction flask in sequence, and the mixture was reacted at 110 °C for 2 h under N₂ atmosphere. After the reaction was completed, the reaction solution was purified by silica gel column chromatography to give compound **12a** (1.6 g). MS (ESI, [M+H]⁺) m/z: 562.21.

### Step 2: Preparation of compound 12b

Referring to the reaction of step 9 in Example 17, compound **17h** was replaced with compound **12a** to give compound **12b** (1.8 g). MS (ESI, [M+H]⁺) m/z: 532.33.

### Step 3: Preparation of compound 12c

Referring to the reaction of step 6 in Example 17, compound **17e** was replaced with compound **12b** to give compound **12c** (1.1 g). MS (ESI, [M+H]⁺) m/z: 418.31.

### Step 4: Preparation of compound 12

Referring to the reaction of step 5 in Example 8, compound 8d was replaced with compound 12c to give compound 12 (0.32 g). MS (ESI, [M+H]⁺) m/z: 673.19. ¹H NMR (500 MHz, DMSO) δ 11.07 (s, 1H), 8.57 (s, 2H), 7.71 (d, J = 8.0 Hz, 1H), 7.30 (d, J = 8.1 Hz, 1H), 7.27-7.22 (m, 2H), 7.06-6.98 (m, 4H), 6.37 (d, J = 8.3 Hz, 2H), 5.19 (d, J = 5.5 Hz, 1H), 4.79 (p, J = 6.3 Hz, 1H), 4.60 (dd, J = 11.8, 5.0 Hz, 1H), 4.17 (s, 2H), 4.06 (s, 2H), 3.95 (t, J = 7.1 Hz, 2H), 3.51 (t, J = 6.1 Hz, 2H), 3.00 (q, J = 7.1 Hz, 3H), 2.77 (ddd, J = 17.2, 11.9, 5.3 Hz, 1H), 2.61 (dt, J = 17.3, 4.2 Hz, 1H), 2.56-2.52 (m, 1H), 2.25-2.16 (m, 1H), 1.59 (s, 6H), 1.41 (d, J = 6.5 Hz, 3H).

### Examples 13-16: Synthesis of Compounds 13-16

Referring to the synthetic method for compound **12,** the following compounds were synthesized by using compound **12c** as a starting material and replacing **z1** with the following intermediates:

| **Examples** | **Intermediates** | **Compound structure** |
|---|---|---|
| **13** | 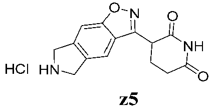 | 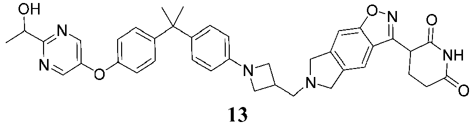 |
| **14** | 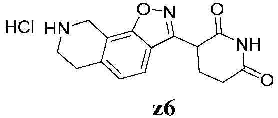 | 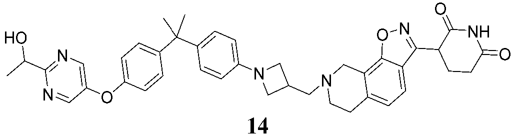 |
| **15** | 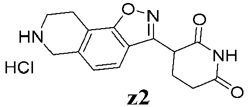 | 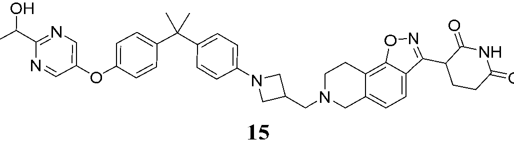 |
| **16** | 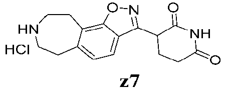 | 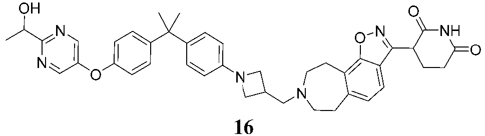 |

| **Examples** | **Data analysis** |
|---|---|
| **13** | MS(ESI, [M+H]⁺) m/z: 673.44 |
| | ¹H NMR (500 MHz, DMSO) δ 11.06 (s, 1H), 8.56 (s, 2H), 7.62 (d, J = 21.8 Hz, 2H), 7.24 (d, J = 8.6 Hz, 2H), 7.06 - 6.97 (m, 4H), 6.35 (d, J = 8.2 Hz, 2H), 5.19 (d, J = 5.5 Hz, 1H), 4.79 (p, J = 6.4 Hz, 1H), 4.55 (dd, J = 11.9, 5.0 Hz, 1H), 3.99 - 3.87 (m, 6H), 3.49 (dd, J = 7.1, 4.2 Hz, 2H), 2.95 (s, 3H), 2.77 (ddd, J = 17.2, 11.9, 5.3 Hz, 1H), 2.60 (dt, J = 17.3, 4.3 Hz, 1H), 2.47 (dd, J = 12.0, 4.2 Hz, 1H), 2.23 - 2.15 (m, 1H), 1.59 (s, 6H), 1.41 (d, J = 6.6 Hz, 3H). |
| **14** | MS(ESI, [M+H]⁺) m/z: 687.21 |
| | ¹H NMR (500 MHz, DMSO) δ 11.06 (s, 1H), 8.56 (s, 2H), 7.59 (d, J = 8.1 Hz, 1H), 7.28 - 7.21 (m, 2H), 7.14 (d, J = 8.2 Hz, 1H), 7.01 (dd, J = 11.0, 8.5 Hz, 4H), 6.36 (d, J = 8.2 Hz, 2H), 5.19 (d, J = 5.5 Hz, 1H), 4.79 (p, J = 6.3 Hz, 1H), 4.56 (dd, J = 11.9, 5.0 Hz, 1H), 3.93 (t, J = 7.3 Hz, 2H), 3.82 (s, 2H), 3.49 (t, J = 6.4 Hz, 2H), 3.05 (p, J = 7.1 Hz, 1H), 2.95 (t, J = 5.8 Hz, 2H), 2.84 (d, J = 7.4 Hz, 2H), 2.76 (dt, J = 17.5, 5.4 Hz, 3H), 2.60 (dt, J = 17.3, 4.2 Hz, 1H), 2.46 (dd, J = 12.0, 4.4 Hz, 1H), 2.19 (dt, J = 13.4, 4.8 Hz, 1H), 1.59 (s, 6H), 1.41 (d, J = 6.5 Hz, 3H). |
| **15** | MS(ESI, [M+H]⁺) m/z: 687.14 |
| | ¹H NMR (500 MHz, DMSO) δ 11.07 (s, 1H), 8.56 (s, 2H), 7.58 (d, J = 8.1 Hz, 1H), 7.24 (d, J = 8.4 Hz, 2H), 7.10 (d, J = 8.2 Hz, 1H), 7.05 - 6.97 (m, 4H), 6.35 (d, J = 8.2 Hz, 2H), 5.21 (d, J = 5.6 Hz, 1H), 4.79 (p, J = 6.4 Hz, 1H), 4.55 (dd, J = 11.9, 5.0 Hz, 1H), 3.92 (t, J = 7.4 Hz, 2H), 3.73 (s, 2H), 3.47 (t, J = 6.4 Hz, 2H), 3.07 - 2.96 (m, 3H), 2.86 - 2.72 (m, 5H), 2.60 (dt, J = 17.3, 4.3 Hz, 1H), 2.48 - 2.44 (m, 1H), 2.18 (dq, J = 13.8, 4.8 Hz, 1H), 1.58 (s, 6H), 1.41 (d, J = 6.6 Hz, 3H). |
| **16** | MS(ESI, [M+H]⁺) m/z: 701.32 |
| | ¹H NMR (500 MHz, DMSO) δ 11.06 (s, 1H), 8.56 (s, 2H), 7.54 (d, J = 8.0 Hz, 1H), 7.29 - 7.21 (m, 2H), 7.18 (d, J = 8.1 Hz, 1H), 7.01 (dd, J = 10.4, 8.0 Hz, 4H), 6.35 (d, J = 8.5 Hz, 2H), 5.19 (d, J = 5.5 Hz, 1H), 4.79 (p, J = 6.4 Hz, 1H), 4.54 (dd, J = 11.9, 5.0 Hz, 1H), 3.89 (t, J = 7.4 Hz, 2H), 3.44 (t, J = 6.4 Hz, 2H), 3.18 - 3.11 (m, 2H), 3.03 (d, J = 6.3 Hz, 2H), 2.99 - 2.89 (m, 1H), 2.77 (tq, J = 11.8, 5.5 Hz, 3H), 2.65 (ddt, J = 25.8, 17.3, 9.0 Hz, 5H), 2.46 (dd, J = 12.1, 4.3 Hz, 1H), 2.18 (dq, J = 13.4, 4.7 Hz, 1H), 1.59 (s, 6H), 1.41 (d, J = 6.5 Hz, 3H). |

### Example 17: Synthesis of Compound 17

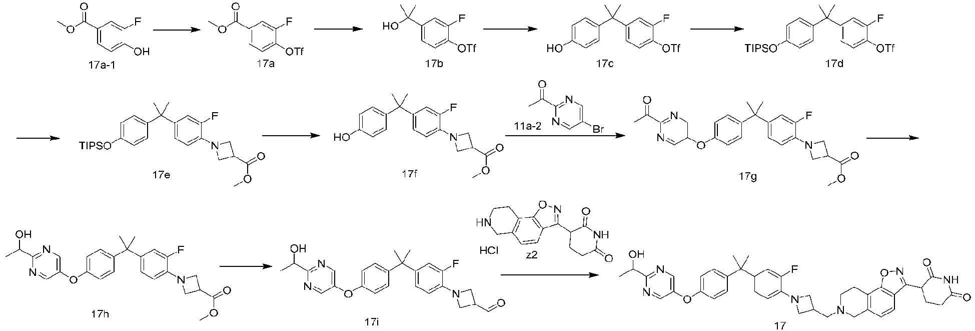

### Step 1: Preparation of intermediate 17a

**17a-1** (10 g), DCM (100 mL), pyridine (13 mL), and DMAP (0.7 g) were added to a single-necked flask in sequence, and trifluoromethanesulfonic anhydride (10 mL) was added slowly in an ice bath. The mixture was reacted at room temperature. After the reaction was completed, the system was extracted with DCM and water, and the organic phase was separated. The mixture was washed with saturated brine, dried over anhydrous sodium sulfate, filtered, and concentrated under reduced pressure to give intermediate **17a** (16.5 g). MS (ESI, [M+H]⁺) *m*/*z:* 303.21.

### Step 2: Preparation of intermediate 17b

**17a** (16.5 g) and toluene (200 mL) were added to a three-necked flask in sequence, and methylmagnesium bromide (45 mL) was slowly added under nitrogen atmosphere in an ice bath. The mixture was reacted at room temperature. After the reaction was completed, the reaction solution was slowly poured into an aqueous ammonium chloride solution, the aqueous phase was extracted with ethyl acetate, the organic phase was separated, washed with saturated brine, dried over anhydrous sodium sulfate, filtered, and concentrated under reduced pressure, and the residue was purified by silica gel column chromatography to give intermediate **17b** (9.1 g). MS (ESI, [M+H]⁺) *m*/*z:* 303.13.

### Step 3: Preparation of intermediate 17c

**17b** (9.1 g), phenol (5.6 g), 1,2-dichloroethane (100 mL), and trifluoromethylsulfonic acid (5.2 mL) were added to a single-necked flask in sequence, and the mixture was reacted at room temperature. After the reaction was completed, the system was extracted with DCM and water. The organic phase was separated, concentrated under reduced pressure, and purified by silica gel column chromatography to give intermediate **17c** (9.5 g). MS (ESI, [M+H]⁺) *m*/*z:* 379.24.

### Step 4: Preparation of intermediate 17d

**17c** (2 g), DMF (20 mL), imidazole (1 g), and TIPS-Cl (1.5 g) were added to a single-necked flask in sequence, and the mixture was heated to 50 °C and reacted. After the reaction was completed, the system was extracted with ethyl acetate and water. The organic phase was separated, concentrated under reduced pressure, and purified by silica gel column chromatography to give intermediate **17d** (3.9 g). MS (ESI, [M+H]⁺) *m*/*z*: 535.32.

### Step 5: Preparation of intermediate 17e

**17d** (2 g), methyl azetidine-3-carboxylate hydrochloride (1.05 g), Pd₂(dba)₃ (0.3 g), Xphos (0.3 g), Cs₂CO₃ (4.8 g), and 1,4-dioxane (20 mL) were added to a single-necked flask in sequence, and the mixture was heated to 120 °C and reacted under N₂ atmosphere. After the reaction was completed, the mixture was concentrated by evaporation under reduced pressure to remove the solvent, and the residue was purified by silica gel column chromatography to give intermediate **17e** (0.85 g). MS (ESI, [M+H]⁺) *m*/*z: 500.25.*

### Step 6: Preparation of intermediate 17f

**17e** (0.85 g), tetrahydrofuran (10 mL), and TBAF (5 mL) were added to a single-necked flask in sequence, and the mixture was reacted at room temperature. After the reaction was completed, the system was extracted with ethyl acetate and water. The organic phase was separated and concentrated by evaporation under reduced pressure to remove the solvent, and the residue was purified by silica gel column chromatography to give intermediate **17f** (0.5 g). MS (ESI, [M+H]⁺) *m*/*z:* 344.33.

### Step 7: Preparation of intermediate 17g

**17f** (0.5 g), **11a-2** (0.33 g), potassium carbonate (1.5 g), and DMF (15 mL) were added to a single-necked flask in sequence, and the mixture was heated to 80 °C and reacted under N₂ atmosphere. After the reaction was completed, the system was extracted with ethyl acetate and water. The organic phase was separated, washed with saturated brine, dried over anhydrous sodium sulfate, filtered, and concentrated by evaporation under reduced pressure to remove the solvent, and the residue was purified by column chromatography to give intermediate **17g** (0.3 g). MS (ESI, [M+H]⁺) *m*/*z:* 464.51.

### Step 8: Preparation of intermediate 17h

**17g** (0.3 g), methanol (5 mL), and sodium borohydride (12 mg) were added to a single-necked flask in sequence, and the mixture was reacted at room temperature. After the reaction was completed, the reaction solution was slowly poured into a saturated aqueous ammonium chloride solution. The aqueous phase was extracted with dichloromethane. The organic phase was separated, washed with saturated brine, dried over anhydrous sodium sulfate, filtered, and concentrated by evaporation under reduced pressure to remove the solvent, thus giving intermediate **17h** (0.25 g). MS (ESI, [M+H]⁺) *m*/*z:* 466.32.

### Step 9: Preparation of intermediate 17i

**17h** (0.25 g) and dichloromethane (5 mL) were added to a three-necked flask in sequence, and then DIBAL-H (0.55 mL) was added dropwise at -78 °C under nitrogen atmosphere. The mixture was reacted at -78 °C. After the reaction was completed, the reaction solution was slowly poured into a saturated aqueous ammonium chloride solution. The aqueous phase was extracted with dichloromethane. The organic phase was separated, washed with saturated brine, dried over anhydrous sodium sulfate, filtered, and concentrated by evaporation under reduced pressure to remove the solvent, thus giving intermediate **17i** (0.18 g). MS (ESI, [M+H]⁺) *m*/*z:* 436.32.

### Step 10: Preparation of compound 17

Intermediate **17i** (180 mg), intermediate **z2** (140 mg), sodium acetate (35 mg), DCE/iPrOH = 5:1 (5 mL, v:v), and sodium triacetoxyborohydride (260 mg) were added to a single-necked flask in sequence, and the mixture was reacted at room temperature. After the reaction was completed, the mixture was purified by silica gel column chromatography to give compound 17 (75 mg). MS (ESI, [M+H]⁺) *m*/*z:* 705.41. ¹H NMR (500 MHz, DMSO-*d*₆) δ 11.07 (s, 1H), 8.58 (s, 2H), 7.58 (d, *J =* 8.2 Hz, 1H), 7.25 (d, *J =* 8.7 Hz, 2H), 7.06 (dd, *J* = 41.9, 8.5 Hz, 3H), 6.90-6.79 (m, 2H), 6.47 (t, *J* = 9.1 Hz, 1H), 5.21 (d, *J* = 5.5 Hz, 1H), 4.79 (p, *J* = 6.4 Hz, 1H), 4.56 (dd, *J* = 11.9, 5.0 Hz, 1H), 4.15-3.95 (m, 2H), 3.72 (s, 2H), 3.58 (t, *J* = 6.6 Hz, 2H), 3.00 (q, *J* = 10.5 Hz, 3H), 2.89-2.69 (m, 5H), 2.60 (dt, *J=* 17.3, 4.3 Hz, 1H), 2.18 (dq, *J* = 14.1, 5.0 Hz, 1H), 1.59 (s, 6H), 1.41 (d, *J* = 6.6 Hz, 3H).

### Example 18: Synthesis of Compound 18

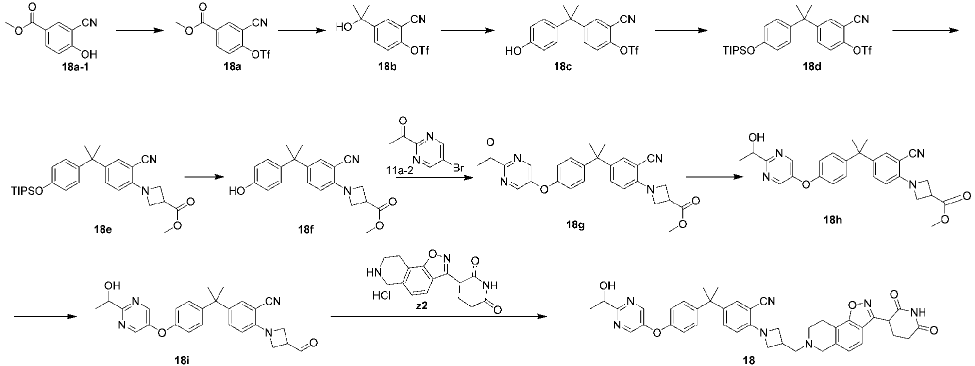

Referring to the reactions of steps 1-10 in Example 17b, **17a-1** was replaced with **18a-1** to finally give compound **18.**

**18a:** MS (ESI, [M+H]⁺) *m*/*z*: 310.05.

**18b:** MS (ESI, [M+H]⁺) *m*/*z*: 310.12.

**18c:** MS (ESI, [M+H]⁺) *m*/*z:* 386.12.

**18d:** MS (ESI, [M+H]⁺) *m*/*z:* 542.31.

**18e:** MS (ESI, [M+H]⁺) *m*/*z:* 507.43.

**18f:** MS (ESI, [M+H]⁺) *m*/*z*: 351.33.

**18g:** MS (ESI, [M+H]⁺) *m*/*z:* 471.21.

**18h:** MS (ESI, [M+H]⁺) *m*/*z:* 473.24.

**18i:** MS(ESI, [M+H]⁺) *m*/*z:* 443.31.

Compound **18:** MS (ESI, [M+H]⁺) *m*/*z:* 712.32. ¹H NMR (500 MHz, DMSO-*d*₆) *δ* 11.07 (s, 1H), 8.58 (s, 2H), 7.58 (d, *J=* 8.1 Hz, 1H), 7.33 (d, *J* = 2.3 Hz, 1H), 7.25 (d, *J* = 8.4 Hz, 2H), 7.21 (dd, *J =* 8.9, 2.3 Hz, 1H), 7.10 (d, *J* = 8.2 Hz, 1H), 7.02 (d, *J =* 8.3 Hz, 2H), 6.52 (d, *J =* 8.9 Hz, 1H), 5.21 (d, *J =* 5.5 Hz, 1H), 4.80 (p, *J* = 6.3 Hz, 1H), 4.56 (dd, *J* = 11.9, 5.0 Hz, 1H), 4.22 (t, *J =* 7.8 Hz, 2H), 3.85-3.68 (m, 4H), 3.03 (dt, *J* = 33.5, 6.6 Hz, 3H), 2.78 (ddd, *J* = 29.3, 11.4, 6.2 Hz, 5H), 2.60 (dt, *J* = 17.4, 4.2 Hz, 1H), 2.46 (dd, *J* = 12.1, 4.4 Hz, 1H), 2.19 (dq, *J* = 13.7, 4.8 Hz, 1H), 1.59 (s, 6H), 1.41 (d, *J* = 6.5 Hz, 3H).

### Example 19: Synthesis of Compound 19

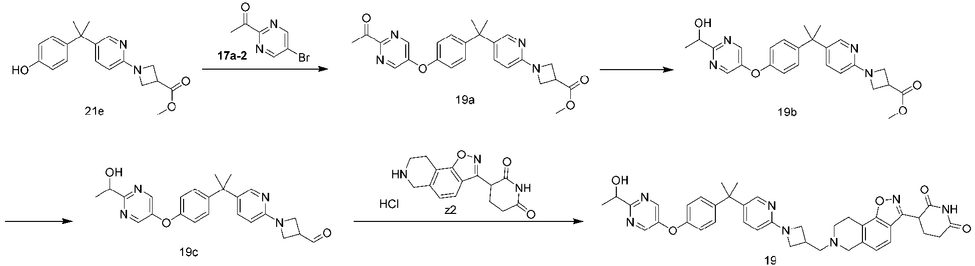

Referring to the reactions of steps 7-10 in Example 17, intermediate **17f** was replaced with intermediate **21e** to finally give compound **19.**

**19a:** MS (ESI, [M+H]⁺) *m*/*z:* 447.31.

**19b:** MS (ESI, [M+H]⁺) *m*/*z:* 449.23.

**19c:** MS (ESI, [M+H]⁺) *m*/*z:* 419.34.

Compound **19:** MS (ESI, [M+H]⁺) *m*/*z:* 688.33. ¹H NMR (500 MHz, DMSO-*d*₆) *δ* 11.07 (s, 1H), 8.57 (s, 2H), 7.98 (d, J = 2.5 Hz, 1H), 7.58 (d, J = 8.1 Hz, 1H), 7.31 (dd, J = 8.6, 2.5 Hz, 1H), 7.29-7.23 (m, 2H), 7.10 (d, J = 8.3 Hz, 1H), 7.06-6.96 (m, 2H), 6.31 (d, J = 8.7 Hz, 1H), 5.21 (d, J = 5.5 Hz, 1H), 4.83-4.74 (m, 1H), 4.56 (dd, J = 11.9, 4.9 Hz, 1H), 4.03 (t, J = 7.9 Hz, 2H), 3.73 (s, 2H), 3.61 (dd, J = 7.9, 5.6 Hz, 2H), 3.01 (dt, J = 20.7, 6.5 Hz, 3H), 2.93-2.70 (m, 5H), 2.60 (dt, J = 17.3, 4.2 Hz, 1H), 2.46 (dd, J = 12.1, 4.4 Hz, 1H), 2.18 (dq, J = 13.6, 4.9 Hz, 1H), 1.61 (s, 6H), 1.41 (d, J = 6.5 Hz, 3H).

### Example 20: Synthesis of Compound 20

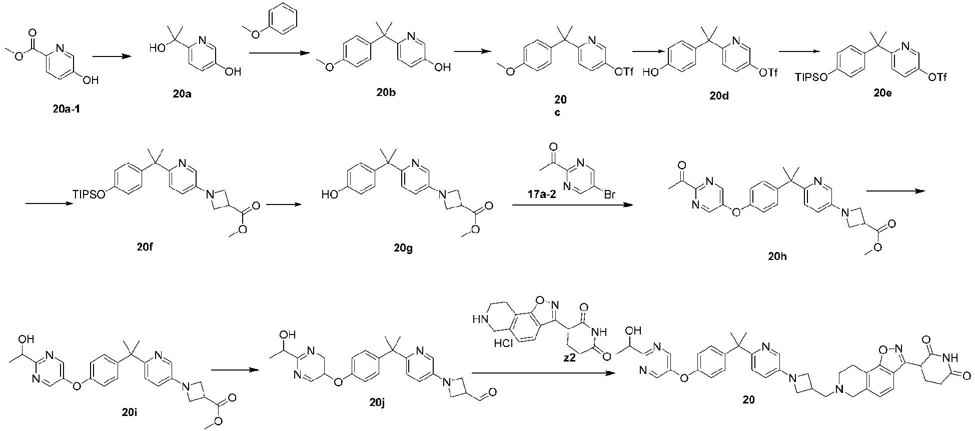

### Step 1: Preparation of intermediate 20a

Referring to the reaction of step 2 in Example 17, **17a** was replaced with intermediate **20a-1** to give **20a.**

MS (ESI, [M+H]⁺) *m*/*z:* 154.37.

### Step 2: Preparation of intermediate 20b

**20a** (18 g), anisole (50 mL), and trifluoromethylsulfonic acid (16 mL) were added to a single-necked flask in sequence, and the mixture was reacted at room temperature. After the reaction was completed, the system was extracted with dichloromethane and water. The organic phase was separated and purified by silica gel column chromatography to give intermediate **20b** (8.5 g).

MS (ESI, [M+H]⁺) *m*/*z:* 244.34.

### Step 3: Preparation of intermediate 20c

Referring to the reaction of step 1 in Example 17, **17a-1** was replaced with **20b** to give intermediate **20c** (9.3 g). MS (ESI, [M+H]⁺) *m*/*z:* 376.33.

### Step 4: Preparation of intermediate 20d

**20c** (9.3 g), dichloromethane (100 mL), and boron tribromide (2 M, 37 mL) were added to a single-necked flask in sequence, and the mixture was reacted at room temperature. After the reaction was completed, the system was extracted with dichloromethane and water. The organic phase was separated, dried over anhydrous sodium sulfate, filtered, and concentrated by evaporation under reduced pressure to remove the solvent, thus giving intermediate **20d** (7.8 g). MS (ESI, [M+H]⁺) *m*/*z:* 362.52.

Referring to the reactions of steps 4-10 in Example 17, **17c** was replaced with **20d** and Xphos of step 5 in Example 17 was replaced with BINAP to give compound 20.

**20e:** MS (ESI, [M+H]⁺) *m*/*z:* 518.32.

**20f:** MS (ESI, [M+H]⁺) *m*/*z:* 483.31.

**20g:** MS (ESI, [M+H]⁺) *m*/*z:* 327.25.

**20h:** MS (ESI, [M+H]⁺) *m*/*z:* 447.33.

**20i:** MS(ESI, [M+H]⁺) *m*/*z:* 449.32.

**20j:** MS(ESI, [M+H]⁺) *m*/*z:* 419.33.

Compound **20:** MS (ESI, [M+H]⁺) *m*/*z:* 688.50. ¹H NMR (500 MHz, DMSO-*d*₆) δ 11.07 (s, 1H), 8.64 (s, 2H), 8.40 (d, J = 2.9 Hz, 1H), 7.58 (d, J = 8.2 Hz, 1H), 7.44 (dd, J = 8.8, 3.0 Hz, 1H), 7.17 (d, J = 8.8 Hz, 1H), 7.09 (d, J = 8.3 Hz, 1H), 7.06 - 7.01 (m, 2H), 6.39 - 6.33 (m, 2H), 5.23 (d, J = 5.5 Hz, 1H), 4.85 - 4.77 (m, 1H), 4.55 (dd, J = 11.9, 5.0 Hz, 1H), 3.92 (t, J = 7.4 Hz, 2H), 3.72 (s, 2H), 3.47 (dd, J = 7.2, 5.6 Hz, 2H), 3.01 (dt, J = 17.2, 6.2 Hz, 3H), 2.85 - 2.73 (m, 5H), 2.60 (dt, J = 17.3, 4.2 Hz, 1H), 2.46 (dd, J = 12.1, 4.5 Hz, 1H), 2.23 - 2.15 (m, 1H), 1.64 (s, 6H), 1.41 (d, J = 6.5 Hz, 3H).

### Example 21: Synthesis of Compound 21

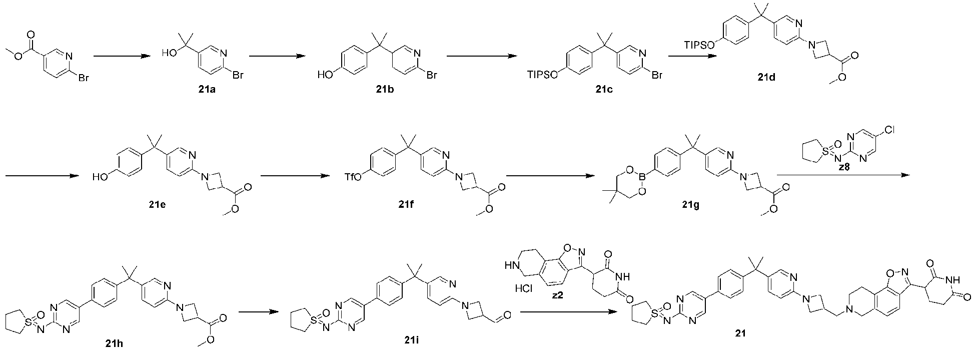

### Steps 1-5: Preparation of intermediate 21e

Referring to the reactions of steps 2-6 in Example 17, **17a** was replaced with methyl 6-bromonicotinate and Xphos of step 5 in Example 17 was replaced with BINAP to give **21e.**

**21a:** MS(ESI, [M+H]⁺) *m*/*z:* 216.05.

**21b:** MS(ESI, [M+H]⁺) *m*/*z:* 292.06.

**21c:** MS(ESI, [M+H]⁺) *m*/*z:* 448.45.

**21d:** MS(ESI, [M+H]⁺) *m*/*z:* 483.30.

**21e:** MS(ESI, [M+H]⁺) *m*/*z:* 327.22.

### Step 6: Preparation of intermediate 21f

Referring to the reaction of step 1 in Example 17, **17a-1** was replaced with **21e** to give intermediate **21f** (2.0 g). **21f:** MS(ESI, [M+H]⁺) *m*/*z:* 459.09.

### Step 7: Preparation of intermediate 21g

**21f** (2 g), bis(neopentyl glycolato)diboron (1.183 g), PdCl₂(dppf)-CH₂Cl₂ (0.713 g), potassium acetate (1.284 g), and 1,4-dioxane (30 mL) were added to a single-necked flask in sequence, and the mixture was heated to 100 °C and reacted under N² atmosphere. After the reaction was completed, the system was extracted with ethyl acetate and water. The organic phase was separated and purified by silica gel column chromatography to give intermediate **21g** (1.3 g). MS(ESI, [M+H]⁺) *m*/*z:* 423.31.

### Step 8: Preparation of intermediate 21h

**21g** (500 mg), intermediate **z8** (274 mg), Pd₂(dba)₃ (217 mg), Xphos (226 mg), Cs₂CO₃ (1157 mg), and 1,4-dioxane (5 mL) were added to a single-necked flask in sequence, and the mixture was heated to 100 °C and reacted under N² atmosphere. After the reaction was completed, the mixture was purified by silica gel column chromatography to give intermediate **21h** (0.59 g). MS(ESI, [M+H]⁺) *m*/*z:* 506.25.

### Steps 9-10: Preparation of compound 21

Referring to the reactions of steps 9-10 in Example 17, **17h** was replaced with **21h** to finally give compound **21** (100 mg). **21i:** MS (ESI, [M+H]⁺) *m*/*z:* 476.34. Compound **21:** MS (ESI, [M+H]⁺) *m*/*z:* 745.32. ¹H NMR (500 MHz, DMSO-*d*₆) *δ* 11.07 (s, 1H), 8.77 (s, 2H), 8.02 (d, *J* = 2.5 Hz, 1H), 7.58 (dd, *J* = 8.1, 5.8 Hz, 3H), 7.37 - 7.28 (m, 3H), 7.10 (d, *J* = 8.3 Hz, 1H), 6.31 (d, *J =* 8.7 Hz, 1H), 4.56 (dd, *J =* 11.9, 5.0 Hz, 1H), 4.03 (t, *J =* 7.9 Hz, 2H), 3.73 (s, 2H), 3.68 - 3.57 (m, 4H), 3.38 (dt, *J =* 14.1, 7.2 Hz, 2H), 3.01 (dt, *J =* 21.1, 6.5 Hz, 3H), 2.87 - 2.71 (m, 5H), 2.60 (dt, *J* = 17.3, 4.3 Hz, 1H), 2.46 (dd, *J =* 12.2, 4.3 Hz, 1H), 2.29 - 2.06 (m, 5H), 1.63 (s, 6H).

### Example 22: Synthesis of Compound 22

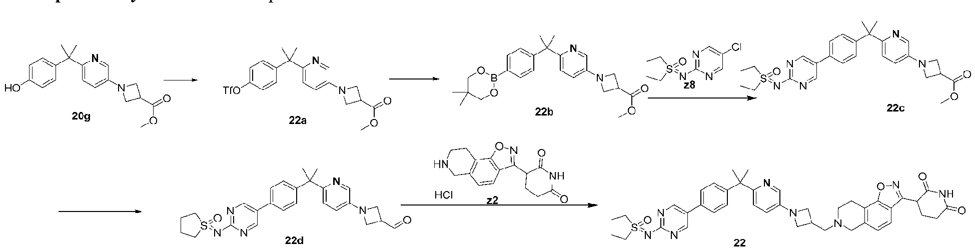

### Step 1: Preparation of intermediate 22a

Referring to the reaction of step 1 in Example 17, **17a-1** was replaced with **20g** to give intermediate **22a** (2.1 g). MS(ESI, [M+H]⁺) *m*/*z:* 459.09.

### Steps 2-5: Preparation of intermediate 22c

Referring to the reactions of steps 7-8 in Example 21, **21f** was replaced with **22a** to finally give compound 22 (83 mg). **22b:** MS(ESI, [M+H]⁺) *m*/*z:* 423.33.

**22c:** MS(ESI, [M+H]⁺) *m*/*z*: 506.31.

**22d:** MS(ESI, [M+H]⁺) *m*/*z:* 476.34.

Compound **22:** MS (ESI, [M+H]⁺) *m*/*z:* 745.32. ¹H NMR (500 MHz, DMSO) δ 11.07 (s, 1H), 8.75 (s, 2H), 7.76 (d, J = 2.9 Hz, 1H), 7.60 - 7.52 (m, 3H), 7.32 - 7.25 (m, 2H), 7.07 (dd, J = 24.7, 8.4 Hz, 2H), 6.76 (dd, J = 8.6, 2.9 Hz, 1H), 4.55 (dd, J = 11.9, 4.9 Hz, 1H), 3.99 (t, J = 7.5 Hz, 2H), 3.73 (s, 2H), 3.63 (dt, J = 13.6, 7.1 Hz, 2H), 3.56 (dd, J = 7.3, 5.6 Hz, 2H), 3.38 (dt, J = 14.1, 7.2 Hz, 2H), 3.08 (p, J = 6.9 Hz, 1H), 2.99 (t, J = 5.8 Hz, 2H), 2.87 - 2.72 (m, 5H), 2.60 (dt, J = 17.3, 4.3 Hz, 1H), 2.48 - 2.42 (m, 1H), 2.28 - 2.06 (m, 5H), 1.66 (s, 6H).

### Example 23: Synthesis of Compound 23

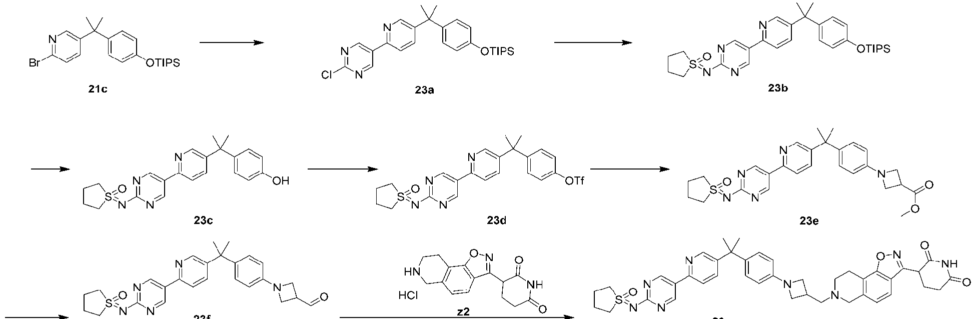

### Step 1: Preparation of intermediate 23a

Intermediate **21c** (6.5 g), 2-chloropyrimidine-5-boronic acid pinacol ester (5.23 g), PdCl₂(dppf)-CH₂Cl₂ (2.367 g), K₂CO₃ (6.01 g), 1,4-dioxane (60 mL), and water (12.00 mL) were added to a single-necked flask in sequence, and the mixture was heated to 85 °C and reacted under N₂ atmosphere. After the reaction was completed, the system was extracted with ethyl acetate and water. The organic phase was separated and purified by silica gel column chromatography to give intermediate **23a** (0.88 g). MS(ESI, [M+H]⁺) *m*/*z:* 482.31.

### Step 2: Preparation of intermediate 23b

**23a** (0.88 g), 1-iminotetrahydrothiophene 1-oxide (0.435 g), XPhos-Pd-G3 (0.154 g), Cs₂CO₃ (1.784 g), and 1,4-dioxane (10 mL) were added to a single-necked flask in sequence, and the mixture was heated to 100 °C and reacted under N₂ atmosphere. After the reaction was completed, the mixture was purified by silica gel column chromatography to give intermediate 23b (0.9 g). MS(ESI, [M+H]⁺) *m*/*z:* 565.25.

### Step 3: Preparation of intermediate 23c

Referring to the reaction of step 6 in Example 17, **17e** was replaced with **23b** to give intermediate **23c** (0.62 g). MS(ESI, [M+H]⁺) *m*/*z:* 409.21.

### Step 4: Preparation of intermediate 23d

Referring to the reaction of step 1 in Example 17, **17a-1** was replaced with **23c** to give intermediate **23d** (0.7 g). MS(ESI, [M+H]⁺) *m*/*z:* 541.08.

### Step 5: Preparation of intermediate 23e

Referring to the reaction of step 5 in Example 17, **17d** was replaced with **23d** to give intermediate **23e** (0.22 g). MS(ESI, [M+H]⁺) *m*/*z:* 506.40.

### Steps 6-7: Preparation of intermediate 23f

Referring to the reactions of steps 9-10 in Example 17, **17h** was replaced with **23e** to finally give compound **23** (15 mg). **23f:** MS(ESI, [M+H]⁺) *m*/*z*: 476.62.

Compound **23:** MS (ESI, [M+H]⁺) *m*/*z:* 745.33. ¹H NMR (500 MHz, DMSO-*d*₆) *δ* 11.07 (s, 1H), 9.07 (s, 2H), 8.50 (d, *J* = 2.5 Hz, 1H), 7.85 (d, *J* = 8.4 Hz, 1H), 7.65 (dd, *J =* 8.4, 2.4 Hz, 1H), 7.58 (d, *J =* 8.2 Hz, 1H), 7.09 (dd, *J =* 13.0, 8.3 Hz, 3H), 6.38 (d, *J* = 8.2 Hz, 2H), 4.56 (dd, *J =* 11.9, 5.0 Hz, 1H), 3.93 (t, *J =* 7.4 Hz, 2H), 3.73 (s, 2H), 3.63 (dt, *J =* 13.6, 6.9 Hz, 2H), 3.48 (t, *J* = 6.4 Hz, 2H), 3.40 (dt, *J =* 13.6, 6.8 Hz, 2H), 3.01 (d, *J =* 22.0 Hz, 3H), 2.87 - 2.71 (m, 5H), 2.60 (dd, *J =* 17.7, 4.5 Hz, 1H), 2.47 (s, 1H), 2.27 - 2.06 (m, 5H), 1.65 (s, 6H).

### Example 24: Synthesis of Compound 24

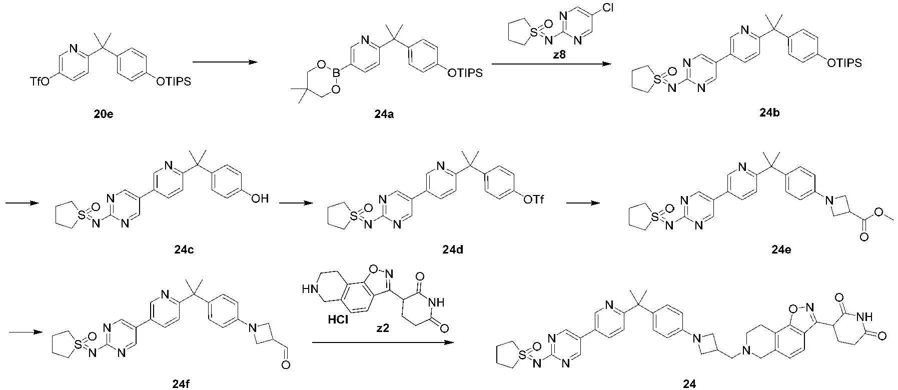

### Step 1: Preparation of intermediate 24a

**20e** (2.0 g), bis(neopentyl glycolato)diboron (2.9 g), potassium acetate (2.1 g), Pd(dppf)Cl₂ (1.1 g), and 1,4-dioxane (50 mL) were added to a reaction flask in sequence, and the mixture was reacted at 100 °C for 2 h. After the reaction was completed, the reaction solution was filtered and extracted with ethyl acetate. The organic phase was separated, washed with saturated brine, concentrated, and then purified by silica gel column chromatography to give intermediate **24a** (1.7 g). MS(ESI, [M+H]⁺) m/z: 482.31.

### Step 2: Preparation of compound 24b

Referring to the reaction of step 8 in Example 21, **21g** was replaced with **24a** to give compound **24b** (1.12 g). MS(ESI, [M+H]⁺) m/z: 565.31.

### Step 3: Preparation of intermediate 24c

Referring to the reaction of step 6 in Example 17, **17e** was replaced with **24b** to give compound **24c** (0.74 g). MS(ESI, [M+H]⁺) m/z: 409.19.

### Step 4: Preparation of intermediate 24d

Intermediate **24c** (0.48 g), 4-dimethylaminopyridine (17.7 mg), triethylamine (391 mg), N-phenyl-bis(trifluoromethanesulfonimide) (514 mg), and dichloromethane (25 mL) were added to a reaction flask in sequence, and the mixture was reacted at room temperature for 2 h. After the reaction was completed, water was added to the reaction solution. The organic phase was separated, washed with saturated brine, concentrated, and then purified by silica gel column chromatography to give compound **24d** (0.52 g). MS(ESI, [M+H]⁺) m/z: 541.12.

### Step 5: Preparation of intermediate 24e

Referring to the reaction of step 5 in Example 17, **17d** was replaced with **24d** to give compound **24e** (0.28 g). MS(ESI, [M+H]⁺) m/z: 506.21.

### Steps 6-7: Preparation of compound 24

Referring to the reactions of steps 9-10 in Example 17, **17h** was replaced with **24e** to finally give compound **24** (0.12 g). **24f:** MS(ESI, [M+H]⁺) m/z: 476.21.

Compound **24:** MS (ESI, [M+H]⁺) m/z: 745.18. ¹H NMR (500 MHz, DMSO) δ 11.07 (s, 1H), 8.86 - 8.79 (m, 3H), 7.96 (dd, *J =* 8.4, 2.5 Hz, 1H), 7.58 (d, *J =* 8.2 Hz, 1H), 7.20 (d, *J =* 8.3 Hz, 1H), 7.12 - 7.04 (m, 3H), 6.39 - 6.33 (m, 2H), 4.55 (dd, *J* = 11.9, 5.0 Hz, 1H), 3.92 (t, *J* = 7.4 Hz, 2H), 3.72 (s, 2H), 3.64 (dt, *J* = 13.6, 7.0 Hz, 2H), 3.47 (dd, *J =* 7.1, 5.6 Hz, 2H), 3.39 (dt, *J =* 13.6, 6.8 Hz, 2H), 3.01 (dt, *J =* 18.1, 6.5 Hz, 3H), 2.83 - 2.73 (m, 5H), 2.60 (dt, *J =* 17.3, 4.4 Hz, 1H), 2.49 - 2.43 (m, 1H), 2.29 - 2.08 (m, 5H), 1.66 (s, 6H).

Referring to the synthetic method for compound **22,** the following compounds were synthesized by using **22b** as a starting material and replacing **z8** with the structures shown in the table below:

| **Examples** | **Starting material** | **Compound structure** |
|---|---|---|
| **25** | 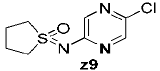 | 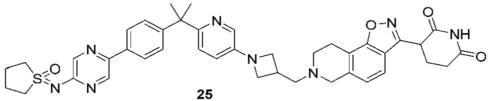 |
| **26** | 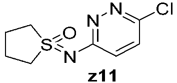 | 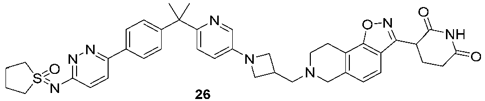 |
| **27** | 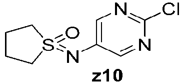 | 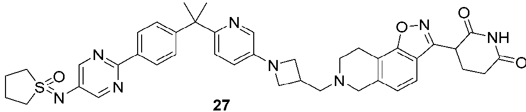 |
| **28** | 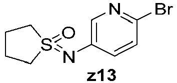 | 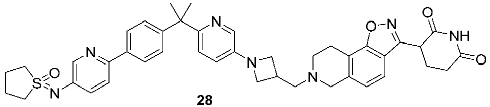 |
| **29** | 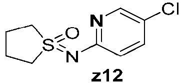 | 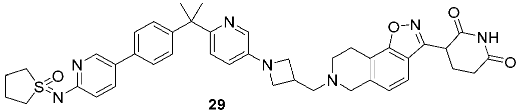 |
| **30** | 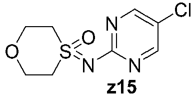 | 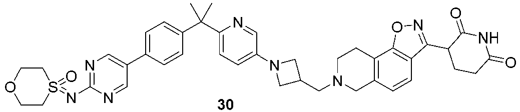 |
| **31** | 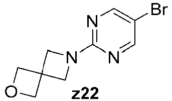 | 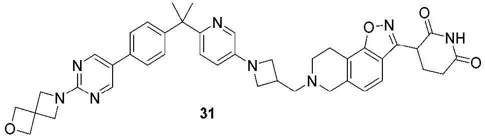 |
| **32** | 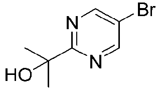 | 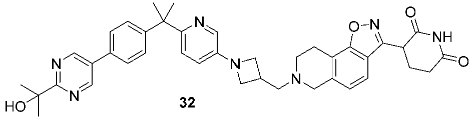 |
| **33** | 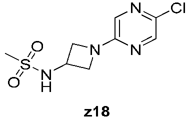 | 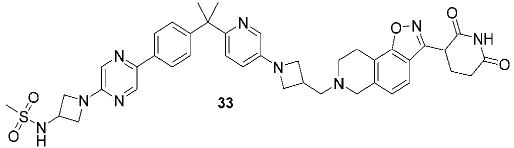 |

| **Examples** | **Data analysis** |
|---|---|
| **25** | MS(ESI, [M+H]⁺) *m*/*z:* 745.34 |
| | ¹H NMR (500 MHz, DMSO) δ 11.07 (s, 1H), 8.67 (d, J = 1.5 Hz, 1H), 8.14 (d, J = 1.5 Hz, 1H), 7.90 - 7.83 (m, 2H), 7.76 (d, J = 2.9 Hz, 1H), 7.58 (d, J = 8.1 Hz, 1H), 7.32 - 7.24 (m, 2H), 7.10 (d, J = 8.2 Hz, 1H), 7.03 (d, J = 8.6 Hz, 1H), 6.76 (dd, J = 8.6, 2.9 Hz, 1H), 4.56 (dd, J = 11.9, 4.9 Hz, 1H), 3.99 (t, J = 7.5 Hz, 2H), 3.73 (s, 2H), 3.66 - 3.52 (m, 4H), 3.43 - 3.33 (m, 2H), 3.14 - 3.03 (m, 1H), 2.99 (d, J = 6.4 Hz, 2H), 2.87 - 2.71 (m, 5H), 2.60 (dt, J = 17.3, 4.3 Hz, 1H), 2.46 (dd, J = 12.1, 4.4 Hz, 1H), 2.30 - 2.05 (m, 5H), 1.66 (s, 6H). |
| **26** | MS(ESI, [M+H]⁺) *m*/*z:* 745.32 |
| | ¹H NMR (500 MHz, DMSO) δ 11.07 (s, 1H), 7.97 - 7.89 (m, 3H), 7.76 (d, J = 2.9 Hz, 1H), 7.58 (d, J = 8.2 Hz, 1H), 7.35 - 7.28 (m, 2H), 7.12 - 7.02 (m, 3H), 6.77 (dd, J = 8.6, 2.9 Hz, 1H), 4.56 (dd, J = 11.9, 5.0 Hz, 1H), 4.00 (t, J = 7.5 Hz, 2H), 3.73 (s, 2H), 3.63 (dt, J = 13.8, 7.3 Hz, 2H), 3.56 (dd, J = 7.4, 5.6 Hz, 2H), 3.41 (dt, J = 14.1, 7.2 Hz, 2H), 3.14 - 3.04 (m, 1H), 2.99 (t, J = 5.8 Hz, 2H), 2.78 (ddd, J = 29.0, 11.5, 6.2 Hz, 5H), 2.60 (dt, J = 17.2, 4.3 Hz, 1H), 2.46 (dd, J = 12.1, 4.4 Hz, 1H), 2.31 - 2.08 (m, 5H), 1.67 (s, 6H). |
| **27** | MS(ESI, [M+H]⁺) *m*/*z:* 745.33 |
| | ¹H NMR (500 MHz, DMSO) δ 11.07 (s, 1H), 8.44 (s, 2H), 8.16 (d, J = 8.1 Hz, 2H), 7.76 (d, J = 2.9 Hz, 1H), 7.58 (d, J = 8.2 Hz, 1H), 7.30 (d, J = 8.2 Hz, 2H), 7.09 (d, J = 8.2 Hz, 1H), 7.02 (d, J = 8.6 Hz, 1H), 6.76 (dd, J = 8.5, 2.9 Hz, 1H), 4.55 (dd, J = 11.9, 5.0 Hz, 1H), 3.99 (t, J = 7.5 Hz, 2H), 3.73 (s, 2H), 3.63 - 3.44 (m, 4H), 3.39 (dt, J = 13.5, 6.8 Hz, 2H), 3.15 - 3.04 (m, 1H), 2.99 (t, J = 6.0 Hz, 2H), 2.88 - 2.70 (m, 5H), 2.67 - 2.57 (m, 1H), 2.47 (s, 1H), 2.29 - 2.11 (m, 5H), 1.67 (s, 6H). |
| **28** | MS(ESI, [M+H]⁺) *m*/*z:* 744.33 |
| | ¹H NMR (500 MHz, DMSO) δ 11.07 (s, 1H), 8.23 (d, J = 2.7 Hz, 1H), 7.86 (d, J = 8.4 Hz, 2H), 7.79 - 7.70 (m, 2H), 7.58 (d, J = 8.2 Hz, 1H), 7.35 (dd, J = 8.5, 2.8 Hz, 1H), 7.26 (d, J = 8.4 Hz, 2H), 7.09 (d, J = 8.2 Hz, 1H), 7.02 (d, J = 8.5 Hz, 1H), 6.75 (dd, J = 8.6, 2.9 Hz, 1H), 4.55 (dd, J = 11.8, 5.0 Hz, 1H), 3.99 (t, J = 7.5 Hz, 2H), 3.73 (s, 2H), 3.55 (dd, J = 7.3, 5.6 Hz, 2H), 3.48 - 3.38 (m, 2H), 3.34 (d, J = 2.0 Hz, 1H), 3.31 - 3.27 (m, 1H), 3.07 (h, J = 6.9 Hz, 1H), 2.99 (t, J = 5.9 Hz, 2H), 2.83 - 2.73 (m, 5H), 2.60 (dt, J = 17.2, 4.2 Hz, 1H), 2.46 (dd, J = 12.0, 4.3 Hz, 1H), 2.26 - 2.09 (m, 5H), 1.66 (s, 6H). |
| **29** | MS(ESI, [M+H]⁺) *m*/*z:* 744.35 |
| | ¹H NMR (500 MHz, DMSO) δ 11.07 (s, 1H), 8.40 (d, J = 2.6 Hz, 1H), 7.82 (dd, J = 8.5, 2.6 Hz, 1H), 7.76 (d, J = 2.9 Hz, 1H), 7.58 (d, J = 8.2 Hz, 1H), 7.50 (d, J = 8.2 Hz, 2H), 7.25 (d, J = 8.3 Hz, 2H), 7.10 (d, J = 8.3 Hz, 1H), 7.04 (d, J = 8.6 Hz, 1H), 6.76 (dd, J = 8.5, 3.6 Hz, 2H), 4.55 (dd, J = 11.9, 5.0 Hz, 1H), 3.99 (t, J = 7.5 Hz, 2H), 3.73 (s, 2H), 3.63 - 3.51 (m, 4H), 3.36 - 3.33 (m, 1H), 3.31 - 3.27 (m, 1H), 3.08 (p, J = 6.8 Hz, 1H), 2.99 (d, J = 6.0 Hz, 2H), 2.78 (ddd, J = 28.7, 11.2, 6.2 Hz, 5H), 2.60 (dt, J = 17.2, 4.2 Hz, 1H), 2.46 (dd, J = 12.1, 4.3 Hz, 1H), 2.20 (ddt, J = 14.3, 8.6, 4.6 Hz, 3H), 2.15 - 2.01 (m, 2H), 1.66 (s, 6H). |
| **30** | MS(ESI, [M+H]⁺) *m*/*z:* 761.23 |
| | ¹H NMR (500 MHz, DMSO) δ 11.07 (s, 1H), 8.74 (s, 2H), 7.76 (d, J = 2.9 Hz, 1H), 7.61 - 7.51 (m, 3H), 7.28 (d, J = 8.4 Hz, 2H), 7.10 (d, J = 8.3 Hz, 1H), 7.05 (d, J = 8.5 Hz, 1H), 6.76 (dd, J = 8.6, 2.9 Hz, 1H), 4.56 (dd, J = 11.9, 5.0 Hz, 1H), 4.10 (dtd, J = 12.8, 6.5, 4.2 Hz, 2H), 4.04 - 3.89 (m, 4H), 3.88 - 3.79 (m, 2H), 3.73 (s, 2H), 3.65 - 3.52 (m, 4H), 3.07 (dd, J = 14.0, 7.0 Hz, 1H), 2.99 (d, J = 6.0 Hz, 2H), 2.78 (ddd, J = 29.1, 11.4, 6.4 Hz, 5H), 2.60 (dt, J = 17.3, 4.2 Hz, 1H), 2.18 (dq, J = 13.7, 4.7 Hz, 1H), 1.66 (s, 6H). |
| **31** | MS(ESI, [M+H]⁺) *m*/*z:* 725.29 |
| | ¹H NMR (500 MHz, DMSO) δ 11.07 (s, 1H), 8.63 (s, 2H), 7.75 (d, J = 2.9 Hz, 1H), 7.58 (d, J = 8.1 Hz, 1H), 7.51 - 7.46 (m, 2H), 7.28 - 7.23 (m, 2H), 7.10 (d, J = 8.3 Hz, 1H), 7.02 (d, J = 8.5 Hz, 1H), 6.75 (dd, J = 8.6, 2.9 Hz, 1H), 4.73 (s, 4H), 4.55 (dd, J = 12.0, 5.0 Hz, 1H), 4.23 (s, 4H), 3.99 (t, J = 7.5 Hz, 2H), 3.73 (s, 2H), 3.55 (t, J = 6.4 Hz, 2H), 3.12 - 3.05 (m, 1H), 2.99 (s, 2H), 2.86 - 2.72 (m, 5H), 2.65 - 2.57 (m, 1H), 2.48 - 2.45 (m, 1H), 2.22 - 2.14 (m, 1H), 1.65 (s, 6H). |
| **32** | MS(ESI, [M+H]⁺) *m*/*z:* 686.24 |
| | ¹H NMR (500 MHz, DMSO) δ 11.07 (s, 1H), 9.06 (s, 2H), 7.76 (d, J = 3.0 Hz, 1H), 7.70 - 7.64 (m, 2H), 7.58 (d, J = 8.2 Hz, 1H), 7.38 - 7.31 (m, 2H), 7.08 (dd, J = 14.3, 8.4 Hz, 2H), 6.77 (dd, J = 8.6, 3.0 Hz, 1H), 5.07 (s, 1H), 4.55 (dd, J = 11.9, 4.9 Hz, 1H), 3.99 (t, J = 7.5 Hz, 2H), 3.73 (s, 2H), 3.56 (dd, J = 7.3, 5.6 Hz, 2H), 3.08 (p, J = 6.9 Hz, 1H), 2.99 (t, J = 5.9 Hz, 2H), 2.78 (ddd, J = 29.1, 11.2, 6.1 Hz, 5H), 2.60 (dt, J = 17.2, 4.2 Hz, 1H), 2.46 (dd, J = 12.2, 4.4 Hz, 1H), 2.19 (dq, J = 8.8, 4.4 Hz, 1H), 1.68 (s, 6H), 1.53 (s, 6H). |
| **33** | MS(ESI, [M+H]⁺) *m*/*z*: 776.14 |
| | ¹H NMR (500 MHz, DMSO) δ 11.07 (s, 1H), 8.60 (d, J = 1.5 Hz, 1H), 7.97 (d, J = 1.5 Hz, 1H), 7.91 - 7.86 (m, 1H), 7.84 - 7.80 (m, 2H), 7.75 (d, J = 2.9 Hz, 1H), 7.58 (d, J = 8.1 Hz, 1H), 7.30 - 7.24 (m, 2H), 7.09 (d, J = 8.3 Hz, 1H), 7.02 (d, J = 8.5 Hz, 1H), 6.75 (dd, J = 8.7, 2.9 Hz, 1H), 4.55 (dd, J = 11.9, 5.0 Hz, 1H), 4.39 (d, J = 5.0 Hz, 3H), 4.02 - 3.91 (m, 4H), 3.73 (s, 2H), 3.55 (dd, J = 7.3, 5.6 Hz, 2H), 3.08 (p, J = 6.8 Hz, 1H), 2.99 (t, J = 5.9 Hz, 2H), 2.95 (s, 3H), 2.85 - 2.72 (m, 5H), 2.60 (dt, J = 17.3, 4.2 Hz, 1H), 2.46 (dd, J = 12.2, 4.4 Hz, 1H), 2.18 (dq, J = 13.6, 4.8 Hz, 1H), 1.65 (s, 6H). |

### Example 34: Synthesis of Compound 34

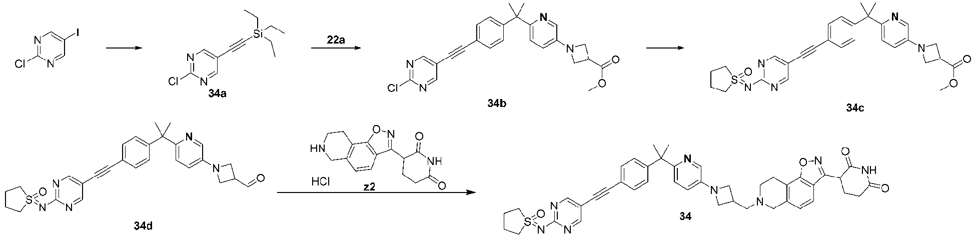

### Step 1: Preparation of compound 34a

2-Chloro-5-iodopyrimidine (3.7 g), triethylsilylacetylene (1.5 g), PdCl₂(PPh₃)₂ (0.85 g), copper(I) iodide (0.46 g), and DMF/triethylamine (2:1, 50 mL, v:v) were added to a reaction flask in sequence. The mixture was reacted at 100 °C for 2 h under nitrogen atmosphere. The reaction solution was diluted with water and ethyl acetate. The organic phase was separated, washed with saturated brine, and concentrated, and the residue was purified by silica gel column chromatography to give compound **34a** (1.6 g). MS(ESI, [M+H]⁺) m/z: 253.31.

### Step 2: Preparation of compound 34b

**22a** (450 mg), **34a** (400 mg), PdCl₂(PPh₃)₂ (71 mg), copper(I) iodide (38 mg), triethylamine (0.5 mL), potassium fluoride (177 mg), and DMF (10 mL) were added to a reaction flask in sequence, and the mixture was reacted at 100 °C for 2 h under nitrogen atmosphere. The reaction solution was diluted with water and EA. The organic phase was separated, washed with saturated brine, and concentrated, and the residue was purified by silica gel column chromatography to give compound **34b** (496 mg). MS(ESI, [M+H]⁺) m/z: 447.31.

### Step 3: Preparation of intermediate 34c

Referring to the preparation of **z8** in Preparation Example 1, 5-chloro-2-iodopyrimidine was replaced with **34b** to give compound **34c** (533 mg). MS(ESI, [M+H]⁺) m/z: 530.10.

### Steps 4-5: Preparation of compound 35

Referring to the reactions of steps 9-10 in Example 17, **17i** was replaced with **34c** to finally give compound **34** (75 mg). **34d:** MS(ESI, [M+H]⁺) *m*/*z*: 500.34.

Compound **34:** MS (ESI, [M+H]⁺) *m*/*z:* 769.32. ¹H NMR (500 MHz, DMSO) δ 11.07 (s, 1H), 8.63 (s, 2H), 7.75 (d, *J* = 2.9 Hz, 1H), 7.58 (d, *J* = 8.1 Hz, 1H), 7.47 - 7.37 (m, 2H), 7.29 - 7.20 (m, 2H), 7.10 (d, *J* = 8.2 Hz, 1H), 7.02 (d, *J =* 8.6 Hz, 1H), 6.76 (dd, *J =* 8.6, 2.9 Hz, 1H), 4.56 (dd, *J =* 11.9, 5.0 Hz, 1H), 3.99 (t, *J =* 7.5 Hz, 2H), 3.73 (s, 2H), 3.62 (dt, *J =* 12.3, 6.5 Hz, 2H), 3.56 (dd, *J =* 7.3, 5.6 Hz, 2H), 3.40 (dt, *J =* 14.0, 7.1 Hz, 2H), 3.08 (p, *J* = 7.0 Hz, 1H), 2.99 (d, *J =* 5.8 Hz, 2H), 2.78 (ddd, *J* = 29.1, 10.9, 6.2 Hz, 5H), 2.60 (dt, *J =* 17.3, 4.3 Hz, 1H), 2.46 (dd, *J* = 12.2, 4.5 Hz, 1H), 2.28 - 2.06 (m, 5H), 1.64 (s, 6H).

### Example 35: Synthesis of Compound 35

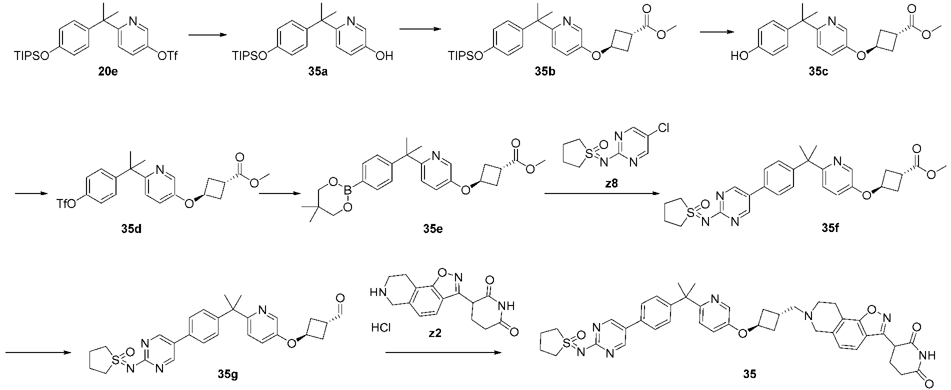

### Step 1: Preparation of intermediate 35a

**20e** (3.0 g), tetrahydrofuran (100 mL), and lithium tri-sec-butylborohydride (3.3 g) were added to a three-necked flask in sequence, and the mixture was heated to 80 °C and reacted. After the reaction was completed, the reaction solution was cooled to room temperature and purified by silica gel column chromatography to give intermediate **35a** (2.0 g). MS(ESI, [M+H]⁺) *m*/*z:* 386.21.

### Step 2: Preparation of intermediate 35b

**35a** (1.9 g), tert-butyl cis-3-hydroxycyclobutanecarboxylate (1.3 g), triphenylphosphine (2.6 g), and dioxane (20 mL) were added to a three-necked flask in sequence. The system was purged with nitrogen, and DEAD (1.94 mL) was slowly added in an ice bath. The mixture was heated to 80 °C and reacted. After the reaction was completed, the mixture was purified by silica gel column chromatography to give intermediate **35b** (1.85 g). MS(ESI, [M+H]⁺) *m*/*z:* 498.25.

### Step 3: Preparation of intermediate 35c

Referring to the reaction of step 6 in Example 17, **17e** was replaced with **35b** to give intermediate **35c** (1.5 g). MS(ESI, [M+H]⁺) *m*/*z:* 342.21.

### Step 4: Preparation of intermediate 35d

Referring to the reaction of step 1 in Example 17, **17a-1** was replaced with **35c** to give intermediate **35d** (1.05 g). MS(ESI, [M+H]⁺) *m*/*z:* 474.31.

### Steps 5-6: Preparation of intermediate 35f

Referring to the reaction in steps 7-8 of Example 21, **21f** was replaced with **35d** to give intermediate **35f** (0.23 g). **35e:** MS(ESI, [M+H]⁺) *m*/*z:* 438.31.

**35f:** MS(ESI, [M+H]⁺) *m*/*z:* 521.25.

### Steps 7-8: Preparation of compound 35

Referring to the reactions of steps 9-10 in Example 17, **17h** was replaced with **35f** to give compound **35** (78 mg). **35g:** MS(ESI, [M+H]⁺) *m*/*z:* 491.34.

Compound **35:** MS (ESI, [M+H]⁺) *m*/*z:* 760.32. ¹H NMR (500 MHz, DMSO-d₆) δ 11.07 (s, 1H), 8.75 (s, 2H), 8.13 (t, *J* = 1.8 Hz, 1H), 7.63 - 7.50 (m, 3H), 7.29 (d, *J* = 8.3 Hz, 2H), 7.17 (d, *J* = 1.8 Hz, 2H), 7.09 (d, *J* = 8.3 Hz, 1H), 4.89 (p, *J* = 6.1 Hz, 1H), 4.55 (dd, *J =* 11.9, 5.0 Hz, 1H), 3.71 (s, 2H), 3.62 (dt, *J =* 13.5, 6.9 Hz, 2H), 3.38 (dt, *J* = 13.5, 6.8 Hz, 2H), 3.04 - 2.85 (m, 2H), 2.83 - 2.72 (m, 3H), 2.71 - 2.55 (m, 4H), 2.46 (dd, *J* = 12.2, 4.4 Hz, 1H), 2.37 - 2.02 (m, 9H), 1.68 (s, 6H).

### Example 36: Synthesis of Compound 36

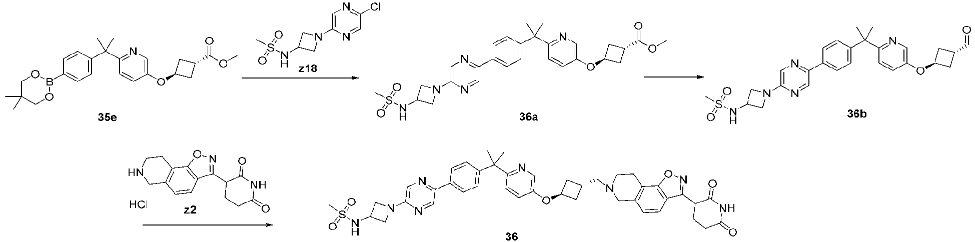

### Step 1: Preparation of intermediate 36a

Referring to the reaction of step 8 in Example 21, **21g** was replaced with **35e** and **z8** was replaced with intermediate **z18** to give intermediate **36a** (0.24 g). MS(ESI, [M+H]⁺) *m*/*z:* 552.25.

### Steps 2-3: Preparation of compound 36

Referring to the reactions of steps 9-10 in Example 17, **17h** was replaced with **36a** to give compound **36** (73 mg). **36b:** MS(ESI, [M+H]⁺) *m*/*z:* 522.31.

Compound **36:** MS (ESI, [M+H]⁺) *m*/*z:* 791.21. ¹H NMR (500 MHz, DMSO-*d*₆) δ 11.07 (s, 1H), 8.60 (d, J = 1.5 Hz, 1H), 8.13 (dd, J = 2.7, 0.9 Hz, 1H), 7.97 (d, J = 1.5 Hz, 1H), 7.90 - 7.86 (m, 1H), 7.85 - 7.81 (m, 2H), 7.57 (d, J = 8.1 Hz, 1H), 7.29 - 7.25 (m, 2H), 7.18 - 7.12 (m, 2H), 7.09 (d, J = 8.2 Hz, 1H), 4.88 (p, J = 6.2 Hz, 1H), 4.55 (dd, J = 11.8, 5.0 Hz, 1H), 4.39 (d, J = 5.2 Hz, 3H), 3.94 (q, J = 3.0 Hz, 2H), 3.70 (s, 2H), 2.95 (s, 5H), 2.77 (dp, J = 17.2, 6.0 Hz, 3H), 2.69 - 2.57 (m, 4H), 2.47 (dd, J = 5.2, 3.2 Hz, 1H), 2.27 (d, J = 7.1 Hz, 2H), 2.19 (ddd, J = 18.0, 9.7, 5.2 Hz, 3H), 1.68 (s, 6H).

### Example 37: Synthesis of Compound 37

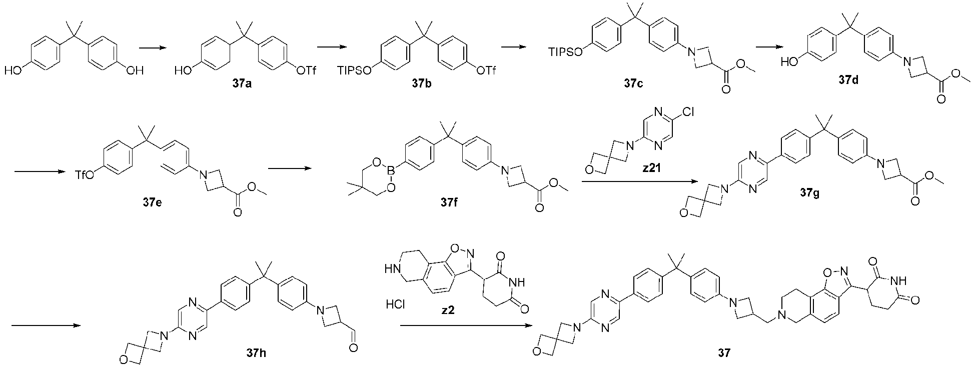

### Step 1: Preparation of intermediate 37a

Bisphenol A (20 g), dichloromethane (200 mL), and DIPEA (46 mL) were added to a single-necked flask in sequence, and then trifluoromethanesulfonic anhydride (37 g) was added slowly in an ice bath. The mixture was reacted at room temperature. After the reaction was completed, the system was extracted with dichloromethane and water, and the organic phase was separated, washed with saturated brine, dried over anhydrous sodium sulfate, filtered, and concentrated by evaporation under reduced pressure to remove the solvent, thus giving intermediate **37a** (18 g). MS(ESI, [M+H]⁺) *m*/*z:* 361.45.

### Steps 2-4: Preparation of intermediate 37d

Referring to the reactions of steps 4-6 in Example 17, **17c** was replaced with **37a** to give intermediate **37d** (1.5 g). **37b:** MS(ESI, [M+H]⁺) *m*/*z:* 517.23.

**37c:** MS(ESI, [M+H]⁺) *m*/*z:* 482.30.

**37d:** MS(ESI, [M+H]⁺) *m*/*z:* 326.22.

### Step 5: Preparation of intermediate 37e

Referring to the reaction of step 1 in Example 17, **17a-1** was replaced with **37d** to give intermediate **37e** (2.1 g). MS(ESI, [M+H]⁺) *m*/*z:* 458.09.

### Step 6: Preparation of intermediate 37f

Referring to the reaction of step 7 in Example 21, **21f** was replaced with **37e** to give intermediate **37f** (1.3 g). MS(ESI, [M+H]⁺) *m*/*z:* 422.31.

### Step 7: Preparation of intermediate 37g

Referring to the reaction of step 8 in Example 21, **21g** was replaced with **37f** and **z8** was replaced with **z21** to give intermediate **37g** (0.66 g). MS(ESI, [M+H]⁺) *m*/*z:* 485.34.

### Steps 8-9: Preparation of compound 37

Referring to the reactions of steps 9-10 in Example 17, **17h** was replaced with **37g** to give compound **37** (53 mg). **37h:** MS(ESI, [M+H]⁺) *m*/*z:* 455.21.

Compound **37:** MS (ESI, [M+H]⁺) *m*/*z:* 724.41. ¹H NMR (500 MHz, DMSO) δ 11.07 (s, 1H), 8.59 (d, *J =* 1.5 Hz, 1H), 7.93 (d, *J* = 1.5 Hz, 1H), 7.81 (d, *J* = 8.2 Hz, 2H), 7.58 (d, *J* = 8.1 Hz, 1H), 7.26 (d, *J* = 8.2 Hz, 2H), 7.07 (dd, *J=* 31.7, 8.3 Hz, 3H), 6.35 (d, *J* = 8.3 Hz, 2H), 4.73 (s, 4H), 4.56 (dd, *J* = 11.9, 5.0 Hz, 1H), 4.24 (s, 4H), 3.92 (t, J = 7.4 Hz, 2H), 3.73 (s, 2H), 3.48 (q, *J=* 9.1 Hz, 2H), 3.00 (s, 3H), 2.91 - 2.70 (m, 5H), 2.62 (t, *J* = 4.1 Hz, 1H), 2.18 (dq, *J =* 13.6, 4.7 Hz, 1H), 1.60 (s, 6H).

The following examples were synthesized with reference to the synthetic method for compound **37** by using **37f** as a starting material and replacing **z21** with the structures in the table below (or simultaneously replacing **z2** with **z24** and the like):

| **Examples** | **Starting material** | **Compound structure** |
|---|---|---|
| **38** | 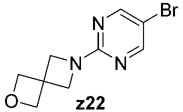 | 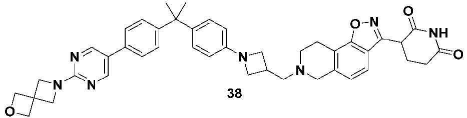 |
| **39** | 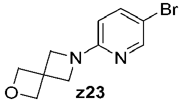 | 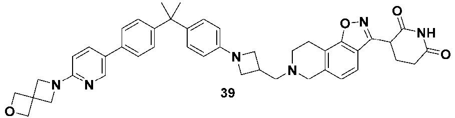 |
| **40** | 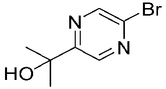 | 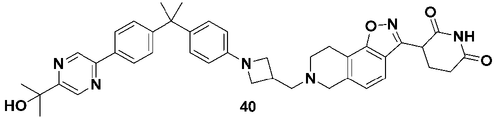 |
| **41** | 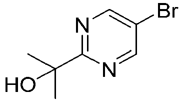 | 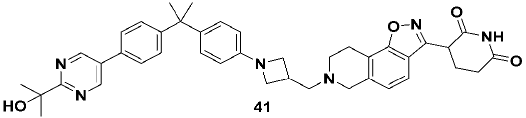 |
| **42** | 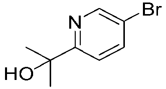 | 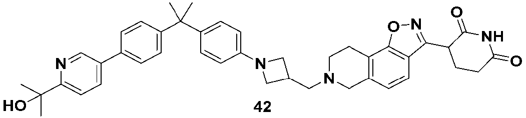 |
| **43** | 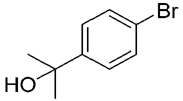 | 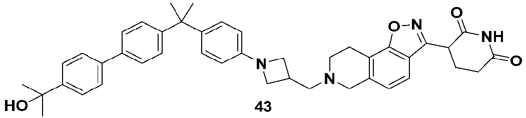 |
| **44** | 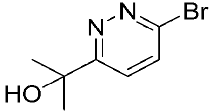 | 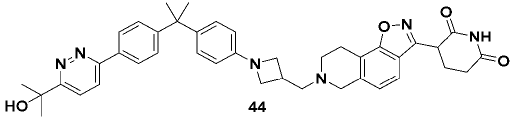 |
| **45** | 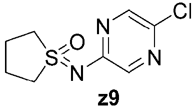 | 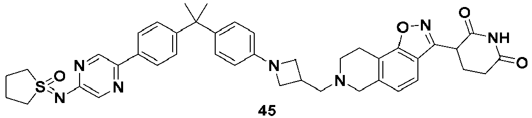 |
| **46** | 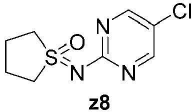 | 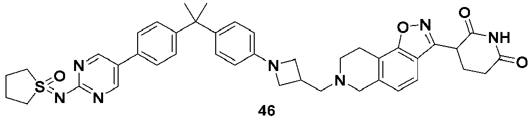 |
| **47** | 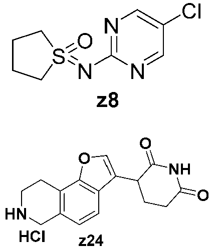 | 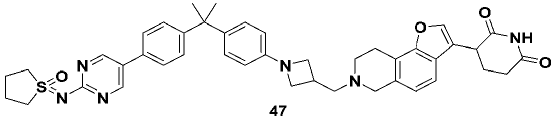 |
| **48** | 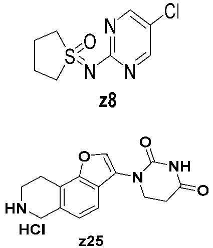 | 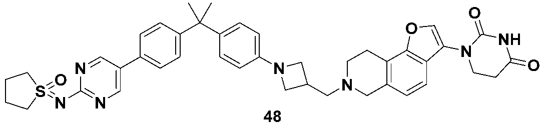 |

| **Examples** | **Data analysis** |
|---|---|
| **38** | MS (ESI, [M+H] ⁺) m/z: 724.36 |
| | ¹H NMR (500 MHz, DMSO) δ 11.07 (s, 1H), 8.64 (s, 2H), 7.58 (d, J = 8.2 Hz, 1H), 7.49 (d, J = 8.3 Hz, 2H), 7.25 (d, J = 8.3 Hz, 2H), 7.10 (d, J = 8.3 Hz, 1H), 7.03 (d, J = 8.3 Hz, 2H), 6.35 (d, J = 8.3 Hz, 2H), 4.73 (s, 4H), 4.56 (dd, J = 11.9, 5.0 Hz, 1H), 4.23 (s, 4H), 3.92 (t, J = 7.4 Hz, 2H), 3.72 (s, 2H), 3.47 (t, J = 6.4 Hz, 2H), 3.01 (d, J = 21.3 Hz, 3H), 2.89 - 2.72 (m, 5H), 2.60 (dt, J = 17.4, 4.3 Hz, 1H), 2.49 - 2.44 (m, 1H), 2.19 (dt, J = 13.5, 4.4 Hz, 1H), 1.60 (s, 6H). |
| **39** | MS (ESI, [M+H] ⁺) m/z: 723.31 |
| | ¹H NMR (500 MHz, DMSO) δ 11.07 (s, 1H), 8.36 (d, J = 2.5 Hz, 1H), 7.78 (dd, J = 8.6, 2.5 Hz, 1H), 7.58 (d, J = 8.1 Hz, 1H), 7.46 (d, J = 8.3 Hz, 2H), 7.23 (d, J = 8.2 Hz, 2H), 7.07 (dd, J = 28.2, 8.4 Hz, 3H), 6.45 (d, J = 8.6 Hz, 1H), 6.35 (d, J = 8.3 Hz, 2H), 4.72 (s, 4H), 4.55 (dd, J = 11.9, 5.0 Hz, 1H), 4.12 (s, 4H), 3.92 (t, J = 7.4 Hz, 2H), 3.72 (s, 2H), 3.47 (t, J = 6.3 Hz, 2H), 3.01 (dt, J = 17.3, 6.4 Hz, 3H), 2.85 - 2.71 (m, 5H), 2.60 (dt, J = 17.4, 4.3 Hz, 1H), 2.48 - 2.45 (m, 1H), 2.18 (dq, J = 13.4, 4.8 Hz, 1H), 1.60 (s, 6H). |
| **40** | MS (ESI, [M+H] +) m/z: 685.35 |
| | ¹H NMR (500 MHz, DMSO) δ 11.07 (s, 1H), 9.06 (d, J = 1.5 Hz, 1H), 8.92 (d, J = 1.5 Hz, 1H), 8.04 - 7.96 (m, 2H), 7.58 (d, J = 8.1 Hz, 1H), 7.39 - 7.32 (m, 2H), 7.13 - 7.02 (m, 3H), 6.41 - 6.33 (m, 2H), 5.45 (s, 1H), 4.55 (dd, J = 11.9, 5.0 Hz, 1H), 3.93 (t, J = 7.4 Hz, 2H), 3.72 (s, 2H), 3.48 (dd, J = 7.2, 5.6 Hz, 2H), 3.01 (dt, J = 18.0, 6.4 Hz, 3H), 2.83 - 2.71 (m, 5H), 2.60 (dt, J = 17.3, 4.3 Hz, 1H),2.48(m, 1H), 2.18 (dq, J = 13.4, 4.8 Hz, 1H), 1.63 (s, 6H), 1.50 (s, 6H). |
| **41** | MS (ESI, [M+H] ⁺) m/z: 685.33 |
| | ¹H NMR (500 MHz, DMSO) δ 11.07 (s, 1H), 9.07 (s, 2H), 7.71 - 7.65 (m, 2H), 7.58 (d, J = 8.2 Hz, 1H), 7.38 - 7.32 (m, 2H), 7.12 - 7.03 (m, 3H), 6.40 - 6.35 (m, 2H), 5.07 (s, 1H), 4.55 (dd, J = 11.9, 5.0 Hz, 1H), 3.93 (t, J = 7.4 Hz, 2H), 3.72 (s, 2H), 3.52 - 3.45 (m, 2H), 3.08 - 2.96 (m, 3H), 2.84 - 2.72 (m, 5H), 2.60 (dt, J = 17.3, 4.3 Hz, 1H), 2.48 - 2.44 (m, 1H), 2.19 (dq, J = 8.6, 4.3 Hz, 1H), 1.63 (s, 6H), 1.53 (s, 6H). |
| **42** | MS (ESI, [M+H] ⁺) m/z: 684.35 |
| | ¹H NMR (500 MHz, DMSO) δ 11.07 (s, 1H), 8.74 (dd, J = 2.4, 0.8 Hz, 1H), 7.99 (dd, J = 8.3, 2.4 Hz, 1H), 7.70 (dd, J = 8.2, 0.8 Hz, 1H), 7.64 - 7.56 (m, 3H), 7.35 - 7.28 (m, 2H), 7.15 - 7.04 (m, 3H), 6.43 - 6.33 (m, 2H), 5.23 (s, 1H), 4.55 (dd, J = 11.9, 5.0 Hz, 1H), 3.93 (t, J = 7.4 Hz, 2H), 3.72 (s, 2H), 3.48 (dd, J = 7.1, 5.6 Hz, 2H), 3.01 (dt, J = 18.3, 6.2 Hz, 3H), 2.88 - 2.72 (m, 5H), 2.60 (dt, J = 17.3, 4.2 Hz, 1H), 2.55 - 2.52 (m, 1H), 2.19 (td, J = 7.9, 3.7 Hz, 1H), 1.62 (s, 6H), 1.46 (s, 6H). |
| **43** | MS (ESI, [M+H] ⁺) m/z: 683.61 |
| | ¹H NMR (500 MHz, DMSO) δ 11.07 (s, 1H), 7.61 - 7.50 (m, 7H), 7.30 - 7.24 (m, 2H), 7.13 - 7.03 (m, 3H), 6.39 - 6.33 (m, 2H), 5.01 (s, 1H), 4.55 (dd, J = 11.9, 5.0 Hz, 1H), 3.92 (t, J = 7.4 Hz, 2H), 3.72 (s, 2H), 3.47 (dd, J = 7.1, 5.7 Hz, 2H), 3.01 (dt, J = 17.8, 6.5 Hz, 3H), 2.84 - 2.72 (m, 5H), 2.60 (dt, J = 17.3, 4.2 Hz, 1H), 2.49 - 2.43 (m, 1H), 2.18 (dq, J = 13.6, 5.0 Hz, 1H), 1.61 (s, 6H), 1.44 (s, 6H). |
| **44** | MS (ESI, [M+H] ⁺) m/z: 685.34 |
| | ¹H NMR (500 MHz, DMSO) δ 11.07 (s, 1H), 8.14 (d, J = 8.9 Hz, 1H), 8.02 (d, J = 8.3 Hz, 2H), 7.93 (d, J = 9.0 Hz, 1H), 7.58 (d, J = 8.1 Hz, 1H), 7.38 (d, J = 8.3 Hz, 2H), 7.08 (dd, J = 16.5, 8.4 Hz, 3H), 6.37 (d, J = 8.6 Hz, 2H), 5.49 (s, 1H), 4.55 (dd, J = 11.9, 5.0 Hz, 1H), 3.93 (t, J = 7.4 Hz, 2H), 3.73 (s, 2H), 3.51 - 3.46 (m, 2H), 3.01 (dt, J = 19.0, 6.3 Hz, 3H), 2.88 - 2.71 (m, 5H), 2.60 (dt, J = 17.2, 4.2 Hz, 1H), 2.49 - 2.43 (m, 1H), 2.23 - 2.13 (m, 1H), 1.64 (s, 6H), 1.57 (s, 6H). |
| **45** | MS (ESI, [M+H] ⁺) m/z: 744.31 |
| | ¹H NMR (500 MHz, DMSO) δ 11.07 (s, 1H), 8.67 (d, J = 1.5 Hz, 1H), 8.14 (d, J = 1.5 Hz, 1H), 7.90 - 7.84 (m, 2H), 7.58 (d, J = 8.2 Hz, 1H), 7.32 - 7.26 (m, 2H), 7.10 (d, J = 8.2 Hz, 1H), 7.05 (d, J = 8.5 Hz, 2H), 6.38 - 6.33 (m, 2H), 4.55 (dd, J = 11.9, 5.0 Hz, 1H), 3.93 (t, J = 7.4 Hz, 2H), 3.72 (s, 2H), 3.61 (dt, J = 13.6, 7.0 Hz, 2H), 3.48 (t, J = 6.3 Hz, 2H), 3.38 (dt, J = 13.4, 6.9 Hz, 2H), 3.08 - 2.96 (m, 3H), 2.77 (ddd, J = 22.7, 10.4, 5.0 Hz, 5H), 2.60 (dt, J = 17.3, 4.3 Hz, 1H), 2.46 (dd, J = 12.1, 4.4 Hz, 1H), 2.29 - 2.08 (m, 5H), 1.61 (s, 6H). |
| **46** | MS (ESI, [M+H] ⁺) m/z: 744.34 |
| | ¹H NMR (500 MHz, DMSO) δ 11.07 (s, 1H), 8.76 (s, 2H), 7.57 (dd, J = 8.3, 6.4 Hz, 3H), 7.33 - 7.24 (m, 2H), 7.07 (dd, J = 23.9, 8.4 Hz, 3H), 6.43 - 6.29 (m, 2H), 4.55 (dd, J = 11.9, 5.0 Hz, 1H), 3.93 (t, J = 7.4 Hz, 2H), 3.73 (s, 2H), 3.63 (dt, J = 13.7, 7.2 Hz, 2H), 3.55 - 3.46 (m, 2H), 3.38 (dt, J = 14.3, 7.1 Hz, 2H), 3.01 (dt, J = 18.3, 6.5 Hz, 3H), 2.89 - 2.70 (m, 5H), 2.60 (dt, J = 17.3, 4.4 Hz, 1H), 2.46 (dd, J = 12.1, 4.3 Hz, 1H), 2.29 - 2.05 (m, 5H), 1.61 (s, 6H). |
| **47** | MS (ESI, [M+H] ⁺) m/z: 743.31 |
| | ¹H NMR (500 MHz, DMSO) δ 10.87 (s, 1H), 8.76 (s, 2H), 7.83 (s, 1H), 7.59 - 7.54 (m, 2H), 7.33 (d, J = 8.0 Hz, 1H), 7.30 - 7.26 (m, 2H), 7.05 (d, J = 8.5 Hz, 2H), 6.95 (d, J = 8.1 Hz, 1H), 6.39 - 6.34 (m, 2H), 4.10 (dd, J = 11.9, 4.9 Hz, 1H), 3.92 (t, J = 7.4 Hz, 2H), 3.68 - 3.59 (m, 4H), 3.50 - 3.45 (m, 2H), 3.38 (dt, J = 14.0, 7.2 Hz, 2H), 3.02 (p, J = 6.9 Hz, 1H), 2.96 (t, J = 5.9 Hz, 2H), 2.81 - 2.70 (m, 5H), 2.60 - 2.54 (m, 1H), 2.34 - 2.21 (m, 3H), 2.16 - 2.07 (m, 3H), 1.61 (s, 6H). |
| **48** | MS (ESI, [M+H] ⁺) m/z: 744.21 |
| | ¹H NMR (500 MHz, DMSO) δ 10.50 (s, 1H), 8.76 (s, 2H), 8.06 (s, 1H), 7.59 - 7.54 (m, 2H), 7.36 (d, J = 8.1 Hz, 1H), 7.30 - 7.26 (m, 2H), 7.07 - 7.02 (m, 2H), 6.99 (d, J = 8.2 Hz, 1H), 6.39 - 6.33 (m, 2H), 3.93 (t, J = 7.4 Hz, 2H), 3.83 (t, J = 6.7 Hz, 2H), 3.70 - 3.58 (m, 4H), 3.47 (dd, J = 7.1, 5.6 Hz, 2H), 3.38 (dt, J = 14.2, 7.2 Hz, 2H), 2.99 (dt, J = 29.1, 6.5 Hz, 3H), 2.77 (t, J = 6.7 Hz, 6H), 2.28 - 2.20 (m, 2H), 2.12 (qt, J = 7.7, 2.9 Hz, 2H), 1.61 (s, 6H). |

### Example 49: Synthesis of Compound 49

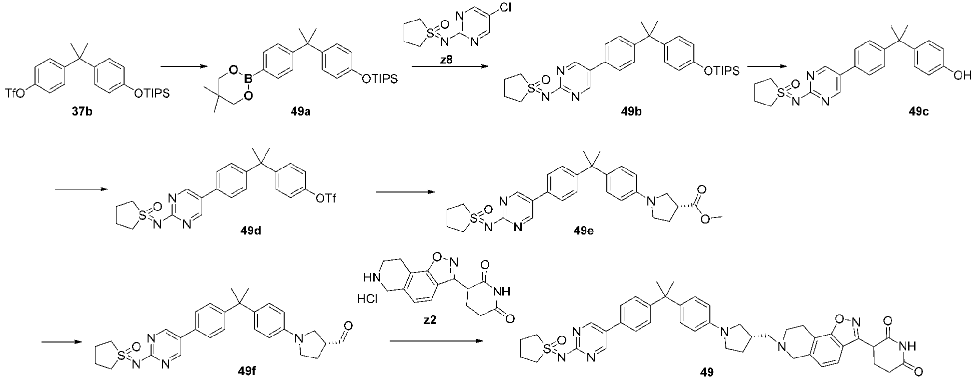

### Step 1: Preparation of intermediate 49a

Referring to the reaction of step 7 in Example 21, **21f was** replaced with **37b** to give intermediate **49a** (15.3 g). MS(ESI, [M+H]⁺) *m*/*z:* 481.31.

### Step 2: Preparation of intermediate 49b

Referring to the reaction of step 8 in Example 21, **21g** was replaced with **49a** to give intermediate **49b** (0.67 g). MS(ESI, [M+H]⁺) *m*/*z:* 564.33.

### Step 3: Preparation of intermediate 49c

Referring to the reaction of step 6 in Example 17, **17e** was replaced with **49b** to give compound **49c** (0.53 g). MS(ESI, [M+H]⁺) m/z: 408.19.

### Step 4: Preparation of intermediate 49d

Intermediate **49c** (0.48 g), 4-dimethylaminopyridine (13 mg), triethylamine (500 mg), N-phenyl-bis(trifluoromethanesulfonimide) (514 mg), and dichloromethane (25 mL) were added to a reaction flask in sequence, and the mixture was reacted at room temperature for 2 h. After the reaction was completed, the reaction solution was purified by silica gel column chromatography to give compound **49d** (0.52 g). MS(ESI, [M+H]⁺) m/z: 540.15.

### Step 5: Preparation of intermediate 49e

Intermediate **49d** (0.46 g), (R)-methylpyrrolidine-3-methyl ester hydrochloride (0.21 g), Pd₂(dba)₃ (83 mg), Ruphos (87 mg), cesium carbonate (0.7 g), and 1,4-dioxane (10 mL) were added to a reaction flask in sequence, and the mixture was reacted at 110 °C for 2 h under nitrogen atmosphere. After the reaction was completed, the reaction solution was purified by silica gel column chromatography to give compound **49e** (0.33 g). MS(ESI, [M+H]⁺) m/z: 519.21.

### Step 6: Preparation of intermediate 49f

Intermediate **49e** (0.25 g) and dichloromethane (20 mL) were added to a reaction flask in sequence, and the mixture was cooled to -78 °C. DIBAL-H (0.68 mL, 1.5 M) was slowly added under nitrogen atmosphere, and the mixture was reacted at -78 °C for 1 h. After the reaction was completed, the reaction solution was poured into a saturated ammonium chloride solution to quench the reaction, and a saturated potassium sodium tartrate solution was added. The mixture was fully stirred, and the organic phase was separated, washed with saturated brine, and concentrated to give **49f** (0.23 g). MS(ESI, [M+H]⁺) m/z: 489.33.

### Step 7: Preparation of compound 49

Referring to the reaction of step 10 in Example 17, **17i** was replaced with **49f** to give compound **49** (73 mg). HRMS(ESI, [M+H]⁺) *m*/*z:* 758.35. ¹H NMR (500 MHz, DMSO-*d*₆) *δ* 11.07 (s, 1H), 8.75 (s, 2H), 7.56 (dd, *J =* 12.9, 8.3 Hz, 3H), 7.30 - 7.21 (m, 2H), 7.10 (d, *J =* 8.2 Hz, 1H), 7.06 - 7.00 (m, 2H), 6.45 (d, *J =* 8.7 Hz, 2H), 4.56 (dd, *J =* 11.9, 5.0 Hz, 1H), 3.76 (q, *J =* 16.0 Hz, 2H), 3.62 (dt, *J =* 13.7, 7.2 Hz, 2H), 3.37 (ddd, *J =* 10.8, 8.8, 4.2 Hz, 3H), 3.30 - 3.26 (m, 1H), 3.21 (dt, *J* = 9.3, 7.4 Hz, 1H), 3.00 (q, *J* = 5.8 Hz, 3H), 2.89 - 2.65 (m, 4H), 2.65 - 2.53 (m, 3H), 2.49 - 2.43 (m, 1H), 2.21 (dtt, *J* = 13.4, 8.5, 4.5 Hz, 3H), 2.12 (qd, *J* = 7.6, 3.1 Hz, 3H), 1.74 (dq, *J =* 12.1, 7.6 Hz, 1H), 1.60 (s, 6H).

### Example 50: Synthesis of Compound 50

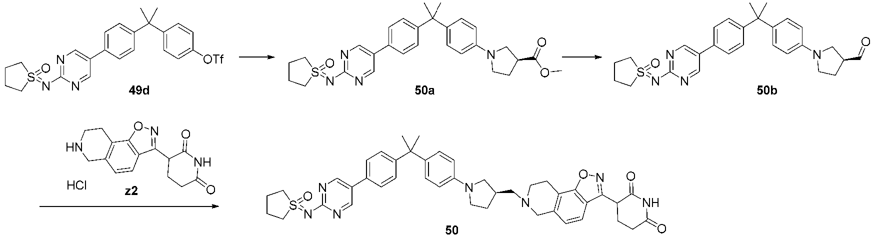

Referring to the reactions of steps 5-7 in Example 49, (R)-methylpyrrolidine-3-methyl ester hydrochloride was replaced with (S)-methylpyrrolidine-3-methyl ester hydrochloride to finally give compound 50.

**50a:** MS(ESI, [M+H]⁺) m/z: 519.33.

**50b:** MS(ESI, [M+H]⁺) *m*/*z:* 489.15.

Compound **50:** HRMS (ESI, [M+H]⁺) m/z: 758.3498. ¹H NMR (500 MHz, DMSO-d₆) *δ* 11.07 (s, 1H), 8.75 (s, 2H), 7.56 (dd, *J* = 12.9, 8.3 Hz, 3H), 7.30 - 7.21 (m, 2H), 7.10 (d, *J* = 8.2 Hz, 1H), 7.06 - 7.00 (m, 2H), 6.45 (d, *J =* 8.7 Hz, 2H), 4.56 (dd, *J =* 11.9, 5.0 Hz, 1H), 3.76 (q, *J* = 16.0 Hz, 2H), 3.62 (dt, *J =* 13.7, 7.2 Hz, 2H), 3.37 (ddd, *J =* 10.8, 8.8, 4.2 Hz, 3H), 3.30 - 3.26 (m, 1H), 3.21 (dt, *J =* 9.3, 7.4 Hz, 1H), 3.00 (q, *J* = 5.8 Hz, 3H), 2.89 - 2.65 (m, 4H), 2.65 - 2.53 (m, 3H), 2.49 - 2.43 (m, 1H), 2.21 (dtt, *J =* 13.4, 8.5, 4.5 Hz, 3H), 2.12 (qd, *J* = 7.6, 3.1 Hz, 3H), 1.74 (dq, *J* = 12.1, 7.6 Hz, 1H), 1.60 (s, 6H).

### Example 51: Synthesis of Compound 51

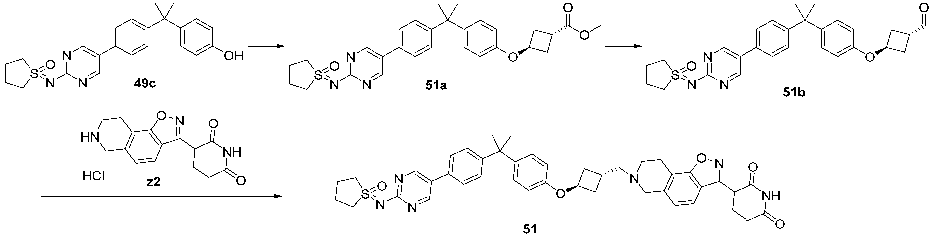

### Step 1: Preparation of intermediate 51a

Referring to the reaction of step 1 in Example 11, bisphenol A was replaced with **49c** to give intermediate **51a** (1.73 g). MS(ESI, [M+H]⁺) *m*/*z*: 520.11.

### Steps 2-3: Preparation of intermediate 51b

Referring to the reaction of steps 9-10 in Example 17, **17h** was replaced with **51a** to give compound **51** (55 mg). **51b:** MS(ESI, [M+H]⁺) *m*/*z:* 490.34.

Compound **51:** MS (ESI, [M+H]⁺) *m*/*z:* 759.33. ¹H NMR (500 MHz, DMSO) δ 11.07 (s, 1H), 8.76 (s, 2H), 7.57 (d, *J* = 8.3 Hz, 3H), 7.31 - 7.26 (m, 2H), 7.16 - 7.07 (m, 3H), 6.77 - 6.71 (m, 2H), 4.80 (p, *J* = 6.2 Hz, 1H), 4.56 (dd, *J* = 11.8, 5.0 Hz, 1H), 3.71 (s, 1H), 3.64 - 3.58 (m, 2H), 3.38 (dt, *J =* 13.7, 6.9 Hz, 2H), 2.99 (s, 2H), 2.76 (ddd, *J =* 17.2, 11.9, 5.3 Hz, 3H), 2.72 - 2.56 (m, 4H), 2.49 - 2.44 (m, 1H), 2.33 - 2.16 (m, 7H), 2.15 - 2.03 (m, 2H), 1.63 (s, 6H).

The following **Examples 52-70** were synthesized with reference to the synthetic method for compound **37** by using **37f** as a starting material and **z21** replaced with the structures in the table below:

| **Examples** | **Starting material** | **Compound structure** |
|---|---|---|
| **52** | 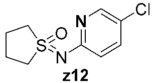 | 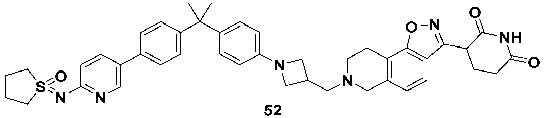 |
| **53** | 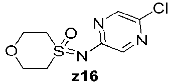 | 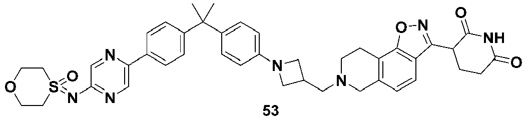 |
| **54** | 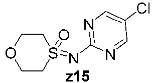 | 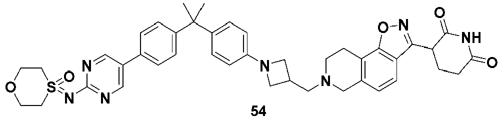 |
| **55** | 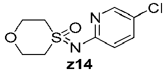 | 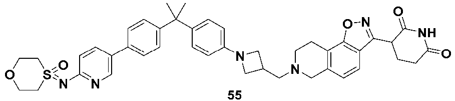 |
| **56** | 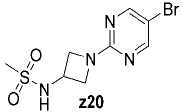 | 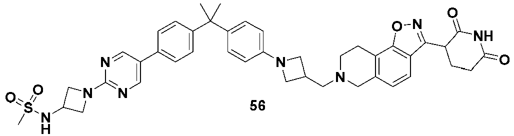 |
| **57** | 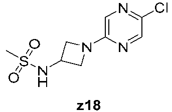 | 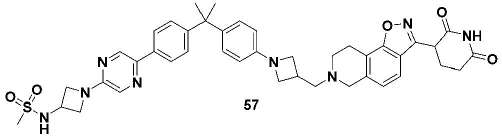 |
| **58** | 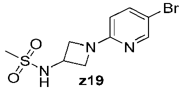 | 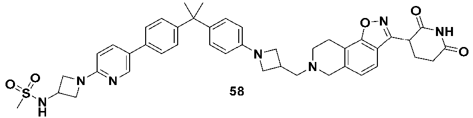 |
| **59** | 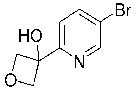 | 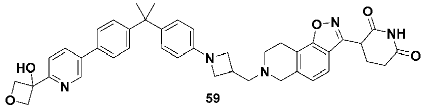 |
| **60** | 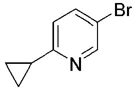 | 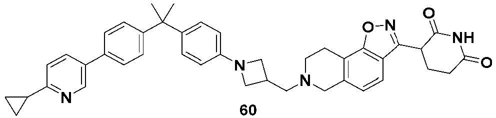 |
| **61** | 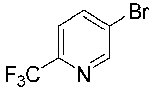 | 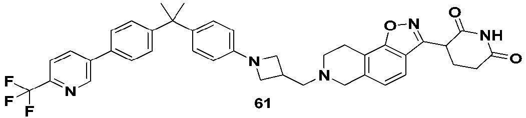 |
| **62** | 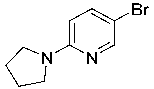 | 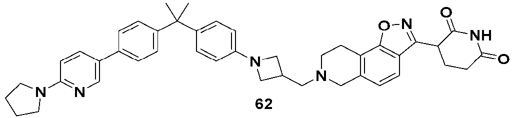 |
| **63** | 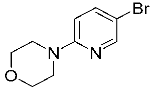 | 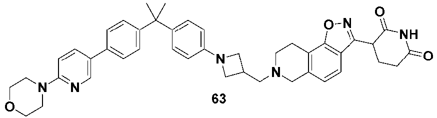 |
| **64** | 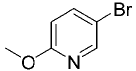 | 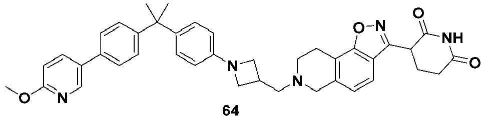 |
| **65** | 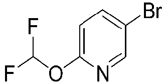 | 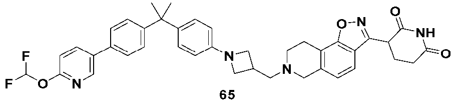 |
| **66** | 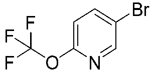 | 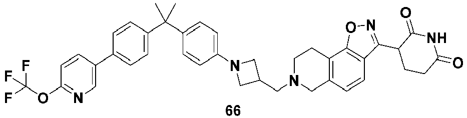 |
| **67** | 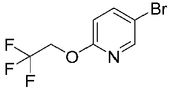 | 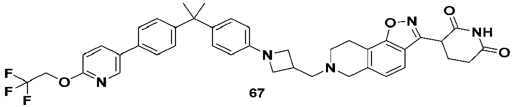 |
| **68** | 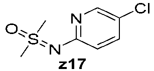 | 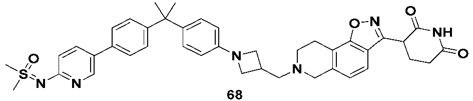 |
| **69** | 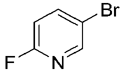 | 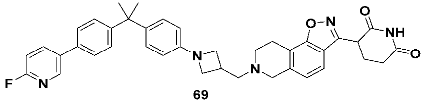 |
| **70** | 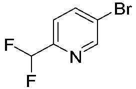 | 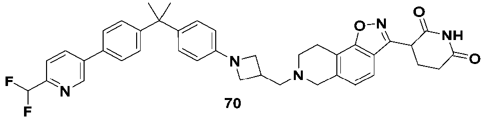 |

| **Examples** | **Data analysis** |
|---|---|
| **52** | MS (ESI, [M+H] ⁺) m/z: 743.25 |
| | ¹H NMR (500 MHz, DMSO) δ 11.07 (s, 1H), 8.41 (d, J = 2.6 Hz, 1H), 7.82 (dd, J = 8.5, 2.6 Hz, 1H), 7.58 (d, J = 8.1 Hz, 1H), 7.51 (d, J = 8.2 Hz, 2H), 7.30 - 7.23 (m, 2H), 7.07 (dd, J = 22.4, 8.3 Hz, 3H), 6.77 (d, J = 8.4 Hz, 1H), 6.36 (d, J = 8.3 Hz, 2H), 4.55 (dd, J = 11.9, 4.9 Hz, 1H), 3.92 (t, J = 7.3 Hz, 2H), 3.72 (s, 2H), 3.57 (dt, J = 13.4, 6.9 Hz, 2H), 3.47 (t, J = 6.4 Hz, 2H), 3.34 (d, J = 4.8 Hz, 2H), 3.01 (dt, J = 17.9, 6.4 Hz, 3H), 2.85 - 2.72 (m, 5H), 2.60 (dt, J = 17.3, 4.2 Hz, 1H), 2.46 (dd, J = 12.1, 4.4 Hz, 1H), 2.19 (qd, J = 9.5, 4.6 Hz, 3H), 2.10 (q, J = 7.4 Hz, 2H), 1.61 (s, 6H). |
| **53** | MS (ESI, [M+H] ⁺) m/z: 760.49 |
| | ¹H NMR (500 MHz, DMSO) δ 11.07 (s, 1H), 8.64 (d, J = 1.5 Hz, 1H), 8.16 (d, J = 1.5 Hz, 1H), 7.90 - 7.84 (m, 2H), 7.58 (d, J = 8.2 Hz, 1H), 7.29 (d, J = 8.4 Hz, 2H), 7.10 (d, J = 8.3 Hz, 1H), 7.07 - 7.02 (m, 2H), 6.36 (d, J = 8.5 Hz, 2H), 4.56 (dd, J = 11.9, 5.0 Hz, 1H), 4.11 (ddd, J = 12.9, 6.1, 3.4 Hz, 2H), 4.00 - 3.90 (m, 4H), 3.87 - 3.79 (m, 2H), 3.73 (s, 2H), 3.60 (ddd, J = 12.6, 8.2, 3.4 Hz, 2H), 3.48 (t, J = 6.4 Hz, 2H), 2.99 (s, 3H), 2.87 - 2.70 (m, 5H), 2.60 (dt, J = 17.3, 4.3 Hz, 1H), 2.49 - 2.44 (m, 1H), 2.19 (dq, J = 8.7, 4.7 Hz, 1H), 1.61 (s, 6H). |
| **54** | MS (ESI, [M+H] ⁺) m/z: 760.56 |
| | ¹H NMR (500 MHz, DMSO) δ 11.07 (s, 1H), 8.75 (s, 2H), 7.57 (t, J = 8.2 Hz, 3H), 7.31 - 7.25 (m, 2H), 7.10 (d, J = 8.3 Hz, 1H), 7.05 (d, J = 8.5 Hz, 2H), 6.39 - 6.32 (m, 2H), 4.55 (dd, J = 11.9, 5.0 Hz, 1H), 4.11 (ddd, J = 12.9, 6.3, 3.2 Hz, 2H), 3.99 - 3.90 (m, 4H), 3.86 - 3.80 (m, 2H), 3.72 (s, 2H), 3.60 (ddd, J = 12.3, 7.9, 3.2 Hz, 2H), 3.48 (dd, J = 7.2, 5.6 Hz, 2H), 3.01 (dt, J = 18.1, 6.4 Hz, 3H), 2.84 - 2.73 (m, 5H), 2.60 (dt, J = 17.3, 4.3 Hz, 1H), 2.46 (dd, J = 12.2, 4.4 Hz, 1H), 2.18 (dq, J = 13.8, 4.7 Hz, 1H), 1.61 (s, 6H). |
| **55** | MS (ESI, [M+H] ⁺) m/z: 759.33 |
| | ¹H NMR (500 MHz, DMSO) δ 11.07 (s, 1H), 8.37 (d, J = 2.6 Hz, 1H), 7.84 (dd, J = 8.5, 2.6 Hz, 1H), 7.58 (d, J = 8.2 Hz, 1H), 7.55 - 7.47 (m, 2H), 7.30 - 7.23 (m, 2H), 7.13 - 7.02 (m, 3H), 6.80 (d, J = 8.4 Hz, 1H), 6.41 - 6.33 (m, 2H), 4.55 (dd, J = 11.9, 5.0 Hz, 1H), 4.09 (ddd, J = 12.6, 6.6, 3.5 Hz, 2H), 3.94 (dt, J = 14.9, 7.6 Hz, 4H), 3.84 - 3.68 (m, 4H), 3.59 - 3.45 (m, 4H), 3.08 - 2.95 (m, 3H), 2.88 - 2.73 (m, 5H), 2.60 (dt, J = 17.2, 4.3 Hz, 1H), 2.46 (dd, J = 12.0, 4.2 Hz, 1H), 2.18 (dq, J = 13.4, 4.4 Hz, 1H), 1.61 (s, 6H). |
| **56** | MS (ESI, [M+H] ⁺) m/z: 775.34 |
| | ¹H NMR (500 MHz, DMSO) δ 11.07 (s, 1H), 8.66 (s, 2H), 7.88 (d, J = 8.2 Hz, 1H), 7.58 (d, J = 8.1 Hz, 1H), 7.51 (d, J = 8.3 Hz, 2H), 7.26 (d, J = 8.3 Hz, 2H), 7.10 (d, J = 8.3 Hz, 1H), 7.04 (d, J = 8.3 Hz, 2H), 6.35 (d, J = 8.3 Hz, 2H), 4.56 (dd, J = 11.9, 5.0 Hz, 1H), 4.43 - 4.28 (m, 3H), 4.01 - 3.88 (m, 4H), 3.73 (s, 2H), 3.47 (t, J = 6.3 Hz, 2H), 3.08 - 2.97 (m, 3H), 2.95 (s, 3H), 2.87 - 2.71 (m, 5H), 2.60 (dt, J = 17.2, 4.3 Hz, 1H), 2.46 (dd, J = 12.3, 4.4 Hz, 1H), 2.18 (dt, J = 13.6, 4.7 Hz, 1H), 1.60 (s, 6H). |
| **57** | MS (ESI, [M+H] ⁺) m/z: 775.28 |
| | ¹H NMR (500 MHz, DMSO) δ 11.07 (s, 1H), 8.61 (d, J = 1.5 Hz, 1H), 7.97 (d, J = 1.5 Hz, 1H), 7.92 - 7.86 (m, 1H), 7.85 - 7.80 (m, 2H), 7.57 (d, J = 8.1 Hz, 1H), 7.30 - 7.25 (m, 2H), 7.09 (d, J = 8.3 Hz, 1H), 7.07 - 7.02 (m, 2H), 6.39 - 6.32 (m, 2H), 4.55 (dd, J = 11.9, 5.0 Hz, 1H), 4.40 (t, J = 5.3 Hz, 3H), 3.98 - 3.90 (m, 4H), 3.72 (s, 2H), 3.47 (dd, J = 7.1, 5.6 Hz, 2H), 3.01 (dt, J = 17.7, 6.4 Hz, 3H), 2.95 (s, 3H), 2.84 - 2.72 (m, 5H), 2.60 (dt, J = 17.3, 4.3 Hz, 1H), 2.46 (dd, J = 12.2, 4.4 Hz, 1H), 2.18 (dq, J = 13.8, 4.9 Hz, 1H), 1.61 (s, 6H). |
| **58** | MS (ESI, [M+H] ⁺) m/z: 774.34 |
| | ¹H NMR (500 MHz, DMSO) δ 11.07 (s, 1H), 8.37 (d, J = 2.5 Hz, 1H), 7.92 - 7.77 (m, 2H), 7.58 (d, J = 8.2 Hz, 1H), 7.47 (d, J = 8.4 Hz, 2H), 7.24 (d, J = 8.4 Hz, 2H), 7.10 (d, J = 8.2 Hz, 1H), 7.08 - 7.00 (m, 2H), 6.49 (d, J = 8.6 Hz, 1H), 6.35 (d, J = 8.6 Hz, 2H), 4.56 (dd, J = 11.9, 5.0 Hz, 1H), 4.39 - 4.23 (m, 3H), 3.92 (t, J = 7.4 Hz, 2H), 3.83 (dd, J = 8.1, 5.3 Hz, 2H), 3.73 (s, 2H), 3.47 (t, J = 6.4 Hz, 2H), 2.99 (s, 3H), 2.94 (s, 3H), 2.88 - 2.70 (m, 5H), 2.60 (dt, J = 17.4, 4.4 Hz, 1H), 2.46 (dd, J = 12.2, 4.3 Hz, 1H), 2.19 (dt, J = 13.4, 4.1 Hz, 1H), 1.61 (d, J = 5.9 Hz, 6H). |
| **59** | MS (ESI, [M+H] ⁺) m/z: 698.29 |
| | ¹H NMR (500 MHz, DMSO) δ 11.07 (s, 1H), 8.93 - 8.88 (m, 1H), 8.06 (dd, J = 8.2, 2.4 Hz, 1H), 7.68 - 7.55 (m, 4H), 7.36 - 7.30 (m, 2H), 7.13 - 7.03 (m, 3H), 6.56 (s, 1H), 6.40 - 6.34 (m, 2H), 4.95 (d, J = 6.1 Hz, 2H), 4.68 (d, J = 6.1 Hz, 2H), 4.55 (dd, J = 11.9, 5.0 Hz, 1H), 3.93 (t, J = 7.4 Hz, 2H), 3.72 (s, 2H), 3.48 (dd, J = 7.1, 5.6 Hz, 2H), 3.01 (dt, J = 18.8, 6.4 Hz, 3H), 2.84 - 2.72 (m, 5H), 2.60 (dt, J = 17.3, 4.3 Hz, 1H), 2.48 - 2.44 (m, 1H), 2.18 (dq, J = 13.5, 4.7 Hz, 1H), 1.62 (s, 6H). |
| **60** | MS (ESI, [M+H] ⁺) m/z: 666.34 |
| | ¹H NMR (500 MHz, DMSO) δ 11.07 (s, 1H), 8.67 (d, J = 2.4 Hz, 1H), 7.87 (dd, J = 8.1, 2.5 Hz, 1H), 7.56 (dd, J = 12.4, 8.1 Hz, 3H), 7.34 (d, J = 8.1 Hz, 1H), 7.28 (d, J = 8.1 Hz, 2H), 7.09 (d, J = 8.3 Hz, 1H), 7.05 (d, J = 8.3 Hz, 2H), 6.36 (d, J = 8.3 Hz, 2H), 4.55 (dd, J = 11.9, 5.0 Hz, 1H), 3.92 (t, J = 7.4 Hz, 2H), 3.72 (s, 2H), 3.47 (t, J = 6.3 Hz, 2H), 3.00 (dt, J = 17.1, 6.2 Hz, 3H), 2.89 - 2.70 (m, 5H), 2.60 (dt, J = 17.3, 4.3 Hz, 1H), 2.46 (dd, J = 12.2, 4.4 Hz, 1H), 2.15 (dtd, J = 34.4, 8.7, 4.9 Hz, 2H), 1.61 (s, 6H), 1.04 - 0.88 (m, 4H). |
| **61** | MS (ESI, [M+H] ⁺) m/z: 694.30 |
| | ¹H NMR (500 MHz, DMSO) δ 11.07 (s, 1H), 9.06 (d, J = 2.3 Hz, 1H), 8.32 (dd, J = 8.1, 2.3 Hz, 1H), 7.96 (d, J = 8.1 Hz, 1H), 7.77 - 7.68 (m, 2H), 7.58 (d, J = 8.2 Hz, 1H), 7.42 - 7.34 (m, 2H), 7.14 - 7.04 (m, 3H), 6.42 - 6.34 (m, 2H), 4.56 (dd, J = 11.9, 5.0 Hz, 1H), 3.93 (t, J = 7.4 Hz, 2H), 3.73 (s, 2H), 3.56 - 3.45 (m, 2H), 3.01 (dt, J = 19.1, 5.3 Hz, 3H), 2.86 - 2.70 (m, 5H), 2.60 (dt, J = 17.2, 4.2 Hz, 1H), 2.50 - 2.44 (m, 1H), 2.18 (tt, J = 9.6, 4.2 Hz, 1H), 1.63 (s, 6H). |
| **62** | MS (ESI, [M+H] ⁺) m/z: 695.37 |
| | ¹H NMR (500 MHz, DMSO) δ 11.07 (s, 1H), 8.37 (d, J = 2.5 Hz, 1H), 7.76 (dd, J = 8.7, 2.6 Hz, 1H), 7.58 (d, J = 8.2 Hz, 1H), 7.46 (d, J = 8.3 Hz, 2H), 7.28 - 7.18 (m, 2H), 7.10 (d, J = 8.3 Hz, 1H), 7.04 (d, J = 8.3 Hz, 2H), 6.50 (d, J = 8.8 Hz, 1H), 6.35 (d, J = 8.3 Hz, 2H), 4.56 (dd, J = 11.9, 5.0 Hz, 1H), 3.92 (t, J = 7.4 Hz, 2H), 3.73 (s, 2H), 3.52 - 3.45 (m, 2H), 3.45 - 3.37 (m, 4H), 3.01 (d, J = 19.6 Hz, 3H), 2.90 - 2.70 (m, 5H), 2.60 (dt, J = 17.4, 4.3 Hz, 1H), 2.46 (dd, J = 12.1, 4.4 Hz, 1H), 2.18 (dq, J = 13.7, 4.8 Hz, 1H), 1.95 (td, J = 8.1, 4.8 Hz, 4H), 1.60 (s, 6H). |
| **63** | MS (ESI, [M+H] ⁺) m/z: 711.25 |
| | ¹H NMR (500 MHz, DMSO) δ 11.07 (s, 1H), 8.43 (d, J = 2.5 Hz, 1H), 7.83 (dd, J = 8.8, 2.6 Hz, 1H), 7.58 (d, J = 8.2 Hz, 1H), 7.53 - 7.47 (m, 2H), 7.27 - 7.21 (m, 2H), 7.10 (d, J = 8.3 Hz, 1H), 7.04 (d, J = 8.4 Hz, 2H), 6.90 (d, J = 8.9 Hz, 1H), 6.39 - 6.32 (m, 2H), 4.55 (dd, J = 11.9, 5.0 Hz, 1H), 3.92 (t, J = 7.4 Hz, 2H), 3.77 - 3.67 (m, 6H), 3.48 (q, J = 4.0 Hz, 6H), 3.01 (dt, J = 17.2, 6.3 Hz, 3H), 2.86 - 2.71 (m, 5H), 2.60 (dt, J = 17.2, 4.3 Hz, 1H), 2.46 (dd, J = 12.2, 4.5 Hz, 1H), 2.18 (dq, J = 13.7, 4.8 Hz, 1H), 1.60 (s, 6H). |
| **64** | MS (ESI, [M+H]⁺) m/z: 656.33 |
| | ¹H NMR (500 MHz, DMSO) δ 11.07 (s, 1H), 8.44 (dd, J = 2.5, 0.7 Hz, 1H), 7.96 (dd, J = 8.6, 2.6 Hz, 1H), 7.58 (d, J = 8.2 Hz, 1H), 7.56 - 7.51 (m, 2H), 7.31 - 7.25 (m, 2H), 7.10 (d, J = 8.3 Hz, 1H), 7.08 - 7.02 (m, 2H), 6.89 (dd, J = 8.6, 0.7 Hz, 1H), 6.39 - 6.33 (m, 2H), 4.55 (dd, J = 11.9, 5.0 Hz, 1H), 3.92 (t, J = 7.4 Hz, 2H), 3.88 (s, 3H), 3.72 (s, 2H), 3.47 (dd, J = 7.1, 5.6 Hz, 2H), 3.01 (dt, J = 17.6, 6.4 Hz, 3H), 2.84 - 2.73 (m, 5H), 2.60 (dt, J = 17.3, 4.3 Hz, 1H), 2.49 - 2.44 (m, 1H), 2.18 (dq, J = 13.4, 4.8 Hz, 1H), 1.61 (s, 6H). |
| **65** | MS (ESI, [M+H]⁺) m/z: 692.31 |
| | ¹H NMR (500 MHz, DMSO) δ 11.07 (s, 1H), 8.54 (d, J = 2.5 Hz, 1H), 8.18 (dd, J = 8.5, 2.6 Hz, 1H), 7.89 - 7.56 (m, 4H), 7.33 - 7.29 (m, 2H), 7.16 (d, J = 8.5 Hz, 1H), 7.10 (d, J = 8.3 Hz, 1H), 7.07 - 7.03 (m, 2H), 6.39 - 6.34 (m, 2H), 4.55 (dd, J = 11.9, 5.0 Hz, 1H), 3.93 (t, J = 7.4 Hz, 2H), 3.72 (s, 2H), 3.48 (dd, J = 7.1, 5.6 Hz, 2H), 3.01 (dt, J = 18.2, 6.5 Hz, 3H), 2.84 - 2.73 (m, 5H), 2.60 (dt, J = 17.3, 4.3 Hz, 1H), 2.49 - 2.44 (m, 1H), 2.18 (dq, J = 13.6, 4.9 Hz, 1H), 1.62 (s, 6H). |
| **66** | MS (ESI, [M+H]⁺) m/z: 710.23 |
| | ¹H NMR (500 MHz, DMSO) δ 11.07 (s, 1H), 8.65 (d, J = 2.6 Hz, 1H), 8.27 (dd, J = 8.5, 2.6 Hz, 1H), 7.62 (d, J = 8.3 Hz, 2H), 7.58 (d, J = 8.2 Hz, 1H), 7.36 (d, J = 8.5 Hz, 1H), 7.32 (d, J = 8.3 Hz, 2H), 7.10 (d, J = 8.3 Hz, 1H), 7.05 (d, J = 8.5 Hz, 2H), 6.36 (d, J = 8.5 Hz, 2H), 4.56 (dd, J = 11.9, 5.0 Hz, 1H), 3.93 (t, J = 7.4 Hz, 2H), 3.72 (s, 2H), 3.48 (t, J = 6.4 Hz, 2H), 3.01 (dt, J = 18.6, 6.1 Hz, 3H), 2.84 - 2.73 (m, 5H), 2.60 (dt, J = 17.3, 4.3 Hz, 1H), 2.46 (dd, J = 11.9, 4.4 Hz, 1H), 2.18 (dq, J = 13.7, 4.8 Hz, 1H), 1.62 (s, 6H). |
| **67** | MS (ESI, [M+H]⁺) m/z: 724.32 |
| | ¹H NMR (500 MHz, DMSO) δ 11.07 (s, 1H), 8.51 - 8.45 (m, 1H), 8.07 (dd, J = 8.6, 2.5 Hz, 1H), 7.57 (dd, J = 8.5, 7.1 Hz, 3H), 7.33 - 7.27 (m, 2H), 7.12 - 7.03 (m, 4H), 6.40 - 6.34 (m, 2H), 5.03 (q, J = 9.1 Hz, 2H), 4.55 (dd, J = 11.9, 5.0 Hz, 1H), 3.93 (t, J = 7.4 Hz, 2H), 3.72 (s, 2H), 3.48 (dd, J = 7.1, 5.6 Hz, 2H), 3.01 (dt, J = 18.4, 6.5 Hz, 3H), 2.84 - 2.73 (m, 5H), 2.60 (dt, J = 17.4, 4.3 Hz, 1H), 2.50 - 2.43 (m, 1H), 2.18 (dq, J = 13.7, 4.8 Hz, 1H), 1.61 (s, 6H). |
| **68** | MS (ESI, [M+H]⁺) m/z: 717.71 |
| | ¹H NMR (500 MHz, DMSO) δ 11.07 (s, 1H), 8.39 (dd, J = 2.6, 0.8 Hz, 1H), 7.80 (dd, J = 8.5, 2.6 Hz, 1H), 7.58 (d, J = 8.1 Hz, 1H), 7.53 - 7.48 (m, 2H), 7.28 - 7.23 (m, 2H), 7.10 (d, J = 8.3 Hz, 1H), 7.08 - 7.03 (m, 2H), 6.70 (dd, J = 8.5, 0.8 Hz, 1H), 6.39 - 6.33 (m, 2H), 4.55 (dd, J = 11.9, 5.0 Hz, 1H), 3.92 (t, J = 7.4 Hz, 2H), 3.72 (s, 2H), 3.48 (dd, J = 7.1, 5.6 Hz, 2H), 3.39 (s, 6H), 3.01 (dt, J = 17.7, 6.4 Hz, 3H), 2.84 - 2.73 (m, 5H), 2.60 (dt, J = 17.3, 4.3 Hz, 1H), 2.49 - 2.43 (m, 1H), 2.18 (dq, J = 13.5, 4.8 Hz, 1H), 1.61 (s, 6H). |
| **69** | MS (ESI, [M+H]⁺) m/z: 644.31 |
| | ¹H NMR (500 MHz, DMSO) δ 11.07 (s, 1H), 8.51 (d, J = 2.6 Hz, 1H), 8.24 (td, J = 8.2, 2.6 Hz, 1H), 7.64 - 7.54 (m, 3H), 7.35 - 7.29 (m, 2H), 7.26 (dd, J = 8.5, 2.9 Hz, 1H), 7.13 - 7.01 (m, 3H), 6.40 - 6.32 (m, 2H), 4.55 (dd, J = 11.9, 5.0 Hz, 1H), 3.93 (t, J = 7.4 Hz, 2H), 3.72 (s, 2H), 3.48 (dd, J = 7.2, 5.7 Hz, 2H), 3.01 (dt, J = 18.0, 6.3 Hz, 3H), 2.83 - 2.71 (m, 5H), 2.64 - 2.58 (m, 1H), 2.46 (dd, J = 12.2, 4.5 Hz, 1H), 2.23 - 2.15 (m, 1H), 1.62 (s, 6H). |
| **70** | MS (ESI, [M+H]⁺) m/z: 676.25 |
| | ¹H NMR (500 MHz, DMSO) δ 11.07 (s, 1H), 8.97 (d, J = 2.3 Hz, 1H), 8.24 (dd, J = 8.1, 2.3 Hz, 1H), 7.76 (d, J = 8.1 Hz, 1H), 7.73 - 7.65 (m, 2H), 7.57 (d, J = 8.2 Hz, 1H), 7.39 - 7.33 (m, 2H), 7.14 - 7.03 (m, 3H), 6.41 - 6.34 (m, 2H), 4.55 (dd, J = 11.9, 5.0 Hz, 1H), 3.93 (t, J = 7.4 Hz, 2H), 3.72 (s, 2H), 3.48 (dd, J = 7.1, 5.6 Hz, 2H), 3.01 (dt, J = 19.0, 6.0 Hz, 3H), 2.85 - 2.72 (m, 5H), 2.60 (dt, J = 17.3, 4.3 Hz, 1H), 2.52 (s, 1H), 2.23 - 2.15 (m, 1H), 1.63 (s, 6H). |

### Example 71: Synthesis of Compound 71

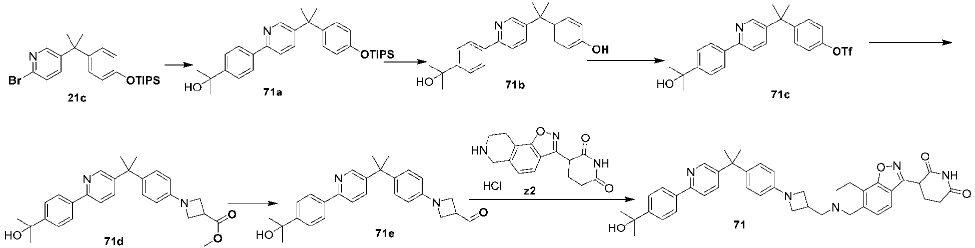

### Step 1: Preparation of intermediate 71a

Intermediate **21c** (684 mg), 4-(2-hydroxy-2-propyl)phenylboronic acid pinacol ester (400 mg), PdCl₂(dppf)-CH₂Cl₂ (125 mg), K₂CO₃ (633 mg), dioxane (5 mL), and water (1 mL) were added to a single-necked flask in sequence, and the mixture was heated to 85 °C and reacted under N₂ atmosphere. After the reaction was completed, a reaction solution containing **71a** was obtained and directly used in the next step. MS(ESI, [M+H]⁺) *m*/*z:* 504.25.

### Step 2: Preparation of intermediate 71b

TBAF (6.08 mL) was added to the reaction solution containing intermediate **71a** obtained in the previous step, and the mixture was reacted at room temperature. After the reaction was completed, the system was extracted with ethyl acetate and water. The organic phase was separated and purified by silica gel column chromatography to give intermediate **71b** (0.32 g). MS(ESI, [M+H]⁺) *m*/*z:* 348.30.

### Step 3: Preparation of intermediate 71c

**71b** (0.32 g), dichloromethane (5 mL), triethylamine (0.385 mL), and DMAP (92 mg) were added to a single-necked flask in sequence, and then N-phenylbis(trifluoromethanesulfonyl)imide (0.362 g) was added. The mixture was reacted at room temperature. After the reaction was completed, the mixture was purified by silica gel column chromatography to give intermediate **71c** (0.36 g). MS(ESI, [M+H]⁺) *m*/*z:* 480.18.

### Step 4: Preparation of intermediate 71d

Referring to the reaction of step 5 in Example 17, **17d** was replaced with **71c** to give intermediate **71d** (0.32 g). MS(ESI, [M+H]⁺) *m*/*z:* 445.27.

### Steps 6-7: Preparation of compound 71

Referring to the reactions of steps 9-10 in Example 17, **17h** was replaced with **71d** to give compound **71** (50 mg). **71e:** MS(ESI, [M+H]⁺) *m*/*z:* 415.33.

Compound **71:** HRMS (ESI, [M+H]⁺) *m*/*z:* 684.3519. ¹H NMR (500 MHz, DMSO-d₆) *δ* 11.07 (s, 1H), 8.50 (d, *J =* 2.5 Hz, 1H), 8.00 - 7.92 (m, 2H), 7.80 (d, *J* = 8.2 Hz, 1H), 7.63 (dd, *J* = 8.3, 2.5 Hz, 1H), 7.56 (dd, *J* = 14.2, 8.3 Hz, 3H), 7.09 (t, *J =* 8.9 Hz, 3H), 6.41 - 6.33 (m, 2H), 5.04 (s, 1H), 4.56 (dd, *J* = 11.9, 5.0 Hz, 1H), 3.93 (t, *J* = 7.4 Hz, 2H), 3.72 (s, 2H), 3.55 - 3.44 (m, 2H), 3.01 (dt, *J =* 19.5, 6.2 Hz, 3H), 2.89 - 2.68 (m, 5H), 2.60 (dt, *J =* 17.3, 4.2 Hz, 1H), 2.46 (dd, *J* = 12.1, 4.4 Hz, 1H), 2.18 (dq, *J =* 13.6, 4.3 Hz, 1H), 1.66 (s, 6H), 1.45 (s, 6H).

### Example 72: Synthesis of Compound 72

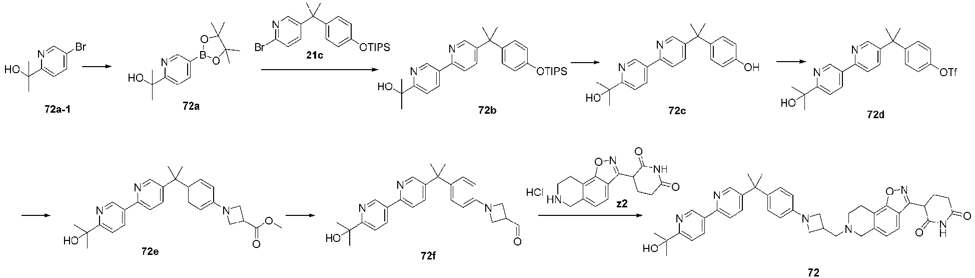

### Step 1: Preparation of intermediate 72a

Referring to the reaction of step 1 in Example 24, **20e** was replaced with **72a-1,** and after the reaction was completed, the reaction solution was filtered and concentrated to give intermediate **72a,** which was directly used in the next step.

### Step 2: Preparation of compound 72b

Intermediate **72a** (3.2 g), intermediate **21c** (4.1 g), potassium carbonate (2.5 g), Pd(dppf)Cl₂ (0.67 g), 1,4-dioxane (20 mL), and water (4 mL) were added to a reaction flask in sequence, and the mixture was reacted at 100 °C for 2 h. After the reaction was completed, the reaction solution was purified by silica gel column chromatography to give compound **72b** (3.5 g). MS(ESI, [M+H]⁺) m/z: 505.27.

### Step 3: Preparation of compound 72c

Referring to the reaction of step 6 in Example 17, **17e** was replaced with **72b** to give compound **72c** (2.7 g). MS(ESI, [M+H]⁺) m/z: 349.31.

### Step 4: Preparation of compound 72d

Referring to the reaction of step 3 in Example 71, **71b** was replaced with **72c** for reaction at 0 °C to give compound **72d** (0.5 g). MS(ESI, [M+H]⁺) m/z: 481.16.

### Step 5: Preparation of compound 72e

Referring to the reaction of step 5 in Example 17, **17d** was replaced with **72d,** and Xphos was replaced with Ruphos to give compound **72e** (0.26 g). MS(ESI, [M+H]⁺) m/z: 446.24.

### Step 6: Preparation of intermediate 72f

Referring to the reaction of step 9 in Example 17, **17h** was replaced with **72e** to give **72f** (0.21 g). MS(ESI, [M+H]⁺) m/z: 416.24

### Step 7: Preparation of compound 72

Referring to the reaction of step 10 in Example 17, **17i** was replaced with **72f to** give compound **72** (0.08 g). MS(ESI, [M+H]⁺) m/z: 685.31. ¹H NMR (500 MHz, DMSO) δ 11.07 (s, 1H), 9.11 (d, *J* = 2.3 Hz, 1H), 8.55 (d, *J* = 2.5 Hz, 1H), 8.35 (dd, *J=* 8.3, 2.3 Hz, 1H), 7.89 (d, *J* = 8.3 Hz, 1H), 7.74 (d, *J* = 8.3 Hz, 1H), 7.68 (dd, *J* = 8.4, 2.5 Hz, 1H), 7.58 (d, *J=* 8.1 Hz, 1H), 7.13 - 7.05 (m, 3H), 6.43 - 6.36 (m, 2H), 5.26 (s, 1H), 4.55 (dd, *J=* 11.9, 5.0 Hz, 1H), 3.93 (t, *J =* 7.4 Hz, 2H), 3.72 (s, 2H), 3.48 (dd, *J* = 7.2, 5.6 Hz, 2H), 3.01 (dt, *J* = 19.8, 6.2 Hz, 3H), 2.84 - 2.73 (m, 5H), 2.64 - 2.58 (m, 1H), 2.46 (dd, *J* = 12.0, 4.3 Hz, 1H), 2.18 (dq, *J* = 13.6, 4.6 Hz, 1H), 1.66 (s, 6H), 1.47 (s, 6H).

### Example 73: Synthesis of Compound 73

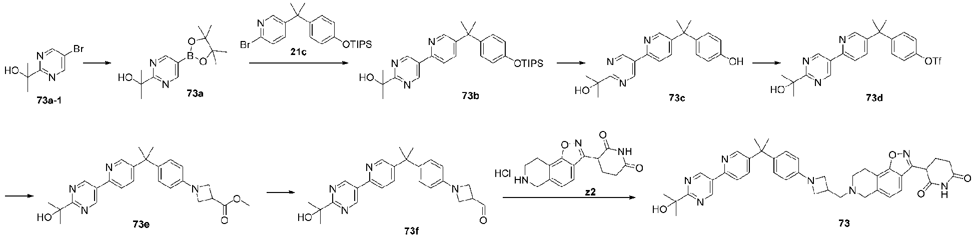

Referring to steps 1-7 of Example 72, **72a-1** was replaced with **73a-1** to give compound **73** (0.1 g).

**73b:** MS(ESI, [M+H]⁺) m/z: 506.27.

**73c:** MS(ESI, [M+H]⁺) m/z: 350.3.

**73d:** MS(ESI, [M+H]⁺) m/z: 482.16.

**73e:** MS(ESI, [M+H]⁺) m/z: 447.29.

**73f:** MS(ESI, [M+H]⁺) m/z: 417.33.

Compound **73:** MS (ESI, [M+H]⁺) m/z: 686.31. ¹H NMR (500 MHz, DMSO) δ 11.07 (s, 1H), 9.36 (s, 2H), 8.58 (d, J = 2.4 Hz, 1H), 8.00 (d, J = 8.3 Hz, 1H), 7.75 (dd, J = 8.3, 2.5 Hz, 1H), 7.57 (d, J = 8.1 Hz, 1H), 7.14 - 7.05 (m, 3H), 6.41 - 6.36 (m, 2H), 5.12 (s, 1H), 4.55 (dd, J = 11.9, 5.0 Hz, 1H), 3.93 (t, J = 7.4 Hz, 2H), 3.72 (s, 2H), 3.52 - 3.45 (m, 2H), 3.01 (dt, J = 20.9, 6.6 Hz, 3H), 2.84 - 2.71 (m, 5H), 2.60 (dt, J = 17.3, 4.3 Hz, 1H), 2.46 (dd, J = 12.3, 4.3 Hz, 1H), 2.18 (dq, J = 13.8, 4.8 Hz, 1H), 1.67 (s, 6H), 1.53 (s, 6H).

### Example 74: Synthesis of Compound 74

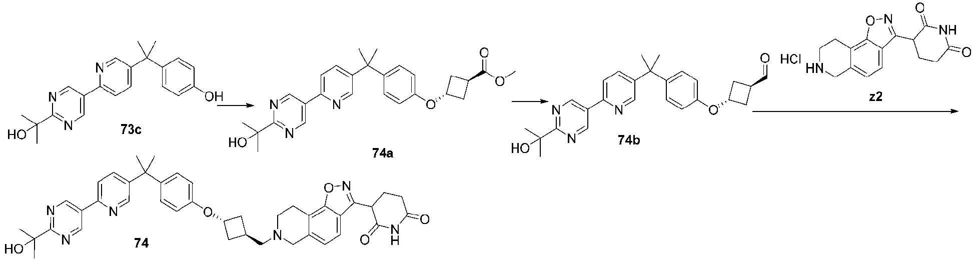

### Step 1: Preparation of compound 74a

**73c** (0.5 g), triphenylphosphine (1.12 g), tert-butyl cis-3-hydroxycyclobutanecarboxylate (0.56 g), and dioxane (10 mL) were added to a reaction flask in sequence, and diisopropyl azodicarboxylate (0.86 g) was slowly added at room temperature under N₂ atmosphere. The mixture was reacted at 100 °C for 3 h. After the reaction was completed, the reaction solution was directly mixed with silica gel and purified by silica gel column chromatography to give compound **74a** (0.18 g). MS(ESI, [M+H]⁺) m/z: 462.30.

### Steps 2-3: Preparation of compound 74

Referring to the reactions of steps 2-3 in Example 51, **51a** was replaced with **74a** to give compound **74** (0.1 g). **74b:** MS(ESI, [M+H]⁺) m/z: 432.31.

Compound **74:** MS (ESI, [M+H]⁺) m/z: 701.28. ¹H NMR (500 MHz, DMSO) δ 11.07 (s, 1H), 9.36 (s, 2H), 8.59 (d, J = 2.5 Hz, 1H), 8.01 (d, J = 8.3 Hz, 1H), 7.76 (dd, J = 8.3, 2.5 Hz, 1H), 7.57 (d, J = 8.2 Hz, 1H), 7.17 (d, J = 8.5 Hz, 2H), 7.09 (d, J = 8.2 Hz, 1H), 6.77 (d, J = 8.4 Hz, 2H), 5.12 (s, 1H), 4.81 (p, J = 6.2 Hz, 1H), 4.55 (dd, J = 11.9, 5.0 Hz, 1H), 3.70 (s, 2H), 2.98 (t, J = 5.9 Hz, 2H), 2.83 - 2.72 (m, 3H), 2.69 - 2.57 (m, 4H), 2.46 (dd, J = 12.2, 4.3 Hz, 1H), 2.30 - 2.14 (m, 5H), 1.69 (s, 6H), 1.53 (s, 6H).

### Example 75: Synthesis of Compound 75

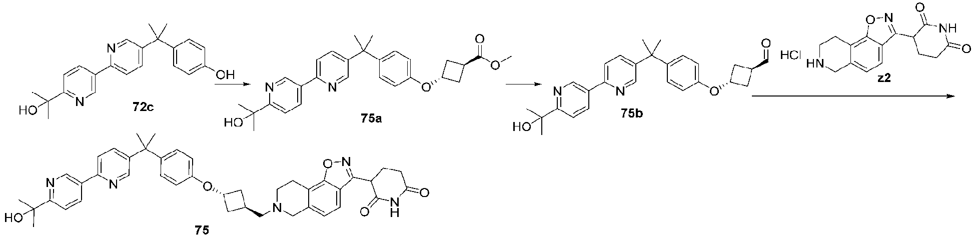

Referring to the method of steps 1-3 in Example 74, intermediate 73c was replaced with intermediate 72c to finally give compound **75** (0.06 g).

**75a:** MS(ESI, [M+H]⁺) m/z: 461.33.

**75b:** MS(ESI, [M+H]⁺) m/z: 431.33.

Compound **75:** MS (ESI, [M+H]⁺) m/z: 700.71.

¹H NMR (500 MHz, DMSO) δ 11.07 (s, 1H), 9.13 - 9.08 (m, 1H), 8.55 (d, J = 2.5 Hz, 1H), 8.35 (dd, J = 8.3, 2.3 Hz, 1H), 7.90 (d, J = 8.4 Hz, 1H), 7.74 (dd, J = 8.3, 0.8 Hz, 1H), 7.70 (dd, J = 8.4, 2.5 Hz, 1H), 7.57 (d, J = 8.2 Hz, 1H), 7.21 - 7.15 (m, 2H), 7.09 (d, J = 8.2 Hz, 1H), 6.80 - 6.74 (m, 2H), 5.26 (s, 1H), 4.81 (p, J = 6.1 Hz, 1H), 4.55 (dd, J = 11.9, 5.0 Hz, 1H), 3.70 (s, 2H), 2.98 (t, J = 5.9 Hz, 2H), 2.83 - 2.72 (m, 3H), 2.69 - 2.56 (m, 4H), 2.48 - 2.44 (m, 1H), 2.30 - 2.14 (m, 5H), 1.68 (s, 6H), 1.47 (s, 6H).

### Test Example 1: Inhibitory or Degradation Effects of Compounds on Proteins in Cells

### 1.1. Assay for inhibitory or degradation activity against AR-V7 protein in 22RV1 cells

22RV1 cells in a good growth state were taken, washed with PBS, and digested with pancreatin, and a 1640 complete medium containing 10% FBS was added to terminate the digestion. The cells were collected into a centrifuge tube, adjusted to a cell density of 2 × 10⁵ cells/mL, seeded onto a 384-well plate (40 µL/well), and incubated overnight at 37 °C. Compounds were added using a nanoliter pipettor such that the final concentrations of the compounds were 1000 nM-0.061 nM; 2 duplicate wells were set, and meanwhile, a control was set. After the cells were cultured for another 24 h, the supernatant was discarded, 40 µL of polyoxymethylene (manufacturer: Sangon Biotech) was added to each well, and the cells were incubated at room temperature for 20 min. The supernatant was discarded, 40 µL of PBS was added to each well, and the cells were washed twice. 40 µL of glacial methanol (manufacturer: Merck) was added to each well, and the cells were incubated at room temperature for 10 min. The supernatant was discarded, 40 µL of TBST was added to each well, and the cells were washed twice. 40 µL of 2% BSA-TBST blocking buffer was added to each well, and the cells were incubated at room temperature for 60 min. The supernatant was discarded, 20 µL of a mixed solution of GAPDH mouse antibody (manufacturer: R&D) and AR-V7 rabbit antibody (manufacturer: CST) prepared in a blocking buffer was added to each well, and the cells were incubated overnight at 4 °C. The supernatant was discarded, 40 µL of TBST was added to each well, and the cells were washed twice. 20 µL of a mixed solution of anti-mouse IgG (H+L) (Dylight^{™} 680 Conjugate) antibody and anti-rabbit IgG (H+L) (Dylight^{™} 800 4× PEG Conjugate) antibody (manufacturer: CST) prepared in a blocking buffer was added to each well, and the cells were incubated at room temperature for 45 min in the dark. The supernatant was discarded, 40 µL of TBST was added to each well, and the cells were washed twice. Then, 40 µL of PBS was added to each well, and the cells were washed once. Detection was performed using an Azure Sapphire^{™} dual-mode multi-spectral laser imaging system (In-Cell WB-800 nm, 680 nm), the four-parameter analysis was performed, dose-response curves were fit, and IC₅₀ or DC₅₀ was calculated.

### 1.2. Assay for inhibitory or degradation activity against AR protein in VCaP cells

The detection was performed using the HTRF HUMAN ANDROGEN RECEPTOR DETECTION KIT (cisbio, 64ANDRPEG), 4× Supplemented Lysis buffer was prepared using the Blocking reagent stock solution (100×) and Lysis buffer stock solution (4×), and 1× Supplemented Lysis buffer was prepared using the 4× Supplemented Lysis buffer and ddH₂O. Premixed antibody solutions were prepared using Detection buffer, Androgen Receptor Eu Cryptate antibody, and Androgen Receptor d2 antibody.

VCaP cells in a good growth state were taken, washed with PBS, and digested with pancreatin, and a phenol redfree DMEM complete medium containing 2% CSS-FBS was added to terminate the digestion. The cells were collected into a centrifuge tube, adjusted to a cell density of 1 × 10⁶ cells/mL, and seeded onto a 96-well plate (100 µL/well). The cells were incubated overnight in a cell incubator. Compounds were added using a nanoliter pipettor such that the final concentrations of the compounds were 1000 nM-0.32 nM; 2 duplicate wells were set, and meanwhile, a control was set. After another 24 h of incubation in the cell incubator, the medium was pipetted out, the 1× Supplemented Lysis buffer was added at 40 µL/well to lyse the cells. After the cells were oscillated for 40 min at room temperature, 16 µL of the cell lysate was added to a 384-well plate, the Premixed antibody solutions were added at 4 µL/well, and the plate was left to stand at 25 °C for 2 h. Fluorescence values were measured at 665 nm/620 nm using an Envision microplate reader, the four-parameter analysis was performed, the dose-response curves were fit, and IC₅₀ or DC50 (the concentration of the drug when the degradation rate reached 50%) was calculated.

### 1.3. Assay for degradation activity against GSPT1 protein in 22RV1 cells

22RV1 cells in a good growth state were taken, washed with PBS, and digested with pancreatin, and a 1640 complete medium containing 10% FBS was added to terminate the digestion. The cells were collected into a centrifuge tube, adjusted to a cell density of 8 × 10⁵ cells/mL, seeded onto a 6-well plate (1 mL/well), and incubated overnight at 37 °C. The compounds were prepared in the 1640 complete medium containing 10% FBS and added to the cells (1 mL/well), such that the final concentrations of the compounds were 1000 nM-8 nM (4 concentration points in total) and 200 nM-0.32 nM (5 concentration points in total). Meanwhile, a DMSO control group was set. After the cells were cultured for another 6 h, the supernatant was discarded, and the cells were washed twice with PBS. 50 µL of RIPA lysis buffer (containing 1× phosphatase inhibitor and 1× PMSF) was added, and the cells were lysed on ice for 30 min and centrifuged at 12000 rpm at 4 °C for 20 min. The supernatant protein samples were collected, adjusted to a protein concentration of 0.7 µg/µL by BCA quantification, and stored in an ultra-low temperature refrigerator at -80 °C. GSPT1 protein expression was detected using fully automated quantitative protein expression analysis. 3.0 µL of cell protein stock solution was taken, and 3 µL of 0.1× Sample Buffer (manufacturer: ProteinSimple) was added, followed by the addition of 1.5 µL of 5× Master Mix (manufacturer: ProteinSimple) to achieve a final cell protein concentration of 0.28 µg/µL. The prepared samples were denatured at 95 °C for 5 min. After the denaturation was completed, the samples were taken out, cooled on ice for 5 min, vortexed, shaken, mixed well, centrifuged briefly, and placed on ice for later use. The primary antibodies were diluted with Antibody Diluent II and placed on ice for later use. The primary antibodies were GSPT1 (manufacturer: CST, 1:20) and GAPDH (manufacturer: CST, 1:400). The secondary antibody was goat anti-rabbit secondary antibody (manufacturer: ProteinSimple), with a 1:1 mixture of Lumino-S and Peroxide as the color development solution. The prepared reagents were sequentially added to a detection plate (manufacturer: ProteinSimple), centrifuged at 1000 g at room temperature for 10 min, and tested on a machine. Western band processing was performed using fully automated protein expression analysis software Compass for SW. An electrophoretogram was automatically simulated based on the signals, and the degradation rate (%) was calculated using the following formula: degradation rate (%) = (control group GSPT1/GAPDH - administration group GSPT1/GAPDH)/control group GSPT1/GAPDH × 100%. Inhibition rates and sample concentrations were plotted using GraphPad Prism 8 software.

The test results show that the compounds of the present application (e.g., example compounds 41, 42, 73, and 74, etc.) have the degradation activity against the GSPT1 protein.

### Test Example 2: Inhibitory Activity for In Vitro Cell Proliferation

### 2.1 Assay for inhibitory activity for VCaP cell proliferation

VCaP cells in a good growth state were taken, washed with PBS, and digested with pancreatin, and a phenol redfree DMEM complete medium containing 5% CSS-FBS was added to terminate the digestion. The cells were collected into a centrifuge tube, adjusted to a cell density of 1 × 10⁵ cells/mL, and seeded onto a 96-well plate (100 µL/well). Meanwhile, compounds were added using a nanoliter pipettor and then subjected to a 2-fold gradient dilution such that the final concentrations of the compounds were 1000 nM-0.061 nM; 2 duplicate wells were set, and meanwhile, a control was set. After another 168 h of incubation in the cell incubator, the detection reagent CCK-8 (manufacturer: Dojindo, 10 µL/well) was added. After 7.0 h of incubation in the cell incubator, absorbance values were measured at 450 nm using a PerkinElmer Envision microplate reader, the four-parameter analysis was performed, the dose-response curves were fit, and IC50 was calculated. The results are shown in Table 1A.

### 2.2. Assay for inhibitory activity for 22RV1 cell proliferation

22RV1 cells in a good growth state were taken, washed with PBS, and digested with pancreatin, and a 1640 complete medium containing 5% FBS was added to terminate the digestion. The cells were collected into a centrifuge tube, adjusted to a cell density of 5 × 10⁴ cells/mL, and seeded onto a 96-well plate (100 µL/well). Meanwhile, compounds were added using a nanoliter pipettor and then subjected to a 2-fold gradient dilution such that the final concentrations of the compounds were 1000 nM-0.061 nM; 2 duplicate wells were set, and meanwhile, a control was set. After another 168 h of incubation in the cell incubator, the detection reagent CellCounting-Lite 2.0 (manufacturer: Vazyme, 50 µL/well) was added. After 10 min of incubation at room temperature, luminescence values were measured using a PerkinElmer Envision microplate reader, the four-parameter analysis was performed, dose-response curves were fit, and IC50 was calculated. The results are shown in Table 1.

**Table 1. Inhibitory activity of compounds for 22RV1 cell proliferation**

| Compound No. | 22RV1 (IC50, nM) | Compound No. | 22RV1 (IC50, nM) | Compound No. | 22RV1 (IC50, nM) |
|---|---|---|---|---|---|
| 11 | <100 | 37 | <100 | 58 | <100 |
| 13 | <100 | 38 | <100 | 59 | <100 |
| 15 | <100 | 39 | <100 | 60 | <100 |
| 17 | <100 | 40 | <100 | 61 | <100 |
| 18 | <100 | 41 | <100 | 62 | <100 |
| 21 | <100 | 42 | <100 | 63 | <100 |
| 22 | <100 | 43 | <100 | 64 | <100 |
| 23 | <100 | 44 | <100 | 65 | <100 |
| 24 | <100 | 45 | <100 | 66 | <100 |
| 25 | <100 | 46 | <100 | 67 | <100 |
| 26 | <100 | 47 | <100 | 68 | <100 |
| 27 | <100 | 48 | <100 | 69 | <100 |
| 28 | <100 | 50 | <100 | 70 | <100 |
| 29 | <100 | 51 | <100 | 71 | <100 |
| 30 | <100 | 52 | <100 | 72 | <100 |
| 31 | <100 | 53 | <100 | 73 | <100 |
| 32 | <100 | 54 | <100 | 74 | <100 |
| 33 | <100 | 55 | <100 | 75 | <100 |
| 34 | <100 | 56 | <100 | | |
| 36 | <100 | 57 | <100 | | |

**Table 1A. Inhibitory activity of compounds for VCaP cell proliferation**

| Compound No. | VCaP (IC50, nM) |
|---|---|
| 42 | <10 |
| 73 | <10 |

The test results show that the compounds of the present application, e.g., the example compounds, have the inhibitory activity for the proliferation of VCaP cells and 22RV1 cells.

### Test Example 3: In Vitro Metabolic Stability

Liver microsome incubation samples were each prepared by mixing a PBS buffer (pH 7.4), a liver microsome solution (0.5 mg/mL; species: human, rat, and mouse), a test compound, and an NADPH+ MgCl₂ solution and incubated at 37 °C and 300 rpm for 1 h. Zero-hour samples were each prepared by mixing a PBS buffer (pH 7.4), a liver microsome solution (0.5 mg/mL), and a test compound. An acetonitrile solution containing an internal standard was added to the samples, and supernatants were prepared by protein precipitation, diluted, and then assayed by LC/MS/MS. The results are shown in Tables 2 and 3.

**Table 2**

| Compound No. | MLM (mouse), remaining amount % at 60 min | Compound No. | MLM (mouse), remaining amount % at 60 min |
|---|---|---|---|
| 11 | >70% | 53 | >70% |
| 37 | >70% | 55 | >70% |
| 38 | >70% | 56 | >70% |
| 39 | >70% | 57 | >70% |
| 40 | >70% | 58 | >70% |
| 41 | >70% | 70 | >70% |
| 42 | >70% | 71 | >70% |
| 43 | >70% | 73 | >70% |
| 44 | >70% | 74 | >70% |
| 52 | >70% | | |

**Table 3**

| Compound No. | HLM (human), remaining amount % at 60 min | Compound No. | HLM (human), remaining amount % at 60 min |
|---|---|---|---|
| 23 | >60% | 44 | >60% |
| 32 | >60% | 45 | >60% |
| 37 | >60% | 53 | >60% |
| 39 | >60% | 70 | >60% |
| 40 | >60% | 71 | >60% |
| 41 | >60% | 72 | >60% |
| 42 | >60% | 73 | >60% |
| 43 | >60% | 74 | >60% |

The test results show that the compounds of the present application, including the example compounds, have stable *in vitro* metabolism.

### Test Example 4: In Vivo pharmacokinetic properties

ICR mice weighing 18-22 g were randomized into groups of 6 after 3-5 days of acclimatization. The mice in the intragastric administration group were intragastrically given the test compound solution at a dose of 10 mg/kg, and the mice in the intravenous injection administration group were intravenously injected with the test compound solution at a dose of 1 mg/kg.

The blood collection time points for the intragastric administration group were 15 min, 1 h, 4 h, 6 h, 8 h, 10 h, and 24 h after the administration, and the blood collection time points for the intravenous injection administration group were 5 min, 0.25 h, 1 h, 4 h, 6 h, 10 h, and 24 h after the administration. Blood was collected from the orbit to prepare plasma samples to be tested.

30 µL of each of the plasma samples to be tested and a standard curve sample were taken, and an acetonitrile solution containing an internal standard was added. Supernatants were obtained by protein precipitation, diluted, and then assayed by LC/MS/MS. Pharmacokinetic parameters were fitted using a non-compartmental model. The results are shown in Table 4.

**Table 4. Pharmacokinetic test results of compounds in mice**

| Compound No. | Intravenous administration to mice (0-24 h), 1 mpk | | | | Intragastric administration to mice (0-24 h), 10 mpk | | | |
|---|---|---|---|---|---|---|---|---|
| | AUC (ng*h/mL) | t_{1/2} (h) | Vₛₛ (L/kg) | CL (L/hr/kg) | AUC (ng*h/mL) | t_{1/2} (h) | Cₘₐₓ (ng/mL) | F |
| 37 | 2100 | 4.15 | 2.5 | 0.468 | 7525 | 4.44 | 537 | 35.83% |
| 39 | 4168 | 4.42 | 1.42 | 0.235 | 13770 | 4.4 | 963 | 33.04% |
| 40 | 2940 | 8.88 | 3.43 | 0.291 | 15088 | 9.08 | 924 | 51.32% |
| 41 | 2771 | 7.31 | 3.1 | 0.326 | 15067 | 6.83 | 966 | 54.37% |
| 42 | 3465 | 8.55 | 2.81 | 0.251 | 20694 | 7.61 | 1276 | 59.73% |
| 44 | 1508 | 3.94 | 2.87 | 0.655 | 8666 | / | 598 | 57.45% |
| 73 | 1523 | 3.68 | 2.93 | 0.650 | 14456 | / | 922 | 94.91% |
| 74 | 2747 | 5.67 | 2.42 | 0.347 | 21319 | / | 1439 | 77.61% |

The test results show that the compounds of the present application, e.g., the example compounds, have good *in vivo* pharmacokinetic properties in animals, e.g., mice; preferably, AUC (0-24 h) was > 10000 ng × h/mL; and/or, the bioavailability F was > 30%.

### Test Example 5: Pharmacodynamic Evaluation in Subcutaneous Xenograft Tumor Model Using 22RV1 Human Prostate Cancer Cells Implanted into Castrated Nude Mice

22RV1 cells were subcutaneously inoculated in the right axilla of SPF-grade male nude mice (source: Shanghai Lingchang Biotech Co., Ltd.) at 5 × 10⁶ cells/mouse. When the mean tumor volume reached about 200 mm³, the animals were subjected to castration surgery. When the tumors continued to grow to about 300 mm³, the animals were grouped.

The day of grouping was defined as day 0, and intragastric administration was started on day 0 (d0) and performed once daily (at a dose of 0.5-3 mpk). The tumor volume and body weight were measured twice to thrice every week, and the data were recorded. The general behavior of the mice was observed and recorded every day. After the experiment was completed (e.g., on day 17), the tumors were removed, weighed, and photographed.

The detection parameters and the calculation formulas are as follows:$Tumor volume TV \left({mm}^{3}\right) = 1 / 2 \times \left(a \times {b}^{2}\right) ,$ where a represents the long diameter of the tumor, and b represents the short diameter of the tumor.

Relative tumor volume RTV = TVₜ/TV₀, wherein TV₀ represents the tumor volume on day 0, and TVₜ represents the tumor volume at each measurement.

Relative tumor proliferation rate T/C (%) = (T_{RTV}/C_{RTV}) × 100%, where T_{RTV} represents RTV of the treatment group, and C_{RTV} represents RTV of the vehicle control group.

Tumor volume inhibition rate TGI (TV) (%), TGI (%) = [1 - (T - T0)/(C - C0)] × 100%, where T represents the mean tumor volume of each treatment group; T0 represents the mean tumor volume of each treatment group on d0; C represents the mean tumor volume of the control group; C0 represents the mean tumor volume of the control group on d0.

Tumor weight inhibition rate TGI (TW) (%), TGI (%) = (1 - TWt/TWc) × 100%, where TWt represents the tumor weight of each treatment group; TWc represents the tumor weight of the control group.

Weight change rate WCR (%) = (Wtt - Wt₀)/Wt₀ × 100%, where Wt₀ represents the body weight of the mouse on day 0, and Wtt represents the body weight of the mouse at each measurement. The test results show that the compounds of the present application (e.g., Examples 41, 42, 73, and 74) have the inhibitory activity against tumors *in vivo,* with TGI (TW) ≥ 85% and TGI (TV) ≥ 95% on day 19 at a dose of 2 mpk; meanwhile, the body weight of the mice in each administration group was relatively stable.

## Claims

1. A compound of formula II, a stereoisomer thereof, or a pharmaceutically acceptable salt thereof: 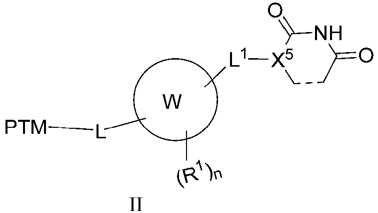 wherein,
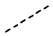 represents a single bond or double bond;
ring W is selected from the group consisting of: 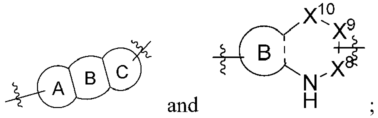
ring A is absent or selected from the group consisting of C₃₋₁₅ cycloalkenyl, 3- to 15-membered heterocycloalkenyl, phenyl, and 5- to 6-membered heteroaryl;
ring B is selected from the group consisting of phenyl and 5- to 6-membered heteroaryl;
ring C is absent or selected from 5- to 6-membered heteroaryl;
X⁸ is selected from the group consisting of -C(O)- and -CH₂-;
X⁹ is selected from the group consisting of -C(R^{d}R^{e})-, -N(R^{e})-, -O-, and -S-;
X¹⁰ is selected from the group consisting of a bond, -CH₂-, -NH-, -O-, and -S-;
R^{d} and R^{e} are each independently selected from the group consisting of hydrogen, deuterium, halogen, -OH, - NH₂, -CN, C₁₋₁₀ alkyl, C₁₋₁₀ alkoxy, (C₁₋₁₀ alkyl)NH-, (C₁₋₁₀ alkyl)₂N-, and C₁₋₁₀ haloalkyl, or R^{d} and R^{e} are linked to each other to form C₃₋₁₀ cycloalkyl or 3- to 10-membered heterocycloalkyl;
each R¹ is independently selected from the group consisting of deuterium, halogen, -OH, -NH₂, -CN, and the following groups optionally substituted with one or more substituents: C₁₋₁₀ alkyl, C₁₋₁₀ alkoxy, (C₁₋₁₀ alkyl)NH-, (C₁₋₁₀ alkyl)₂N-, C₁₋₁₀ haloalkyl, C₂₋₁₀ alkenyl, C₂₋₁₀ alkynyl, C₃₋₁₀ cycloalkyl, and 3- to 10-membered heterocycloalkyl;
n is selected from the group consisting of 0, 1, 2, and 3;
X⁵ is selected from the group consisting of C(R^{f}) and N;
R^{f} is selected from the group consisting of H, halogen, deuterium, and C₁₋₆ alkyl optionally substituted with one or more substituents;
L¹ is selected from the group consisting of a bond, -NH-, -O-, -S-, -CONH-, and -CON(C₁₋₆ alkyl)-;
L is selected from a connecting group;
PTM is selected from a small molecule compound targeting GSPT1.

2. The compound, the stereoisomer thereof, or the pharmaceutically acceptable salt thereof according to claim 1, being selected from a compound of formula III, a stereoisomer thereof, or a pharmaceutically acceptable salt thereof: 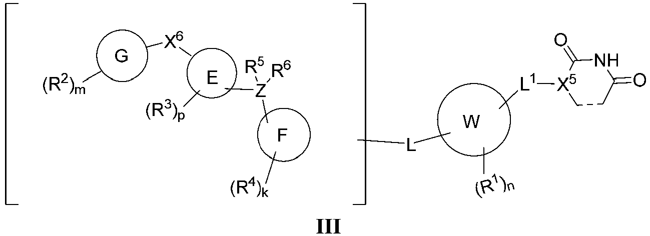
wherein X⁶ is selected from the group consisting of a bond, -O-, -S-, -N(R^{t})-, -C₁₋₆ alkylene-, -C₂₋₆ alkenylene-, -C₂₋₆ alkynylene-, -N(R^{t})C₁₋₆ alkylene-, -OC₁₋₆ alkylene-, -SC₁₋₆ alkylene-, and -N(R^{t})C(O)-;
R^{t} is selected from the group consisting of H and C₁₋₆ alkyl;
R², R³, and R⁴ are each independently selected from the group consisting of halogen, -CN, and the following groups optionally substituted with one or more substituents: R^{v}-, R^{v}O-, R^{v}S-, R^{s}R^{v}N-, R^{v}C(O)-, R^{v}S(O)₂-, R^{v}S(O)-, R^{v}=N-, R^{v}OC(O)-, R^{v}C(O)O-, R^{v}S(O)O-, R^{v}OS(O)-, R^{v}S(O)₂O-, R^{v}OS(O)₂-, R^{s}R^{v}NC(O)-, R^{v}C(O)NH-, R^{v}OC(O)NH-, R^{v}NHC(O)O-, R^{v}S(O)NH-, R^{s}R^{v}NS(O)-, R^{v}S(O)₂NH-, R^{s}R^{v}NS(O)₂-, and R^{s}R^{v}S(O)=N-;
R^{s} and R^{v} are each independently selected from the group consisting of H, C₁₋₁₂ alkyl, C₂₋₁₂ alkenyl, C₂₋₁₂ alkynyl, C₃₋₁₂ cycloalkyl, C₃₋₁₂ cycloalkyl C₁₋₃ alkylene-, 3- to 12-membered heterocycloalkyl, 3- to 12-membered heterocycloalkyl C₁₋₃ alkylene-, C₃₋₁₂ cycloalkenyl, C₃₋₁₂ cycloalkenyl C₁₋₃ alkylene-, 3- to 12-membered heterocycloalkenyl, 3- to 12-membered heterocycloalkenyl C₁₋₃ alkylene-, C₆₋₁₂ aryl, C₆₋₁₂ aryl C₁₋₃ alkylene-, 5- to 12-membered heteroaryl, and 5- to 12-membered heteroaryl C₁₋₃ alkylene-;
m, p, and k are each independently selected from the group consisting of 0, 1, 2, 3, 4, 5, and 6;
ring G is selected from the group consisting of C₃₋₁₂ cycloalkyl, 3- to 12-membered heterocycloalkyl, C₃₋₁₂ cycloalkenyl, 3- to 12-membered heterocycloalkenyl, C₆₋₁₂ aryl, and 5- to 12-membered heteroaryl;
ring E and ring F are each independently selected from the group consisting of C₆₋₁₂ aryl and 5- to 12-membered heteroaryl;
Z is selected from the group consisting of -C-, -O-, -S-, -N-, -OC₁₋₆ alkylene-, and -NC(O)-;
R⁵ and R⁶ are each independently absent or each independently selected from the group consisting of H, =O, halogen, -OH, -NH₂, -CN, C₁₋₁₀ alkyl, C₂₋₁₀ alkenyl, and C₂₋₁₀ alkynyl, or R⁵ and R⁶ are linked to each other to form the following group optionally substituted with one or more substituents: C₃₋₁₀ cycloalkyl or 3- to 10-membered heterocycloalkyl.

3. The compound, the stereoisomer thereof, or the pharmaceutically acceptable salt thereof according to claim 1 or 2, wherein ring A is absent or selected from the group consisting of C₃₋₁₂ cycloalkenyl, 3- to 12-membered heterocycloalkenyl, phenyl, and 5- to 6-membered heteroaryl;
or, ring A is absent or selected from the group consisting of C₅₋₁₅ cycloalkenyl, 5- to 15-membered heterocycloalkenyl, phenyl, and 5- to 6-membered heteroaryl;
or, ring A is absent or selected from the group consisting of C₅₋₁₀ cycloalkenyl, 5- to 10-membered heterocycloalkenyl, phenyl, and 5- to 6-membered heteroaryl;
or, ring A is absent or selected from the group consisting of C₅₋₉ cycloalkenyl, 5- to 9-membered heterocycloalkenyl, phenyl, and 5- to 6-membered heteroaryl;
or, ring A is absent or selected from the group consisting of C₅₋₇ cycloalkenyl, 5- to 9-membered heterocycloalkenyl, phenyl, and 5- to 6-membered heteroaryl;
or, ring A is absent or selected from the group consisting of C₅₋₆ cycloalkenyl, 5- to 10-membered heterocycloalkenyl, phenyl, and 5- to 6-membered heteroaryl;
or, ring A is absent or selected from the group consisting of C₅₋₆ cycloalkenyl, 5- to 9-membered heterocycloalkenyl, phenyl, and 5- to 6-membered heteroaryl;
or, ring A is absent or selected from the group consisting of C₅₋₆ cycloalkenyl, 5- to 9-membered heterocycloalkenyl, phenyl, and 5-membered heteroaryl;
or, ring A is absent or selected from the group consisting of C₅₋₆ cycloalkenyl, 5- to 9-membered heterocycloalkenyl, phenyl, pyrrolyl, pyrazolyl, furanyl, and oxazolyl;
or, ring A is absent or selected from the group consisting of C₅ cycloalkenyl, C₆ cycloalkenyl, 5-, 6-, 7-, 8-, or 9-membered heterocycloalkenyl, phenyl, pyrrolyl, pyrazolyl, furanyl, and oxazolyl;
or, ring A is absent or selected from the group consisting of cyclopentenyl, monocyclohexenyl, bicyclohexenyl, dihydropyrrolyl, tetrahydropyridinyl, tetrahydroazepinyl, azaspirooctenyl, azaspirononenyl, phenyl, pyrrolyl, pyrazolyl, furanyl, oxazolyl, and dihydrooxazinyl; and/or
ring B is selected from the group consisting of phenyl and 6-membered heteroaryl;
or, ring B is selected from the group consisting of phenyl and pyridinyl;
or, ring B is selected from phenyl; and/or
or, ring C is absent or selected from 5-membered heteroaryl;
or, ring C is absent or selected from the group consisting of isoxazolyl, pyrazolyl, and furanyl;
optionally, the structural fragment 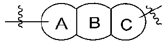 is selected from the group consisting of 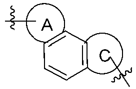 and 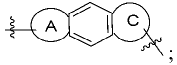
or, the structural fragment 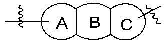 is selected from the group consisting of 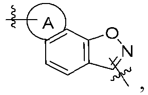 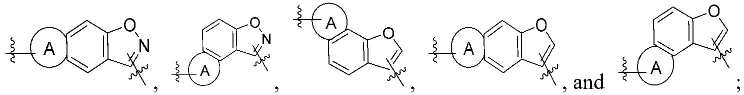
or, the structural fragment 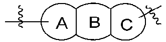 is selected from the group consisting of 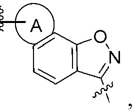 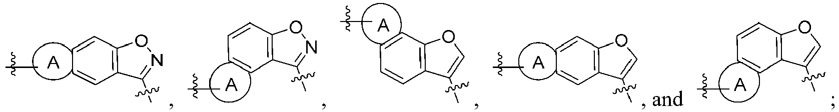
or, the structural fragment 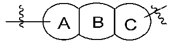 is selected from the group consisting of 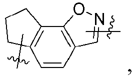 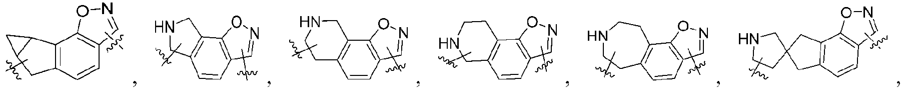 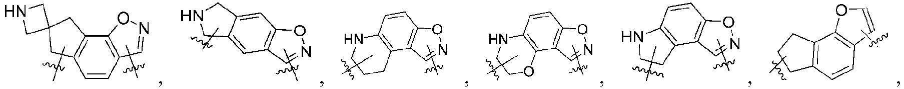 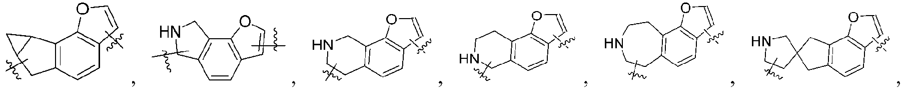 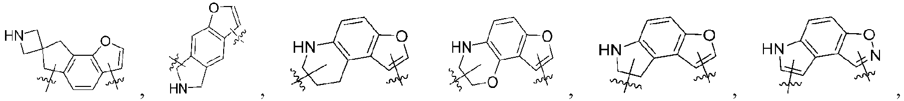 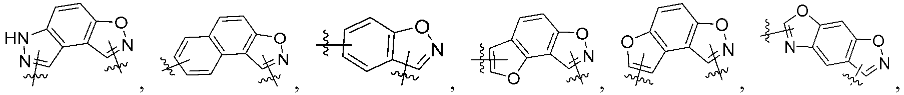 phenyl, pyridinyl,

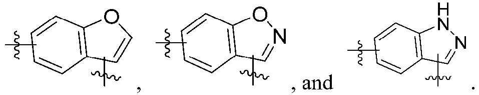

4. The compound, the stereoisomer thereof, or the pharmaceutically acceptable salt thereof according to any one of claims 1 to 3, wherein the structural fragment 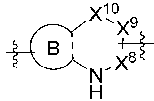 is selected from the group consisting of 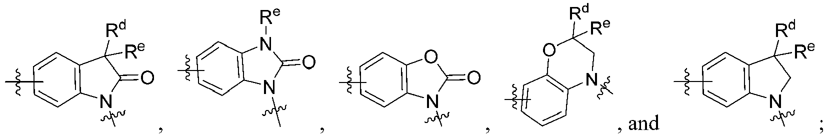
optionally, X⁹ is selected from the group consisting of -C(R^{d}R^{e})-, -N(R^{e})-, and -O-; and/or
X¹⁰ is selected from the group consisting of a bond and -O-;
optionally, R^{d} and R^{e} are each independently selected from the group consisting of hydrogen, deuterium, halogen, -OH, -NH₂, -CN, C₁₋₆ alkyl, C₁₋₆ alkoxy, (C₁₋₆ alkyl) NH-, (C₁₋₆ alkyl)₂N-, and C₁₋₆ haloalkyl, or R^{d} and R^{e} are linked to each other to form C₃₋₆ cycloalkyl or 3- to 6-membered heterocycloalkyl;
or, R^{d} and R^{e} are each independently selected from the group consisting of hydrogen, deuterium, halogen, -OH, -NH₂, -CN, and C₁₋₆ alkyl, or R^{d} and R^{e} are linked to each other to form C₃₋₄ cycloalkyl;
or, R^{d} and R^{e} are each independently selected from the group consisting of hydrogen, F, and methyl, or R^{d} and R^{e} are linked to each other to form cyclopropyl.

5. The compound, the stereoisomer thereof, or the pharmaceutically acceptable salt thereof according to any one of claims 1 to 4, wherein each R¹ is independently selected from the group consisting of deuterium, halogen, - OH, -NH₂, -CN, and the following groups optionally substituted with one or more substituents: C₁₋₆ alkyl, C₁₋₆ alkoxy, (C₁₋₁₀ alkyl) NH-, (C₁₋₁₀ alkyl)₂N-, C₁₋₆ haloalkyl, C₂₋₆ alkenyl, C₂₋₆ alkynyl, C₃₋₆ cycloalkyl, and 3- to 6-membered heterocycloalkyl;
or, each R¹ is independently selected from the group consisting of deuterium, halogen, -OH, -NH₂, -CN, and the following groups optionally substituted with one or more substituents: C₁₋₆ alkyl, C₁₋₆ alkoxy, and C₁₋₆ haloalkyl;
or, each R¹ is independently selected from the group consisting of halogen, -OH, -NH₂, -CN, C₁₋₄ alkyl, C₁₋₄ alkoxy, and C₁₋₄ haloalkyl;
or, each R¹ is independently selected from the group consisting of halogen, -OH, -NH₂, -CN, C₁₋₃ alkyl, C₁₋₃ alkoxy, and C₁₋₃ haloalkyl;
or, each R¹ is independently selected from the group consisting of halogen, -OH, -NH₂, -CN, and C₁₋₃ alkyl;
or, each R¹ is independently selected from the group consisting of halogen and C₁₋₃ alkyl;
or, each R¹ is independently selected from the group consisting of fluorine, chlorine, bromine, -OH, -NH₂, and -CN;
optionally, n is selected from the group consisting of 0, 1, and 2;
or, n is selected from the group consisting of 0 and 1.

6. The compound, the stereoisomer thereof, or the pharmaceutically acceptable salt thereof according to any one of claims 1 to 5, wherein R^{f} is selected from the group consisting of H, fluorine, chlorine, bromine, deuterium, and C₁₋₃ alkyl optionally substituted with one or more substituents;
or, R^{f} is selected from the group consisting of H, fluorine, chlorine, bromine, deuterium, and C₁₋₃ alkyl, wherein the C₁₋₃ alkyl is optionally substituted with one or more of the following groups: halogen, -OH, -NH₂, and -CN; or, R^{f} is selected from the group consisting of H, fluorine, chlorine, bromine, deuterium, and C₁₋₃ alkyl; or, R^{f} is selected from the group consisting of H, fluorine, deuterium, and methyl;
or, R^{f} is selected from H;
optionally, X⁵ is selected from the group consisting of CH and N.

7. The compound, the stereoisomer thereof, or the pharmaceutically acceptable salt thereof according to any one of claims 1 to 6, wherein L¹ is selected from the group consisting of a bond, -NH-, and -CONH-; or, L¹ is selected from a bond.

8. The compound, the stereoisomer thereof, or the pharmaceutically acceptable salt thereof according to any one of claims 1 to 7, wherein L is selected from the group consisting of the following groups optionally substituted with one or more substituents: C₁₋₅₀ alkylene, C₂₋₅₀ alkenylene, and C₂₋₅₀ alkynylene, wherein optionally, 1 or more -CH₂- of the C₁₋₅₀ alkylene, C₂₋₅₀ alkenylene, or C₂₋₅₀ alkynylene are independently optionally replaced by -O-, C₃₋₁₅ cycloalkyl, 3- to 15-membered heterocycloalkyl, 4- to 15-membered heterocycloalkenyl, C₆₋₁₅ aryl, 5-to 15-membered heteroaryl, -NH-, -N(C₁₋₆ alkyl)-, or -S-;
or, L is selected from the group consisting of the following groups optionally substituted with one or more substituents: C₁₋₃₀ alkylene, C₂₋₃₀ alkenylene, and C₂₋₃₀ alkynylene, wherein optionally, 1 or more -CH₂- of the C₁₋₃₀ alkylene, C₂₋₃₀ alkenylene, or C₂₋₃₀ alkynylene are independently optionally replaced by -O-, C₃₋₁₂ cycloalkyl, 3- to 12-membered heterocycloalkyl, 4- to 12-membered heterocycloalkenyl, C₆₋₁₂ aryl, 5- to 12-membered heteroaryl, -NH-, -N(C₁₋₆ alkyl)-, or -S-;
or, L is selected from the group consisting of the following groups optionally substituted with one or more substituents: C₁₋₂₀ alkylene, C₂₋₂₀ alkenylene, and C₂₋₂₀ alkynylene, wherein optionally, 1 or more -CH₂- of the C₁₋₂₀ alkylene, C₂₋₂₀ alkenylene, or C₂₋₂₀ alkynylene are independently optionally replaced by -O-, C₃₋₁₀ cycloalkyl, 3- to 11-membered heterocycloalkyl, 4- to 10-membered heterocycloalkenyl, C₆₋₁₀ aryl, 5- to 10-membered heteroaryl, -NH-, -N(C₁₋₆ alkyl)-, or -S-;
or, L is selected from the group consisting of the following groups optionally substituted with one or more substituents: C₁₋₁₅ alkylene, C₂₋₁₅ alkenylene, and C₂₋₁₅ alkynylene, wherein optionally, 1 or more -CH₂- of the C₁₋₁₅ alkylene, C₂₋₁₅ alkenylene, or C₂₋₁₅ alkynylene are independently optionally replaced by -O-, C₃₋₉ cycloalkyl, 3- to 11-membered heterocycloalkyl, 4- to 8-membered heterocycloalkenyl, C₆₋₈ aryl, 5- to 8-membered heteroaryl, -NH-, -N(C₁₋₄ alkyl)-, or -S-;
or, L is selected from the group consisting of the following groups optionally substituted with one or more substituents: C₁₋₁₀ alkylene, C₂₋₁₀ alkenylene, and C₂₋₁₀ alkynylene, wherein optionally, 1 or more -CH₂- of the C₁₋₁₀ alkylene, C₂₋₁₀ alkenylene, or C₂₋₁₀ alkynylene are independently optionally replaced by -O-, C₃₋₉ cycloalkyl, 3- to 11-membered heterocycloalkyl, 4- to 6-membered heterocycloalkenyl, C₆ aryl, 5- to 6-membered heteroaryl, -NH-, -N(C₁₋₃ alkyl)-, or -S-;
or, L is selected from the group consisting of the following groups optionally substituted with one or more substituents: C₁₋₆ alkylene, C₂₋₆ alkenylene, and C₂₋₆ alkynylene, wherein optionally, 1 or more -CH₂- of the C₁₋₆ alkylene, C₂₋₆ alkenylene, or C₂₋₆ alkynylene are independently optionally replaced by -O-, C₃₋₉ cycloalkyl, 3-to 11-membered heterocycloalkyl, 4- to 6-membered heterocycloalkenyl, C₆ aryl, 5- to 6-membered heteroaryl, -NH-, -N(C₁₋₃ alkyl)-, or -S-;
or, L is selected from the group consisting of the following groups optionally substituted with one or more substituents: C₁₋₄ alkylene, C₂₋₄ alkenylene, and C₂₋₄ alkynylene, wherein optionally, 1 or more (e.g., 1 or 2, 1 or 3, etc.) -CH₂- of the C₁₋₄ alkylene, C₂₋₄ alkenylene, or C₂₋₄ alkynylene are independently optionally replaced by -O-, C₄₋₆ cycloalkyl, 4- to 6-membered heterocycloalkyl, 4- to 6-membered heterocycloalkenyl, C₆ aryl, 5- to 6-membered heteroaryl, -NH-, -N(C₁₋₃ alkyl)-, or -S-;
or, L is selected from the group consisting of C₁₋₁₀ alkylene and C₂₋₁₀ alkynylene optionally substituted with one or more substituents, wherein optionally, 1 or more -CH₂- of the C₁₋₁₀ alkylene or C₂₋₁₀ alkynylene are independently optionally replaced by a group selected from the group consisting of -O-, C₃₋₁₂ cycloalkyl, 4- to 12-membered heterocycloalkyl, 4- to 12-membered heterocycloalkenyl, -NH-, -N(C₁₋₆ alkyl)-, and -S-;
or, L is selected from the group consisting of C₁₋₆ alkylene and C₂₋₆ alkynylene optionally substituted with one or more substituents, wherein optionally, 1 or more -CH₂- of the C₁₋₆ alkylene or C₂₋₆ alkynylene are independently optionally replaced by a group selected from the group consisting of -O-, C₃₋₁₀ cycloalkyl, 4- to 11-membered heterocycloalkyl, 5- to 6-membered heterocycloalkenyl, -NH-, -N(C₁₋₃ alkyl)-, and -S-;
or, L is selected from the group consisting of C₁₋₆ alkylene and C₂₋₆ alkynylene optionally substituted with one or more substituents, wherein optionally, 1 or more -CH₂- of the C₁₋₆ alkylene or C₂₋₆ alkynylene are independently optionally replaced by a group selected from the group consisting of -O-, C₃₋₁₀ cycloalkyl, 4- to 11-membered heterocycloalkyl, -NH-, -N(C₁₋₃ alkyl)-, and -S-;
optionally, in the definition of L, the substituent is selected from the group consisting of halogen, =O, -OH, - NH₂, -CN, C₁₋₆ alkyl, C₁₋₆ alkoxy, C₁₋₆ haloalkyl, (C₁₋₆ alkyl) NH-, (C₁₋₆ alkyl)₂N-, C₃₋₁₂ cycloalkyl, and 4- to 12-membered heterocycloalkyl.

9. The compound, the stereoisomer thereof, or the pharmaceutically acceptable salt thereof according to any one of claims 1 to 8, wherein L is selected from -LNK¹-Cy¹-LNK-Cy²-LNK^{Z}-Cy³-, wherein
Cy¹, Cy², or Cy³ is each independently selected from the group consisting of a bond and the following groups optionally substituted with one or more R^{b}: C₃₋₁₂ cycloalkyl, 4- to 12-membered heterocycloalkyl, and 4- to 12-membered heterocycloalkenyl;
LNK, LNK¹, and LNK² are each independently selected from the group consisting of a bond, -NH-, -O-, -S-, and the following groups optionally substituted with one or more R^{c}: C₁₋₁₂ alkylene, C₂₋₁₂ alkenylene, C₂₋₁₂ alkynylene, and C₁₋₁₂ heteroalkylene;
R^{b} and R^{c} are each independently selected from the group consisting of halogen, =O, -OH, -NH₂, -CN, C₁₋₆ alkyl, C₁₋₆ alkoxy, C₁₋₆ haloalkyl, (C₁₋₆ alkyl) NH-, (C₁₋₆ alkyl)₂N-, C₃₋₁₂ cycloalkyl, and 4- to 12-membered heterocycloalkyl;
or, L is selected from the group consisting of -Cy¹-, -Cy²-, -LNK¹-, -Cy¹-LNK-, -Cy¹-Cy²-, -LNK¹-Cy¹-LNK-, -LNK-Cy²-LNK²-, -Cy¹-Cy² -LNK²-, -LNK¹-Cy¹-Cy²-, -Cy¹-LNK-Cy²-, -LNK¹-Cy¹-Cy²-LNK²-, -LNK¹-Cy¹-LNK-Cy²-, -Cy¹-LNK-Cy²-LNK²-, -Cy¹-Cy²-Cy³-, and -Cy¹-Cy²-LNK²-Cy³-;
or, LNK, LNK¹, and LNK² are each independently selected from the group consisting of a bond, -NH-, -O-, -S-, and the following groups optionally substituted with one or more R^{c}: C₁₋₁₀ alkylene, C₂₋₁₀ alkenylene, C₂₋₁₀ alkynylene, and C₁₋₁₀ heteroalkylene;
or, LNK, LNK¹, and LNK² are each independently selected from the group consisting of a bond, -NH-, -O-, -S-, and the following groups optionally substituted with one or more R^{c}: C₁₋₆ alkylene, C₂₋₆ alkenylene, C₂₋₆ alkynylene, and C₁₋₆ heteroalkylene;
or, LNK, LNK¹, and LNK² are each independently selected from the group consisting of a bond, -NH-, -O-, -S-, and the following groups optionally substituted with one or more R^{c}: C₁₋₄ alkylene, C₂₋₄ alkenylene, C₂₋₄ alkynylene, and C₁₋₄ heteroalkylene;
or, LNK, LNK¹, and LNK² are each independently selected from the group consisting of a bond, -NH-, -O-, -S-, and the following groups optionally substituted with one or more R^{c}: C₁₋₆ alkylene, C₂₋₆ alkynylene, and C₁₋₆ heteroalkylene;
or, LNK, LNK¹, and LNK² are each independently selected from the group consisting of a bond, -NH-, -O-, -S-, and the following groups optionally substituted with one or more R^{c}: C₁₋ₐ alkylene, C₂₋₄ alkynylene, and C₁₋₄ heteroalkylene;
or, LNK, LNK¹, and LNK² are each independently selected from the group consisting of a bond, -NH-, -O-, and the following groups optionally substituted with one or more R^{c}: C₁₋₃ alkylene, C₂₋₃ alkynylene, and C₁₋₃ heteroalkylene;
or, LNK, LNK¹, and LNK² are each independently selected from the group consisting of a bond, -NH-, -O-, and the following groups optionally substituted with one or more R^{c}: C₁₋₂ alkylene, C₂ alkynylene, and C₁₋₂ heteroalkylene;
or, LNK, LNK¹, and LNK² are each independently selected from the group consisting of a bond, -NH-, -O-, - NHCH₂-, -CH₂NHCH₂-, -CH₂-, -CH₂CH₂-, ethynylene, -C(O)-, and -C(O)CH₂-; and/or Cy¹, Cy², or Cy³ is each independently selected from the group consisting of a bond and the following groups optionally substituted with one or more R^{b}: C₃₋₁₁ cycloalkyl, 4- to 12-membered heterocycloalkyl, and 4- to 11-membered heterocycloalkenyl;
or, Cy¹, Cy², or Cy³ is each independently selected from the group consisting of a bond and the following groups optionally substituted with one or more R^{b}: C₄₋₁₀ cycloalkyl, 4- to 11-membered heterocycloalkyl, and 5- to 6-membered heterocycloalkenyl;
or, Cy¹, Cy², or Cy³ is each independently selected from the group consisting of a bond and the following groups optionally substituted with one or more R^{b}: C₄₋₉ cycloalkyl, 4- to 11-membered heterocycloalkyl, and 5- to 6-membered heterocycloalkenyl;
or, Cy¹, Cy², or Cy³ is each independently selected from the group consisting of a bond and the following groups optionally substituted with one or more R^{b}: C₄₋₆ cycloalkyl, C₉ cycloalkyl, 4- to 11-membered heterocycloalkyl, and 6-membered heterocycloalkenyl;
or, Cy¹, Cy², or Cy³ is each independently selected from the group consisting of a bond and the following groups optionally substituted with one or more R^{b}: cyclobutyl, cyclopentyl, cyclohexyl, spirononyl, azetidinyl, pyrrolidinyl, piperidinyl, tetrahydropyridinyl, piperazinyl, monoazaspiroheptyl, monoazaspirooctyl, monoazaspirononyl, diazaspirononyl, monoazaspirodecyl, diazaspirodecyl, monoazaspiroundecyl, diazaspiroundecyl, monoazabicyclohexyl, octahydrocyclopentapyrrolyl, diazabicyclooctyl, and monoazabicyclononyl;
or, Cy¹, Cy², or Cy³ is each independently selected from the group consisting of a bond, 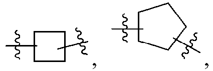 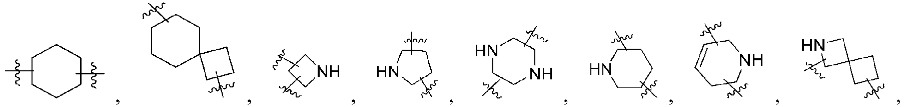 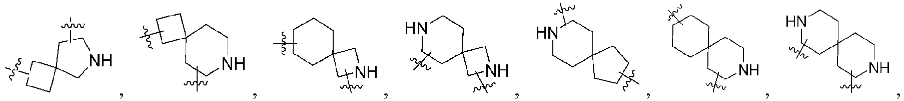 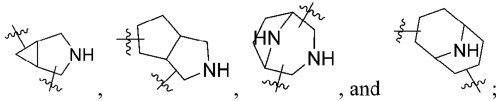 and/or
R^{d} and R^{c} are each independently selected from the group consisting of halogen, =O, -OH, -NH₂, -CN, C₁₋₆ alkyl, C₁₋₆ alkoxy, C₁₋₆ haloalkyl, (C₁₋₆ alkyl) NH-, (C₁₋₆ alkyl)₂N-, C₃₋₁₀ cycloalkyl, and 4- to 10-membered heterocycloalkyl;
or, R^{d} and R^{c} are each independently selected from the group consisting of halogen, =O, -OH, -NH₂, -CN, C₁₋₆ alkyl, C₁₋₆ alkoxy, C₁₋₆ haloalkyl, (C₁₋₆ alkyl) NH-, and (C₁₋₆ alkyl)₂N-;
or, R^{d} and R^{c} are each independently selected from the group consisting of halogen, =O, -OH, -NH₂, -CN, C₁₋₄ alkyl, C₁₋₄ alkoxy, C₁₋₄ haloalkyl, (C₁₋₄ alkyl) NH-, and (C₁₋₄ alkyl)₂N-;
or, R^{d} and R^{c} are each independently selected from the group consisting of halogen, =O, -OH, -NH₂, -CN, and C₁₋₃ alkyl;
or, R^{d} and R^{c} are each independently selected from the group consisting of halogen, =O, -OH, -NH₂, and -CN; or, R^{d} and R^{c} are each independently selected from =O;
optionally, L is selected from the group consisting of a bond, -NHCH₂-, -CH₂NHCH₂-, -CH₂-, -C(O)CH₂-,

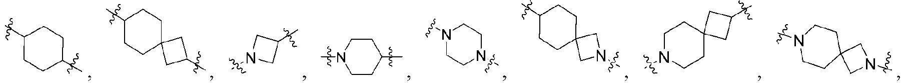

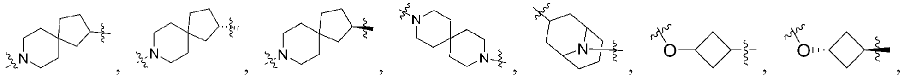

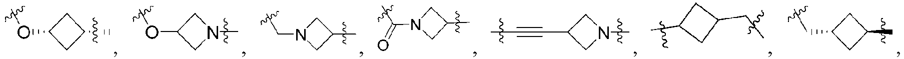

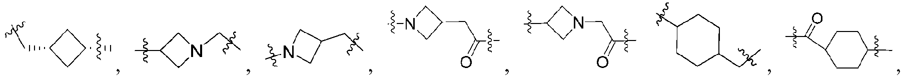

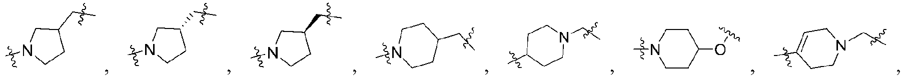

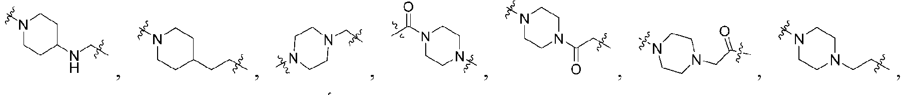

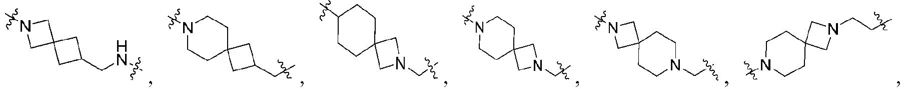

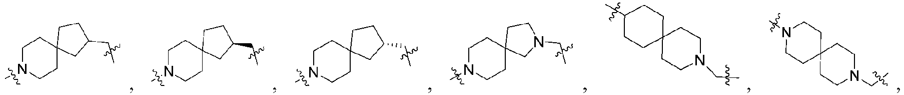

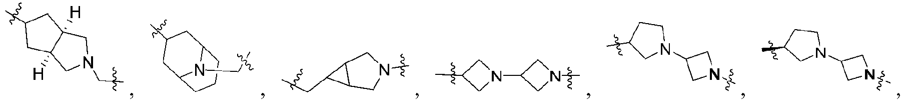

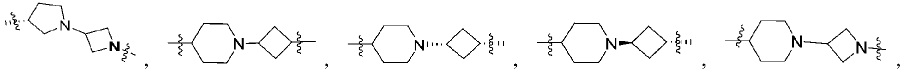

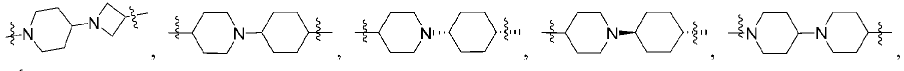

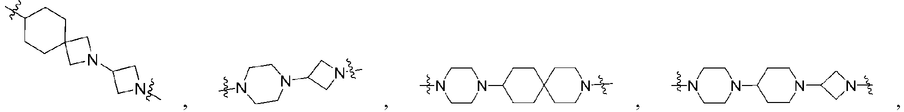

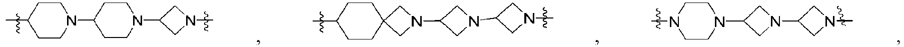

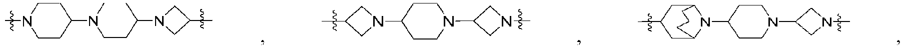

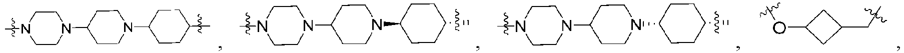

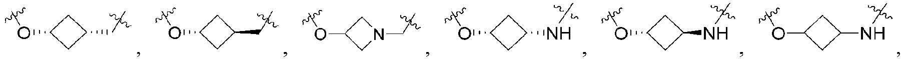

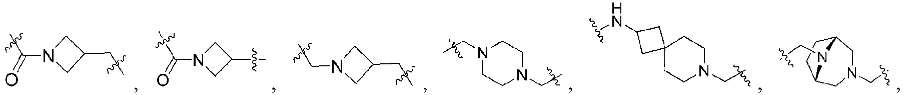

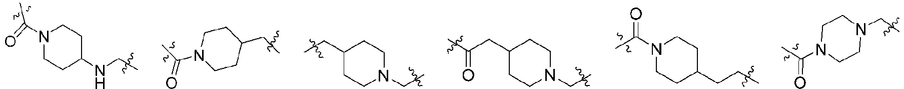

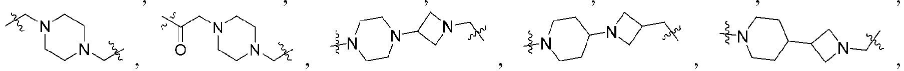

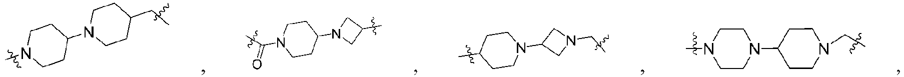

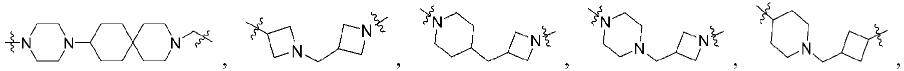

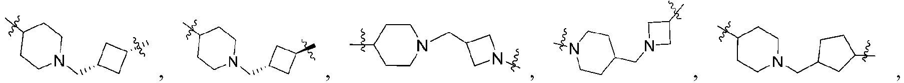

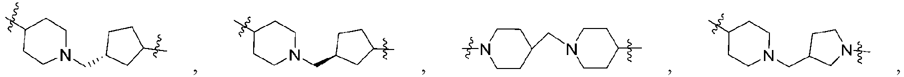

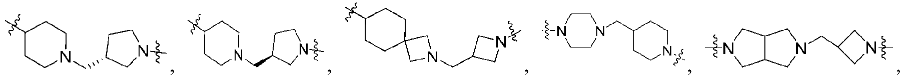

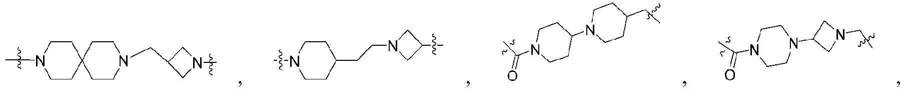

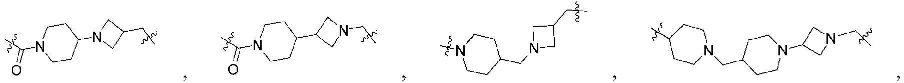

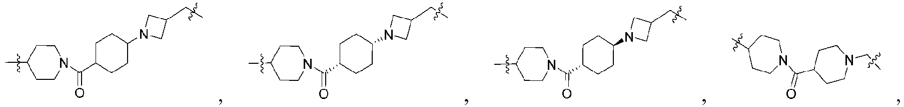

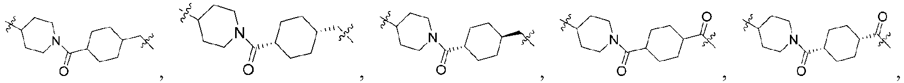

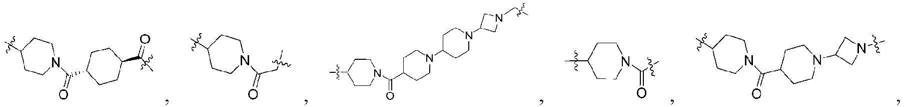

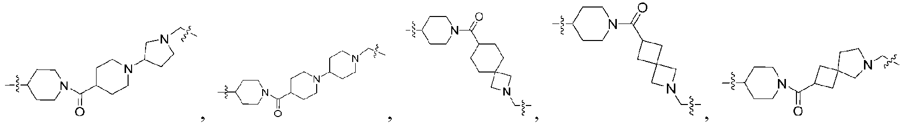

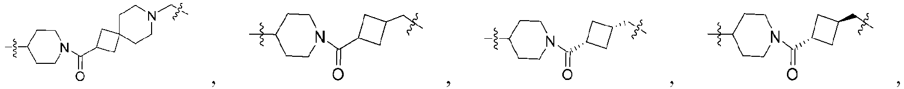

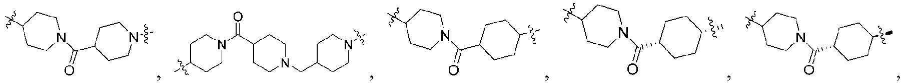

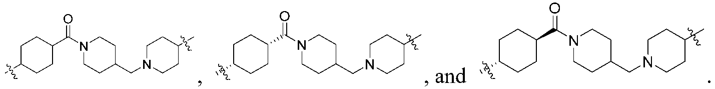

10. The compound, the stereoisomer thereof, or the pharmaceutically acceptable salt thereof according to any one of claims 1 to 9, wherein X⁶ is selected from the group consisting of a bond, -O-, -S-, -N(R^{t})-, -C₁₋₄ alkylene-, - C₂₋₄ alkenylene-, -C₂₋₄ alkynylene-, -N(R^{t})C₁₋₄ alkylene-, -OC₁₋₄ alkylene-, -SC₁₋₄ alkylene-, and -N(R^{t})C(O)-; or, X⁶ is selected from the group consisting of a bond, -O-, -S-, -N(R^{t})-, -C₁₋₃ alkylene-, -C₂₋₃ alkenylene-, -C₂₋₃ alkynylene-, -N(R^{t})C₁₋₃ alkylene-, -OC₁₋₃ alkylene-, -SC₁₋₃ alkylene-, and -N(R^{t})C(O)-;
or, X⁶ is selected from the group consisting of a bond, -O-, -S-, -N(R^{t})-, -C₁₋₃ alkylene-, -C₂₋₃ alkenylene-, -C₂₋₃ alkynylene-, -N(R^{t})C₁₋₃ alkylene-, -OC₁₋₃ alkylene-, and -N(R^{t})C(O)-;
or, X⁶ is selected from the group consisting of a bond, -O-, -S-, -N(R^{t})-, -CH₂-, -C₂ alkynylene-, -N(R^{t})C₁₋₂ alkylene-, -OC₁₋₂ alkylene-, and -N(R^{t})C(O)-;
or, X⁶ is selected from the group consisting of a bond, -O-, -S-, -NH-, -CH₂-, -NHCH₂-, -N(CH₃)CH₂-, -OCH₂-, -OCH(CH₃)-, and -NHC(O)-;
or, X⁶ is selected from the group consisting of -O-, -S-, -NH-, -C₂₋₄ alkynylene-, -NHC₁₋₄ alkylene-, and -OC₁₋₄ alkylene-;
or, X⁶ is selected from the group consisting of a bond, -O-, -NHC₁₋₃ alkylene-, and -OC₁₋₃ alkylene-;
or, X⁶ is selected from the group consisting of a bond, -O-, -C₂₋₃ alkynylene-, and -OC₁₋₃ alkylene-;
or, X⁶ is selected from the group consisting of a bond, -O-, -ethynylene-, and -OCH₂-;
or, X⁶ is selected from the group consisting of a bond, -O-, -NHCH₂-, and -OCH₂-.

11. The compound, the stereoisomer thereof, or the pharmaceutically acceptable salt thereof according to any one of claims 1 to 10, wherein R^{t} is selected from the group consisting of H and C₁₋₆ alkyl;
or, R^{t} is selected from the group consisting of H and C₁₋₃ alkyl; or, R^{t} is selected from the group consisting of H and methyl.

12. The compound, the stereoisomer thereof, or the pharmaceutically acceptable salt thereof according to any one of claims 1 to 11, wherein each R² is independently selected from the group consisting of halogen, -CN, and the following groups optionally substituted with one or more R': R^{v}-, R^{v}O-, R^{v}S-, R^{s}R^{v}N-, R^{v}C(O)-, R^{v}S(O)₂-, R^{v}S(O)-, R^{v}=N-, R^{v}OC(O)-, R^{v}C(O)O-, R^{v}S(O)O-, R^{v}OS(O)-, R^{v}S(O)₂O-, R^{v}OS(O)₂-, R^{s}R^{v}NC(O)-, R^{v}C(O)NH-, R^{v}OC(O)NH-, R^{v}NHC(O)O-, R^{v}S(O)NH-, R^{s}R^{v}NS(O)-, R^{v}S(O)₂NH-, R^{s}R^{v}NS(O)₂-, and R^{s}R^{v}S(O)=N-;
or, each R² is independently selected from the group consisting of halogen, -CN, and the following groups optionally substituted with one or more R': R^{v}-, R^{v}O-, R^{v}C(O)-, R^{v}=N-, R^{v}S(O)₂NH-, and R^{s}R^{v}S(O)=N-; optionally, R^{s} and R^{v} are each independently selected from the group consisting of H, C₁₋₁₀ alkyl, C₂₋₁₀ alkenyl, C₂₋₁₀ alkynyl, C₃₋₁₂ cycloalkyl, C₃₋₁₂ cycloalkyl C₁₋₃ alkylene-, 3- to 12-membered heterocycloalkyl, 3- to 12-membered heterocycloalkyl C₁₋₃ alkylene-, C₃₋₁₂ cycloalkenyl, C₃₋₁₂ cycloalkenyl C₁₋₃ alkylene-, 3- to 12-membered heterocycloalkenyl, 3- to 12-membered heterocycloalkenyl C₁₋₃ alkylene-, C₆₋₁₀ aryl, C₆₋₁₀ aryl C₁₋₃ alkylene, 5- to 10-membered heteroaryl, and 5- to 10-membered heteroaryl C₁₋₃ alkylene-;
or, R^{s} and R^{v} are each independently selected from the group consisting of H, C₁₋₆ alkyl, C₂₋₆ alkenyl, C₂₋₆ alkynyl, C₃₋₁₀ cycloalkyl, C₃₋₁₀ cycloalkyl C₁₋₃ alkylene-, 3- to 10-membered heterocycloalkyl, 3- to 10-membered heterocycloalkyl C₁₋₃ alkylene-, C₃₋₁₀ cycloalkenyl, C₃₋₁₀ cycloalkenyl C₁₋₃ alkylene-, 3- to 10-membered heterocycloalkenyl, 3- to 10-membered heterocycloalkenyl C₁₋₃ alkylene-, C₆₋₁₀ aryl, C₆₋₁₀ aryl C₁₋₃ alkylene-, 5- to 10-membered heteroaryl, and 5- to 10-membered heteroaryl C₁₋₃ alkylene-;
or, each R^{s} is independently selected from the group consisting of H and C₁₋₆ alkyl; or, each R^{s} is independently selected from H;
or, R^{s} and R^{v} are each independently selected from the group consisting of H, C₁₋₆ alkyl, C₂₋₆ alkenyl, C₃₋₆ cycloalkyl, 3- to 10-membered heterocycloalkyl, C₆₋₁₀ aryl, and 5- to 6-membered heteroaryl;
or, R^{s} and R^{v} are each independently selected from the group consisting of H, C₁₋₄ alkyl, C₃₋₄ cycloalkyl, 4- to 7-membered heterocycloalkyl, and 5- to 6-membered heteroaryl;
or, R^{s} and R^{v} are each independently selected from the group consisting of H, methyl, ethyl, n-propyl, isopropyl, isobutyl, ethenyl, cyclopropyl, cyclobutyl, cyclopentyl, oxetanyl, azetidinyl, pyrrolidinyl, tetrahydrothienyl, 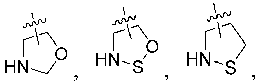 piperidinyl, 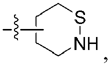 morpholinyl, piperazinyl, 1,4-oxathianyl, 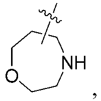 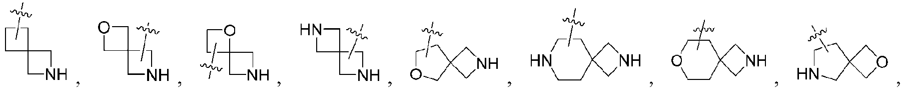 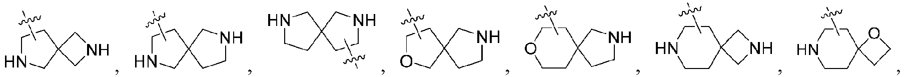 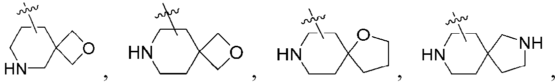 pyrazolyl, imidazolyl, oxazolyl, isoxazolyl, triazolyl, oxadiazolyl, thiadiazolyl, and phenyl;
or, R^{s} and R^{v} are each independently selected from the group consisting of H, methyl, ethyl, n-propyl, isopropyl, isobutyl, ethenyl, cyclopropyl, cyclobutyl, cyclopentyl, oxetanyl, azetidinyl, pyrrolidinyl, 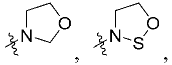 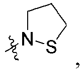 piperidinyl, 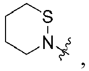 morpholinyl, piperazinyl, 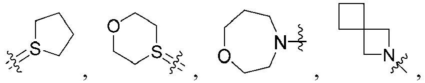 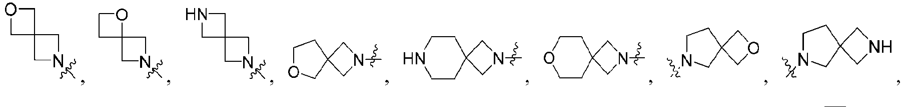 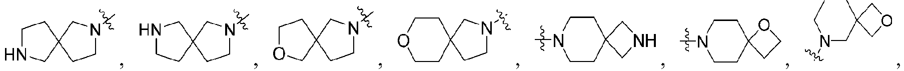 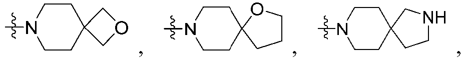 pyrazolyl, imidazolyl, oxazolyl, isoxazolyl, triazolyl, oxadiazolyl, and thiadiazolylphenyl.

13. The compound, the stereoisomer thereof, or the pharmaceutically acceptable salt thereof according to any one of claims 1 to 12, wherein each R² is independently selected from the group consisting of halogen, -OH, -NH₂, -CN, and the following groups optionally substituted with one or more substituents R': C₁₋₆ alkyl, C₁₋₆ alkyl O-, 4- to 10-membered heterocycloalkyl O-, C₁₋₆ alkyl S-, C₁₋₆ alkyl NH-, (C₁₋₆ alkyl)₂N-, C₃₋₁₀ cycloalkyl NH-, 4-to 10-membered heterocycloalkyl NH-, C₁₋₆ alkyl C(O)-, C₃₋₁₀ cycloalkyl C(O)-, 5- to 10-membered heterocycloalkyl C(O)-, C₁₋₆ alkyl S(O)₂-, H₂NC(O)-, C₁₋₆ alkyl NHC(O)-, (C₁₋₆ alkyl)₂NC(O)-, C₂₋₆ alkenyl C(O)NH-, C₁₋₆ alkyl S(O)₂NH-, C₃₋₁₀ cycloalkyl-S(O)₂NH-, H₂NS(O)₂-, (C₁₋₆ alkyl)₂S(O)=N-, 4- to 12-membered heterocycloalkyl=N-, C₃₋₁₀ cycloalkyl, 4- to 12-membered heterocycloalkyl, and 5- to 10-membered heteroaryl;
or, each R² is independently selected from the group consisting of halogen, -OH, -NH₂, -CN, and the following groups optionally substituted with one or more substituents R': C₁₋₆ alkyl, C₁₋₆ alkyl O-, 4- to 6-membered heterocycloalkyl O-, C₁₋₆ alkyl S-, C₁₋₆ alkyl NH-, (C₁₋₆ alkyl)₂N-, C₃₋₆ cycloalkyl NH-, 4- to 6-membered heterocycloalkyl NH-, C₁₋₆ alkyl C(O)-, C₃₋₆ cycloalkyl C(O)-, 5- to 6-membered heterocycloalkyl C(O)-, C₁₋₆ alkyl S(O)₂-, H₂NC(O)-, C₁₋₆ alkyl NHC(O)-, (C₁₋₆ alkyl)₂NC(O)-, C₂₋₆ alkenyl C(O)NH-, C₁₋₆ alkyl S(O)₂NH-, C₃₋₆ cycloalkyl-S(O)₂NH-, H₂NS(O)₂-, (C₁₋₆ alkyl)₂S(O)=N-, 4- to 10-membered heterocycloalkyl=N-, C₃₋₆ cycloalkyl, 4- to 10-membered heterocycloalkyl, and 5- to 6-membered heteroaryl;
or, each R² is independently selected from the group consisting of the following groups: halogen, C₁₋₄ alkyl, C₁₋₄ alkyl O-, C₁₋₃ alkyl C(O)-, C₁₋₃ alkyl S(O)₂NH-, (C₁₋₃ alkyl)₂S(O)=N-, 5- to 6-membered heterocycloalkyl=N-, C₃₋₄ cycloalkyl, 4- to 7-membered heterocycloalkyl, and 5-membered heteroaryl, wherein the C₁₋₄ alkyl, C₁₋₄ alkyl O-, C₁₋₃ alkyl C(O)-, C₁₋₃ alkyl S(O)₂NH-, (C₁₋₃ alkyl)₂S(O)=N-, 5- to 6-membered heterocycloalkyl=N-, C₃₋₄ cycloalkyl, 4- to 7-membered heterocycloalkyl, or 5-membered heteroaryl is optionally substituted with one or more of the following groups: halogen, -OH, -NH₂, -CN, C₁₋₃ alkyl, =O, and C₁₋₃ alkyl S(O)₂NH-;
or, each R² is independently selected from the group consisting of halogen and the following groups optionally substituted with one or more R': C₁₋₆ alkyl, C₁₋₆ alkyl O-, C₁₋₆ alkyl C(O)-, C₁₋₆ alkyl S(O)₂NH-, (C₁₋₆ alkyl)₂S(O)=N-, 5- to 7-membered heterocycloalkyl=N-, C₃₋₆ cycloalkyl, 4- to 7-membered heterocycloalkyl, and 5- to 6-membered heteroaryl;
or, each R² is independently selected from the group consisting of halogen, -OH, -NH₂, -CN, and the following groups optionally substituted with one or more substituents R': methyl, ethyl, n-propyl, isopropyl, isobutyl, 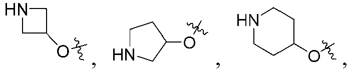 CH₃S-, CH₃NH-, CH₃CH₂NH-, (CH₃)₂CHNH-, (CH₃)₂N-, 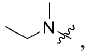 cyclopropyl-NH-, 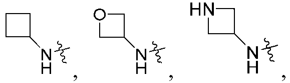 CH₃C(O)-, CH₃CH₂C(O)-, (CH₃)₂CHC(O)-, cyclopropyl-C(O)-, cyclobutyl-C(O)-, cyclopentyl-C(O)-, 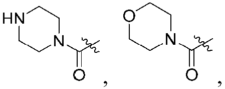 CH₃S(O)₂-, H₂NC(O)-, CH₃NHC(O)-, CH₃CH₂NHC(O)-, (CH₃)₂NC(O)-, 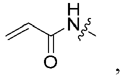 CH₃S(O)₂NH-, cyclopropyl-S(O)₂NH-, H₂NS(O)₂-, (CH₃)₂S(O)-N-, 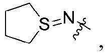 cyclopropyl, 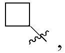 azetidinyl, pyrrolidinyl, 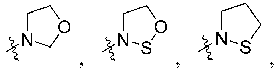 piperidinyl, 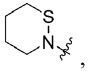 morpholinyl, piperazinyl, 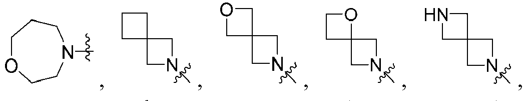 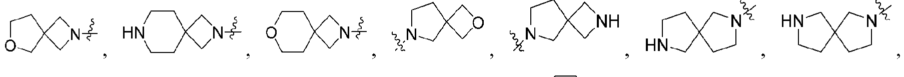 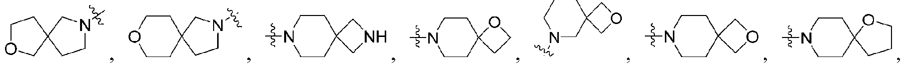 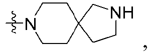 pyrazolyl, imidazolyl, oxazolyl, isoxazolyl, triazolyl, oxadiazolyl, thiadiazolyl, CH₃O-, CH₃CH₂O-, 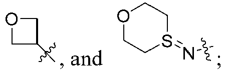
optionally, each R' is independently selected from the group consisting of halogen, -OH, -NH₂, -CN, =O, and the following groups optionally substituted with one or more substituents: C₁₋₆ alkyl, C₁₋₆ alkyl O-C₁₋₆ alkyl-, C₁₋₆ alkyl O-, C₁₋₆ alkyl S-, (C₁₋₆ alkyl)NH-, (C₁₋₆ alkyl)₂N-, C₁₋₆ alkyl C(O)-, C₁₋₆ alkyl S(O)₂-, C₁₋₆ alkyl C(O)NH-, C₁₋₆ alkyl S(O)₂NH-, C₁₋₆ alkyl S(O)NH-, (C₁₋₆ alkyl)₂S(O)=N-, C₁₋₆ alkyl NHC(O)-, C₁₋₆ alkyl NHS(O)₂-, C₁₋₆ alkyl NHS(O)-, C₁₋₆ alkyl C(O)O-, C₁₋₆ alkyl S(O)₂O-, C₁₋₆ alkyl S(O)O-, C₁₋₆ alkyl OC(O)-, C₁₋₆ alkyl OS(O)₂-, C₁₋₆ alkyl OS(O)-, C₁₋₆ alkyl NHC(O)O-, C₁₋₆ alkyl OC(O)NH-, C₃₋₁₀ cycloalkyl, 3- to 10-membered heterocycloalkyl, 3- to 10-membered heterocycloalkenyl, C₆₋₁₀ aryl, and 5- to 10-membered heteroaryl;
or, each R' is independently selected from the group consisting of halogen, -OH, -NH₂, -CN, =O, C₁₋₆ alkyl optionally substituted with one or more hydroxyl or halogen, C₁₋₆ alkyl OC₁₋₆ alkylene-, C₁₋₆ alkyl O-, (C₁₋₆ alkyl)NH-, (C₁₋₆ alkyl)₂N-, C₁₋₆ alkyl S(O)₂-, C₃₋₆ cycloalkyl, and 3- to 6-membered heterocycloalkyl optionally substituted with one or more halogen;
or, each R' is independently selected from the group consisting of halogen, -OH, -NH₂, -CN, =O, C₁₋₆ alkyl, C₁₋₆ alkyl O-, (C₁₋₆ alkyl)NH-, C₁₋₆ alkyl S(O)₂NH-, C₃₋₆ cycloalkyl, and 3- to 6-membered heterocycloalkyl;
or, each R' is independently selected from the group consisting of halogen, -OH, -NH₂, -CN, =O, C₁₋₄ alkyl, and C₁₋₄ alkyl S(O)₂NH-; or, each R' is independently selected from the group consisting of -F, -OH, -NH₂, -CN, =O, methyl, ethyl, HOCH₂-, CH₃OCH₂-, CH₃CH₂O-, (CH₃)₂N-, CH₃S(O)₂-, cyclopropyl, cyclobutyl, cyclopentyl, azetidinyl, 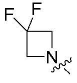 , -Cl, and CH₃S(O)₂NH-;
or, each R² is independently selected from the group consisting of -F, -CN, -NH₂, methyl, CH₃CH₂-, CH₃CH(NH₂)-, CH₃CH(OH)-, (CH₃)₂C(OH)-, CH₃CH₂CH(OH)-, (CH₃)₂CHCH(OH)-, 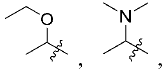 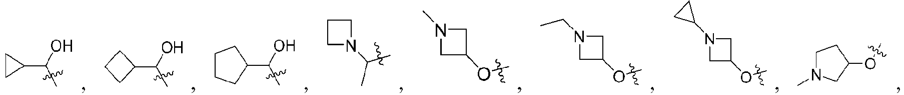 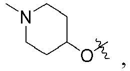 CH₃S-, CH₃NH-, CH₃CH₂NH-, (CH₃)₂CHNH-, 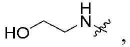 (CH₃)₂N-, 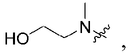 cyclopropyl-NH-, 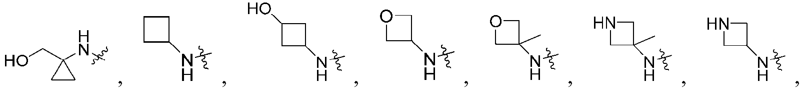 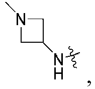 CH₃C(O)-, CH₃CH₂C(O)-, (CH₃)₂CHC(O)-, cyclopropyl-C(O)-, cyclobutyl-C(O)-, cyclopentyl-C(O)-, 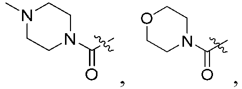 CH₃S(O)₂-, H₂NC(O)-, CH₃NHC(O)-, CH₃CH₂NHC(O)-, (CH₃)₂NC(O)-, 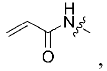 CH₃S(O)₂NH-, cyclopropyl-S(O)₂NH-, H₂NS(O)₂-, (CH₃)₂S(O)=N-, 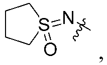 cyclopropyl, 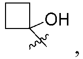 azetidinyl, 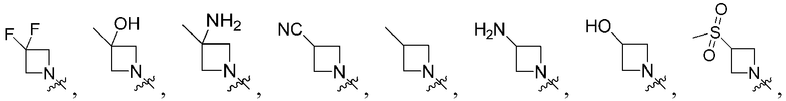 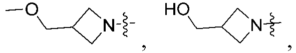 pyrrolidinyl, 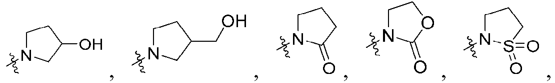 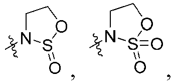 piperidinyl, 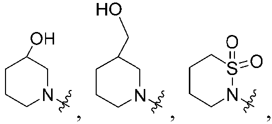 morpholinyl, 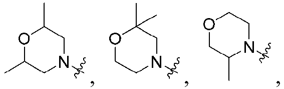 piperazinyl, 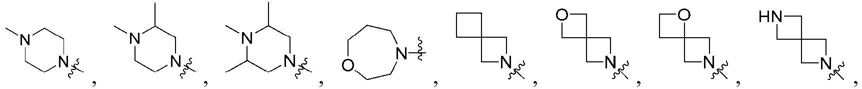 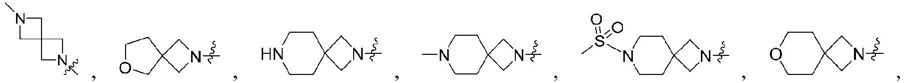 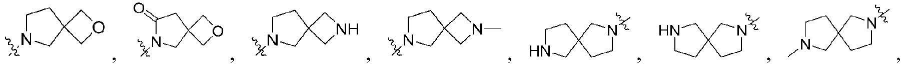 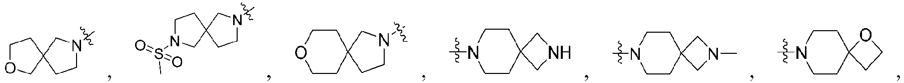 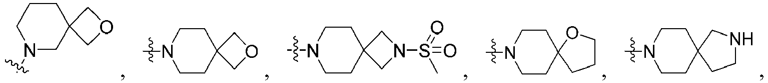 pyrazolyl, imidazolyl, oxazolyl, isoxazolyl, triazolyl, oxadiazolyl, thiadiazolyl, 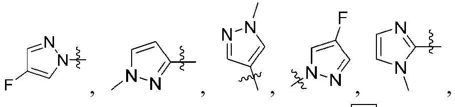 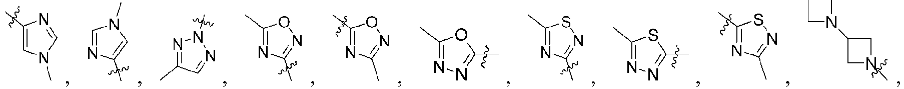 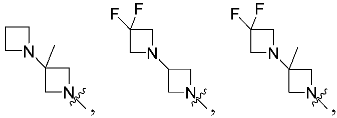 -Cl, -CHF₂, -CF₃, CH₃O-, CHF₂O-, CF₃O-, CF₃CH₂O-, 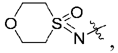 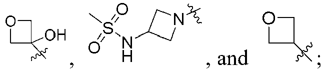
or, each R² is independently selected from the group consisting of -F, CH₃CH(OH)-, (CH₃)₂C(OH)-, -CHF₂, - CF₃, CH₃O-, CHF₂O-, CF₃O-, CF₃CH₂O-, CH₃C(O)-, CH₃S(O)₂NH-, (CH₃)₂S(O)=N-, 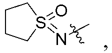 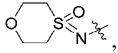 cyclopropyl,

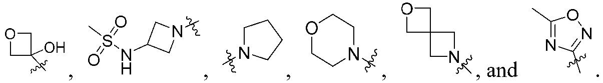

14. The compound, the stereoisomer thereof, or the pharmaceutically acceptable salt thereof according to any one of claims 1 to 13, wherein each R³ is independently selected from the group consisting of halogen, -OH, -NH₂, -CN, C₁₋₆ alkyl, C₁₋₆ alkoxy, C₁₋₆ haloalkyl, C₃₋₆ cycloalkyl, 3- to 6-membered heterocycloalkyl, C₆₋₁₀ aryl, and 5- to 6-membered heteroaryl;
or, each R³ is independently selected from the group consisting of halogen, -OH, -NH₂, -CN, C₁₋₆ alkyl, C₁₋₆ alkoxy, and C₁₋₆ haloalkyl;
or, each R³ is independently selected from the group consisting of halogen, -OH, -NH₂, -CN, and methyl;
or, each R³ is independently selected from halogen; or, each R³ is independently selected from F.

15. The compound, the stereoisomer thereof, or the pharmaceutically acceptable salt thereof according to any one of claims 1 to 14, wherein each R⁴ is independently selected from the group consisting of halogen, -OH, -NH₂, -CN, C₁₋₆ alkyl, C₁₋₆ alkoxy, C₁₋₆ haloalkyl, C₃₋₆ cycloalkyl, 3- to 6-membered heterocycloalkyl, C₆₋₁₀ aryl, and 5- to 6-membered heteroaryl;
or, each R⁴ is independently selected from the group consisting of halogen, -OH, -NH₂, -CN, C₁₋₆ alkyl, C₁₋₆ alkoxy, and C₁₋₆ haloalkyl;
or, each R⁴ is independently selected from the group consisting of halogen, -OH, -NH₂, -CN, and methyl;
or, each R⁴ is independently selected from the group consisting of halogen, -OH, -NH₂, and -CN;
or, each R⁴ is independently selected from the group consisting of halogen and -CN;
or, each R⁴ is independently selected from the group consisting of -F, -Cl, and -CN.

16. The compound, the stereoisomer thereof, or the pharmaceutically acceptable salt thereof according to any one of claims 1 to 15, wherein m is selected from the group consisting of 0, 1, 2, and 3; or, m is selected from the group consisting of 1 and 2;
optionally, p is selected from the group consisting of 0, 1, and 2; or, p is selected from the group consisting of 0 and 1;
optionally, k is selected from the group consisting of 0, 1, 2, and 3; or, k is selected from the group consisting of 0, 1, and 2.

17. The compound, the stereoisomer thereof, or the pharmaceutically acceptable salt thereof according to any one of claims 1 to 16, wherein ring G is selected from the group consisting of C₄₋₆ cycloalkyl, 4- to 9-membered heterocycloalkyl, phenyl, 5- to 8-membered heteroaryl, and 9- to 10-membered heterocycloalkenyl; or, ring G is selected from the group consisting of C₄₋₆ cycloalkyl, 4- to 9-membered heterocycloalkyl, phenyl, 5- to 8-membered heteroaryl, tetrahydroisoquinolinyl, tetrahydropyridopyrimidinyl, tetrahydropyridopyrazinyl, pyrrolopyrimidinyl, and pyrimidopyrrolidinyl;
or, ring G is selected from the group consisting of phenyl, 5- to 6-membered heteroaryl, and 9- to 10-membered heterocycloalkenyl;
or, ring G is selected from the group consisting of cyclobutyl, cyclopentyl, cyclohexyl, oxetanyl, azetidinyl, pyrrolidinyl, tetrahydrofuranyl, tetrahydropyranyl, piperidinyl, morpholinyl, dioxanyl, 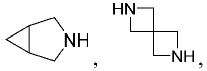 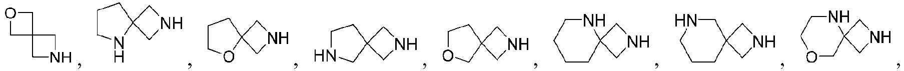 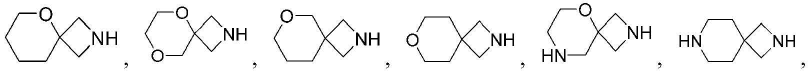 phenyl, thiazolyl, isothiazolyl, oxazolyl, isoxazolyl, imidazolyl, pyrazolyl, triazolyl, oxadiazolyl, thiadiazolyl, pyridinyl, pyrimidinyl, pyrazinyl, pyridazinyl, 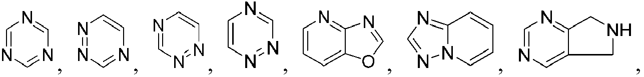 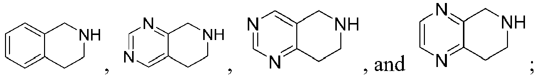
or, ring G is selected from the group consisting of cyclobutyl, cyclopentyl, cyclohexyl, oxetanyl, azetidinyl, pyrrolidinyl, tetrahydrofuranyl, tetrahydropyranyl, piperidinyl, morpholinyl, dioxanyl, 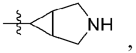 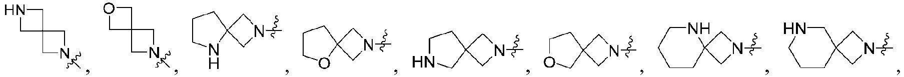 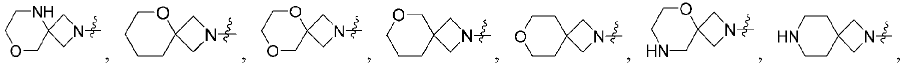 phenyl, thiazolyl, isothiazolyl, oxazolyl, isoxazolyl, imidazolyl, pyrazolyl, triazolyl, oxadiazolyl, thiadiazolyl, pyridinyl, pyrimidinyl, pyrazinyl, pyridazinyl, 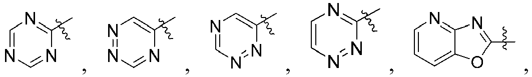 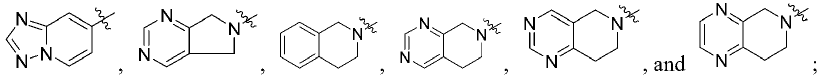
or, ring G is selected from the group consisting of cyclobutyl, cyclopentyl, cyclohexyl, oxetanyl, azetidinyl, pyrrolidinyl, tetrahydrofuranyl, tetrahydropyranyl, piperidinyl, morpholinyl, dioxanyl, 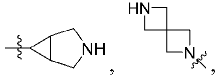 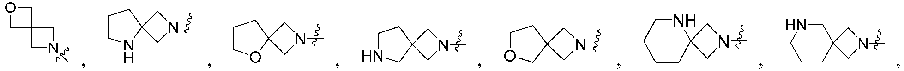 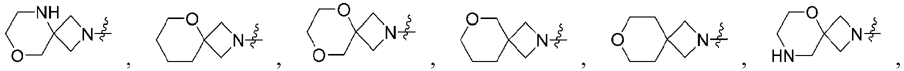 and 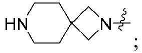
or, ring G is selected from the group consisting of

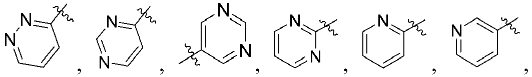

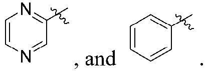

18. The compound, the stereoisomer thereof, or the pharmaceutically acceptable salt thereof according to any one of claims 1 to 17, wherein ring E is selected from the group consisting of C₆₋₁₂ aryl and 5- to 10-membered heteroaryl;
or, ring E is selected from the group consisting of phenyl and 5- to 6-membered heteroaryl;
or, ring E is selected from the group consisting of phenyl and 6-membered heteroaryl;
or, ring E is selected from the group consisting of

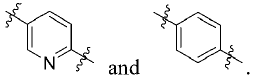

19. The compound, the stereoisomer thereof, or the pharmaceutically acceptable salt thereof according to any one of claims 1 to 18, wherein ring F is selected from the group consisting of C₆₋₁₂ aryl and 5- to 10-membered heteroaryl;
or, ring F is selected from the group consisting of phenyl and 5- to 6-membered heteroaryl;
or, ring F is selected from the group consisting of phenyl and 6-membered heteroaryl;
or, ring F is selected from the group consisting of 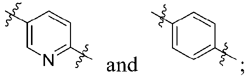
optionally, 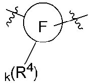 is selected from the group consisting of 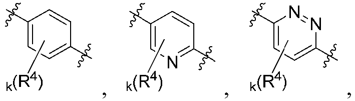 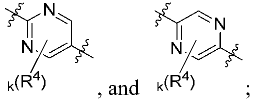
optionally, 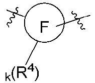 is selected from the group consisting of 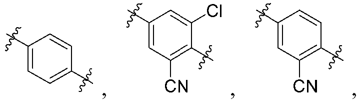 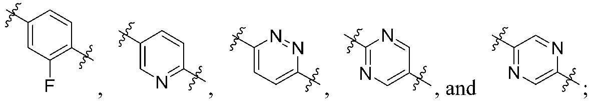
optionally, Z is selected from -C-.

20. The compound, the stereoisomer thereof, or the pharmaceutically acceptable salt thereof according to any one of claims 1 to 19, wherein R⁵ and R⁶ are each independently selected from the group consisting of H, =O, halogen, -OH, -NH₂, -CN, C₁₋₆ alkyl, and C₂₋₆ alkenyl, or R⁵ and R⁶ are linked to each other to form the following group optionally substituted with one or more substituents such as R": C₃₋₆ cycloalkyl or 3- to 6-membered heterocycloalkyl;
or, R⁵ and R⁶ are each independently selected from the group consisting of H, C₁₋₃ alkyl, and C₂₋₃ alkenyl, or R⁵ and R⁶ are linked to each other to form the following group optionally substituted with one or more R": C₃₋₆ cycloalkyl or 4- to 6-membered heterocycloalkyl;
or, R⁵ and R⁶ are each independently selected from the group consisting of H, methyl, ethyl, and ethenyl, or R⁵ and R⁶ are linked to each other to form the following group optionally substituted with one or more R": cyclopropyl, cyclobutyl, cyclopentyl, cyclohexyl, tetrahydropyranyl, or oxetanyl;
optionally, R" is selected from the group consisting of halogen, -OH, -NH₂, -CN, C₁₋₃ alkyl, and C₁₋₃ haloalkyl; or, R⁵ and R⁶ are each independently selected from the group consisting of H, methyl, ethyl, and ethenyl, or R⁵ and R⁶ are linked to each other to form the following group: cyclopropyl, cyclobutyl, cyclopentyl, cyclohexyl, tetrahydropyranyl, or oxetanyl;
or, R⁵ and R⁶ are each independently selected from the group consisting of H and C₁₋₆ alkyl, or R⁵ and R⁶ are linked to each other to form the following group: C₃₋₆ cycloalkyl or 4- to 6-membered heterocycloalkyl;
or, R⁵ and R⁶ are each independently selected from the group consisting of H and C₁₋₃ alkyl, or R⁵ and R⁶ are linked to each other to form the following group: C₃₋₄ cycloalkyl or 4-membered heterocycloalkyl; or, R⁵ and R⁶ are each independently selected from the group consisting of H and C₁₋₃ alkyl, or R⁵ and R⁶ are linked to each other to form 4-membered heterocycloalkyl;
or, R⁵ and R⁶ are each independently selected from methyl, or R⁵ and R⁶ are linked to each other to form oxetanyl;
optionally, the structural moiety 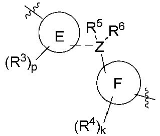 is selected from the group consisting of

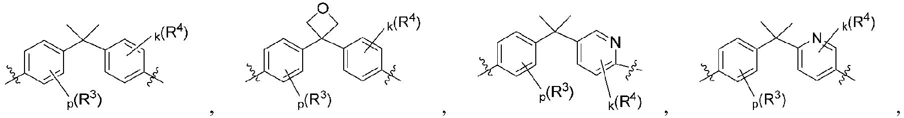

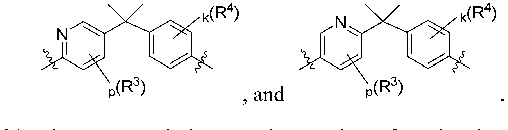

21. The compound, the stereoisomer thereof, or the pharmaceutically acceptable salt thereof according to any one of claims 1 to 20, wherein the compound of formula III, the stereoisomer thereof, or the pharmaceutically acceptable salt thereof is selected from the group consisting of the following compounds, stereoisomers thereof, or pharmaceutically acceptable salts thereof: 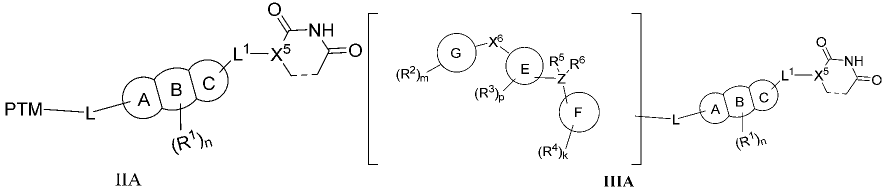 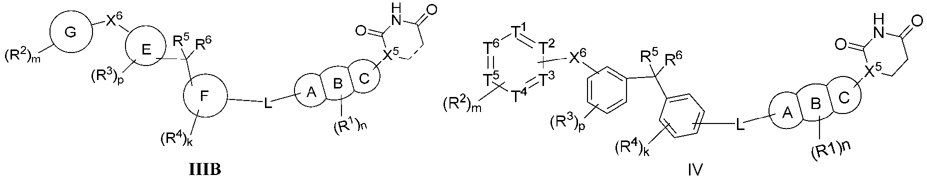 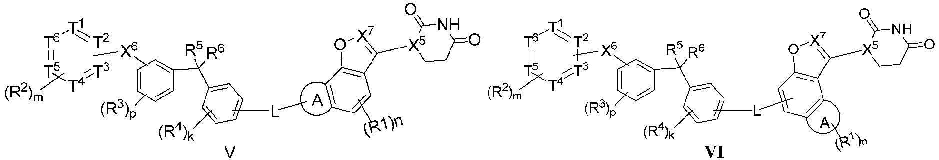 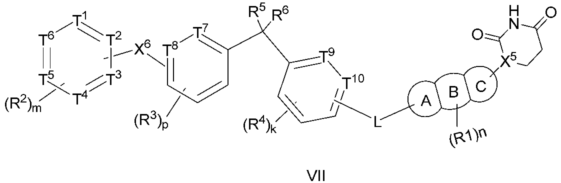 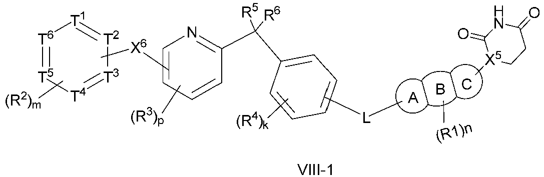 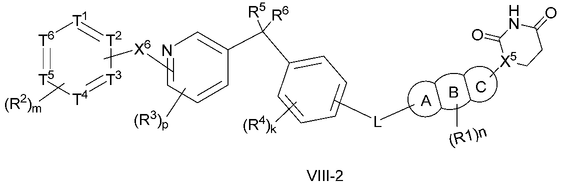 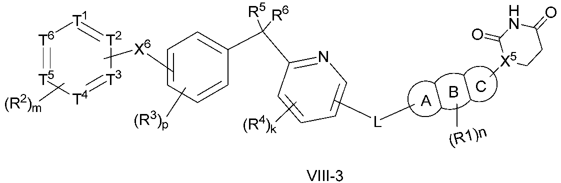 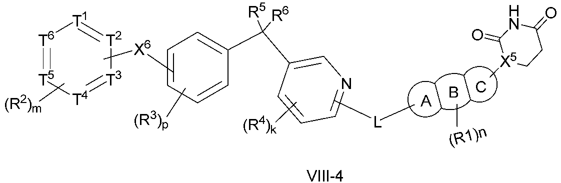
wherein T¹, T², T³, T⁴, T⁵, and T⁶ are each independently selected from the group consisting of CH and N;
X⁷ is selected from the group consisting of CH and N;
T⁷, T⁸, T⁹, and T¹⁰ are each independently selected from the group consisting of CH and N;
preferably,
X³ is C(R^{f}), wherein R^{f} is selected from the group consisting of H, halogen, deuterium, and C₁₋₃ alkyl;
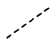 represents a single bond;
L¹ is a bond;
ring A is selected from 5- to 6-membered heterocycloalkenyl containing 1-2 N atoms or O atoms, ring B is selected from phenyl and pyridinyl, and ring C is 5-membered heteroaryl containing 1-2 heteroatoms selected from the group consisting of N, O, and S;
R¹ is selected from the group consisting of deuterium, halogen, -OH, and -NH₂;
n is 0 or 1;
L is selected from the group consisting of 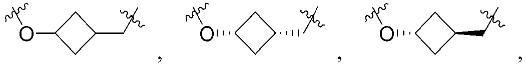 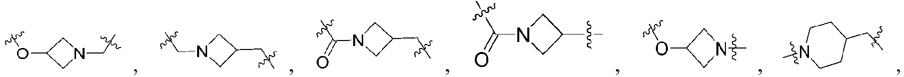 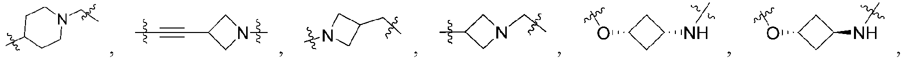 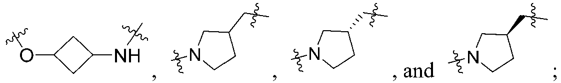
PTM is 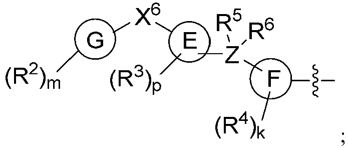
R² is selected from the group consisting of halogen, -CHF₂, -CF₃, methyl, CH₃O-, CHF₂O-, CF₃O-, CF₃CH₂O-, CH₃CH(OH)-, (CH₃)₂C(OH)-, CH₃CH₂CH(OH)-, cyclopropyl, 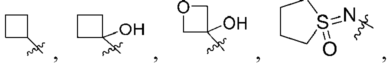 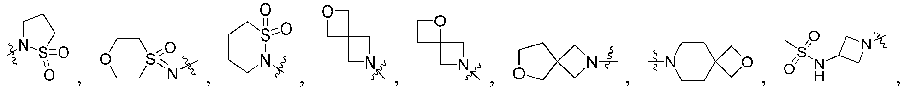 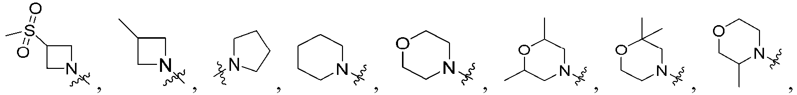 CH₃S(O)₂NH-, and cyclopropyl-S(O)₂NH-;
m is 1;
ring G is selected from the group consisting of phenyl and 5- to 6-membered heteroaryl containing 1-3 N atoms; X⁶ is selected from the group consisting of a bond, -O-, -S-, -C₂₋₃ alkenylene-, and -C₂₋₃ alkynylene-;
ring E is selected from the group consisting of phenyl and 6-membered heteroaryl containing 1-2 N atoms; p is 0;
Z is selected from -C-;
R⁵ and R⁶ are each independently selected from the group consisting of C₁₋₃ alkyl and C₂₋₃ alkenyl;
ring F is selected from the group consisting of phenyl and 6-membered heteroaryl containing 1-2 N atoms;
R⁴ is selected from the group consisting of halogen and -CN;
k is 0 or 1.

22. The compound, the stereoisomer thereof, or the pharmaceutically acceptable salt thereof according to any one of claims 1 to 21, wherein the compound is selected from the group consisting of: 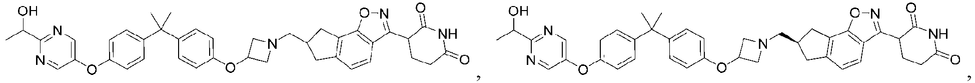 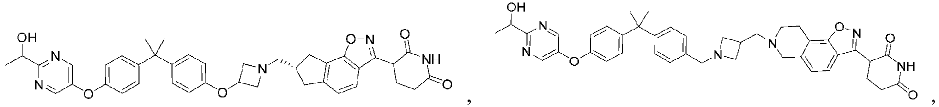 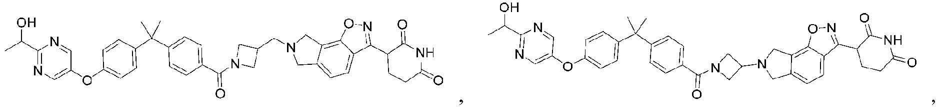 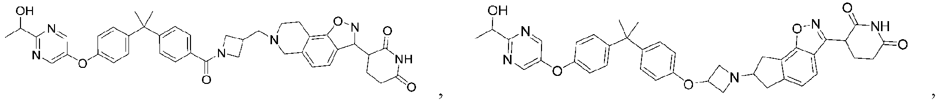 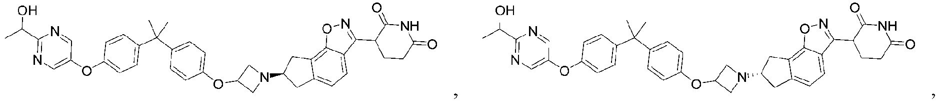 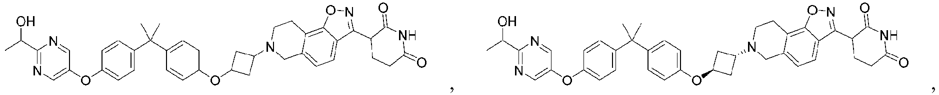 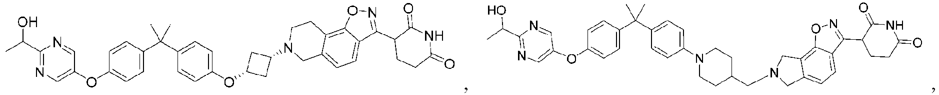 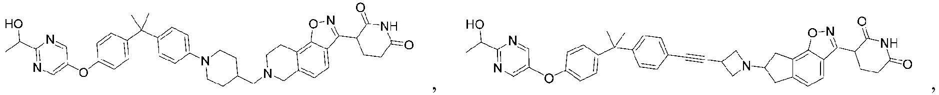 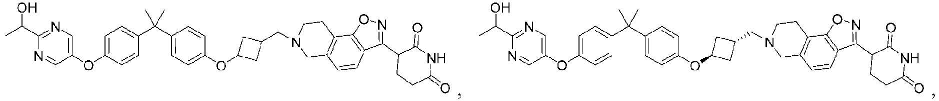 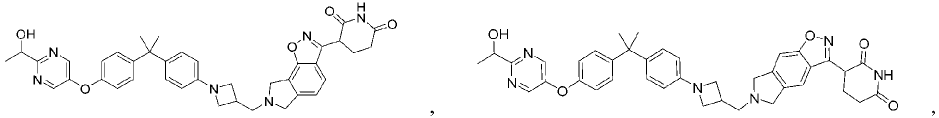 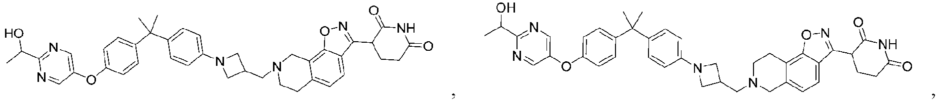 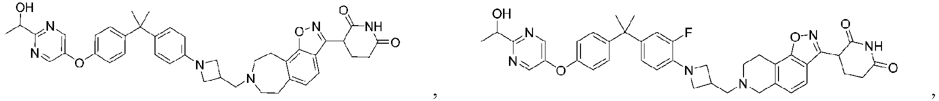 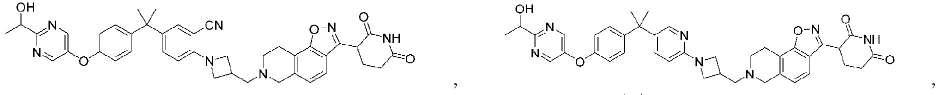 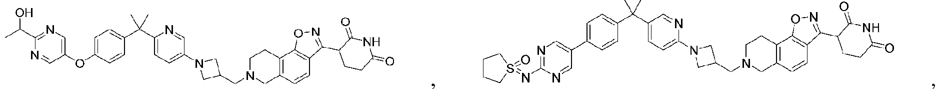 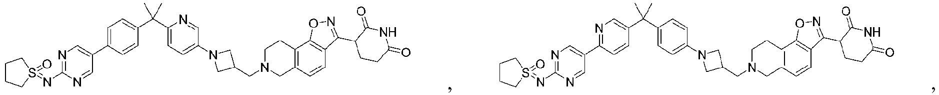 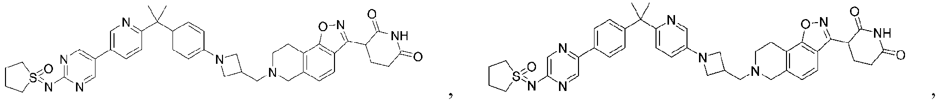 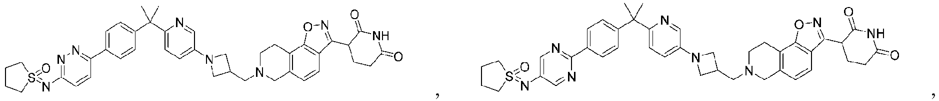 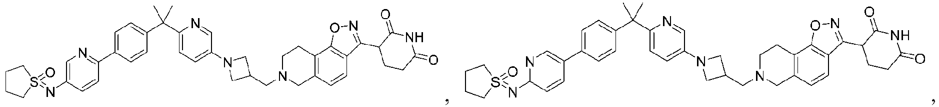 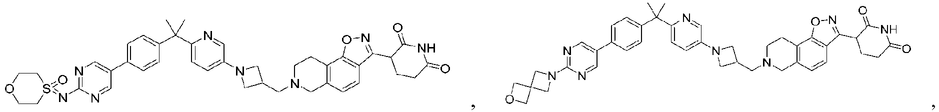 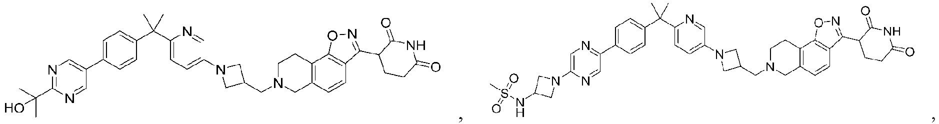 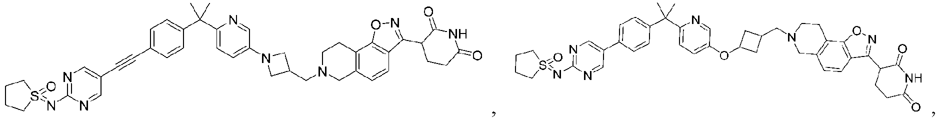 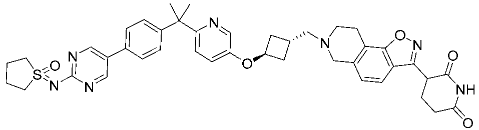 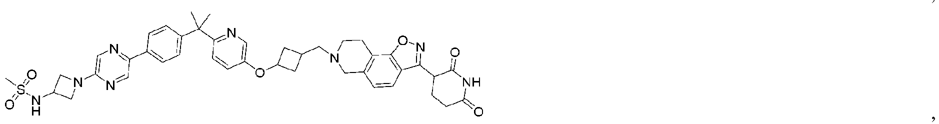 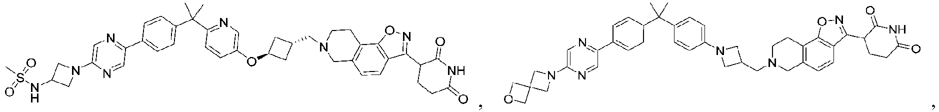 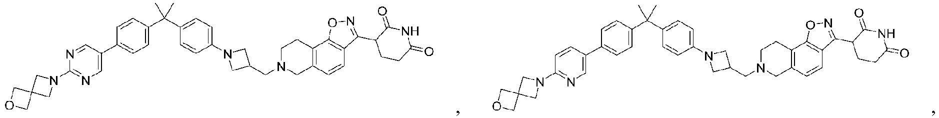 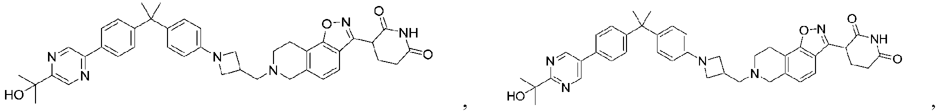 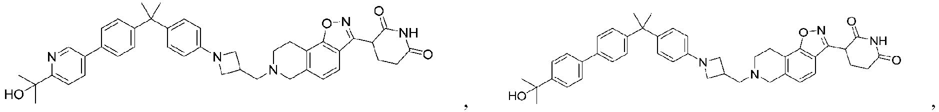 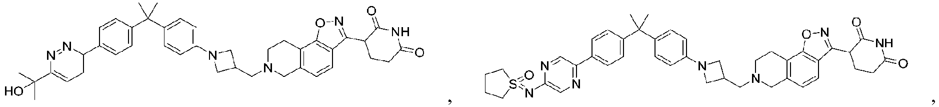 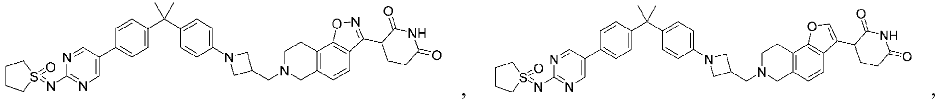 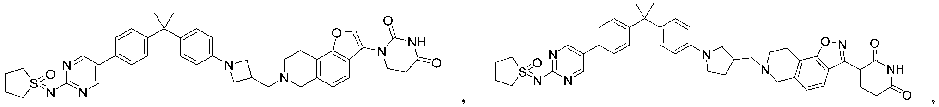 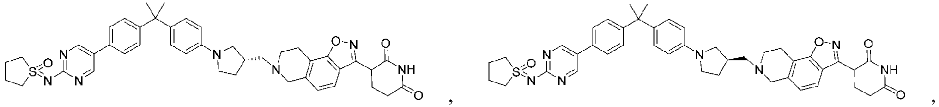 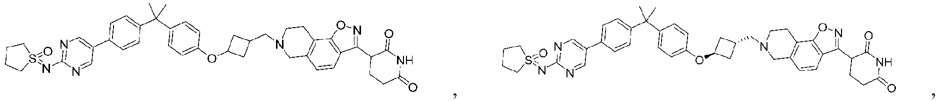 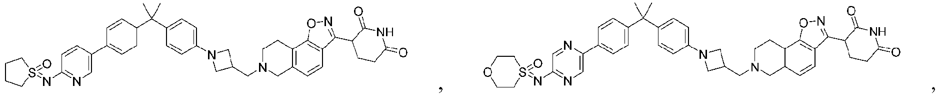 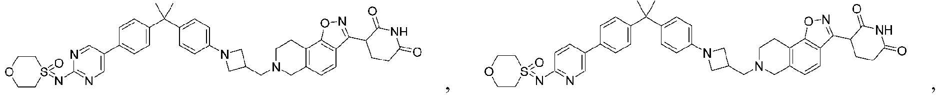 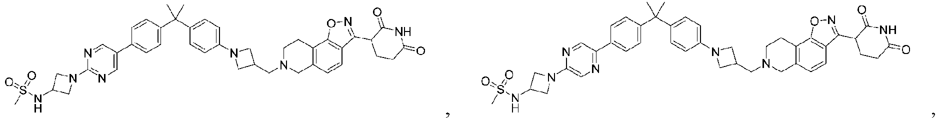 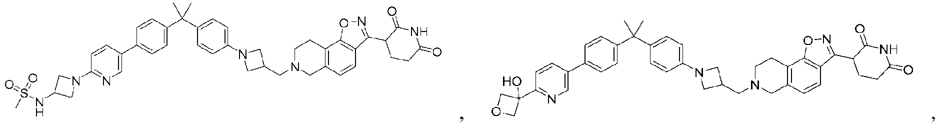 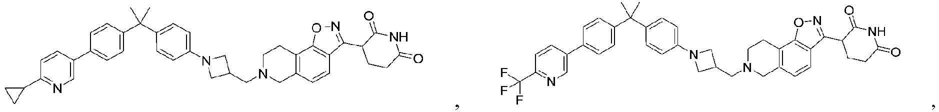 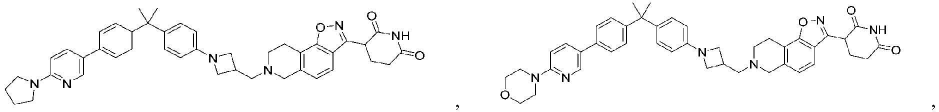 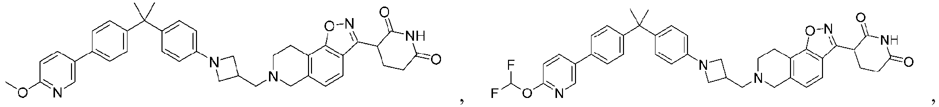 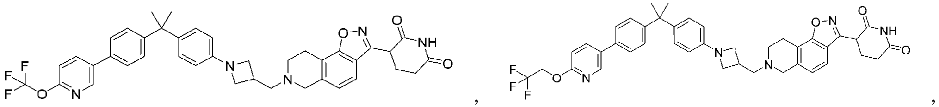 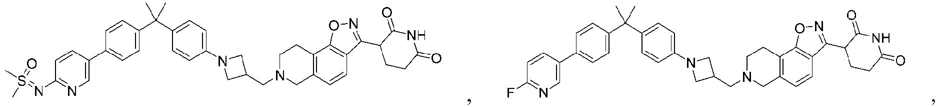 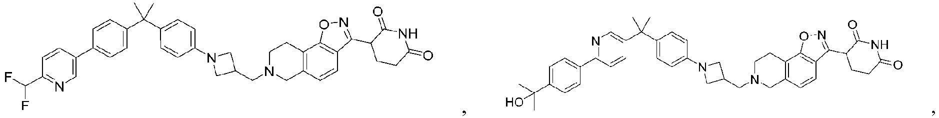 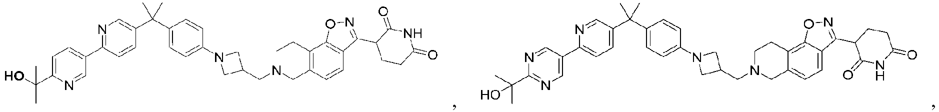 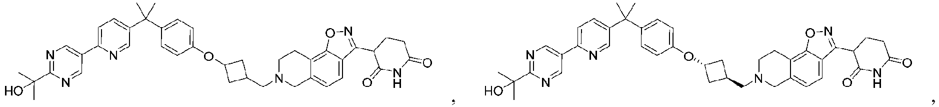 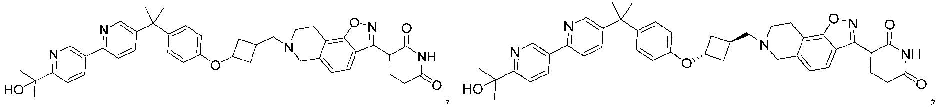 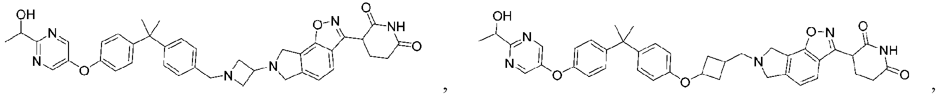 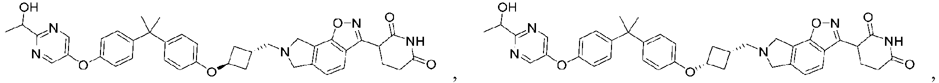 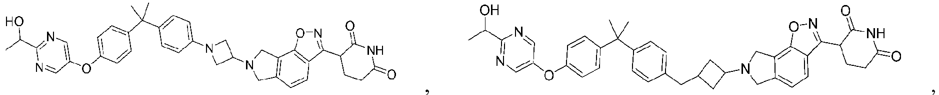 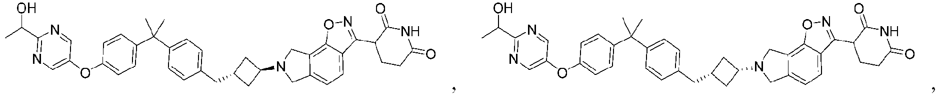 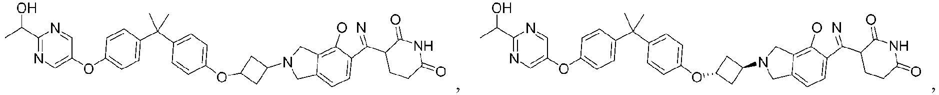 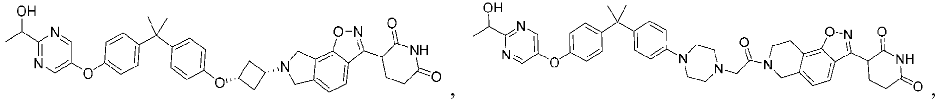 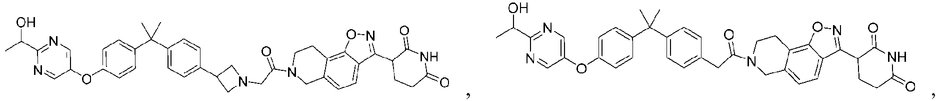 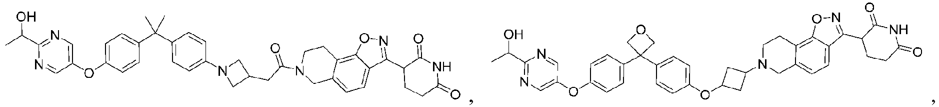 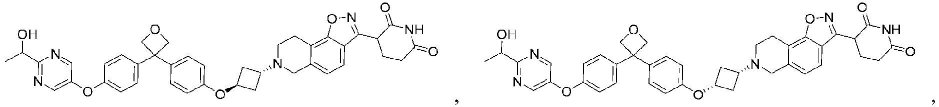 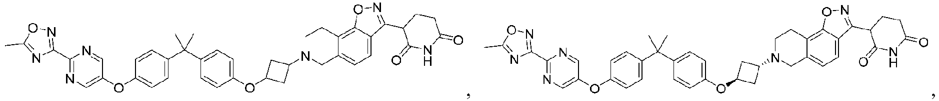 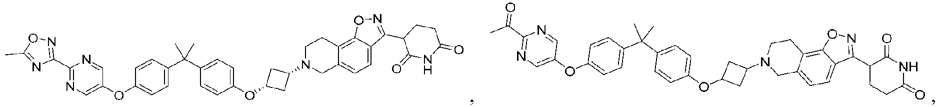 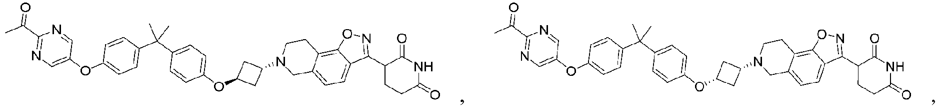 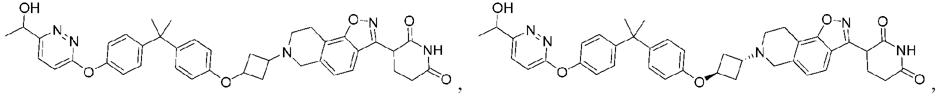 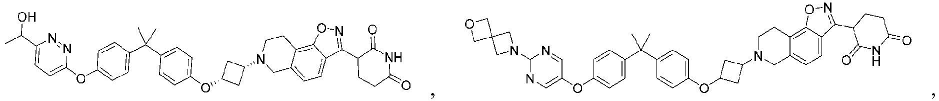 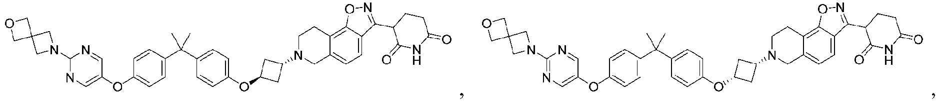 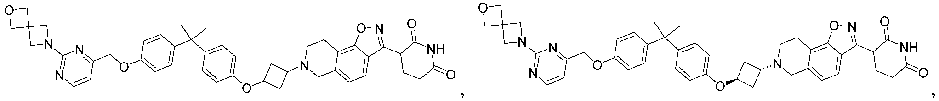 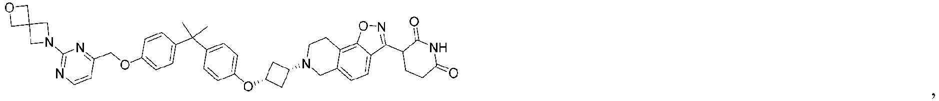 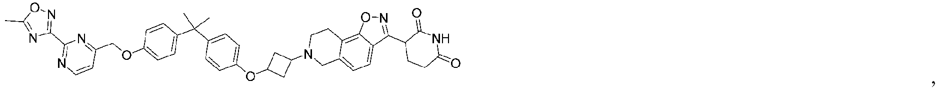 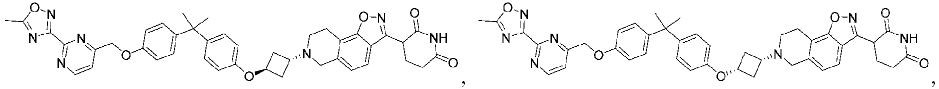 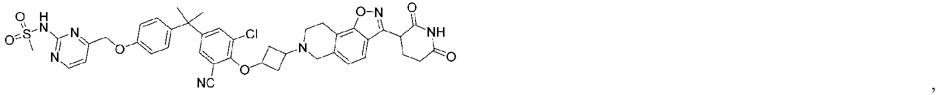 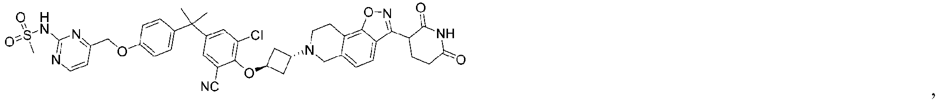 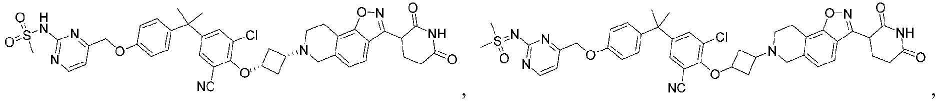 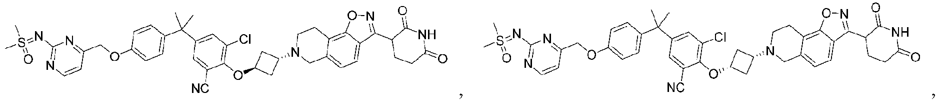 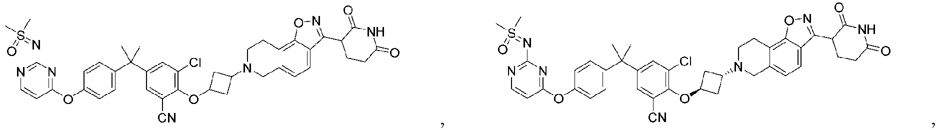 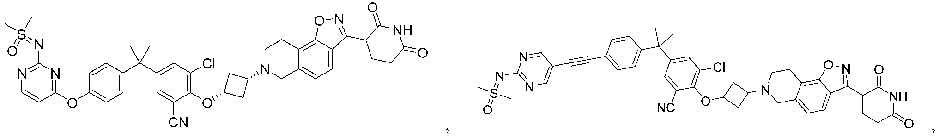 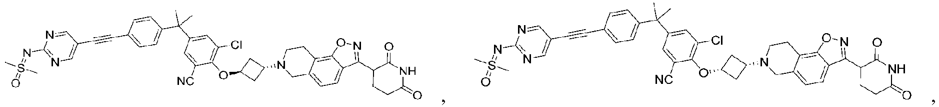 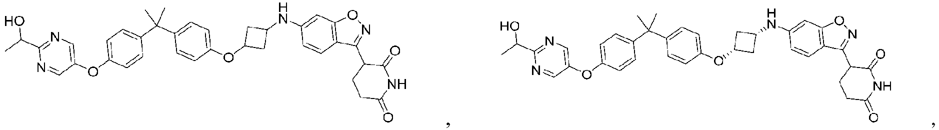 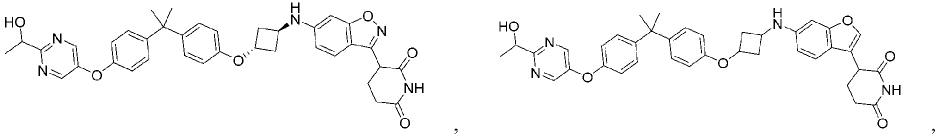 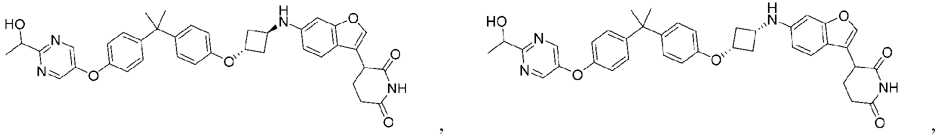 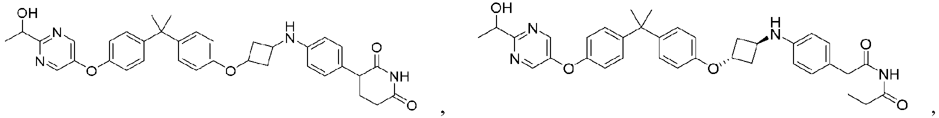 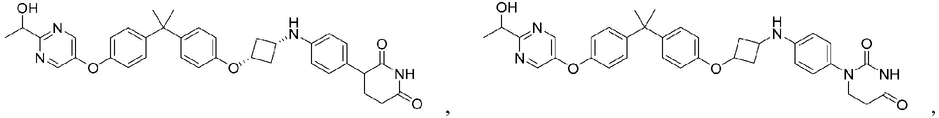 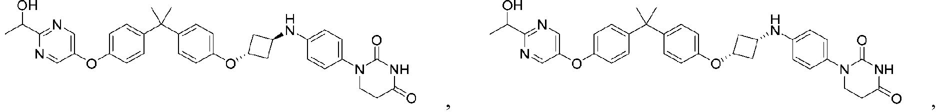 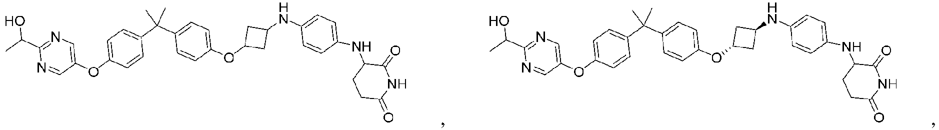 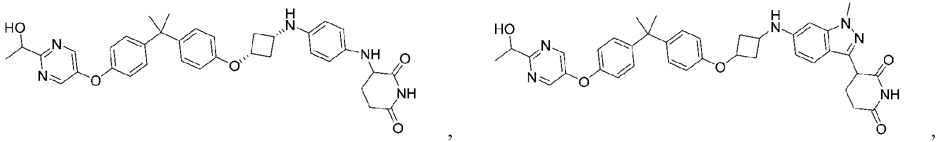 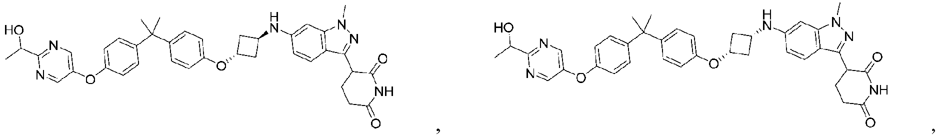 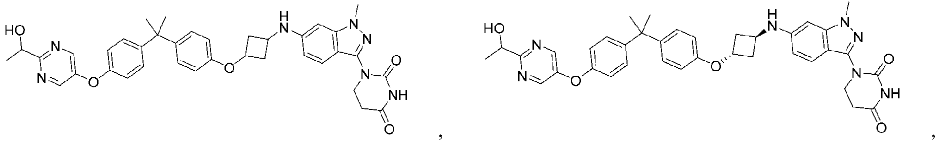 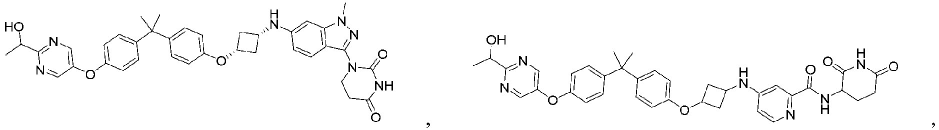 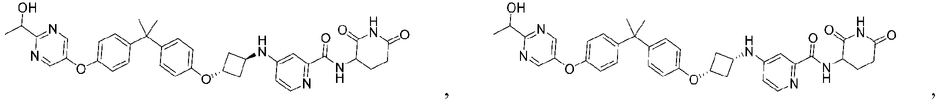 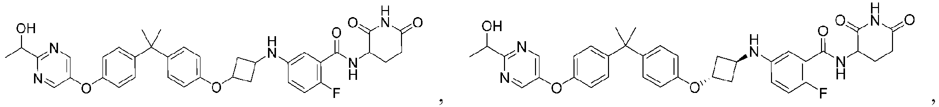 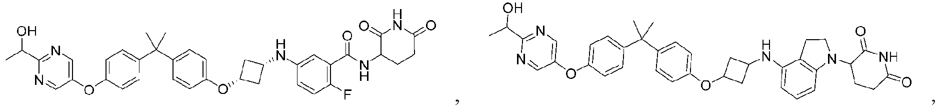 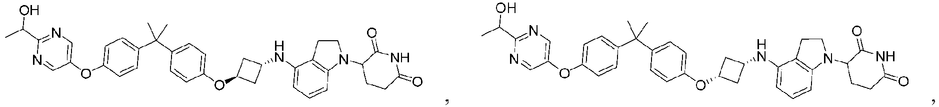 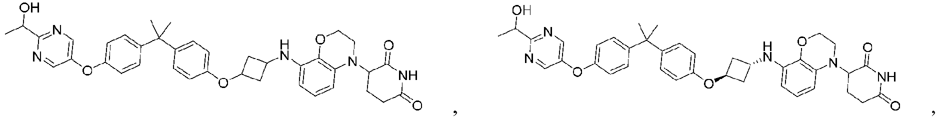 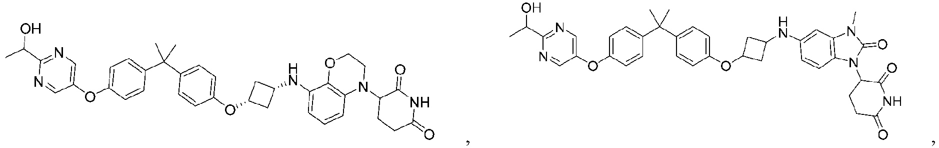 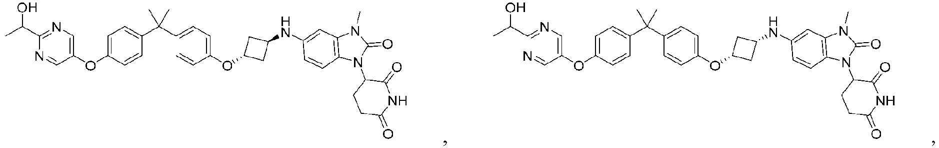 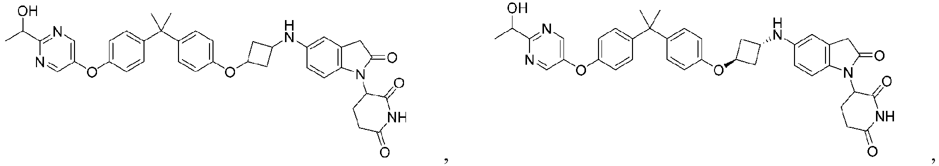 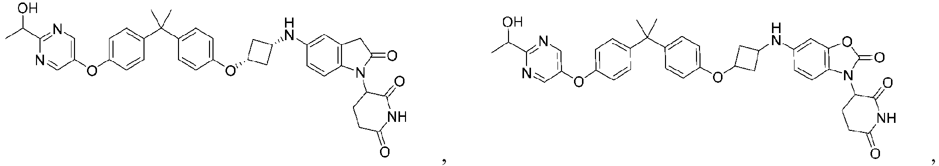 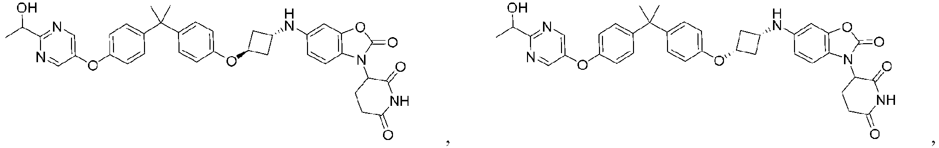 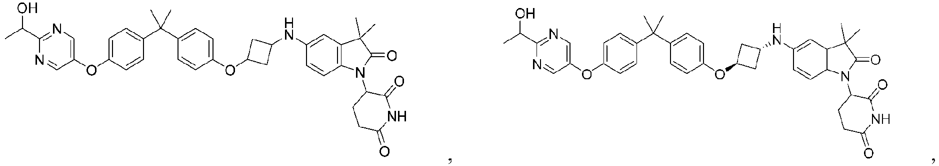 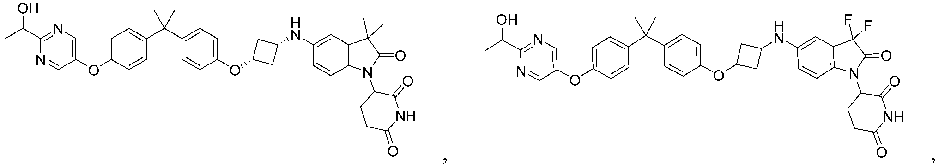 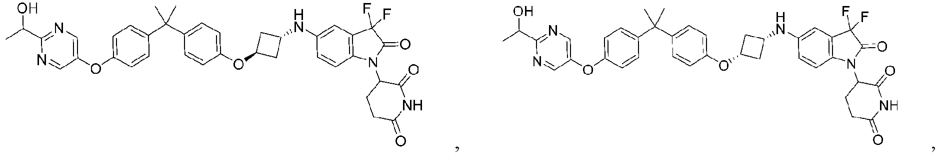 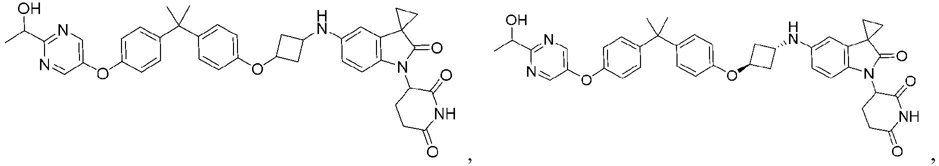 and

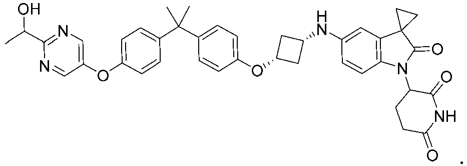

23. A pharmaceutical composition, comprising the compound, the stereoisomer thereof, or the pharmaceutically acceptable salt thereof according to any one of claims 1 to 22, and optionally further comprising a pharmaceutically acceptable excipient.

24. Use of the compound, the stereoisomer thereof, or the pharmaceutically acceptable salt thereof according to any one of claims 1 to 22, or the pharmaceutical composition according to claim 23 for preparing a medicament for preventing or treating a disease, wherein optionally, the disease is selected from a GSPT1-related disease; optionally, the disease is selected from an AR- or ARv7-related disease; preferably, the disease is cancer.

25. A method for treating or preventing a disease in a mammal, comprising administering to a mammal in need of such treatment a therapeutically effective amount of the compound, the stereoisomer thereof, or the pharmaceutically acceptable salt thereof according to any one of claims 1 to 22, or the pharmaceutical composition according to claim 23, wherein optionally, the disease is selected from a GSPT1-related disease; optionally, the disease is selected from an AR- or ARv7-related disease; preferably, the disease is cancer.

26. The compound, the stereoisomer thereof, or the pharmaceutically acceptable salt thereof according to any one of claims 1 to 22, or the pharmaceutical composition according to claim 23 for use in preventing or treating a disease, wherein optionally, the disease is selected from a GSPT1-related disease; optionally, the disease is selected from an AR- or ARv7-related disease; preferably, the disease is cancer.
